# EUROPÄISCHE PATENTANMELDUNG

(11) **EP 3 974 414 A1**
(43) Veröffentlichungstag der Anmeldung: **30.03.2022**
(21) Anmeldenummer: 20198503.3
(22) Anmeldetag: 25.09.2020
(51) Int. Cl.: C07D 231/38, C07D 231/40, C07D 249/14, C07D 401/04, C07D 417/12, C07D 417/14, A01N 43/78, A01N 47/30, A01N 47/36

(54) **5-AMINO SUBSTITUIERTE PYRAZOLE UND TRIAZOLE ALS SCHÄDLINGSBEKÄMPFUNGSMITTEL**

(71) Anmelder: Bayer AG, 51373 Leverkusen (DE)
(72) Erfinder: HEIL, Markus, 42799 Leichlingen (DE); VELTEN, Robert, 40764 Langenfeld (DE); WINTER, Philipp, 51381 Leverkusen (DE); HARSCHNECK, Tobias, 40223 Düsseldorf (DE); LINKA, Marc, 40223 Düsseldorf (DE); LÖSEL, Peter, 51371 Leverkusen (DE); TURBERG, Andreas, 42781 Haan (DE)
(74) Vertreter: BIP Patents

(57) **Zusammenfassung**

Die Erfindung betrifft neue Verbindungen der Formel (I), 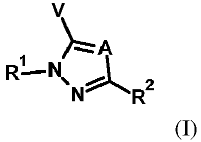 in welcher V, A, R¹, und R² die oben genannten Bedeutungen haben und deren Verwendung zur Bekämpfung von tierischen Schädlingen, vor allem von Arthropoden und insbesondere von Insekten und Spinnentieren.

## Beschreibung

Die vorliegende Erfindung betrifft neue 5-Amino substituierte Pyrazole und -Triazole, Verfahren zu ihrer Herstellung und ihre Verwendung zur Bekämpfung von tierischen Schädlingen, vor allem von Arthropoden und insbesondere von Insekten und Spinnentieren.

Bestimmt Amino substituierte Triazole und -Pyrazole mit Insektizider Wirkung sind bereits bekannt (WO2020083733). Weiterhin wurden in 5-Position substituierte oder unsubstituierte Triazole und -Pyrazole als Insektizide beschrieben (vgl. z.B. WO2009102736, WO2011017504, WO2012109125, WO2013116052). Die gemäß den oben genannten Schriften bereits bekannten Wirkstoffe weisen aber in ihrer Anwendung teils Nachteile auf, sei es, dass sie nur eine geringe Anwendungsbreite aufweisen, sei es, dass sie keine zufriedenstellende insektizide Wirkung aufweisen.

Moderne Insektizide und Akarizide müssen vielen Anforderungen genügen, beispielsweise in Bezug auf Höhe, Dauer und Breite ihrer Wirkung und möglichen Verwendung. Es spielen Fragen der Toxizität, der Nützlings- und Bestäuberschonung, der Umwelteigenschaften, der Aufwandmengen, der Kombinierbarkeit mit anderen Wirkstoffen oder Formulierhilfsmitteln eine Rolle sowie die Frage des Aufwands, der für die Synthese eines Wirkstoffs betrieben werden muss, ferner können Resistenzen auftreten, um nur einige Parameter zu nennen. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen zur Verwendung zur Bekämpfung von tierischen Schädlingen bereitzustellen, durch die das Spektrum der Schädlingsbekämpfungsmittel unter verschiedenen Aspekten ergänzt wird.

Es wurde nun überraschend gefunden, dass bestimmte neue Aminosubstituerte Pyrazole und Triazole gegenüber den bereits bekannten Verbindungen Vorteile aufweisen, zum Beispiel seien bessere biologische oder ökologische Eigenschaften, breitere Anwendungsmethoden, eine bessere insektizide, akarizide Wirkung, sowie eine gute Verträglichkeit gegenüber Nutzpflanzen beispielhaft genannt. Die neuen Aminosubstituierten Pyrazole und Triazole können in Kombination mit weiteren Mitteln zur Verbesserung der Wirksamkeit insbesondere gegen schwierig zu bekämpfende Insekten eingesetzt werden. Die erfindungsgemäßen neuen Verbindungen wurden bislang nicht offenbart.

Gegenstand der vorliegenden Erfindung sind daher neue Verbindungen der Formel (I) 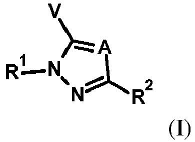 worin (Ausgestaltung 1-1)
- A: für N oder CR^{A} steht; dabei steht
R^{A} für H, Halogen, Cyano, Nitro, SF₅, NR^{d}R^{e}, OR^{a}, S(O)ₙR^{a} oder SO₂NR^{b}R^{c}; oder für C₁-C₆-Alkyl, C₂-C₆-Alkenyl, C₂-C₆-Alkinyl oder C₃-C₇-Cycloalkyl, die alle jeweils unsubstituiert sein können, oder einfach bis 13fach mit Halogen und/oder gegebenenfalls mit 1 bis 3 R^{f} substituiert sein können;
- V: für -NR^{V1}R^{V2}, -N(R^{V1})C(Q¹)R^{b}, -N(R^{b})C(Q¹)R^{V3}, -N(R^{V1})C(Q¹)NR^{b}R^{c}, -N(R^{V1})C(O)OR^{a},-N(R^{V1})C(O)C(O)R^{b}, -N(R^{V1})SO₂R^{a}, -N(R^{b})SO₂R^{V3} oder -N(R^{b})C(O)N(R^{b})R^{V1} steht; dabei steht
R^{V1} für C₁-C₆-Alkyl, das jeweils unsubstituiert sein kann, oder gegebenenfalls einfach bis 13fach mit Halogen und/oder mit 1 bis 2 R^{h} substituiert sein kann; oder für C₂-C₆-Alkenyl, C₂-C₆-Alkinyl, C₃-C₇-Cycloalkyl oder C₅-C₇-Cycloalkenyl, die alle jeweils unsubstituiert sein können, oder einfach bis 13fach mit Halogen und/oder gegebenenfalls mit 1 bis 2 Rⁱ substituiert sein können; oder für Phenyl oder für einen 4- bis 7-gliedrigen gesättigen, teilweise gesättigten oder aromatischen Heterocyclus mit 1 bis 3 Heteroatomen, jeweils unsubstituiert oder einfach bis 5fach substituiert mit Halogen und/oder gegebenenfalls mit 1 bis 2 Rⁱ;
R^{V2} für H; oder für C₁-C₆-Alkyl, C₂-C₆-Alkenyl, C₂-C₆-Alkinyl, C₁-C₆-Alkoxy oder C₃-C₇-Cycloalkyl, alle jeweils unsubstituiert oder einfach bis 13fach substituiert mit Halogen und/oder gegebenenfalls mit 1 bis 3 R^{g}; oder
R^{V1} und R^{V2} zusammen mit dem Stickstoffatom, an das sie gebunden sind, für einen gesättigten, teilweise gesättigten oder aromatischen Heterocyclus mit 3 bis 7 Ringatomen steht, der gegebenenfalls durch weitere Heteroatome aus der Reihe N, O, S(O)ₙ und/oder durch ein oder zwei C=O-Gruppen unterbrochen sein kann und der jeweils unsubstituiert ist oder einfach bis 7fach mit Halogen und/oder gegebenenfalls mit 1 bis 2 Rⁱ substituiert sein kann;
R^{V3} für C₁-C₆-Alkyl, C₂-C₆-Alkenyl, C₂-C₆-Alkinyl oder C₃-C₇-Cycloalkyl, die alle jeweils unsubstituiert sein können, oder einfach bis 13fach mit Halogen und/oder gegebenenfalls mit 1 bis 2 Rⁱ substituiert sein können; oder für einen 4- bis 7-gliedrigen gesättigen oder teilweise gesättigten Heterocyclus mit 1 bis 3 Heteroatomen, jeweils unsubstituiert oder einfach bis 5fach substituiert mit Halogen und/oder gegebenenfalls mit 1 bis 2 Rⁱ;
- Rⁱ: für H steht; oder für C₁-C₆-Alkyl, C₂-C₆-Alkenyl, C₂-C₆-Alkinyl, C₃-C₇-Cycloalkyl, C₁-C₆-Alkylcarbonyl, C₃-C₇-Cycloalkylcarbonyl, C₁-C₆₋Alkoxycarbonyl, C₃-C₇-Cycloalkoxyylcarbonyl, C₁-C₆-Alkylsulfonyl oder C₃-C₇-Cycloalkylsulfonyl steht, die alle jeweils unsubstituiert sein können, oder einfach bis 13fach mit Halogen und/oder gegebenenfalls mit 1 bis 2 Rⁱ substituiert sein können;
- R²: für die Substruktur der allgemeinen Formel -X-Y-Z steht; dabei steht
X für Phenyl oder einen 5- oder 6-gliedrigen heteroaromatischen Ring, alle jeweils unsubstituiert oder substituiert mit 1 bis 3 R^{X}; dabei steht jedes
R^{X} unabhängig voneinander für Halogen, Cyano, Nitro, C(Q¹)R^{a}, C(O)OR^{a}, C(Q¹)NR^{b}R^{c}, NR^{d}R^{e}, OR^{a}, S(O)ₙR^{a} oder SO₂NR^{b}R^{c}; oder für C₁-C₆-Alkyl, C₂-C₆-Alkenyl, C₂-C₆-Alkinyl oder C₃-C₇-Cycloalkyl, alle jeweils unsubstituiert oder einfach bis 13fach substituiert mit Halogen und/oder gegebenenfalls mit 1 bis 3 R^{f};
Y für -CR^{Y1}=N-, wobei N an Z gebunden ist, oder für
-NR^{Y2}-C(=Q^{Y})-, wobei C an Z gebunden ist; dabei steht jedes
R^{Y1} und R^{Y2} für H; oder für C₁-C₆-Alkyl, C₂-C₆-Alkenyl, C₂-C₆-Alkinyl oder C₃-C₇-Cycloalkyl, alle jeweils unsubstituiert oder substituiert mit 1 bis 7 R^{Y11}; dabei steht jedes
R^{Y11} unabhängig voneinander für Halogen, Cyano, C₁-C₄-Alkyl, C₃-C₆-Cycloalkyl, C₁-C₄-Haloalkyl, C₁-C₄-Alkoxy, oder C₁-C₄-Haloalkoxy;
Q^{Y} für O oder S;
Z für die Fragmente der allgemeinen Formel (A1), (A2) oder (A3); 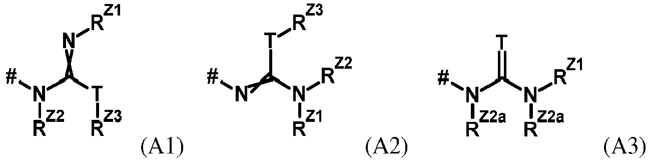 dabei ist # der Anknüpfungspunkt zu Y und dabei steht jedes
T für O oder S;
R^{Z1} unabhängig voneinander für einen 5- bis 10-gliedrigen aromatischen oder heteroaromatischen Ring oder ein bicyclisches Ringsystem, jeweils unsubstituiert oder substituiert mit 1 bis 4 R^{Z11}; dabei steht jedes
R^{Z11} unabhängig voneinander für Halogen, Cyano, Nitro, SF₅, C(Q)R^{a}, C(O)OR^{a}, C(Q¹)NR^{b}R^{c}, NR^{d}R^{e}, OR^{a}, S(O)ₙR^{a} oder SO₂NR^{b}R^{c}; oder für C₁-C₆-Alkyl, C₂-C₆-Alkenyl, C₂-C₆-Alkinyl oder C₃-C₇-Cycloalkyl, alle jeweils unsubstituiert oder substituiert mit 1 bis 5 R^{Z1a}; oder für Phenyl oder einen 5- oder 6-gliedrigen heteroaromatischen Ring, alle jeweils unsubstituiert oder substituiert mit 1 bis 3 R^{Z1a}; oder zwei benachbarte R^{Z11} bilden zusammen eine unsubstituierte oder mit 1 bis 6 R^{Z1a} substituierte lineare C₃-C₅-Alkylen-Gruppe, wobei unabhängig voneinander 1 CH₂-Einheit durch Carbonyl und 1 bis 2 CH₂-Einheiten durch O, S, NH oder N(CH₃) ersetzt sein können; dabei steht jedes
R^{Z1a} für Halogen, Cyano, C₁-C₄-Alkyl, C₁-C₄-Alkoxy, C₁-C₄-Thioalkyl, C₁-C₄-Haloalkyl oder C₁-C₄-Haloalkoxy;
- R^{Z2},: R^{Z2a} und R^{Z3} unabhängig voneinander für H; oder für C(O)R^{a}, C(O)OR^{a}, C(O)NR^{b}R^{c}, S(O)ₙR^{a}; oder für C₁-C₆-Alkyl, C₂-C₆-Alkenyl, C₂-C₆-Alkinyl oder C₃-C₇-Cycloalkyl, alle jeweils unsubstituiert oder substituiert mit 1 bis 5 R^{Z21}; oder für Phenyl, Benzyl oder einen 5- oder 6-gliedrigen heteroaromatischen Ring, alle jeweils unsubstituiert oder substituiert mit 1 bis 4 R^{Z21}; dabei steht jedes
R^{Z21} unabhängig voneinander für Halogen, Cyano, Nitro, SF₅, C(Q)R^{a}, C(O)OR^{a}, C(Q¹)NR^{b}R^{c}, NR^{d}R^{e}, OR^{a}, S(O)ₙR^{a} oder SO₂NR^{b}R^{c}; oder für C₁-C₆-Alkyl, C₂-C₆-Alkenyl, C₂-C₆-Alkinyl oder C₃-C₇-Cycloalkyl; oder
- R^{Z2} und R^{Z3}: bilden zusammen mit der T-C-N-Einheit einen 5- bis 7-gliedrigen Ring; dabei bestehen die R^{Z2}-R^{Z3}-Ringglieder aus Kohlenstoff-Atomen und gegebenenfalls 1 Sauerstoff- oder Schwefel- oder Stickstoff-Atom; dabei ist das Heteroatom nicht direkt an T gebunden; dabei können bis zu 2 Kohlenstoff-Atom-Ringglieder unabhängig voneinander aus C(=O) und C(=S) bestehen und das Schwefel-Atom-Ringglied aus S, S(O) oder S(O)₂ bestehen; dabei ist diese R^{Z2}-R^{Z3}-Einheit unsubstituiert oder substituiert mit 1 bis 4 R^{Z22}; dabei steht jedes
R^{Z22} unabhängig voneinander für Halogen, Hydroxy, Cyano, C₁-C₆-Alkyl, C₃-C₆-Cycloalkyl, C₁-C₆-Haloalkyl, C₁-C₄-Alkenyl, C₁-C₆-Alkoxy, oder C₁-C₆-Haloalkoxy; oder für spirocyclisch gebundenes Heterocyclyl mit 2 bis 7 Ringatomen, wobei 1 bis 2 Sauerstoffatome als Heteroatome enthalten sind; oder
- R^{Z2a} und ein zweites R^{Z2a}: bilden zusammen mit der N-C-N-Einheit einen 5- bis 7-gliedrigen Ring; dabei bestehen die R^{Z2a}-R^{Z2a}-Ringglieder aus Kohlenstoff-Atomen und bis zu 2 Heteroatomen, die unabhängig voneinander aus 1 Sauerstoff-Atom, 1 Schwefel-Atom und bis zu 2 Stickstoff-Atomen ausgewählt werden können; dabei können bis zu 2 Kohlenstoff-Atom-Ringglieder unabhängig voneinander aus C(=O) und C(=S) bestehen und das Schwefel-Atom-Ringglied aus S, S(O) oder S(O)₂ bestehen; dabei ist diese R^{Z2}-R^{Z3}-Einheit unsubstituiert oder substituiert mit 1 bis 5 R^{Z22};
dabei steht jedes
- R^{a}: unabhängig voneinander für C₁-C₆-Alkyl, C₃-C₆-Cycloalkyl, C₂-C₆-Alkenyl, C₂-C₆-Alkinyl, die alle jeweils unsubstituiert sein können, oder einfach bis 13fach mit Halogen und/oder gegebenenfalls mit 1 bis 3 R^{f} substituiert sein können; oder für Phenyl, unsubstituiert oder substituiert mit 1 bis 5 R^{g};
- R^{b} und R^{c}: unabhängig voneinander für H; oder für C₁-C₆-Alkyl, C₃-C₆-Cycloalkyl, C₂-C₆-Alkenyl, C₂-C₆-Alkinyl, die alle jeweils unsubstituiert sein können, oder einfach bis 13fach mit Halogen und/oder gegebenenfalls mit 1 bis 3 R^{f} substituiert sein können; oder R^{b} und R^{c} bilden zusammen einen 3-bis 7-gliedrigen Ring;
- R^{d} und R^{e}: unabhängig voneinander für H, C(Q¹)R^{a}, C(O)OR^{a}; oder für C₁-C₆-Alkyl, C₃-C₆-Cycloalkyl, C₂-C₆-Alkenyl, C₂-C₆-Alkinyl, die alle jeweils unsubstituiert sein können, oder einfach bis 13fach mit Halogen und/oder gegebenenfalls mit 1 bis 3 R^{f} substituiert sein können; oder für Phenyl, unsubstituiert oder substituiert mit 1 bis 5 R^{g}; oder R^{d} und R^{e} bilden zusammen einen 3- bis 7-gliedrigen Ring;
- R^{f}: unabhängig voneinander für Halogen, Cyano, Nitro, C₃-C₆-Cycloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl, C₁-C₄-Alkoxycarbonyl; oder für Phenyl oder einen 5- oder 6-gliedrigen heteroaromatischen Ring, alle jeweils unsubstituiert oder substituiert mit 1 bis 7 R^{g};
- R^{g}: unabhängig voneinander für Halogen, Cyano, Nitro, C₁-C₄-Alkyl, C₃-C₆-Cycloalkyl, C₁-C₄-Haloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl oder C₁-C₄-Alkoxycarbonyl;
- R^{h}: unabhängig voneinander für Halogen, Cyano, Nitro, Hydroxy, OSO₂R^{a}, SO₂NR^{b}R^{c}, N(R^{d})(R^{e}), C(Q¹)NR^{b}R^{c}, N(R^{b})C(Q¹)R^{a}, C(O)OR^{b} OC(O)R^{b} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl, spirocyclisch gebundenes C₂-C₆-Cycloalkyl, spirocyclisch gebundenes Heterocyclyl mit 3 bis 7 Ringatomen, C₃-C₆-Cycloalkylthio, C₃-C₆-Cycloalkoxy, C₃-C₆-Cycloalkylsulfinyl oder C₃-C₆-Cycloalkylsulfonyl, die alle jeweils unsubstituiert sein können oder einfach bis 13fach mit Halogen und/oder gegebenenfalls mit 1 bis 2 R^{g} substituiert sein können; oder für einen 4- bis 7-gliedrigen gesättigen oder teilweise gesättigten Heterocyclus mit 1 bis 4 Heteroatomen, die alle jeweils unsubstituiert sein können oder einfach bis 5fach mit Halogen und/oder gegebenenfalls mit 1 bis 2 R^{g} substituiert sein können;
- Rⁱ: unabhängig voneinander für Halogen, Cyano, Nitro, Hydroxy, OSO₂R^{a}, SC₂NR^{b}R^{c}, N(R^{d})(R^{e}), C(Q¹)NR^{b}R^{c}, N(R^{b})C(Q¹)R^{a}, C(O)R^{a}, C(=N-OR^{b})R^{a}, C(O)OR^{b}, OC(O)R^{a} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl, spirocyclisch gebundenes C₂-C₆-Cycloalkyl, spirocyclisch gebundenes Heterocyclyl mit 3 bis 7 Ringatomen, C₃-C₆-Cycloalkylthio, C₃-C₆-Cycloalkoxy, C₃-C₆-Cycloalkylsulfinyl oder C₃-C₆-Cycloalkylsulfonyl, die alle jeweils unsubstituiert sein können oder einfach bis 13fach mit Halogen und/oder gegebenenfalls mit 1 bis 2 R^{g} substituiert sein können; oder für Phenyl oder einen 4- bis 7-gliedrigen gesättigen, teilweise gesättigten oder aromatischen Heterocyclus mit 1 bis 4 Heteroatomen, die alle jeweils unsubstituiert sein können oder einfach bis 5fach mit Halogen und/oder gegebenenfalls mit 1 bis 2 R^{g} substituiert sein können;
- Q¹: unabhängig voneinander für O, S, NOR^{a} oder NCN;
- Q²: unabhängig voneinander für O oder NOR^{a};
- n: unabhängig voneinander für 0, 1 oder 2.

Bevorzugt (Ausgestaltung 2-1) sind die Verbindungen der Formel (I), in denen
- A: für N oder CR^{A} steht; dabei steht
R^{A} für H, Halogen, Cyano oder SFs; oder für C₁-C₄-Alkyl, C₂-C₄-Alkenyl, C₂-C₆-Alkinyl oder C₃-C₆-Cycloalkyl, die alle jeweils unsubstituiert sein können, oder einfach bis siebenfach mit Halogen und/oder gegebenenfalls mit einem R^{f} substituiert sein können;
- V: für -NR^{V1}R^{V2}, -N(R^{V1})C(Q¹)R^{b}, -N(R^{b})C(Q¹)R^{V3}, -N(R^{V1})C(Q¹)NR^{b}R^{c}, -N(R^{V1})C(O)OR^{a},-N(R^{V1})C(O)C(O)R^{b} oder -N(R^{V1})SO₂R^{a} steht; dabei steht
R^{V1} für C₁-C₆-Alkyl, das jeweils unsubstituiert sein kann, oder einfach bis 5fach mit Halogen und/oder gegebenenfalls mit 1 bis 2 R^{h} substituiert sein kann; oder für C₂-C₆-Alkenyl, C₂-C₆-Alkinyl oder C₃-C₇-Cycloalkyl, die alle jeweils unsubstituiert sein können, oder einfach bis fünffach mit Halogen und/oder gegebenenfalls mit 1 bis 2 Rⁱ substituiert sein können; oder für Phenyl oder für einen Heterocylus aus der Reihe Furanyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidyl, Pyridazinyl, Pyrazinyl, 1,2,3-Triazinyl, 1,2,4-Triazinyl, 1,3,5-Triazinyl, Oxetanyl, Oxolanyl, Oxanyl, Dioxanyl, Thiethanyl, Thiethanyl-1-oxide, Thiethanyl-1,1-oxide, Thiolanyl, Thiolanyl-1-oxide, Thiolanyl-1,1-oxide, Thianyl, Thianyl-1-oxide, Thianyl-1,1-oxide, Pyrrolidinyl, Piperidinyl oder Dihydroisoxazolyl, die alle jeweils unsubstituiert sein können oder gegebenenfalls einfach bis 5fach mit Halogen und/oder mit 1 bis 2 Rⁱ substituiert sein können;
R^{V2} oder für H; oder für C₁-C₄-Alkyl, C₂-C₄-Alkenyl, C₂-C₄-Alkinyl, C₁-C₄-Alkoxy oder C₃-C₆-Cycloalkyl, alle jeweils alle jeweils unsubstituiert oder einfach bis 5fach substituiert mit Halogen und/oder gegebenenfalls mit 1 bis 3 R^{g}; R^{V1} und R^{V2} zusammen mit dem Stickstoffatom, an das sie gebunden sind, für einen Heterocyclus aus der Reihe U-01 bis U-06 steht 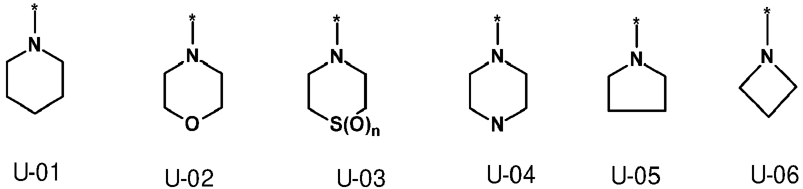 wobei alle U-01 bis U-06 jeweils unsubstituiert sein können oder gegebenenfalls einfach bis 5fach mit Halogen substituiert sein können und/oder gegebenenfalls mit 1 bis 2 Rⁱ
- R^{V3}: für C₁-C₆-Alkyl, C₂-C₆-Alkenyl, C₂-C₆-Alkinyl oder C₃-C₆-Cycloalkyl, die alle jeweils unsubstituiert sein können, oder einfach bis fünffach mit Halogen und/oder gegebenenfalls mit 1 bis 2 Rⁱ substituiert sein können; oder für einen Heterocylus aus der Reihe Oxetanyl, Oxolanyl, Oxanyl, Dioxanyl, Thiethanyl, Thiolanyl, Thianyl, Pyrrolidin-3-yl, Piperidin-4-yl oder Dihydroisoxazolyl jeweils unsubstituiert oder substituiert mit 1 bis 2 Rⁱ
- R¹: für H steht; oder für C₁-C₄-Alkyl, C₂-C₄-Alkenyl, C₂-C₆-Alkinyl oder C₃-C₆-Cycloalkyl steht, die alle jeweils unsubstituiert sein können, oder einfach bis fünffach mit Halogen und/oder gegebenenfalls mit 1 bis 2 Rⁱ substituiert sein können;
- R²: für die Substruktur der allgemeinen Formel -X-Y-Z steht.; dabei steht
X für Phenyl, Pyridyl, Pyrimidyl, Pyridazinyl oder Thienyl, alle jeweils unsubstituiert oder substituiert mit 1 bis 3 R^{X}; dabei steht
R^{X} unabhängig voneinander für Halogen, Cyano, Nitro, C(Q¹)R^{a}, C(O)OR^{a}; oder für C₁-C₄-Alkyl, C₂-C₄-Alkenyl, C₁-C₄-Alkoxy, C₂-C₆-Alkinyl oder C₃-C₆-Cycloalkyl, alle jeweils unsubstituiert oder substituiert mit 1 bis 3 R^{f};
Y für -CR^{Y1}=N-, wobei N an Z gebunden ist, oder für -NR^{Y2}-C(=Q^{Y})-, wobei C an Z gebunden ist; dabei steht jedes
R^{Y1} und R^{Y2} für H; oder für C₁-C₄-Alkyl, C₂-C₆-Alkenyl, C₂-C₄-Alkinyl oder C₃₋C₇-Cycloalkyl, alle jeweils unsubstituiert oder substituiert mit 1 bis 3 R^{Y11}; dabei steht jedes
R^{Y11} unabhängig voneinander für Halogen, Cyano, C₁-C₄-Alkyl, C₃-C₆-Cycloalkyl, C₁-C₄-Haloalkyl, C₁-C₄-Alkoxy, oder C₁-C₄-Haloalkoxy;
Q^{Y} für O oder S;
- Z: für die Fragmente der allgemeinen Formel (A1), (A2) oder (A3); 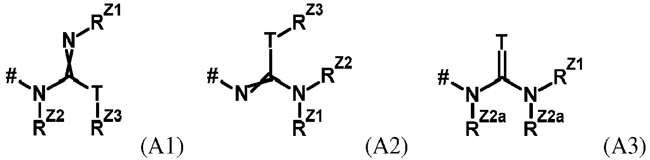 dabei ist # der Anknüpfungspunkt zu Y und dabei steht jedes
T für O oder S;
R^{Z1} für Phenyl, unsubstituiert oder substituiert mit 1 bis 4 R^{Z11}; dabei steht jedes
R^{Z11} unabhängig voneinander für Halogen, Cyano, Nitro, SFs, C(Q¹)R^{a}, C(O)OR^{a}, C(Q¹)NR^{b}R^{c}, NR^{d}R^{e}, OR^{a}, S(O)ₙR^{a} oder SO₂NR^{b}R^{c}; oder für C₁-C₆-Alkyl, C₂-C₆-Alkenyl, C₂-C₆-Alkinyl oder C₃-C₇-Cycloalkyl, alle jeweils unsubstituiert oder substituiert mit 1 bis 5 R^{Z1a}; dabei steht jedes
R^{Z1a} für Halogen, Cyano, C₁-C₃-Alkyl, C₁-C₃-Alkoxy, C₁-C₃-Thioalkyl, C₁-C₃-Haloalkyl oder C₁-C₃-Haloalkoxy;
R^{Z2}, R^{Z2a} und R^{Z3} unabhängig voneinander für H; oder für C(O)R^{a}, C(O)OR^{a}, C(O)NR^{b}R^{c}, S(O)ₙR^{a}; oder für C₁-C₆-Alkyl, C₂-C₆-Alkenyl, C₂-C₆-Alkinyl oder C₃-C₆-Cycloalkyl, alle jeweils unsubstituiert oder substituiert mit 1 bis 4 R^{Z21}; oder für Phenyl oder Benzyl alle jeweils unsubstituiert oder substituiert mit 1 bis 3 R^{Z21}; dabei steht jedes
R^{Z21} unabhängig voneinander für Halogen, Cyano, Nitro, SFs, C(Q¹)R^{a}, C(O)OR^{a}, C(Q¹)NR^{b}R^{c}, NR^{d}R^{e}, OR^{a}, S(O)ₙR^{a} oder SO₂NR^{b}R^{c}; oder für C₁-C₄-Alkyl, C₁-C₄-Haloalkyl, C₁-C₄-Alkenyl, C₂-C₄-Alkinyl oder C₃-C₆-Cycloalkyl; oder
R^{Z2} und R^{Z3} bilden zusammen mit der T-C-N-Einheit einen 5- bis 7-gliedrigen Ring; dabei bestehen die R^{Z2}-R^{Z3}-Ringglieder aus Kohlenstoff-Atomen; dabei können bis zu 2 Kohlenstoff-Atom-Ringglieder unabhängig voneinander aus C(=O) und C(=S) bestehen; dabei ist diese R^{Z2}-R^{Z3}-Einheit unsubstituiert oder substituiert mit 1 bis 4 R^{Z22}, dabei steht jedes
R^{Z22} unabhängig voneinander für Halogen, Hydroxy, Cyano, C₁-C₆-Alkyl, C₃-C₆-Cycloalkyl, C₁-C₆-Haloalkyl, C₁-C₄-Alkenyl, C₁-C₆-Alkoxy, oder C₁-C₆-Haloalkoxy; oder
R^{Z2a} und ein zweites R^{Z2a} bilden zusammen mit der N-C-N-Einheit einen 5- bis 7-gliedrigen Ring; dabei bestehen die R^{Z2a}-R^{Z2a}-Ringglieder aus Kohlenstoff-Atomen; dabei können bis zu 2 Kohlenstoff-Atom-Ringglieder unabhängig voneinander aus C(=O) und C(=S) bestehen; dabei ist diese R^{Z2}-R^{Z3}-Einheit unsubstituiert oder substituiert mit 1 bis 4 R^{Z22};
dabei steht jedes
- R^{a}: unabhängig voneinander für C₁-C₆-Alkyl, C₃-C₆-Cycloalkyl, C₂-C₆-Alkenyl, C₂-C₆-Alkinyl, die alle jeweils unsubstituiert sein können, oder einfach bis 5fach mit Halogen und/oder gegebenenfalls mit einem R^{f}; oder für Phenyl, unsubstituiert oder substituiert mit 1 bis 3 R^{g};
- R^{b} und R^{c}: unabhängig voneinander für H; oder für C₁-C₄-Alkyl, C₃-C₆-Cycloalkyl, C₂-C₄-Alkenyl, C₂-C₄-Alkinyl, die alle jeweils unsubstituiert sein können, oder einfach bis 5fach mit Halogen und/oder gegebenenfalls mit 1 bis 2 R^{f} substituiert sein können; oder R^{b} und R^{c} bilden zusammen einen 3-bis 6-gliedrigen Ring;
- R^{d} und R^{e}: unabhängig voneinander für H, C(Q¹)R^{a}, C(O)OR^{a}; oder für C₁-C₄-Alkyl, C₃-C₆-Cycloalkyl, C₂-C₄-Alkenyl, C₂-C₄-Alkinyl, die alle jeweils unsubstituiert sein können, oder einfach bis 5fach mit Halogen und/oder gegebenenfalls mit 1 bis 2 R^{f} substituiert sein können; oder für Phenyl, unsubstituiert oder substituiert mit 1 bis 3 R^{g}; oder R^{d} und R^{e} bilden zusammen einen 3- bis 6-gliedrigen Ring;
- R^{f}: unabhängig voneinander für Halogen, Cyano, Nitro, C₃-C₆-Cycloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl, C₁-C₄-Alkoxycarbonyl; oder für Phenyl, oder einen 5- oder 6-gliedrigen heteroaromatischen Ring, alle jeweils unsubstituiert oder substituiert mit 1 bis 3 R^{g};
- R^{g}: unabhängig voneinander für Halogen, Cyano, Nitro, C₁-C₄-Alkyl, C₃-C₆-Cycloalkyl, C₁-C₄-Haloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl oder C₁-C₄-Alkoxycarbonyl;
- R^{h}: unabhängig voneinander für Halogen, Cyano, Nitro, Hydroxy, C(Q¹)NR^{b}R^{c}, N(R^{b})C(Q¹)R^{a}, C(O)R^{a}, OC(O)R^{b} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl, spirocyclisch gebundenes C₃-C₆-Cycloalkyl, C₃-C₆-Cycloalkoxy, C₃-C₆-Cycloalkylthio, C₃-C₆-Cycloalkylsulfinyl oder C₃-C₆-Cycloalkylsulfonyl, die alle jeweils unsubstituiert sein können, oder einfach bis siebenfach mit Halogen und/oder gegebenenfalls mit 1 bis 2 R^{g} substituiert sein können; oder für einen Heterocyclus aus der Reihe Oxetanyl, Oxolanyl, Dioxolanyl, Oxanyl, Dioxanyl, Thiethanyl, Thiolanyl, Thianyl oder Dihydroisoxazolyl, die alle jeweils unsubstituiert sein können oder einfach bis 5fach mit Halogen und/oder gegebenenfalls mit 1 R^{g} substituiert sein können;
- Rⁱ: unabhängig voneinander für Halogen, Cyano, Nitro, Hydroxy, C(Q¹)NR^{b}R^{c}, N(R^{c})C(Q¹)R^{a}, C(O)R^{a}, C(=N-OR^{b})R^{a}, C(O)OR^{b}, OC(O)R^{a} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl, spirocyclisch gebundenes C₃-C₆-Cycloalkyl, C₃-C₆-Cycloalkoxy, C₃-C₆-Cycloalkylthio, C₃-C₆-Cycloalkylsulfinyl oder C₃-C₆-Cycloalkylsulfonyl, die alle jeweils unsubstituiert sein können, oder einfach bis siebenfach mit Halogen und/oder gegebenenfalls mit 1 bis 2 R^{g} substituiert sein können; oder für Phenyl oder für einen Heterocyclus aus der Reihe Furanyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidyl, Pyridazinyl, Pyrazinyl, 1,2,3-Triazinyl, 1,2,4-Triazinyl, 1,3,5-Triazinyl, Oxetanyl, Oxolanyl, Dioxolanyl, Oxanyl, Dioxanyl, Thiethanyl, Thiolanyl, Thianyl, oder Dihydroisoxazolyl, alle jeweils unsubstituiert oder substituiert mit 1 bis 3 R^{g};
- Q¹: unabhängig voneinander für O, S, NOR^{a} oder NCN;
- Q²: unabhängig voneinander für O oder NOR^{a};
- n: unabhängig voneinander für 0, 1 oder 2.

Weiter bevorzugt (Ausgestaltung 3-1) sind die Verbindungen der Formel (I), in denen
- A: für N oder CR^{A} steht; dabei steht
R^{A} für H, Halogen oder C₁-C₄-Alkyl;
- V: für -NR^{V1}R^{V2}, -N(R^{V1})C(Q¹)R^{b}, -N(R^{b})C(Q¹)R^{V3} oder -N(R^{V1})C(O)OR^{a} steht; dabei steht
R^{V1} für C₁-C₆-Alkyl, das jeweils unsubstituiert sein kann, oder einfach bis 5fach mit Halogen und/oder gegebenenfalls mit 1 bis 2 R^{h} substituiert sein kann; oder für C₂-C₆-Alkenyl, C₂-C₆-Alkinyl oder C₃-C₆-Cycloalkyl, die alle jeweils unsubstituiert sein können, oder einfach bis 5fach mit Halogen und/oder gegebenenfalls mit 1 bis 2 Rⁱ substituiert sein können; oder für Phenyl oder für einen Heterocylus aus der Reihe Furanyl, Thienyl, Pyrrolyl, Pyrazolyl, Imidazolyl, 1,2,3-Triazolyl, 1,2,4-Triazolyl, Oxazolyl, Isoxazolyl, Thiazolyl, Pyridyl, Pyrimidyl, Oxetanyl, Oxolanyl, Oxan-4-yl, Thiethanyl, Thiethanyl-1-oxide, Thiethanyl-1,1-oxide, Thianyl, Thianyl-1-oxide, Thianyl-1,1-oxide, oder Piperidin-4-yl, die alle jeweils unsubstituiert sein können oder die gegebenenfalls einfach bis 5fach mit Halogen und/oder mit 1 bis 2 Rⁱ substituiert sein können.
R^{V2} für H; oder für C₁-C₄-Alkyl, C₂-C₄-Alkenyl, C₂-C₄-Alkinyl oder C₃-C₆-Cycloalkyl, alle jeweils unsubstituiert oder substituiert mit 1 bis 3 R^{g}; oder
R^{V1} und R^{V2} zusammen mit dem Stickstoffatom, an das sie gebunden sind, für einen Heterocyclus aus der Reihe U-01 bis U-06 steht 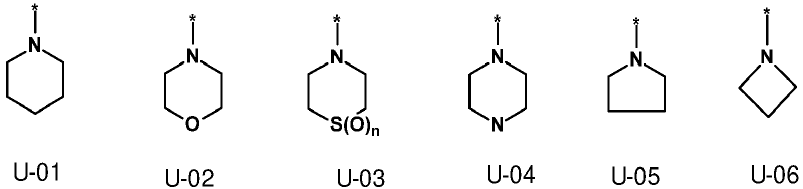 wobei alle U-01 bis U-06 jeweils unsubstituiert sein können oder gegebenenfalls einfach bis 5fach mit Halogen substituiert sein können und/oder gegebenenfalls mit 1 bis 2 Rⁱ
- R^{V3}: für C₁-C₄-Alkyl, C₂-C₄-Alkenyl oder C₃-C₆-Cycloalkyl, die alle jeweils unsubstituiert sein können, oder einfach bis fünffach mit Halogen und/oder gegebenenfalls mit 1 bis 2 Rⁱ substituiert sein können; oder für einen Heterocylus aus der Reihe Oxanyl, Dioxanyl, Thianyl oder Piperidin-4-yl jeweils unsubstituiert oder substituiert mit 1 bis 2 Rⁱ
- R¹: für H steht; oder für C₁-C₄-Alkyl oder C₂-C₄-Alkenyl steht, die alle jeweils unsubstituiert sein können, oder einfach bis fünffach mit Halogen und/oder gegebenenfalls mit 1 bis 2 Rⁱ substituiert sein können;
- R²: für die Substruktur der allgemeinen Formel -X-Y-Z steht; dabei steht
X für Phenyl oder Pyridyl, alle jeweils unsubstituiert oder substituiert mit 1 bis 3 R^{X}; dabei steht
R^{X} unabhängig voneinander für Halogen, Cyano, oder für C₁-C₄-Alkyl, jeweils unsubstituiert oder substituiert mit 1 bis 2 R^{f};
Y für -CR^{Y1}=N-, wobei N an Z gebunden ist, oder für
-NR^{Y2}-C(=Q^{Y})-, wobei C an Z gebunden ist; dabei steht jedes
R^{Y1} und R^{Y2} für H; oder für C₁-C₂-Alkyl jeweils unsubstituiert oder substituiert mit 1 bis 3 R^{Y11}; dabei steht jedes
R^{Y11} unabhängig voneinander für Halogen, Cyano, C₁-C₂-Alkyl oder C₁-C₂-Alkoxy;
Q^{Y} für O oder S;
- Z: für die Fragmente der allgemeinen Formel (A1), (A2) oder (A3); 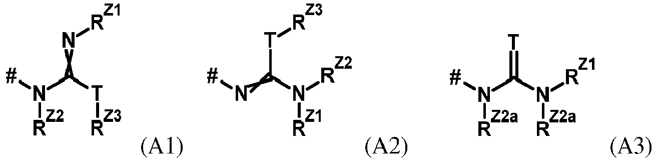 dabei ist # der Anknüpfungspunkt zu Y und dabei steht jedes
T für O oder S;
R^{Z1} für Phenyl, substituiert mit 1 bis 3 R^{Z11}, steht; dabei steht jedes
R^{Z11} unabhängig voneinander für Halogen, Cyano, OR^{a}, SR^{a}, oder für C₁-C₄-Alkyl, C₃-C₆-Cycloalkyl, alle jeweils unsubstituiert oder substituiert mit 1 bis 3 R^{Z1a}; dabei steht jedes
R^{Z1a} für Halogen, C₁-C₃-Alkoxy oder C₁-C₃-Thioalkyl;
R^{Z2}, R^{Z2a} und R^{Z3} unabhängig voneinander für H; oder für C₁-C₄-Alkyl, jeweils unsubstituiert oder substituiert mit 1 bis 3 R^{Z21}; dabei steht jedes
R^{Z21} unabhängig voneinander für Halogen oder C₁-C₃-Alkoxy; oder
R^{Z2} und R^{Z3} bilden zusammen mit der T-C-N-Einheit einen 5- bis 6-gliedrigen Ring; dabei bestehen die R^{Z2}-R^{Z3}-Ringglieder aus Kohlenstoff-Atomen; dabei kann 1 Kohlenstoff-Atom-Ringglied aus C(=O) bestehen; dabei ist diese R^{Z2}-R^{Z3}-Einheit unsubstituiert oder substituiert mit 1 bis 3 R^{Z22}; dabei steht jedes
R^{Z22} unabhängig voneinander für Halogen, C₁-C₃-Alkyl, C₁-C₃-Alkenyl, oder C₁-C₃-Alkoxy;
dabei steht jedes
- R^{a}: unabhängig voneinander für C₁-C₄-Alkyl, C₃-C₆-Cycloalkyl, C₂-C₄-Alkenyl, C₂-C₄-Alkinyl, die alle jeweils unsubstituiert sein können,oder einfach bis 5fach mit Halogen und/oder gegebenenfalls mit einem R^{f} substituiert sein können;
- R^{b}: unabhängig voneinander für H; oder für C₁-C₄-Alkyl, C₃-C₆-Cycloalkyl, C₂-C₄-Alkenyl, C₂-C₄-Alkinyl, die alle jeweils unsubstituiert sein können, oder einfach bis 5fach mit Halogen und/oder gegebenenfalls mit 1 bis 3 R^{f} substituiert sein können;
- R^{f}: unabhängig voneinander für Halogen, Cyano, Nitro, C₃-C₆-Cycloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl, C₁-C₄-Alkoxycarbonyl; oder für Phenyl oder einen 5- oder 6-gliedrigen heteroaromatischen Ring, alle jeweils unsubstituiert oder substituiert mit 1 bis 3 R^{g};
- R^{g}: unabhängig voneinander für Halogen, Cyano, Nitro, C₁-C₄-Alkyl, C₃-C₆-Cycloalkyl, C₁-C₄-Haloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl, C₁-C₄-Alkoxycarbonyl;
- R^{h}: unabhängig voneinander für Halogen, Cyano, Nitro, Hydroxy, C(Q¹)NR^{b}R^{c}, N(R^{b})C(Q¹)R^{a}, C(O)OR^{b} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl oder C₃-C₆-Cycloalkoxy, die alle jeweils unsubstituiert sein können, oder einfach bis fünfach mit Halogen und/oder gegebenenfalls mit 1 R^{g} substituiert sein können;
- Rⁱ: unabhängig voneinander für Halogen, Cyano, Nitro, Hydroxy, C(Q¹)NR^{b}R^{c}, N(R^{b})C(Q¹)R^{a}, C(O)OR^{b} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl oder C₃-C₆-Cycloalkoxy, die alle jeweils unsubstituiert sein können, oder einfach bis fünfach mit Halogen und/oder gegebenenfalls mit 1 R^{g} substituiert sein können; oder für Phenyl oder für einen Heterocyclus aus der Reihe, Thienyl, Pyrazolyl, Pyridyl, Pyrimidyl, Oxetanyl, Oxolanyl, Dioxolanyl, Oxanyl oder Dioxanyl, alle jeweils unsubstituiert oder substituiert mit 1 bis 3 R^{g};
- Q¹: für O;
- Q²: unabhängig voneinander für O oder NOR^{a};
- n: für 0,1,2

Besonders bevorzugt (Ausgestaltung 4-1) sind die Verbindungen der Formel (I), in denen
- A: für N oder CR^{A} steht; dabei steht
R^{A} für H;
- V: für -NR^{V1}R^{V2}, -N(R^{V1})C(O)R^{b} oder -N(R^{b})C(O)R^{V3} steht; dabei steht
R^{V1} für C₁-C₄-Alkyl, das jeweils unsubstituiert sein kann, oder gegebenenfalls einfach bis 5-fach mit Halogen und mit 1 R^{h} substituiert sein kann; oder für C₂-C₄-Alkenyl, C₂-C₆-Alkinyl oder C₃-C₆-Cycloalkyl, die alle jeweils unsubstituiert sein können, oder einfach bis fünffach mit Halogen und/oder gegebenenfalls mit 1 Rⁱ substituiert sein können; oder für Phenyl oder für einen Heterocylus aus der Reihe, Pyridyl, Pyrimidyl, Oxan-4-yl, Thian-4-yl, Thian-1,1-oxid-4-yl oder Piperidin-4-yl die alle jeweils unsubstituiert sein können oder die gegebenenfalls einfach bis 5fach mit Halogen und/oder mit 1 Rⁱ substituiert sein können;
R^{V2} für H; oder für C₁-C₄-Alkyl, unsubstituiert oder substituiert mit 1 bis 3 R^{g}; oder
R^{V1} und R^{V2} zusammen mit dem Stickstoffatom, an das sie gebunden sind, für einen Heterocyclus aus der Reihe U-01, U-05 oder U-06 steht; 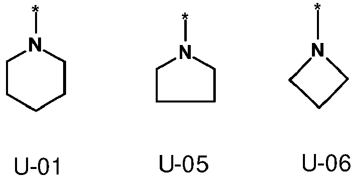 wobei U-01, U-05 und U-06 jeweils unsubstituiert sein können oder gegebenenfalls einfach bis 5fach mit Halogen substituiert sein können und/oder gegebenenfalls mit 1 bis 2 Rⁱ
- R^{V3}: für C₁-C₄-Alkyl oder C₃-C₆-Cycloalkyl, die alle jeweils unsubstituiert sein können, oder einfach bis fünffach mit Halogen und/oder gegebenenfalls mit 1 bis 2 Rⁱ substituiert sein können;
- R¹: für H steht; oder für C₁-C₄-Alkyl steht, das jeweils unsubstituiert sein kann, oder einfach bis dreifach mit Halogen und/oder gegebenenfalls mit 1 bis 2 Rⁱ substituiert sein kann;
- R²: für die Substruktur der allgemeinen Formel -X-Y-Z steht; dabei steht
X für Phenyl, unsubstituiert oder substituiert mit 1 bis 3 R^{X}; dabei steht
R^{X} unabhängig voneinander für Halogen, Cyano, Methyl
Y für -CR^{Y1}=N-, wobei N an Z gebunden ist, oder für
-NR^{Y2}-C(=Q^{Y})-, wobei C an Z gebunden ist; dabei steht jedes
R^{Y1} und R^{Y2} für H;
Q^{Y} für O;
- Z: für die Fragmente der allgemeinen Formel (A1), (A2), (A3); 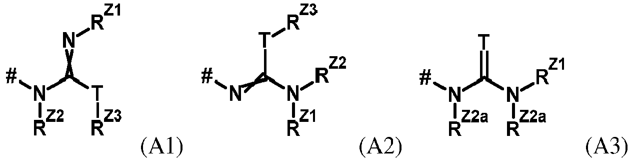 dabei ist # der Anknüpfungspunkt zu Y und dabei steht jedes
T für S;
R^{Z1} für Phenyl, substituiert mit 1 bis 3 R^{Z11}, steht; dabei befindet sich 1 R^{Z11} in 2-Position und dabei steht jedes
R^{Z11} unabhängig voneinander für F, CI, Br, CH₃, CH₂CH₃, CH₂CH₂CH₃, CH(CH₃)₂, Cyclopropyl, OCH₃, OCH₂CH₃, OCH₂CH₂CH₃, OCH(CH₃)₂, CH₂OCH₃ oder CH(CH₃)OCH₃;
R^{Z2}, R^{Z2a} und R^{Z3} unabhängig voneinander für H; oder
R^{Z2} und R^{Z3} bilden zusammen mit der T-C-N-Einheit einen 5- bis 6-gliedrigen Ring; dabei bestehen die R^{Z2}-R^{Z3}-Ringglieder aus Kohlenstoff-Atomen; dabei kann 1 Kohlenstoff-Atom-Ringglied aus C(=O) bestehen; dabei ist diese R^{Z2}-R^{Z3}-Einheit unsubstituiert oder substituiert mit 1 bis 2 R^{Z22}; dabei steht jedes
R^{Z22} unabhängig voneinander für Methyl;
dabei steht jedes
- R^{b}: unabhängig voneinander für H; oder für C₁-C₄-Alkyl, jeweils unsubstituiert oder substituiert mit 1 bis 3 R^{f};
- R^{g}: unabhängig voneinander für Halogen, Cyano, C₁-C₄-Alkyl, C₃-C₆-Cycloalkyl, C₁-C₄-Haloalkyl, C₁-C₄-Alkoxy oder C₁-C₄-Haloalkoxy;
- R^{h}: unabhängig voneinander für Halogen, Cyano, Hydroxy; oder für C₁-C₄-Alkyl, C₁-C₄-Alkoxy oder C₃-C₆-Cycloalkyl, die alle unsubstituiert sein können, oder einfach bis fünfach mit Halogen und/oder gegebenenfalls mit 1 R^{g} substituiert sein können;
- Rⁱ: unabhängig voneinander für Halogen, Cyano, Hydroxy; oder für C₁-C₄-Alkyl, C₁-C₄-Alkoxy oder C₃-C₆-Cycloalkyl, die alle unsubstituiert sein können, oder einfach bis fünfach mit Halogen und/oder gegebenenfalls mit 1 R^{g} substituiert sein können; oder für Phenyl, Thienyl oder Pyridyl, die alle jeweils unsubstituiert sein können oder einfach bis fünfach mit Halogen und/oder gegebenenfalls mit 1 R^{g} substituiert sein können;

Ganz besonders bevorzugt (Ausgestaltung 5-1) sind die Verbindungen der Formel (I), in denen
- A: für N oder CR^{A} steht; dabei steht
R^{A} für H;
- V: für -NR^{V1}R^{V2}, -N(R^{V1})C(=O)R^{b} oder -N(R^{b})C(=O)R^{V3} steht; dabei steht
R^{V1} für C₁-C₄-Alkyl, das jeweils unsubstituiert ist oder das gegebenenfalls einfach bis 5-fach mit F oder Cl, und/oder oder gegebenenfalls einfach mit R^{h} substituiert sein kann; oder für C₃-C₆-Cycloalkyl, das jeweils unsubstituiert sein kann, oder einfach bis dreifach mit F oder Cl, und/oder oder gegebenenfalls einfach mit Rⁱ substituiert sein kann; oder für Phenyl das gegebenenfalls einfach bis dreifach Halogen und/oder mit 1 R^{g}, substituiert sein kann;
R^{V2} für H, Methyl oder Ethyl;
R^{V1} und R^{V2} zusammen mit dem Stickstoffatom, an das sie gebunden sind, für eine Heterocyclus aus der Reihe U-01, U-05 oder U-06 stehen; 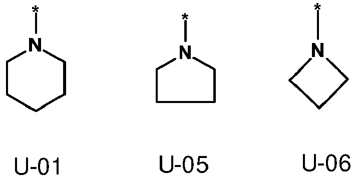
wobei U-01, U-05 und U-06 jeweils unsubstituiert sein können oder gegebenenfalls einfach bis 3fach mit Fluor oder Chlor oder einfach mit CN, C₁-C₄-Alkyl, C₁-C₄-Alkoxy, CF₃ oder C₂F₅ substituiert sein können;
R^{V3} für C₁-C₄-Alkyl oder C₃-C₆-Cycloalkyl, die alle jeweils unsubstituiert sein können, oder einfach bis dreifach mit F oder Cl, oder gegebenenfalls einfach bis zweifach mit R^{h} substituiert sein können;
- R¹: für H steht; oder für C₁-C₄-Alkyl steht, das jeweils unsubstituiert sein kann, oder einfach bis 3fach mit Fluor oder einfach mit CN oder C₁-C₄-Alkoxy, substituiert sein kann;
- R²: für die Substruktur der allgemeinen Formel -X-Y-Z steht; dabei steht
X für Phenyl, unsubstituiert oder substituiert mit 1 bis 2 R^{X}; dabei steht
R^{X} unabhängig voneinander für Fluor, Chlor, Cyano oder Methyl;
- Y: für -CR^{Y1}=N-, wobei N an Z gebunden ist, oder für
-NR^{Y2}-C(=Q^{Y})-, wobei C an Z gebunden ist; dabei steht jedes
R^{Y1} und R^{Y2} für H;
Q^{Y} für O;
- Z: für die Fragmente der allgemeinen Formel (A2) oder (A3); 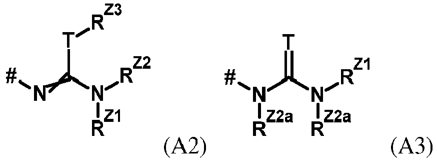 dabei ist # der Anknüpfungspunkt zu Y und dabei steht jedes
- T: für S;
- R^{Z1}: für Phenyl, substituiert mit 1 bis 3 R^{Z11}, steht; dabei befindet sich 1 R^{Z11} in 2-Position und dabei steht jedes
R^{Z11} unabhängig voneinander für F, CI, Br, CH₃, CH₂CH₃, CH₂CH₂CH₃, CH(CH₃)₂, Cyclopropyl, OCH₃, OCH₂CH₃, OCH₂CH₂CH₃, OCH(CH₃)₂ oder CH₂OCH₃;
- R^{Z2}, R^{Z2a} und R^{Z3}: für H; oder
- R^{Z2} und R^{Z3}: bilden zusammen -C(O)CH₂-;
dabei steht jedes
R^{b} und R^{c} unabhängig voneinander für H; oder für Methyl, Ethyl, n-Propyl, iso-Propyl oder Cyclopropyl;
R^{g} unabhängig voneinander für Fluor, Chlor, Cyano, Methyl, Ethyl, n-Propyl, i-Propyl, Methoxy, Ethoxy, CF₃, C₂F₅, OCF₃ oder OC₂F₅;
R^{h} unabhängig voneinander für Fluor, Chlor, Cyano, Methyl, Ethyl, n-Propyl, i-Propyl, Methoxy, Ethoxy, CF₃, C₂F₅, OCF₃ oder OC₂F₅; oder für Cyclohexyl, das unsubstituiert sein kann oder das gegebenenfalls einfach bis 5-fach mit F und/oder gegebenenfalls einfach mit CF₃ substituiert sein kann;
Rⁱ unabhängig voneinander für Fluor, Chlor, Cyano, Methyl, Ethyl, n-Propyl, i-Propyl, t-Butyl, - CF3, C₂F₅, Methoxy, Ethoxy oder OCF₃ ;

Hervorgehoben (Ausgestaltung 6-1) sind die Verbindungen der Formel (I), in denen
- A: für N oder CH steht;
- V: für -NR^{V1}R^{V2}, -N(R^{V1})C(=O)CH₃ oder -N(H)C(=O)R^{V3} steht; dabei steht
R^{V1} für 4,4,4-Trifluorbutyl, 3,3,3-Trifluorpropyl, Methoxyethyl, Cyclohexyl, 4-(Trifluormethyl)cyclohexyl, 4-(Trifluormethyl)cyclohexylmethyl, 4-(tert.Butyl)cyclohexyl, 4,4-Difluorcyclohexyl, oder für 4-(Trifluormethoxy)phenyl, 4-(Pentafluorethoxy)phenyl, 3-(Trifluormethoxy)phenyl, 3,4-Dichlorphenyl;
R^{V2} für H; oder
R^{V1} und R^{V2} zusammen mit dem Stickstoffatom, an das sie gebunden sind, für 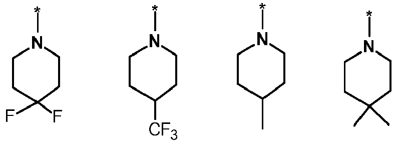 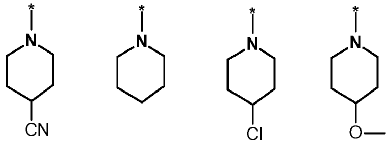 stehen;
- R^{V3}: für 4-(Trifluormethyl)cyclohexyl steht;
- R¹: für H, Methyl, Ethyl, Ethoxymethyl oder Methoxyethyl steht.
- R²: für die Substruktur der allgemeinen Formel -X-Y-Z steht; dabei steht
X für Phenyl
Y für -CH=N-, wobei N an Z gebunden ist, oder für
-NH-C(=O)-, wobei C an Z gebunden ist; dabei steht jedes
Z für die Fragmente der allgemeinen Formel (A2-1) oder (A3-1); 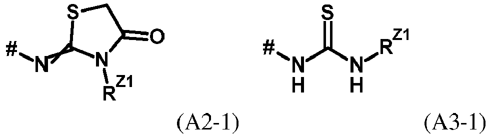 dabei ist # der Anknüpfungspunkt zu Y und dabei steht jedes
R^{Z1} für Phenyl, substituiert mit 1 bis 2 R^{Z11}, steht; dabei befindet sich 1 R^{Z11} in 2-Position und dabei steht jedes
R^{Z11} unabhängig voneinander für Methyl, Methoxy oder iso-Propyl

In einer weiteren bevorzugten Ausführungsform betrifft die Erfindung Verbindungen der allgemeinen Formel (I-1), in denen die Strukturelemente V, R¹, R^{Y1} und R^{Z1} die in Ausgestaltung (1-1) oder die in Ausgestaltung (2-1) oder die in Ausgestaltung (3-1) oder die in Ausgestaltung (4-1) oder die in Ausgestaltung (5-1) angegebenen Bedeutungen haben.

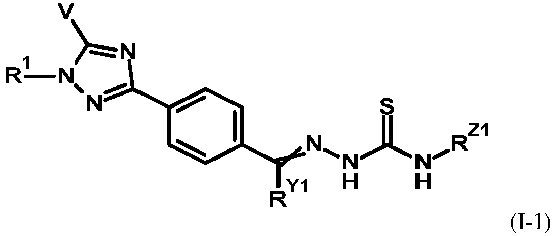

In einer weiteren bevorzugten Ausführungsform betrifft die Erfindung Verbindungen der allgemeinen Formel (I-2), in denen die Strukturelemente Ar, R¹, R^{Y1} und R^{Z1} die in Ausgestaltung (1-1) oder die in Ausgestaltung (2-1) oder die in Ausgestaltung (3-1) oder die in Ausgestaltung (4-1) oder die in Ausgestaltung (5-1) angegebenen Bedeutungen haben.

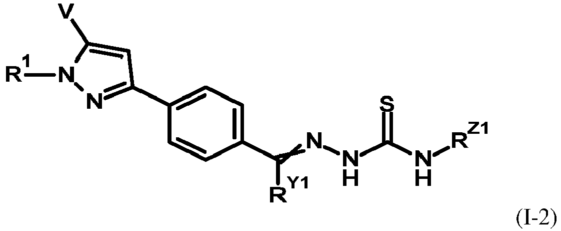

In einer weiteren bevorzugten Ausführungsform betrifft die Erfindung Verbindungen der allgemeinen Formel (I-3), in denen die Strukturelemente V, R¹, R^{Y1} und R^{Z1} die in Ausgestaltung (1-1) oder die in Ausgestaltung (2-1) oder die in Ausgestaltung (3-1) oder die in Ausgestaltung (4-1) oder die in Ausgestaltung (5-1) angegebenen Bedeutungen haben.

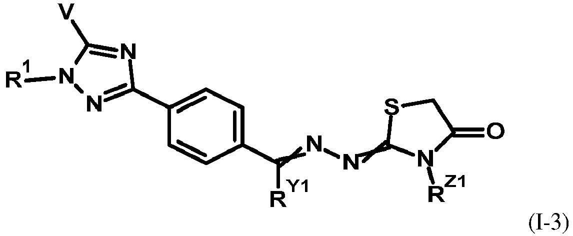

In einer weiteren bevorzugten Ausführungsform betrifft die Erfindung Verbindungen der allgemeinen Formel (I-4), in denen das Strukturelemente V, R¹, R^{Y1} und R^{Z1} die in Ausgestaltung (1-1) oder die in Ausgestaltung (2-1) oder die in Ausgestaltung (3-1) oder die in Ausgestaltung (4-1) oder die in Ausgestaltung (5-1) angegebenen Bedeutungen haben.

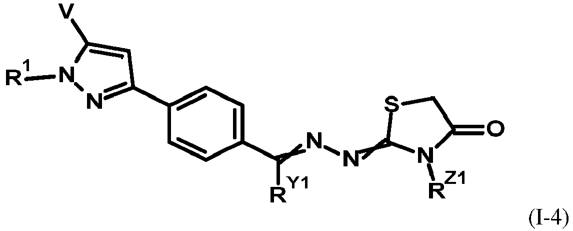

In einer weiteren bevorzugten Ausführungsform betrifft die Erfindung Verbindungen der allgemeinen Formel (I-5), in denen die Strukturelemente V, R¹ und R^{Z1} die in Ausgestaltung (1-1) oder die in Ausgestaltung (2-1) oder die in Ausgestaltung (3-1) oder die in Ausgestaltung (4-1) oder die in Ausgestaltung (5-1) angegebenen Bedeutungen haben.

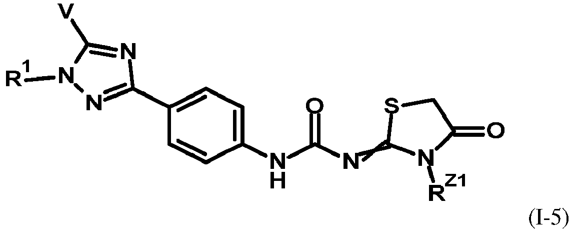

Die Verbindungen der Formel (I) können gegebenenfalls auch in Abhängigkeit von der Art der Substituenten als Stereoisomere, d.h. als geometrische und/oder als optische Isomere oder Isomerengemische in unterschiedlichen Zusammensetzungen vorliegen. Sowohl die reinen Stereoisomeren als auch beliebige Gemische dieser Isomeren sind Gegenstand dieser Erfindung, auch wenn hier im Allgemeinen nur von Verbindungen der Formel (I) die Rede ist.

Vorzugsweise werden jedoch die optisch aktiven, stereoisomeren Formen der Verbindungen der Formel (I) und deren Salze erfindungsgemäß verwendet.

Die Erfindung betrifft daher sowohl die reinen Enantiomeren und Diastereomeren, als auch deren Gemische zur Bekämpfung von tierischen Schädlingen, zu denen Arthropoden und insbesondere Insekten zählen.

Die Verbindungen der Formel (I) können gegebenenfalls in verschiedenen polymorphen Formen oder als Mischung verschiedener polymorpher Formen vorliegen. Sowohl die reinen Polymorphe als auch die Polymorphgemische sind Gegenstand der Erfindung und können erfindungsgemäß verwendet werden.

Sofern nicht an anderer Stelle anders definiert, wird unter dem Begriff **"Alkyl"**, entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sec.-Butyl, tert-Butyl, n-Pentyl, iso-Pentyl, Neopentyl, tert-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste.

Sofern nicht an anderer Stelle anders definiert, wird unter dem Begriff **"Alkenyl"**, entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Sofern nicht an anderer Stelle anders definiert, wird unter dem Begriff **"Alkinyl",** entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Sofern nicht an anderer Stelle anders definiert, wird unter dem Begriff **"Cycloalkyl"**, entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Sofern nicht an anderer Stelle anders definiert, wird unter dem Begriff **"Aryl"** erfindungsgemäß ein aromatischer Rest mit 6 bis 14 Kohlenstoffatomen, vorzugsweise Phenyl, Naphthyl, Anthryl oder Phenanthrenyl, besonders bevorzugt Phenyl, verstanden.

Sofern nicht an anderer Stelle anders definiert, wird unter dem Begriff **"Arylalkyl"** eine Kombination von erfindungsgemäß definierten Resten "Aryl" und "Alkyl" verstanden, wobei der Rest im Allgemeinen über die Alkylgruppe gebunden wird, Beispiele hierfür sind Benzyl, Phenylethyl oder α-Methylbenzyl, wobei Benzyl besonders bevorzugt ist.

Sofern nicht an anderer Stelle anders definiert, bedeutet **"Hetaryl"** eine mono-, bi- oder tricyclische heterocyclische Gruppe aus C-Atomen und mindestens einem Heteroatom, wobei mindestens ein Zyklus aromatisch ist. Bevorzugt enthält die Hetaryl-Gruppe 3, 4, 5 oder 6 C-Atome ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuranyl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl.

Sofern nicht an anderer Stelle anders definiert, bedeutet **"Heterocyclyl"** einen monocyclischen, gesättigten oder teilgesättigten 4-, 5-, 6- oder 7-gliedrigen Ring aus C-Atomen und mindestens einem Heteroatom im Ring. Bevorzugt enthält die Heterocyclyl-Gruppe 3, 4, 5 oder 6 C-Atome und 1 oder 2 Heteroatome aus der Reihe Sauerstoff, Schwefel oder Stickstoff. Beispiele für Heterocyclyl sind Azetidinyl, Azolidinyl, Azinanyl, Oxetanyl, Oxolanyl, Oxanyl, Dioxanyl, Thiethanyl, Thiolanyl, Thianyl, Tetrahydrofuryl.

Sofern nicht an anderer Stelle anders definiert, bedeuten **"Oxo-Heterocyclyl"** bzw. **"Dioxo-Heterocyclyl"** ein Heterocyclyl, das an mindestens einer Position im Ring ein Ring-Atom enthält, das mit einer bzw. zwei (=O) -Gruppen substituiert ist. Bevorzugt ist dabei ein Heteroatom wie beispielsweise Schwefel mit einer oder zwei (=O) -Gruppen substituiert, wobei sich dann die Gruppe -S(=O)- bzw. -S(=O)₂- ergibt, wobei das S-Atom Bestandteil des Rings ist.

Im Rahmen der vorliegenden Erfindung werden unter durch Halogensubstituierte Reste, beispielsweise **"Halogenalkyl",** einfach oder mehrfach bis zur maximal möglichen Substituentenzahl halogenierte Reste verstanden. Bei mehrfacher Halogenierung können die Halogenatome gleich oder verschieden sein. **"Halogen"** steht dabei für Fluor, Chlor, Brom oder Jod, bevorzugt für Fluor oder Chlor.

Unter dem Begriff **"Alkoxy",** entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

### Erläuterung der Verfahren und Zwischenprodukte

### Verfahren A

V, R¹, A, X, R^{Y1}, T und R^{Z1} haben die oben beschriebenen Bedeutungen. Damit entspricht die allgemeine Formel (Ia) der allgemeinen Formel (I) mit R² = X-Y-Z, wobei Y für -CR^{Y1}=N-, Z für das Fragment der allgemeinen Formel (A3) und R^{Z2a} für H stehen.

Verbindungen der allgemeinen Formel (Ia) können entsprechend Abbildung 1 aus Aldehyden (R^{Y1} = H) oder Ketonen der allgemeinen Formel (II) und Hydrazin-Derivaten der allgemeinen Formel (III) in einem geeigneten Lösungsmittel, wie z.B. Ethanol oder Methanol, gegebenenfalls mit einem Katalysator, wie z.B. Essigsäure, und bei geeigneter Temperatur, wie z.B. in einem Bereich von 50 °C bis 80 °C, hergestellt werden. Eine repräsentative Vorschrift dieser Methode findet sich in WO 2013/116053 oder WO 2013/116052.

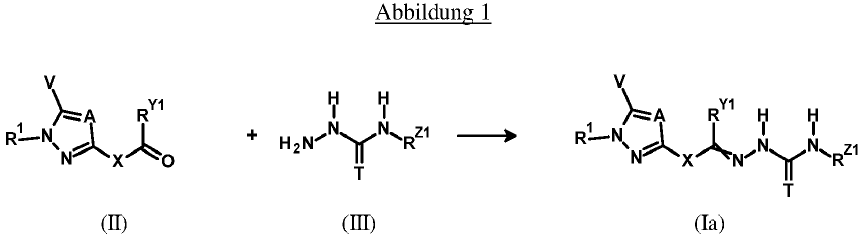

### Verfahren B

V, R¹, A, X, R^{Y1}, R^{Z3} und R^{Z1} haben die oben beschriebenen Bedeutungen. T steht für S. Damit entspricht die allgemeine Formel (Ib) der allgemeinen Formel (I) mit R² = X-Y-Z, wobei Y für -CR^{Y1}=N-, Z für das Fragment der allgemeinen Formel (A2) und R^{Z2} für H stehen.

Verbindungen der allgemeinen Formel (Ib) können entsprechend Abbildung 2 aus Verbindungen der allgemeinen Formel (Ia) und R^{Z3}-LG (LG = Abgangsgruppe) in Gegenwart einer Base, wie z.B. Kaliumcarbonat oder Triethylamin, in einem geeigneten Lösungsmittel, wie z.B. Aceton oder Dichlormethan, und bei geeigneter Temperatur, wie z.B. in einem Bereich von 20 °C bis 60 °C, hergestellt werden. Eine repräsentative Vorschrift dieser Methode findet sich in WO 2013/116053 oder WO 2013/116052.

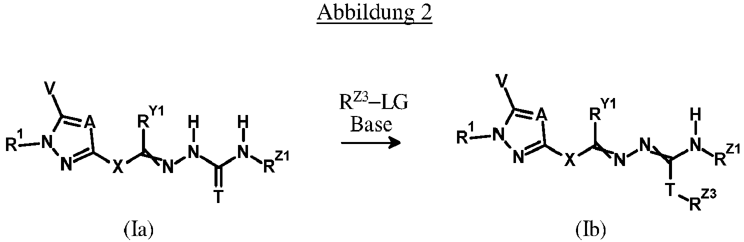

### Verfahren C

V, R¹, A, X, R^{Y1}, und R^{Z1} haben die oben beschriebenen Bedeutungen. Damit entspricht die allgemeine Formel (Ic) der allgemeinen Formel (I) mit R² = X-Y-Z, wobei Y für -CR^{Y1}=N- und Z für das Fragment der allgemeinen Formel (A2-1) stehen.

Verbindungen der allgemeinen Formel (Ic-1 Ic-2, Ic-3 und Ic-4) können entsprechend Abbildung 3 aus Verbindungen der allgemeinen Formel (Ia) mit T = S durch Umsetzung mit einer Verbindung aus der Reihe LG(CH₂)nCO₂Et, LG(CH₂)nLG, LG(CH₂)nC(=O)CH₃ oder LGCH₂(C=CH₂)CH₂LG (LG= Cl, Br; n=1,2) gegebenenfalls in Gegenwart einer Base, wie z.B. Natriumacetat oder Kaliumcarbonat, in einem geeigneten Lösungsmittel, wie z.B. Ethanol, und bei geeigneter Temperatur, wie z.B. in einem Bereich von 50 °C bis 80 °C, hergestellt werden. Eine repräsentative Vorschrift dieser Methode findet sich in WO 2013/116053 oder WO 2013/116052.

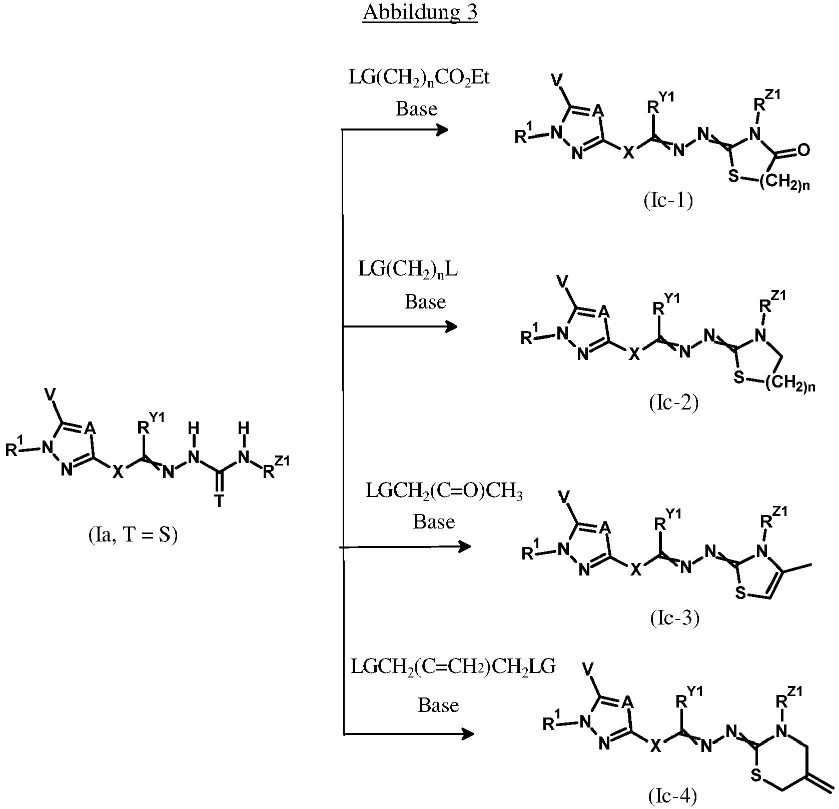

### Verfahren D

V, R¹, A, X, R^{Y1}, R^{Z2}, R^{Z3} und R^{Z1} haben die oben beschriebenen Bedeutungen. Damit entspricht die allgemeine Formel (Id) der allgemeinen Formel (I) mit R² = X-Y-Z, wobei Y für -CR^{Y1}=N- und Z für das Fragment der allgemeinen Formel (A1) stehen.

Verbindungen der allgemeinen Formel (Id) können entsprechend Abbildung 4 aus Aldehyden (R^{Y1} = H) oder Ketonen der allgemeinen Formel (II) und Hydrazin-Derivaten der allgemeinen Formel (IV) in einem geeigneten Lösungsmittel, wie z.B. Ethanol, gegebenenfalls mit einem Katalysator, wie z.B. Essigsäure, und bei geeigneter Temperatur, wie z.B. in einem Bereich von 50 °C bis 80 °C, hergestellt werden. Eine repräsentative Vorschrift dieser Methode findet sich in WO 2016/196280.

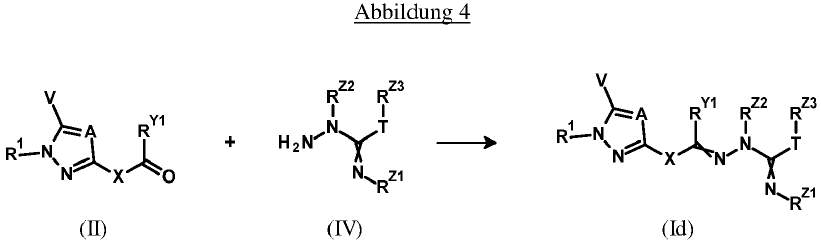

### Verfahren E

V, R¹, A, X, R^{Y2} und R^{Z1} haben die oben beschriebenen Bedeutungen. Damit entspricht die allgemeine Formel (If) der allgemeinen Formel (I) mit R⁴ = X-Y-Z, wobei Y für -NR^{Y2}-C(=O)- und Z für das Fragment der allgemeinen Formel (A2-1) stehen.

Verbindungen der allgemeinen Formel (If) können entsprechend Abbildung 5 hergestellt werden, indem zunächst Amine der allgemeinen Formel (V) mit 4-Nitrophenoxyameisensäurechlorid in einem geeigneten Lösungsmittel, wie z.B. Tetrahydrofuran zu 4-Nitrophenylcarbamaten der allgemeinen Formel (VI) umsetzt werden. Verbindungen der allgemeinen Formel (VI) können anschließend in einem geeigneten Lösungsmittel, wie z.B. Acetonitril, und bei geeigneter Temperatur, wie z.B. in einem Bereich von 0 °C bis 40 °C, mit Iminen der allgemeinen Formel (VII) zu Verbindungen der allgemeinen Formel (If) umgesetzt werden. Eine repräsentative Vorschrift dieser Methode findet sich in WO 2016/033025.

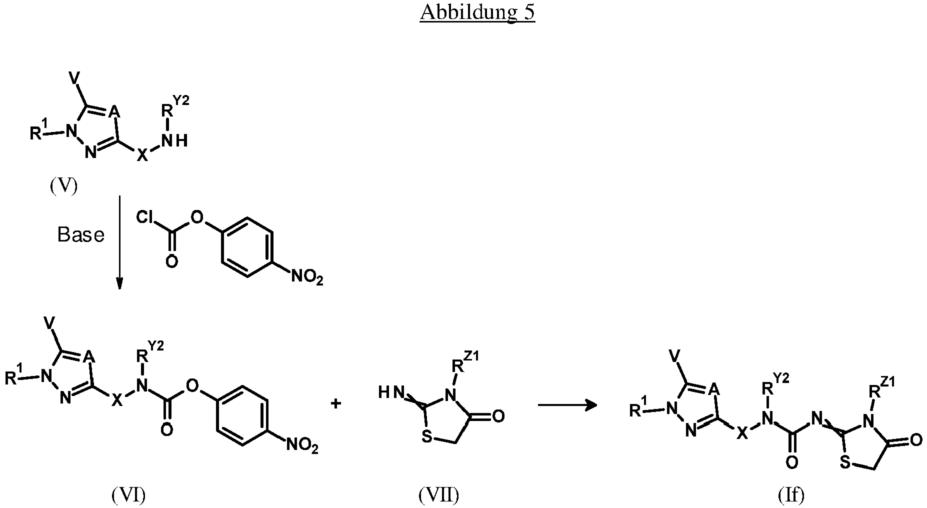

### Verfahren F-1

R^{V1}, R¹, R², A, R^{V1}, X, R^{a} und R^{b} haben die oben beschriebenen Bedeutungen. Damit entsprechen die allgemeine Formel (Ih), (Ii), (Ij) und (Ik) der allgemeinen Formel (I) mit V = NR^{V1}H, NR^{V1}C(O)OR^{a}, NR^{V1}SO₂R^{a}. NR^{V1}C(O)R^{b}

Verbindungen der allgemeinen Formel (Ii), (Ij) und (Ik) können entsprechend Abbildung 6 aus Verbindungen der allgemeinen Formel (Ih) und Säurehalogeniden wie z.B. Carbonsäurechloriden, Anhydriden, wie z.B. Acetanhydrid oder Sulfonsäurechloriden der Formel (XI, LG= Abgangsgruppe) gegebenenfalls in Gegenwart einer Base, wie z.B. Pyridin, in einem geeigneten Lösungsmittel, wie z.B. Chloroform, und bei geeigneter Temperatur, wie z.B. in einem Bereich von 0 °C bis 50 °C, hergestellt werden.

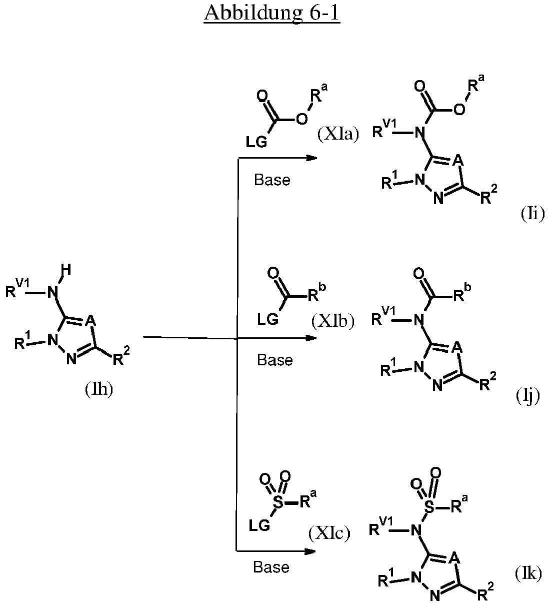

### Verfahren F-2

R^{V1}, R¹, A, X, R^{a} und R^{b} haben die oben beschriebenen Bedeutungen. Damit entsprechen die allgemeine Formel (VIIIb), (VIIIc), (VIIId) und (VIIIe) der allgemeinen Formel (VIII) mit V = NR^{V1}H, NR^{V1}C(O)OR^{a}, NR^{V1}SO₂R^{a}. NR^{V1}C(O)R^{b}

Verbindungen der allgemeinen Formel (VIIIc), (VIIId) und (VIIIe) können entsprechend Abbildung 6 aus Verbindungen der allgemeinen Formel (VIIIb) und Säurehalogeniden wie z.B. Carbonsäurechloriden, Anhydriden, wie z.B. Acetanhydrid oder Sulfonsäurechloriden der Formel (XI, LG= Abgangsgruppe) gegebenenfalls in Gegenwart einer Base, wie z.B. Pyridin, in einem geeigneten Lösungsmittel, wie z.B. Chloroform, und bei geeigneter Temperatur, wie z.B. in einem Bereich von 0 °C bis 50 °C, hergestellt werden.

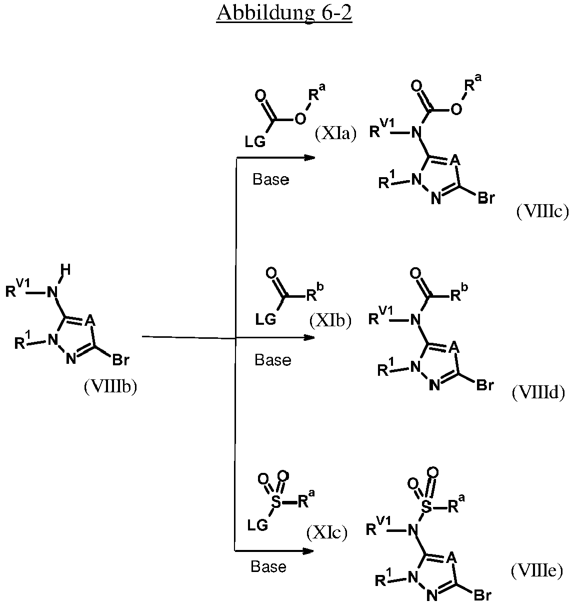

### Verfahren G

V, R¹, A, X und R^{Y1} haben die oben beschriebenen Bedeutungen. L¹ und L² stehen jeweils für H oder stehen zusammen für C(CH₃)₂-C(CH₃)₂

Verbindungen der allgemeinen Formel (II) können entsprechend Abbildung 7 durch eine Suzuki-Kupplung zwischen einer Bromverbindung der allgemeinen Formel (VIII) und einem Boronsäurederivat der allgemeinen Formel (IXa) in Gegenwart eines Pd-Katalysators, wie z.B. Tetrakis(triphenylphosphin)palladium und einer Base, wie z.B. Natriumcarbonat, in einem geeigneten Lösungsmittel, wie z.B. einer Ethanol-Toluol-Mischung, 1,2-Dimethoxyethan oder Dioxan, und bei geeigneter Temperatur, wie z.B. in einem Bereich von 60 °C bis 150 °C, hergestellt werden.

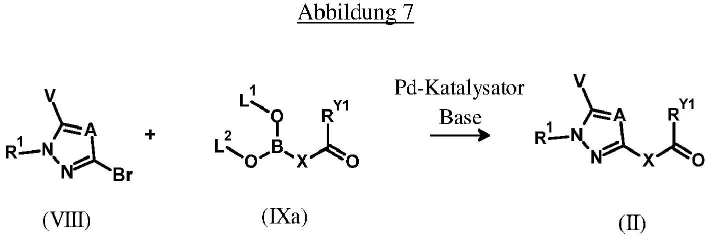

### Verfahren H

V, R¹, A, X, R¹ und R^{Y2} haben die oben beschriebenen Bedeutungen. L¹ und L² stehen jeweils für H oder stehen zusammen für C(CH₃)₂-C(CH₃)₂

Verbindungen der allgemeinen Formel (V) können entsprechend Abbildung 8 durch eine Suzuki-Kupplung zwischen einer Bromverbindung der allgemeinen Formel (VIII) und einem Boronsäurederivat der allgemeinen Formel (IXb) in Gegenwart eines Pd-Katalysators, wie z.B. 1,1'-Bis(diphenylphosphino)ferrocen-palladium und einer Base, wie z.B. Caesiumcarbonat, in einem geeigneten Lösungsmittel, wie z.B. einer Ethanol-Toluol-Mischung, 1,2-Dimethoxyethan oder Dioxan, und bei geeigneter Temperatur, wie z.B. in einem Bereich von 60 °C bis 150 °C, hergestellt werden.

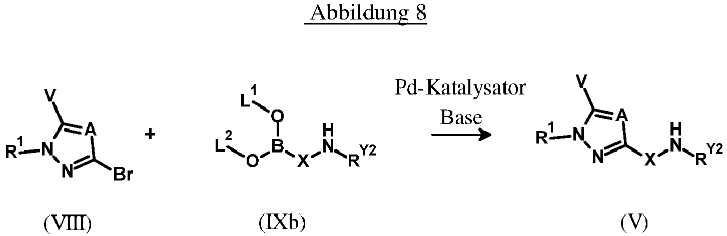

### Verfahren I

V, R¹, R^{V1} und R^{V2} haben die oben beschriebenen Bedeutungen.

Bromtriazole der allgemeinen Formel (VIIIa) können entsprechend Abbildung 9 durch nucleophile Substitution ausgehend von der Dibromtriazolen der allgemeinen Formel (X) und einer Aminoverbindung der Formel (XI), wie z.B. einem primären oder sekundären aliphatischen Amin gegebenenfalls durch Zusatz einer Base, wie z.B. Kaliumcarbonat in einem geeigneten Lösungsmittel, wie z.B. Dimethylacetamid (DMA), und bei geeigneter Temperatur, wie z.B. in einem Bereich von 20 °C bis 150 °C, hergestellt werden.

Dibromtriazolen der allgemeinen Formel (X) können entsprechend Abbildung 9 in Analogie zu WO2017/004500 auch mit aromatischen oder heteroaromatischen Aminen der Formel (XI) durch Zusatz einer starken Base, wie z.B. Lithium trimethyl-N-(trimethylsilyl)silanaminid in einem geeigneten Lösungsmittel, wie z.B. THF bei geeigneter Temperatur, wie z.B. in einem Bereich von -78°C bis 20°C zu Bromtriazolen der allgemeinen Formel (VIIIa) umgesetzt werden.

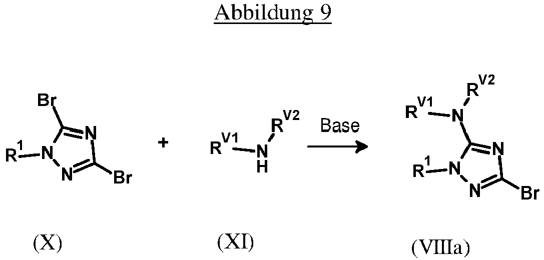

### Verfahren J

R¹ hat die oben beschriebene Bedeutung.

Dibromtriazole der allgemeinen Formel (X) können entsprechend Abbildung 10 in Analogie zu WO2018/87018 aus Dibromtriazol und einer Halogenalkylverbindung der allgemeinen Formel (XII, Hal= Cl,Br,I) in Gegenwart einer Base, wie z.B. Kaliumcarbonat, in einem geeigneten Lösungsmittel, wie z.B. Dimethylformamid, und bei geeigneter Temperatur, wie z.B. in einem Bereich von 20 °C bis 120 °C, hergestellt werden.

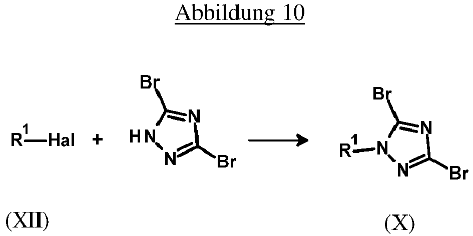

### Verfahren K

R¹, X und R^{b} haben die oben beschriebenen Bedeutungen. L¹ und L² stehen jeweils für H oder stehen zusammen für C(CH₃)₂-C(CH₃)₂.

Pyrazole der allgemeinen Formel (IIa) können entsprechend Abbildung 11 hergestellt werden, indem zunächst Aminopyrazole der allgemeinen Formel (XII) mit Boronsäureestern der allgemeinen Formel (IXc) in Gegenwart eines Palladiumkatalysators, wie z.B. 1,1'-Bis(diphenylphosphino)ferrocen-palladium in einem geeigneten Lösungsmittel, wie z.B. Dioxane und bei geeigneter Reaktionstemperatur, wie z.B. bei 60 °C bis 120 °C zu Pyrazolen der allgemeinen Formel (XIII) umsetzt werden. Verbindungen der allgemeinen Formel (XIII) werden anschließend mit einer Arylhalogenverbindung der allgemeinen Formel (XIV) in Gegenwart eines Palladiumkatalysators, wie z.B. XanthPhos und einer Base, wie z.B. Kaliumkarbonat in einem geeigneten Lösungsmittel, wie z.B. Dioxan bei geeigneter Temperatur, wie z.B. 20°C bis 120 °C umgesetzt, wobei Verbindungen der allgemeinen Formel (XV) erhalten werden. Verbindungen der allgemeinen Formel (XV) werden dann zunächst mit einem geeigneten Reduktionsmittel, wie z.B. Diisobutylaluminiumhydrid in einen geeigneten Lösungsmittel, wie z.B. Tetrahydrofuran zu Alkoholen der allgemeinen Formel (XVI) reduziert und anschließend mit einem Oxidationsmittel, wie z.B. dem Dess-Martin Reagenz in einem geeigneten Lösungsmittel, wie z.B. Dichlormethan zu Verbindungen der allgemeinen Formel (IIa) oxidiert.

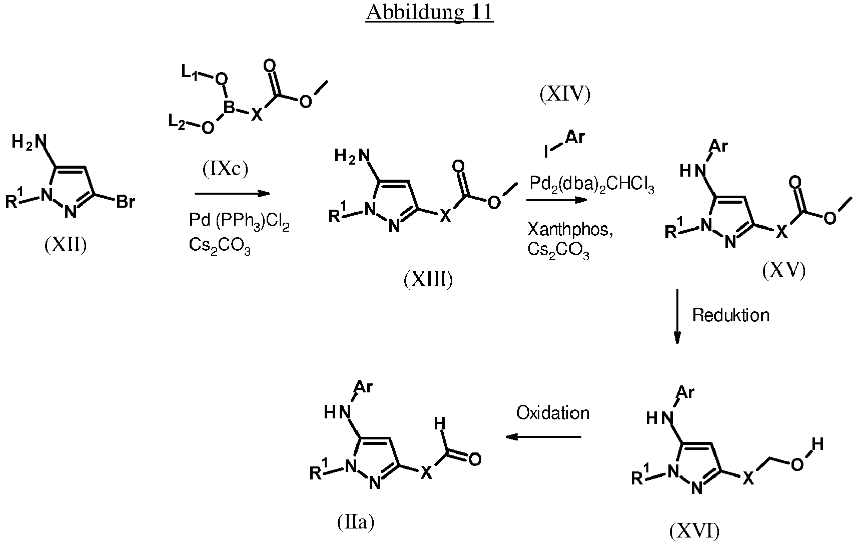

Verbindungen der allgemeinen Formel (III) sind literaturbekannt oder können in Analogie zu literaturbekannten Methoden erhalten werden (vgl. z.B. WO 2013/116053).

Verbindungen der allgemeinen Formel (IV) sind literaturbekannt oder können in Analogie zu literaturbekannten Methoden erhalten werden (vgl. z.B. WO 2013/116053).

Iminothiazolidinone der allgemeinen Formel (VII) sind literaturbekannt oder können in Analogie zu literaturbekannten Methoden erhalten werden (vgl. z.B. US 2014/0274688 oder WO 2016/033025)

Verbindungen der allgemeinen Formel (XII) sind literaturbekannt oder können in Analogie zu literaturbekannten Verfahren erhalten werden. (vgl. z.B. EP 2848615).

Verbindungen der Formel (IXa-c), (XI), (XII) und (XIV) sind käuflich oder können in Analogie zu allgemein bekannten Verfahren erhalten werden.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit sowohl reine Stereoisomere als auch beliebige Gemische dieser Isomere.

### Verfahren und Verwendungen

Die Erfindung betrifft auch Verfahren zur Bekämpfung von tierischen Schädlingen, bei denen man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel jeweils immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung ist der Begriff "Hygiene" so zu verstehen, dass damit jegliche und alle Maßnahmen, Vorschriften und Verfahrensweisen gemeint sind, deren Ziel es ist, Krankheiten, insbesondere Infektionskrankheiten, zu verhindern, und die dazu dienen, die Gesundheit von Menschen und Tieren zu schützen und/oder die Umwelt zu schützen, und/oder die Sauberkeit aufrechterhalten. Erfindungsgemäß schließt dies insbesondere Maßnahmen zur Reinigung, Desinfektion und Sterilisation beispielsweise von Textilien oder harten Oberflächen, insbesondere Oberflächen aus Glas, Holz, Zement, Porzellan, Keramik, Kunststoff oder auch Metall(en) ein, um sicherzustellen, dass diese frei von Hygieneschädlingen und/oder ihren Ausscheidungen sind. Vorzugsweise ausgeschlossen vom Schutzbereich der Erfindung sind in dieser Hinsicht chirurgische oder therapeutische, auf den menschlichen Körper oder die Körper von Tieren anzuwendende Behandlungsvorschriften und diagnostische Vorschriften, die am menschlichen Körper oder den Körpern von Tieren durchgeführt werden.

Der Begriff "Hygienesektor" deckt somit alle Gebiete, technischen Felder und industriellen Anwendungen ab, bei denen diese Hygienemaßnahmen, -vorschriften und -verfahrensweisen wichtig sind, zum Beispiel im Hinblick auf Hygiene in Küchen, Bäckereien, Flughäfen, Badezimmern, Schwimmbecken, Kaufhäusern, Hotels, Krankenhäusern, Ställen, Tierhaltungen usw.

Der Begriff "Hygieneschädling" ist daher so zu verstehen, dass damit ein oder mehrere Tierschädlinge gemeint sind, deren Gegenwart im Hygienesektor problematisch ist, insbesondere aus Gesundheitsgründen. Es ist daher ein Hauptziel, das Vorhandensein von Hygieneschädlingen und/oder das Ausgesetztsein ihnen gegenüber im Hygienesektor zu vermeiden oder auf ein Mindestmaß zu begrenzen. Dies lässt sich insbesondere durch die Anwendung eines Pestizids erreichen, das sich sowohl zum Verhindern eines Befalls als auch zum Bewältigen eines bereits vorhandenen Befalls einsetzen lässt. Man kann auch Zubereitungen verwenden, die eine Exposition gegenüber Schädlingen verhindern oder reduzieren. Hygieneschädlinge schließen zum Beispiel die unten erwähnten Organismen ein.

Der Begriff "Hygieneschutz" deckt somit alle Handlungen ab, mit denen diese Hygienemaßnahmen, -vorschriften und -verfahrensweisen aufrechterhalten und/oder verbessert werden.

Die Verbindungen der Formel (I) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z. B. Acarus spp., z. B. Acarus siro, Aceria kuko, Aceria sheldoni, Aculops spp., Aculus spp., z. B. Aculus fockeui, Aculus schlechtendali, Amblyomma spp., Amphitetranychus viennensis, Argas spp., Boophilus spp., Brevipalpus spp., z. B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z. B. Eotetranychus hicoriae, Epitrimerus pyri, Eutetranychus spp., z. B. Eutetranychus banksi, Eriophyes spp., z. B. Eriophyes pyri, Glycyphagus domesticus, Halotydeus destructor, Hemitarsonemus spp., z. B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z. B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z. B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z. B. Tarsonemus confusus, Tarsonemus pallidus, Tetranychus spp., z. B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z. B. Geophilus spp., Scutigera spp.;
aus der Ordnung oder der Klasse der Collembola z. B. Onychiurus armatus; Sminthurus viridis;
aus der Klasse der Diplopoda z. B. Blaniulus guttulatus;
aus der Klasse der Insecta, z. B. aus der Ordnung der Blattodea z. B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z. B. Periplaneta americana, Periplaneta australasiae, Pycnoscelus surinamensis, Supella longipalpa;
aus der Ordnung der Coleoptera z. B. Acalymma vittatum, Acanthoscelides obtectus, Adoretus spp., Aethina tumida, Agelastica alni, Agrilus spp., z. B. Agrilus planipennis, Agrilus coxalis, Agrilus bilineatus, Agrilus anxius, Agriotes spp., z. B. Agriotes linneatus, Agriotes mancus, Agriotes obscurus Alphitobius diaperinus, Amphimallon solstitialis, Anobium punctatum, Anomala dubia, Anoplophora spp., z. B. Anoplophora glabripennis, Anthonomus spp., z. B. Anthonomus grandis, Anthrenus spp., Apion spp., Apogonia spp., Athous haemorrhoidales, Atomaria spp., z. B. Atomaria linearis, Attagenus spp., Baris caerulescens, Bruchidius obtectus, Bruchus spp., z. B. Bruchus pisorum, Bruchus rufimanus, Cassida spp., Cerotoma trifurcata, Ceutorrhynchus spp., z. B. Ceutorrhynchus assimilis, Ceutorrhynchus quadridens, Ceutorrhynchus rapae, Chaetocnema spp., z. B. Chaetocnema confinis, Chaetocnema denticulata, Chaetocnema ectypa, Cleonus mendicus, Conoderus spp., Cosmopolites spp., z. B. Cosmopolites sordidus, Costelytra zealandica, Ctenicera spp., Curculio spp., z. B. Curculio caryae, Curculio caryatrypes, Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dendroctonus spp., z. B. Dendroctonus ponderosae, Dermestes spp., Diabrotica spp., z. B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z. B. Epilachna borealis, Epilachna varivestis, Epitrix spp., z. B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hoplia argentea, Hylamorpha elegans, Hylotrupes bajulus, Hypera postica, Hypomeces squamosus, Hypothenemus spp., z. B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z. B. Leucoptera coffeella, Limonius ectypus, Lissorhoptrus oryzophilus, Listronotus (=Hyperodes) spp., Lixus spp., Luperodes spp., Luperomorpha xanthodera, Lyctus spp., Megacyllene spp., z. B. Megacyllene robiniae, Megascelis spp., Melanotus spp., z. B. Melanotus longulus oregonensis, Meligethes aeneus, Melolontha spp., z. B. Melolontha melolontha, Migdolus spp., Monochamus spp., Naupactus xanthographus, Necrobia spp., Neogalerucella spp., Niptus hololeucus, O-ryctes rhinoceros, Oryzaephilus surinamensis, Oryzaphagus oryzae, Otiorhynchus spp., z. B. Otiorhynchus cribricollis, Otiorhynchus ligustici, Otiorhynchus ovatus, Otiorhynchus rugosostriarus, Otiorhynchus sulcatus, Oulema spp., z. B. Oulema melanopus, Oulema oryzae, Oxycetoniajucunda, Phaedon cochleariae, Phyllophaga spp., Phyllophaga helleri, Phyllotreta spp., z. B. Phyllotreta armoraciae, Phyllotreta pusilla, Phyllotreta ramosa, Phyllotreta striolata, Popillia japonica, Premnotrypes spp., Prostephanus truncatus, Psylliodes spp., z. B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Scolytus spp., z. B. Scolytus multistriatus, Sinoxylon perforans, Sitophilus spp., z. B. Sitophilus granarius, Sitophilus linearis, Sitophilus oryzae, Sitophilus zeamais, Sphenophorus spp., Stegobium paniceum, Sternechus spp., z. B. Sternechus paludatus, Symphyletes spp., Tanymecus spp., z. B. Tanymecus dilaticollis, Tanymecus indicus, Tanymecus palliatus, Tenebrio molitor, Tenebrioides mauretanicus, Tribolium spp., z. B. Tribolium audax, Tribolium castaneum, Tribolium confusum, Trogoderma spp., Tychius spp., Xylotrechus spp., Zabrus spp., z. B. Zabrus tenebrioides;
aus der Ordnung der Dermaptera z. B. Anisolabis maritime, Forficula auricularia, Labidura riparia;
aus der Ordnung der Diptera z. B. Aedes spp., z. B. Aedes aegypti, Aedes albopictus, Aedes sticticus, Aedes vexans, Agromyza spp., z. B. Agromyza frontella, Agromyza parvicornis, Anastrepha spp., Anopheles spp., z. B. Anopheles quadrimaculatus, Anopheles gambiae, Asphondylia spp., Bactrocera spp., z. B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z. B. Contarinia johnsoni, Contarinia nasturtii, Contarinia pyrivora, Contarinia schulzi, Contarinia sorghicola, Contarinia tritici, Cordylobia anthropophaga, Cricotopus sylvestris, Culex spp., z. B. Culex pipiens, Culex quinquefasciatus, Culicoides spp., Culiseta spp., Cuterebra spp., Dacus oleae, Dasineura spp., z. B. Dasineura brassicae, Delia spp., z. B. Delia antiqua, Delia coarctata, Delia florilega, Delia platura, Delia radicum, Dermatobia hominis, Drosophila spp., z. B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z. B. Liriomyza brassicae, Liriomyza huidobrensis, Liriomyza sativae, Lucilia spp., z. B. Lucilia cuprina, Lutzomyia spp., Mansonia spp., Musca spp., z. B. Musca domestica, Musca domestica vicina, Oestrus spp., Oscinella frit, Paratanytarsus spp., Paralauterborniella subcincta, Pegomya oder Pegomyia spp., z. B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z. B. Rhagoletis cingulata, Rhagoletis completa, Rhagoletis fausta, Rhagoletis indifferens, Rhagoletis mendax, Rhagoletis pomonella, Sarcophaga spp., Simulium spp., z. B. Simulium meridionale, Stomoxys spp., Tabanus spp., Tetanops spp., Tipula spp., z. B. Tipula paludosa, Tipula simplex, Toxotrypana curvicauda;
aus der Ordnung der Hemiptera z. B. Acizzia acaciaebaileyanae, Acizzia dodonaeae, Acizzia uncatoides, Acrida turrita, Acyrthosipon spp., z. B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z. B. Amrasca bigutulla, Amrasca devastans, Anuraphis cardui, Aonidiella spp., z. B. Aonidiella aurantii, Aonidiella citrina, Aonidiella inornata, Aphanostigma piri, Aphis spp., z. B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z. B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z. B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita o-nukii, Chondracris rosea, Chromaphis juglandicola, Chrysomphalus aonidum, Chrysomphalus ficus, Cicadulina mbila, Coccomytilus halli, Coccus spp., z. B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., z. B. Dysaphis apiifolia, Dysaphis plantaginea, Dysaphis tulipae, Dysmicoccus spp., Empoasca spp., z. B. Empoasca abrupta, Empoasca fabae, Empoasca maligna, Empoasca solana, Empoasca stevensi, Eriosoma spp., z. B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z. B. Icerya purchasi, Idiocerus spp., Idioscopus spp., Laodelphax striatellus, Lecanium spp., z. B. Lecanium corni (=Parthenolecanium corni), Lepidosaphes spp., z. B. Lepidosaphes ulmi, Lipaphis erysimi, Lopholeucaspis japonica, Lycorma delicatula, Macrosiphum spp., z. B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z. B. Myzus ascalonicus, Myzus cerasi, Myzus ligustri, Myzus ornatus, Myzus persicae, Myzus nicotianae, Nasonovia ribisnigri, Neomaskellia spp., Nephotettix spp., z. B. Nephotettix cincticeps, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z. B. Paratrioza cockerelli, Parlatoria spp., Pemphigus spp., z. B. Pemphigus bursarius, Pemphigus populivenae, Peregrinus maidis, Perkinsiella spp., Phenacoccus spp., z. B. Phenacoccus madeirensis, Phloeomyzus passerinii, Phorodon humuli, Phylloxera spp., z. B. Phylloxera devastatrix, Phylloxera notabilis, Pinnaspis aspidistrae, Planococcus spp., z. B. Planococcus citri, Prosopidopsylla flava, Protopulvinaria pyriformis, Pseudaulacaspis pentagona, Pseudococcus spp., z. B. Pseudococcus calceolariae, Pseudococcus comstocki, Pseudococcus longispinus, Pseudococcus maritimus, Pseudococcus viburni, Psyllopsis spp., Psylla spp., z. B. Psylla buxi, Psylla mali, Psylla pyri, Pteromalus spp., Pulvinaria spp., Pyrilla spp., Quadraspidiotus spp., z. B. Quadraspidiotus juglansregiae, Quadraspidiotus ostreaeformis, Quadraspidiotus perniciosus, Quesada gigas, Rastrococcus spp., Rhopalosiphum spp., z. B. Rhopalosiphum maidis, Rhopalosiphum oxyacanthae, Rhopalosiphum padi, Rhopalosiphum rufiabdominale, Saissetia spp., z. B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., z. B. Toxoptera aurantii, Toxoptera citricidus, Trialeurodes vaporariorum, Trioza spp., z. B. Trioza diospyri, Typhlocyba spp., Unaspis spp., Viteus vitifolii, Zygina spp.;
aus der Unterordnung der Heteroptera z. B. Aelia spp., Anasa tristis, Antestiopsis spp., Boisea spp., Blissus spp., Calocoris spp., Campylomma livida, Cavelerius spp., Cimex spp., z. B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z. B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z. B. Lygocoris pabulinus, Lygus spp., z. B. Lygus elisus, Lygus hesperus, Lygus lineolaris, Macropes excavatus, Megacopta cribraria, Miridae, Monalonion atratum, Nezara spp., z. B. Nezara viridula, Nysius spp., Oebalus spp., Pentomidae, Piesma quadrata, Piezodorus spp., z. B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z. B. Acromyrmex spp., Athalia spp., z. B. Athalia rosae, Atta spp., Camponotus spp., Dolichovespula spp., Diprion spp., z. B. Diprion similis, Hoplocampa spp., z. B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., z. B. Sirex noctilio, Solenopsis invicta, Tapinoma spp., Technomyrmex albipes, Urocerus spp., Vespa spp., z. B. Vespa crabro, Wasmannia auropunctata, Xeris spp.;
aus der Ordnung der Isopoda z. B. Armadillidium vulgare, Oniscus asellus, Porcellio scaber;
aus der Ordnung der Isoptera z. B. Coptotermes spp., z. B. Coptotermes formosanus, Cornitermes cumulans, Cryptotermes spp., Incisitermes spp., Kalotermes spp., Microtermes obesi, Nasutitermes spp., Odontotermes spp., Porotermes spp., Reticulitermes spp., z. B. Reticulitermes flavipes, Reticulitermes hesperus;
aus der Ordnung der Lepidoptera z. B. Achroia grisella, Acronicta major, Adoxophyes spp., z. B. Adoxophyes orana, Aedia leucomelas, Agrotis spp., z. B. Agrotis segetum, Agrotis ipsilon, Alabama spp., z. B. Alabama argillacea, Amyelois transitella, Anarsia spp., Anticarsia spp., z. B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z. B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z. B. Cydia nigricana, Cydia pomonella, Dalaca noctuides, Diaphania spp., Diparopsis spp., Diatraea saccharalis, Dioryctria spp., z. B. Dioryctria zimmermani, Earias spp., Ecdytolopha aurantium, Elasmopalpus lignosellus, Eldana saccharina, Ephestia spp., z. B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z. B. Euproctis chrysorrhoea, Euxoa spp., Feltia spp., Galleria mellonella, Gracillaria spp., Grapholitha spp., z. B. Grapholita molesta, Grapholita prunivora, Hedylepta spp., Helicoverpa spp., z. B. Helicoverpa armigera, Helicoverpa zea, Heliothis spp., z. B. Heliothis virescens, Hepialus spp., z. B. Hepialus humuli, Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z. B. Leucoptera coffeella, Lithocolletis spp., z. B. Lithocolletis blancardella, Lithophane antennata, Lobesia spp., z. B. Lobesia botrana, Loxagrotis albicosta, Lymantria spp., z. B. Lymantria dispar, Lyonetia spp., z. B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z. B. Ostrinia nubilalis, Panolis flammea, Parnara spp., Pectinophora spp., z. B. Pectinophora gossypiella, Perileucoptera spp., Phthorimaea spp., z. B. Phthorimaea operculella, Phyllocnistis citrella, Phyllonorycter spp., z. B. Phyllonorycter blancardella, Phyllonorycter crataegella, Pieris spp., z. B. Pieris rapae, Platynota stultana, Plodia interpunctella, Plusia spp., Plutella xylostella (=Plutella maculipennis), Podesia spp., z. B. Podesia syringae, Prays spp., Prodenia spp., Protoparce spp., Pseudaletia spp., z. B. Pseudaletia unipuncta, Pseudoplusia includens, Pyrausta nubilalis, Rachiplusia nu, Schoenobius spp., z. B. Schoenobius bipunctifer, Scirpophaga spp., z. B. Scirpophaga innotata, Scotia segetum, Sesamia spp., z. B. Sesamia inferens, Sparganothis spp., Spodoptera spp., z. B. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z. B. Trichoplusia ni, Tryporyza incertulas, Tuta absoluta, Virachola spp.;
aus der Ordnung der Orthoptera oder Saltatoria z. B. Acheta domesticus, Dichroplus spp., Gryllotalpa spp., z. B. Gryllotalpa gryllotalpa, Hieroglyphus spp., Locusta spp., z. B. Locusta migratoria, Melanoplus spp., z. B. Melanoplus devastator, Paratlanticus ussuriensis, Schistocerca gregaria;
aus der Ordnung der Phthiraptera z. B. Damalinia spp., Haematopinus spp., Linognathus spp., Pediculus spp., Phylloxera vastatrix, Phthirus pubis, Trichodectes spp.;
aus der Ordnung der Psocoptera z. B. Lepinotus spp., Liposcelis spp.;
aus der Ordnung der Siphonaptera z. B. Ceratophyllus spp., Ctenocephalides spp., z. B. Ctenocephalides canis, Ctenocephalides felis, Pulex irritans, Tunga penetrans, Xenopsylla cheopis;
aus der Ordnung der Thysanoptera z. B. Anaphothrips obscurus, Baliothrips biformis, Chaetanaphothrips leeuweni, Drepanothrips reuteri, Enneothrips flavens, Frankliniella spp., z. B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z. B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z. B. Scutigerella spp., z. B. Scutigerella immaculata;
Schädlinge aus dem Stamm der Mollusca, z. B. aus der Klasse der Bivalvia, z. B. Dreissena spp.;
sowie aus der Klasse der Gastropoda z. B. Arion spp., z. B. Arion ater rufus, Biomphalaria spp., Bulinus spp., Deroceras spp., z. B. Deroceras laeve, Galba spp., Lymnaea spp., Oncomelania spp., Pomacea spp., Succinea spp.;
Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen/Anwendungsformen

Die vorliegende Erfindung betrifft weiterhin Formulierungen, insbesondere Formulierungen zur Bekämpfung unerwünschter tierischer Schädlinge. Die Formulierung kann auf den tierischen Schädling und/oder in dessen Lebensraum angewendet werden.

Die erfindungsgemäße Formulierung kann dem Endanwender als anwendungsfertige "Anwendungsform" bereitgestellt werden, d.h. die Formulierungen können direkt mittels eines geeigneten Geräts wie einem Sprüh- oder Stäubegerät auf die Pflanzen oder Samen aufgebracht werden. Alternativ dazu können die Formulierungen dem Endanwender in Form von vor der Anwendung vorzugsweise mit Wasser zu verdünnenden Konzentraten bereitgestellt werden. Wenn nicht anders angegeben wird mit dem Ausdruck "Formulierung" somit ein solches Konzentrat bezeichnet, während der Ausdruck "Anwendungsform" eine für den Endanwender anwendungsfertige Lösung bezeichnet, d.h. gewöhnlich eine solche verdünnte Formulierung.

Die erfindungsgemäße Formulierung kann auf herkömmliche Weise hergestellt werden, zum Beispiel durch Mischen der erfindungsgemäßen Verbindung mit einem oder mehreren geeigneten Hilfsstoffen wie z.B. den hier offenbarten.

Die Formulierung umfasst mindestens eine erfindungsgemäße Verbindung und mindestens einen landwirtschaftlich brauchbaren Hilfsstoff, z.B. Träger und/oder Tensid(e).

Ein Träger ist eine feste oder flüssige, natürliche oder synthetische, organische oder anorganische Substanz, die im Allgemeinen inert ist. Der Träger verbessert im Allgemeinen das Ausbringen der Verbindungen, zum Beispiel auf Pflanzen, Pflanzenteile oder Samen. Beispiele für geeignete feste Träger schließen, wobei dies nicht einschränkend ist, Ammoniumsalze, insbesondere Ammoniumsulfate, Ammoniumphosphate und Ammoniumnitrate, gemahlenes natürliches Gestein, wie Kaoline, Tone, Talkum, Kreide, Quarz, Attapulgit, Montmorillonit und Diatomeenerde, Kieselgel, und gemahlenes synthetisches Gestein, wie feinteiliges Siliciumdioxid, Aluminiumoxid und Silicate, ein. Beispiele für typische geeignete feste Träger zur Herstellung von Granulaten schließen, wobei dies nicht einschränkend ist, gebrochene und fraktionierte natürliche Gesteine wie Calcit, Marmor, Bimsstein, Sepiolith und Dolomit, synthetische Granulate anorganischer und organischer Mehle und Granulate organischer Materialien wie Papier, Sägespäne, Kokosnussschalen, Maiskolben und Tabakstängel ein. Beispiele für geeignete flüssige Träger schließen, wobei dies nicht einschränkend ist, Wasser, organische Lösungsmittel und Kombinationen davon ein. Beispiele für geeignete Lösungsmittel schließen polare und unpolare organische chemische Flüssigkeiten, zum Beispiel aus den Klassen der aromatischen und nichtaromatischen Kohlenwasserstoffe (wie Cyclohexan, Paraffine, Alkylbenzole, Xylol, Toluol, Tetrahydronaphthalin, Alkylnaphthaline, chlorierte Aromaten oder chlorierte aliphatische Kohlenwasserstoffe wie Chlorbenzole, Chlorethylene oder Methylenchlorid), Alkohole und Polyole (die auch substituiert, verethert und/oder verestert sein können, wie Ethanol, Propanol, Butanol, Benzylalkohol, Cyclohexanol oder Glykol), Ketone (wie Aceton, Methylethylketon, Methylisobutylketon, Acetophenon oder Cyclohexanon), Ester (einschließlich Fette und Öle) und (Poly)ether, unsubstituierte und substituierte Amine, Amide (wie Dimethylformamid oder Fettsäureamide) und Ester davon, Lactame (wie N-Alkylpyrrolidone, insbesondere N-Methylpyrrolidon) und Lactone, Sulfone und Sulfoxide (wie Dimethylsulfoxid), Öle pflanzlichen oder tierischen Ursprungs, Nitrile (Alkylnitrile wie Acetonitril, Propionnitril, Butyronitril, oder aromatische Nitrile wie Benzonitril), Kohlensäureester (cyclische Kohlensäureester wie Ethylencarbonat, Propylencarbonat, Butylencarbonat, oder Dialkylcarbonsäureester wie Dimethylcarbonat, Diethylcarbonat, Dipropylcarbonat, Dibutylcarbonat, Dioctylcarbonat) ein. Bei dem Träger kann es sich auch um ein verflüssigtes gasförmiges Streckmittel handeln, d.h. eine Flüssigkeit, die bei normaler Temperatur und unter Normaldruck gasförmig ist, zum Beispiel Aerosoltreibmittel wie Halogenkohlenwasserstoffe, Butan, Propan, Stickstoff und Kohlendioxid.

Bevorzugte feste Träger sind aus Tonen, Talkum und Siliciumdioxid ausgewählt.

Bevorzugte flüssige Träger sind aus Wasser, Fettsäureamiden und Estern davon, aromatischen und nichtaromatischen Kohlenwasserstoffen, Lactamen, Lactonen, Kohlensäureestern, Ketonen, (Poly)ethern ausgewählt.

Die Menge an Träger liegt typischerweise im Bereich von 1 bis 99,99 Gew.-%, vorzugsweise 5-99,9 Gew.-%, besonders bevorzugt 10 bis 99,5 Gew.-% und am meisten bevorzugt 20 bis 99 Gew.-% der Formulierung.

Flüssige Träger sind typischerweise in einem Bereich von 20 bis 90 Gew.-%, zum Beispiel 30 bis 80 Gew.-% der Formulierung vorhanden.

Feste Träger sind typischerweise in einem Bereich von 0 bis 50 Gew.-%, vorzugsweise 5 bis 45 Gew.-%, zum Beispiel 10 bis 30 Gew.-% der Formulierung vorhanden.

Umfasst die Formulierung zwei oder mehr Träger, so beziehen sich die umrissenen Bereiche auf die Gesamtmenge an Träger.

Bei dem Tensid kann es sich um ein ionisches (kationisches oder anionisches), amphoteres oder nichtionisches Tensid wie ionische oder nichtionische Emulgatoren, Schaumbilder, Dispersionsmittel, Netzmittel, Penetrationsförderer und beliebige Mischungen davon handeln. Beispiele für geeignete Tenside schließen, wobei dies nicht einschränkend ist, Salze von Polyacrylsäure, ethoxylierte Poly(alpha-substituierte)acrylatderivate, Salze von Lignosulfonsäure (wie Natriumlignosulfonat), Salze von Phenolsulfonsäure oder Naphthalinsulfonsäure, Polykondensate von Ethylenoxid und/oder Propylenoxid mit oder ohne Alkoholen, Fettsäuren oder Fettaminen (zum Beispiel Polyoxyethylenfettsäureester wie Rizinusölethoxylat, Polyoxyethylenfettalkoholether, zum Beispiel Alkylarylpolyglykolether), substituierte Phenole (vorzugsweise Alkylphenole oder Arylphenole), Salze von Sulfobernsteinsäureestern, Taurinderivate (vorzugsweise Alkyltaurate), Phosphorsäureester von polyethoxylierten Alkoholen oder Phenolen, Fettsäureester von Polyolen (wie Fettsäureester von Glycerin, Sorbit oder Saccharose), Sulfate (wie Alkylsulfate und Alkylethersulfate), Sulfonate, (zum Beispiel Alkylsulfonate, Arylsulfonate und Alkylbenzolsulfonate), sulfonierte Polymere von Naphthalin/Formaldehyd, Phosphatester, Proteinhydrolysate, Lignosulfitablaugen und Methylcellulose ein. Wird im vorliegenden Absatz auf Salze verwiesen, so bezieht sich dies vorzugsweise auf die betreffenden Alkali-, Erdalkali- und Ammoniumsalze.

Bevorzugte Tenside sind aus ethoxylierten Poly(alpha-substituierten)acrylatderivaten, Polykondensaten von Ethylenoxid und/oder Propylenoxid mit Alkoholen, Polyoxyethylenfettsäureestern, Alkylbenzolsulfonaten, sulfonierten Polymeren von Naphthalin/Formaldehyd, Polyoxyethylenfettsäureestern wie Rizinusölethoxylat, Natriumlignosulfonat und Arylphenolethoxylat ausgewählt.

Die Menge an Tensid liegt typischerweise im Bereich von 5 bis 40 Gew.-%, zum Beispiel 10 bis 20 Gew.-%, der Formulierung.

Weitere Beispiele für geeignete Hilfsstoffe schließen wasserabweisende Substanzen, Trockenmittel, Bindemittel (Klebstoffe, Haftmittel, Fixiermittel wie Carboxymethylcellulose, natürliche und synthetische Polymere in Form von Pulvern, Granulaten oder Latizes, wie Gummi arabicum, Polyvinylalkohol und Polyvinylacetat, natürliche Phospholipide wie Cephaline und Lecithine und synthetische Phospholipide, Polyvinylpyrrolidon und Tylose), Verdickungsmittel und sekundäre Verdickungsmittel (wie Celluloseether, Acrylsäurederivate, Xanthan, modifizierte Tone, z.B. die unter dem Namen Bentone erhältlichen Produkte, und feinteiliges Siliciumdioxid), Stabilisatoren (z.B. Kältestabilisatoren, Konservierungsstoffe (z.B. Dichlorophon, Benzylalkoholhemiformal, 1,2-Benzisothiazolin-3-on, 2-Methyl-4-isothiazolin-3-on), Antioxidationsmittel, Lichtschutzmittel, insbesondere UV-Schutzmittel, und andere Mittel, die die chemische und/oder physikalische Stabilität verbessern), Farbstoffe oder Pigmente (wie anorganische Pigmente, z.B. Eisenoxid, Titanoxid und Berliner Blau; organische Farbstoffe, z.B. Alizarin-, Azo- und Metallphthalocyaninfarbstoffe), Antischaummittel (z.B. Siliconantischaummittel und Magnesiumstearat), Frostschutzmittel, Kleber, Gibberelline und Verarbeitungshilfsstoffe, Mineral- und Pflanzenöle, Duftstoffe, Wachse, Nährstoffe (einschließlich Spurennährstoffe wie Salze von Eisen, Mangan, Bor, Kupfer, Kobalt, Molybdän und Zink), Schutzkolloide, thixotropische Substanzen, Penetriermittel, Sequestriermittel und Komplexbildner ein.

Die Auswahl an Hilfsstoffen hängt von der vorgesehenen Anwendungsweise der erfindungsgemäßen Verbindung und/oder von den physikalischen Eigenschaften der Verbindung(en) ab. Weiterhin können Hilfsstoffe so gewählt werden, dass sie den Formulierungen bzw. den daraus hergestellten Anwendungsformen bestimmte Eigenschaften (technische, physikalische und/oder biologische Eigenschaften) verleihen. Durch die Auswahl an Hilfsstoffen kann es möglich sein, die Formulierungen an bestimmte Bedürfnisse anzupassen.

Die Formulierung umfasst eine insektizid/akarizid wirksame Menge der erfindungsgemäßen Verbindung(en). Der Begriff "wirksame Menge" bezeichnet eine Menge, die zur Bekämpfung von Schadinsekten/-milben auf kultivierten Pflanzen oder beim Materialschutz ausreicht und die die behandelten Pflanzen nicht wesentlich schädigt. Eine solche Menge kann in einem weiten Bereich variieren und hängt von verschiedenen Faktoren wie der zu bekämpfenden Insekten/-milbenart, der behandelten kultivierten Pflanze bzw. dem behandelten Material, den Klimabedingungen und der jeweils eingesetzten erfindungsgemäßen Verbindung ab. Gewöhnlich enthält die erfindungsgemäße Formulierung 0,01 bis 99 Gew.-%, vorzugsweise 0,05 bis 98 Gew.-%, besonders bevorzugt 0,1 bis 95 Gew.-%, noch mehr bevorzugt 0,5 bis 90 Gew.-%, am meisten bevorzugt 1 bis 80 Gew.-% der erfindungsgemäßen Verbindung. Es ist möglich, dass eine Formulierung zwei oder mehr erfindungsgemäße Verbindungen umfasst. In einem solchen Fall beziehen sich die umrissenen Bereiche auf die Gesamtmenge der Verbindungen der vorliegenden Erfindung.

Die erfindungsgemäße Formulierung kann in einem beliebigen herkömmlichen Formulierungstyp vorliegen, wie Lösungen (z.B. wässrige Lösungen), Emulsionen, Suspensionen auf Wasser- und Ölbasis, Pulvern (z.B. Spritzpulvern, löslichen Pulvern), Stäuben, Pasten, Granulaten (z.B. löslichen Granulaten, Streugranulaten), Suspoemulsionskonzentraten, mit der erfindungsgemäßen Verbindung imprägnierten natürlichen oder synthetischen Produkten, Düngemitteln und außerdem Mikroverkapselungen in polymeren Substanzen. Die erfindungsgemäße Verbindung kann in suspendierter, emulgierter oder gelöster Form vorliegen. Beispiele für bestimmte geeignete Formulierungstypen sind Lösungen, wasserlösliche Konzentrate (z.B. SL, LS), Dispersionskonzentrate (DC), Suspensionen und Suspensionskonzentrate (z.B. SC, OD, OF, FS), Emulsionskonzentrate (z.B. EC), Emulsionen (z.B. EW, EO, ES, ME, SE), Kapseln (z.B. CS, ZC), Pasten, Pastillen, Spritzpulver oder Stäube (z.B. WP, SP, WS, DP, DS), Pressteile (z.B. BR, TB, DT), Granulate (z.B. WG, SG, GR, FG, GG, MG), insektizide Artikel (z.B. LN) sowie Gelformulierungen zur Behandlung von Pflanzenfortpflanzungsmaterial wie Samen (z.B. GW, GF). Diese und andere Formulierungstypen sind von der Food and Agriculture Organization of the United Nations (FAO) definiert. Ein Überblick findet sich im "Catalogue of pesticide formulation types and international coding system", Technical Monograph Nr. 2, 6. Aufl. Mai 2008, Croplife International.

Vorzugsweise liegt die erfindungsgemäße Formulierung in Form einer der folgenden Typen vor: EC, SC, FS, SE, OD, WG, WP, CS, besonders bevorzugt EC, SC, OD, WG, CS.

Weitere Details zu Beispielen für Formulierungstypen und ihre Herstellung finden sich unten. Sind zwei oder mehr erfindungsgemäße Verbindungen vorhanden, so bezieht sich die umrissene Menge an erfindungsgemäßer Verbindung auf die Gesamtmenge der Verbindungen der vorliegenden Erfindung. Dies gilt umgekehrt auch für alle weiteren Komponenten der Formulierung, wenn zwei oder mehr Vertreter einer solchen Komponente, z.B. eines Netz- oder Bindemittels, vorliegen.

### i) Wasserlösliche Konzentrate (SL, LS)

10-60 Gew.-% mindestens einer erfindungsgemäßen Verbindung und 5-15 Gew.-% Tensid (z.B. Polykondensate von Ethylenoxid und/oder Propylenoxid mit Alkoholen) werden in einer solchen Menge Wasser und/oder wasserlöslichem Lösungsmittel (z.B. Alkohole wie Propylenglykol und Carbonaten wie Propylencarbonat) gelöst, so dass sich eine Gesamtmenge von 100 Gew.-% ergibt. Vor der Anwendung wird das Konzentrat mit Wasser verdünnt.

### ii) Dispersionskonzentrate (DC)

5-25 Gew.-% mindestens einer erfindungsgemäßen Verbindung und 1-10 Gew.-% Tensid und/oder Bindemittel (z.B. Polvinylpyrrolidon) werden in einer solchen Menge organischen Lösungsmittels (z.B. Cyclohexan) gelöst, das sich eine Gesamtmenge von 100 Gew.-% ergibt. Das Verdünnen mit Wasser liefert eine Dispersion.

### iii) Emulsionskonzentrate (EC)

15-70 Gew.-% mindestens einer erfindungsgemäßen Verbindung und 5-10 Gew.-% Tensid (z.B. eine Mischung von Calciumdodecylbenzolsulfonat und Riziniusölethoxylat) werden in einer solchen Menge wasserunlöslichem organischem Lösungsmittel (z.B. aromatischem Kohlenwasserstoff oder Fettsäureamid) und, falls erforderlich, zusätzlichem wasserlöslichem Lösungsmittel gelöst, so dass man auf eine Gesamtmenge von 100 Gew.-% kommt. Durch Verdünnen mit Wasser erhält man eine Emulsion.

### iv) Emulsionen (EW, EO, ES)

5-40 Gew.-% mindestens einer erfindungsgemäßen Verbindung und 1-10 Gew.-% Tensid (z.B. eine Mischung von Calciumdodecylbenzolsulfonat und Rizinusölethoxylat, oder Polykondensate von Ethylenoxid und/oder Propylenoxid mit oder ohne Alkoholen) werden in 20-40 Gew.-% wasserunlöslichem organischem Lösungsmittel (z.B. aromatischer Kohlenwasserstoff) gelöst. Die Mischung wird mittels einer Emulgiermaschine zu einer solchen Menge an Wasser gegeben, dass man eine Gesamtmenge von 100 Gew.-% erhält. Bei der erhaltenen Formulierung handelt es sich um eine homogene Emulsion. Vor der Anwendung kann die Emulsion weiter mit Wasser verdünnt werden.

### v) Suspensionen und Suspensionskonzentrate

### v-1) Auf Wasserbasis (SC, FS)

In einem geeigneten Mahlgerät, z. B. einer Kugelmühle, werden 20-60 Gew.-% mindestens einer erfindungsgemäßen Verbindung unter Zugabe von 2-10 Gew.-% Tensid (z.B. Natriumlignosulfonat und Polyoxyethylenfettalkoholether), 0,1-2 Gew.-% Verdickungsmittel (z.B. Xanthan) und Wasser zu einer feinen Wirkstoffsuspension zerkleinert. Das Wasser wird in einer solchen Menge zugegeben, dass man eine Gesamtmenge von 100 Gew.-% erhält. Durch Verdünnen mit Wasser erhält man eine stabile Suspension des Wirkstoffs. Für Formulierungen vom FS-Typ werden bis zu 40 Gew.-% Bindemittel (z.B. Polyvinylalkohol) zugesetzt.

### v-2) Auf Ölbasis (OD, OF)

In einem geeigneten Mahlgerät, z.B. einer Kugelmühle, werden 20-60 Gew.-% mindestens einer erfindungsgemäßen Verbindung unter Zugabe von 2-10 Gew.-% Tensid (z.B. Natriumlignosulfonat und Polyoxyethylenfettalkoholether), 0,1-2 Gew.-% Verdickungsmittel (z.B. modifizierter Ton, insbesondere Bentone, oder Siliciumdioxid) und einem organischen Träger zu einer feinen Wirkstoff-Öl-Suspension zerkleinert. Der organische Träger wird in einer solchen Menge zugefügt, dass man eine Gesamtmenge von 100 Gew.-% erhält. Durch Verdünnen mit Wasser erhält man eine stabile Dispersion des Wirkstoffs.

### vi) Wasserdispergierbare Granulate und wasserlösliche Granulate (WG, SG)

1-90 Gew.-%, vorzugsweise 20-80 Gew.-%, am meisten bevorzugt 50-80 Gew.-% mindestens einer erfindungsgemäßen Verbindung werden unter Zugabe eines Tensids (z.B. Natriumlignosulfonat und Natriumalkylnaphthylsulfonate) und gegebenenfalls Trägermaterial fein gemahlen und mittels typischer technischer Anwendungen wie z.B. Extrusion, Sprühtrocknung, Wirbelschichtgranulation in wasserdispergierbare oder wasserlösliche Granulate überführt. Tensid und Trägermaterial werden in einer solchen Menge eingesetzt, dass man eine Gesamtmenge von 100 Gew.-% erhält. Durch Verdünnen mit Wasser erhält man eine stabile Dispersion bzw. Lösung des Wirkstoffs.

### vii) Wasserdispergierbare Pulver und wasserlösliche Pulver (WP, SP, WS)

50-80 Gew.-% mindestens einer erfindungsgemäßen Verbindung werden in einer Rotor-Stator-Mühle unter Zugabe von 1-20 Gew.-% Tensid (z.B. Natriumlignosulfonat, Natriumalkylnaphthylsulfonate) und einer solchen Menge an festem Träger, z.B. Kieselgel, dass man auf eine Gesamtmenge von 100 Gew.-% kommt, gemahlen. Durch Verdünnen mit Wasser erhält man eine stabile Dispersion bzw. Lösung des Wirkstoffs.

### viii) Gel (GW, GF)

In einer Kugelmühle werden 5-25 Gew.-% mindestens einer erfindungsgemäßen Verbindung unter Zugabe von 3-10 Gew.-% Tensid (z.B. Natriumlignosulfonat), 1-5 Gew.-% Bindemittel (z.B. Carboxymethylcellulose) und einer solchen Menge an Wasser, dass man auf eine Gesamtmenge von 100 Gew.-% kommt, zerkleinert. Hierdurch erhält man eine feine Suspension des Wirkstoffs. Durch Verdünnen mit Wasser erhält man eine stabile Suspension des Wirkstoffs.

### ix) Mikroemulsion (ME)

5-20 Gew.-% mindestens einer erfindungsgemäßen Verbindung werden zu 5-30 Gew.-% organischer Lösungsmittelmischung (z.B. Fettsäuredimethylamid und Cyclohexanon), 10-25 Gew.-% Tensidmischung (z.B. Polyoxyethylenfettalkoholether und Arylphenolethoxylat) und einer solchen Menge an Wasser, dass man auf eine Gesamtmenge von 100 Gew.-% kommt, gegeben. Diese Mischung wird 1 h gerührt, wodurch sich spontan eine thermodynamisch stabile Mikroemulsion bildet.

### x) Mikrokapseln (CS)

Eine Ölphase mit 5-50 Gew.-% mindestens einer erfindungsgemäßen Verbindung, 0-40 Gew.-% wasserunlöslichen organischen Lösungsmittels (z.B. aromatischem Kohlenwasserstoff), 2-15 Gew.-% acrylischen Monomeren (z.B. Methylmethacrylat, Methacrylsäure und einem Di- oder Triacrylat) werden in einer wässrigen Lösung eines Schutzkolloids (z.B. Polyvinylalkohol) dispergiert. Eine mit einem Radikalstarter eingeleitete radikalische Polymerisation führt zur Bildung von Poly(methy)acrylatmikrokapseln. Alternativ dazu wird eine 5-50 Gew.-% mindestens einer erfindungsgemäßen Verbindung, 0-40 Gew.-% wasserunlösliches organisches Lösungsmittel (z.B. aromatischen Kohlenwasserstoff) und ein Isocyanatmonomer (z.B. Diphenylmethen-4,4'-diisocyanat) umfassende Ölphase in einer wässrigen Lösung eines Schutzkolloids (z.B. Polyvinylakohol) dispergiert, dies führt zur Bildung von Polyharnstoffmikrokapseln. Gegebenenfalls kann man auch die Zugabe eines Polyamins (z.B. Hexamethylendiamin) anwenden, um die Bildung von Polyharnstoffmikrokapseln herbeizuführen. Die Monomere machen 1-10 Gew.-% der gesamten CS-Formulierung aus.

### xi) Stäubepulver (DP, DS)

1-10 Gew.-% mindestens einer erfindungsgemäßen Verbindung werden fein gemahlen und innig mit einer solchen Menge an festem Träger, z.B. feinteiligem Kaolin, gemischt, dass man auf eine Gesamtmenge von 100 Gew.-% kommt.

### xii) Granulate (GR, FG)

0,5-30 Gew.-% mindestens einer erfindungsgemäßen Verbindung werden fein gemahlen und mit einer solchen Menge an festem Träger (z.B. Silicat) assoziiert, dass man auf eine Gesamtmenge von 100 Gew.-% kommt.

### xiii) Ultra-Low-Volumen-Flüssigkeiten (UL)

1-50 Gew.-% mindestens einer erfindungsgemäßen Verbindung werden in einer solchen Menge an organischem Lösungsmittel, z.B. aromatischem Kohlenwasserstoff, gelöst, dass man auf eine Gesamtmenge von 100 Gew.-% kommt.

Die Formulierungstypen i) bis xiii) können weitere Hilfsstoffe wie 0,1-1 Gew.-% Konservierungsstoffe, 0,1-1 Gew.-% Antischaummittel, 0,1-1 Gew.-% Farbstoffe und/oder Pigmente und 5-10 Gew.-% Frostschutzmittel umfassen.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbiziden, Düngemitteln, Vogelrepellentien, Phytotonics, Sterilantien, Safenern, Semiochemicals und/oder Pflanzenwachstumsregulatoren verwendet werden, um so z. B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteertrag steigern, die Reife beeinflussen, die Qualität und/oder der Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann, sind auch diese Formen mit umfasst, auch wenn sie nicht in jedem Fall explizit genannt wurden. Alle genannten Mischungspartner können außerdem, wenn sie auf Grund ihrer funktionellen Gruppen dazu imstande sind, gegebenenfalls mit geeigneten Basen oder Säuren Salze bilden.

### Insektizide/Akarizide/Nematizide

Die hier mit ihrem "Common Name" genannten Wirkstoffe sind bekannt und beispielsweise im Pestizidhandbuch ("The Pesticide Manual" 16th Ed., British Crop Protection Council 2012) beschrieben oder im Internet recherchierbar (z. B. http://www.alanwood.net/pesticides). Die Klassifizierung basiert auf dem zum Zeitpunkt der Einreichung dieser Patentanmeldung gültigen IRAC Mode of Action Classification Scheme.
(1) Acetylcholinesterase(AChE)-Inhibitoren, vorzugsweise Carbamate ausgewählt aus Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanat, Furathiocarb, Isoprocarb, Methiocarb, Methomyl, Metolcarb, O-xamyl, Pirimicarb, Propoxur, Thiodicarb, Thiofanox, Triazamat, Trimethacarb, XMC und Xylylcarb, oder Organophosphate ausgewählt aus Acephat, Azamethiphos, Azinphos-ethyl, Azinphos-methyl, Cadusafos, Chlorethoxyfos, Chlorfenvinphos, Chlormephos, Chlorpyrifos-methyl, Coumaphos, Cyanophos, Demeton-S-methyl, Diazinon, Dichlorvos/DDVP, Dicrotophos, Dimethoat, Dimethylvinphos, Disulfoton, EPN, Ethion, Ethoprophos, Famphur, Fenamiphos, Fenitrothion, Fenthion, Fosthiazat, Heptenophos, Imicyafos, Isofenphos, Isopropyl-O-(methoxyaminothio-phosphoryl)salicylat, Isoxathion, Malathion, Mecarbam, Methamidophos, Methidathion, Mevinphos, Monocrotophos, Naled, Omethoat, Oxydemeton-methyl, Parathion-methyl, Phenthoat, Phorat, Phosalon, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Blocker, vorzugsweise Cyclodien-organochlorine ausgewählt aus Chlordan und Endosulfan, oder Phenylpyrazole (Fiprole) ausgewählt aus Ethiprol und Fipronil.
(3) Natrium-Kanal-Modulatoren, vorzugsweise Pyrethroide ausgewählt aus Acrinathrin, Allethrin, d-cistrans-Allethrin, d-trans-Allethrin, Bifenthrin, Bioallethrin, Bioallethrin-S-cyclopentenyl-Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomer], Deltamethrin, Empenthrin [(EZ)-(1R)-Isomer], Esfenvalerat, Etofenprox, Fenpropathrin, Fenvalerat, Flucythrinat, Flumethrin, tau-Fluvalinat, Halfenprox, Imiprothrin, Kadethrin, Momfluorothrin, Permethrin, Phenothrin [(1R)-trans-Isomer], Prallethrin, Pyrethrine (Pyrethrum), Resmethrin, Silafluofen, Tefluthrin, Tetramethrin, Tetramethrin [(1R)-Isomer], Tralomethrin und Transfluthrin, oder DDT oder Methoxychlor.
(4) Kompetitive Modulatoren des nicotinischen Acetylcholin-Rezeptors (nAChR), vorzugsweise Neonicotinoide ausgewählt aus Acetamiprid, Clothianidin, Dinotefuran, Imidacloprid, Nitenpyram, Thiacloprid und Thiamethoxam, oder Nicotin, oder Sulfoximine ausgewählt aus Sulfoxaflor, oder Butenolide ausgewählt aus Flupyradifuron, oder Mesoionics ausgewählt aus Triflumezopyrim.
(5) Allosterische Modulatoren des nicotinischen Acetylcholin-Rezeptors (nAChR), vorzugsweise Spinosyne ausgewählt aus Spinetoram und Spinosad.
(6) Allosterische Modulatoren des Glutamat-abhängigen Chloridkanals (GluCl), vorzugsweise Avermectine/Milbemycine ausgewählt aus Abamectin, Emamectin-benzoat, Lepimectin und Milbemectin.
(7) Juvenilhormon-Mimetika, vorzugsweise Juvenilhormon-Analoge ausgewählt aus Hydropren, Kinopren und Methopren, oder Fenoxycarb oder Pyriproxyfen.
(8) Verschiedene nicht spezifische (multi-site) Inhibitoren, vorzugsweise Alkylhalogenide ausgewählt aus Methylbromid und andere Alkylhalogenide, oder Chloropicrin oder Sulfurylfluorid oder Borax oder Brechweinstein oder Methylisocyanaterzeuger ausgewählt aus Diazomet und Metam.
(9) TRPV-Kanal-Modulatoren chordotonaler Organe, vorzugsweise Pyridinazomethane, ausgewählt aus Pymetrozin und Pyrifluquinazon oder Pyropene ausgewählt aus Afidopyropen.
(10) CHS1 betreffende Milbenwachstumsinhibitoren ausgewählt aus Clofentezin, Hexythiazox, Diflovidazin und Etoxazol.
(11) Mikrobielle Disruptoren der Insektendarmmembran ausgewählt aus *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis* und *B.t.*-Pflanzenproteine ausgewählt aus Cry1Ab, Cry1Ac, Cry1Fa, Cry1A.105, Cry2Ab, VIP3A, mCry3A, Cry3Ab, Cry3Bb und Cry34Ab1/35Ab1.
(12) Inhibitoren der mitochondrialen ATP-Synthase, vorzugsweise ATP-Disruptoren ausgewählt aus Diafenthiuron, oder Organozinnverbindungen ausgewählt aus Azocyclotin, Cyhexatin und Fenbutatin-oxid, oder Propargit oder Tetradifon.
(13) Entkoppler der oxidativen Phoshorylierung durch Störung des Protonengradienten ausgewählt aus Chlorfenapyr, DNOC und Sulfluramid.
(14) Blocker des nicotinischen Acetylcholinrezeptorkanals ausgewählt aus Bensultap, Cartap-hydrochlorid, Thiocyclam und Thiosultap-Natrium.
(15) CHS1 betreffende Inhibitoren der Chitinbiosynthese, vorzugsweise Benzoylharnstoffe, ausgewählt aus Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1 ausgewählt aus Buprofezin.
(17) Häutungsdisruptor (insbesondere bei Dipteren, d. h. Zweiflüglern) ausgewählt aus Cyromazin.
(18) Ecdyson-Rezeptor-Agonisten, vorzugsweise Diacylhydrazine, ausgewählt aus Chromafenozid, Halofenozid, Methoxyfenozid und Tebufenozid.
(19) Oktopamin-Rezeptor-Agonisten ausgewählt aus Amitraz.
(20) Mitochondriale Komplex-III-Elektronentransportinhibitoren ausgewählt aus Hydramethylnon, Acequinocyl, Fluacrypyrim und Bifenazat.
(21) Mitochondriale Komplex-I-Elektronentransportinhibitoren, vorzugsweise METI-Akarizide und Insektizide ausgewählt aus Fenazaquin, Fenpyroximat, Pyrimidifen, Pyridaben, Tebufenpyrad und Tolfenpyrad, oder Rotenon (Derris).
(22) Blocker des spannungsabhängigen Natriumkanals, vorzugsweise Oxadiazine ausgewählt aus Indoxacarb oder Semicarbazone ausgewählt aus Metaflumizon.
(23) Inhibitoren der Acetyl-CoA-Carboxylase, vorzugsweise Tetron- und Tetramsäurederivate ausgewählt aus Spirodiclofen, Spiromesifen, Spiropidion und Spirotetramat.
(24) Inhibitoren des mitochondrialen Komplex-IV-Elektronentransports, vorzugsweise Phosphide ausgewählt aus Aluminiumphosphid, Calciumphosphid, Phosphin und Zinkphosphid, oder Cyanide ausgewählt aus Calciumcyanid, Kaliumcyanid und Natriumcyanid.
(25) Inhibitoren des mitochondrialen Komplex-II-Elektronentransports, vorzugsweise beta-Ketonitrilderivate ausgewählt aus Cyenopyrafen und Cyflumetofen, oder Carboxanilide ausgewählt aus Pyflubumid.
(28) Ryanodinrezeptor-Modulatoren, vorzugsweise Diamide ausgewählt aus Chlorantraniliprol, Cyantraniliprol, Cyclaniliprol, Flubendiamid und Tetraniliprol.
(29) Modulatoren chordotonaler Organe (mit undefinierter Zielstruktur) ausgewählt aus Flonicamid.
(30) Allosterische Modulatoren des GABA-abhängigen Chloridkanals, vorzugsweise meta-Diamide ausgewählt aus Broflanilid oder Isoxazole ausgewählt aus Fluxametamid.
(31) Baculoviren, vorzugsweise Granuloviren (GVs) ausgewählt aus *Cydia pomonella* GV und *Thaumatotibia leucotreta* (GV) oder Nukleopolyhedroviren (NPVs) ausgewählt aus *Anticarsia gemmatalis* MNPV und *Helicoverpa armigera* NPV.
(32) Allosterische Modulatoren (Stelle II) des nikotinischen Acetylcholinrezeptors ausgewählt aus GSomega/kappa-HXTX-Hv1a-Peptid.
(33) Weitere Wirkstoffe ausgewählt aus Acynonapyr, Afoxolaner, Azadirachtin, Benclothiaz, Benzoximat, Benzpyrimoxan, Bromopropylat, Chinomethionat, Chloroprallethrin, Cryolit, Cyclobutrifluram, Cycloxaprid, Cyetpyrafen, Cyhalodiamid, Cyproflanilide (CAS 2375110-88-4), Dicloromezotiaz, Dicofol, Dimpropyridaz, epsilon-Metofluthrin, epsilon-Momfluthrin, Flometoquin, Fluazaindolizin, Flucypyriprol (CAS 1771741-86-6), Fluensulfon, Flufenerim, Flufenoxystrobin, Flufiprol, Fluhexafon, Fluopyram, Flupyrimin, Fluralaner, Fufenozid, Flupentiofenox, Guadipyr, Heptafluthrin, Imidaclothiz, Iprodion, Isocycloseram, kappa-Bifenthrin, kappa-Tefluthrin, Lotilaner, Meperfluthrin, Nicofluprol (CAS 1771741-86-6), Oxazosulfyl, Paichongding, Pyridalyl, Pyrifluquinazon, Pyriminostrobin, Sarolaner, Spidoxamat, Spirobudiclofen, Tetramethylfluthrin, Tetrachlorantraniliprol, Tigolaner, Tioxazafen, Thiofluoximate, Tyclopyrazoflor, Iodmethan; des Weiteren Präparate auf Basis von *Bacillus firmus* (I-1582, Votivo) und Azadirachtin (BioNeem), sowie folgende Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635) (CAS 885026-50-6), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494) (CAS 872999-66-1), 3-(4-Chlor-2,6-dimethylphenyl)-4-hydroxy-8-methoxy-1,8-diazaspiro[4.5]dec-3-en-2-on (bekannt aus WO 2010052161) (CAS 1225292-17-0), 3-(4-Chlor-2,6-dimethylphenyl)-8-methoxy-2-oxo-1,8-diazaspiro[4.5]dec-3-en-4-yl-ethylcarbonat (bekannt aus EP 2647626) (CAS1440516-42-6), PF1364 (bekannt aus JP2010/018586) (CAS 1204776-60-2), (3E)-3-[1-[(6-Chlor-3-pyridyl)methyl]-2-pyridyliden]-1,1,1-trifluorpropan-2-on (bekannt aus WO2013/144213) (CAS 1461743-15-6), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926) (CAS 1226889-14-0), 5-Brom-4-chlor-N-[4-chlor-2-methyl-6-(methylcarbamoyl)phenyl]-2-(3-chlor-2-pyridyl)pyrazol-3-carboxamid (bekannt aus CN103232431) (CAS 1449220-44-3), 4-[5-(3,5-Dichlorphenyl)-4,5-dihydro-5-(trifluormethyl)-3-isoxazolyl]-2-methyl-N-(cis-1-oxido-3-thietanyl)benzamid, 4-[5-(3,5-Dichlorphenyl)-4,5-dihydro-5-(trifluormethyl)-3-isoxazolyl]-2-methyl-N-(trans-1-oxido-3-thietanyl)benzamid und 4-[(5S)-5-(3,5-Dichlorphenyl)-4,5-dihydro-5-(trifluormethyl)-3-isoxazolyl]-2-methyl-N-(cis-1-oxido-3-thietanyl)benzamid (bekannt aus WO 2013/050317 A1) (CAS 1332628-83-7), N-[3-Chlor-1-(3-pyridinyl)-1H-pyrazol-4-yl]-N-ethyl-3-[(3,3,3-trifluorpropyl)sulfinyl]propanamid, (+)-N-[3-Chlor-1-(3-pyridinyl)-1H-pyrazol-4-yl]-N-ethyl-3-[(3,3,3-trifluorpropyl)sulfinyl]propanamid und (-)-N-[3-Chlor-1-(3-pyridinyl)-1H-pyrazol-4-yl]-N-ethyl-3-[(3,3,3-trifluorpropyl)sulfinyl]propanamid (bekannt aus WO 2013/162715 A2, WO 2013/162716 A2, US 2014/0213448 A1) (CAS 1477923-37-7), 5-[[(2E)-3-Chlor-2-propen-1-yl]amino]-1-[2,6-dichlor-4-(trifluormethyl)phenyl]-4-[(trifluormethyl)sulfinyl]-1H-pyrazol-3-carbonitrile (bekannt aus CN 101337937 A) (CAS 1105672-77-2), 3-Brom-N-[4-chlor-2-methyl-6-[(methylamino)thioxomethyl]phenyl]-1-(3-chlor-2-pyridinyl)-1H-pyrazol-5-carboxamid, (Liudaibenjiaxuanan, bekannt aus CN 103109816 A) (CAS 1232543-85-9); N-[4-Chlor-2-[[(1,1-dimethylethyl)amino]carbonyl]-6-methylphenyl]-1-(3-chlor-2-pyridinyl)-3-(fluormethoxy)-1H-pyrazol-5-carboxamid (bekannt aus WO 2012/034403 A1) (CAS 1268277-22-0), N-[2-(5-Amino-1,3,4-thiadiazol-2-yl)-4-chlor-6-methylphenyl]-3-brom-1-(3-chlor-2-pyridinyl)-1H-pyrazol-5-carboxamid (bekannt aus WO 2011/085575 A1) (CAS 1233882-22-8), 4-[3-[2,6-Dichlor-4-[(3,3-dichlor-2-propen-1-yl)oxy]phenoxy]propoxy]-2-methoxy-6-(trifluormethyl)pyrimidin (bekannt aus CN 101337940 A) (CAS 1108184-52-6); (2E)- und 2(Z)-2-[2-(4-Cyanophenyl)-1-[3-(trifluormethyl)phenyl]ethyliden]-N-[4-(difluormethoxy)phenyl]hydrazincarboxamid (bekannt aus CN 101715774 A) (CAS 1232543-85-9); Cyclopropancarbonsäure-3-(2,2-dichlorethenyl)-2,2-dimethyl-4-(1H-benzimidazol-2-yl)phenylester (bekannt aus CN 103524422 A) (CAS 1542271-46-4); (4aS)-7-Chlor-2,5-dihydro-2-[[(methoxycarbonyl)[4-[(trifluormethyl)thio]phenyl]amino]carbonyl]indeno[1,2-e][1,3,4]oxadiazin-4a(3H)-carbonsäuremethylester (bekannt aus CN 102391261 A) (CAS 1370358-69-2); 6-Desoxy-3-O-ethyl-2,4-di-O-methyl-1-[N-[4-[1-[4-(1,1,2,2,2-pentafluorethoxy)phenyl]-1H-1,2,4-triazol-3-yl]phenyl]carbamat]-α-L-mannopyranose (bekannt aus US 2014/0275503 A1) (CAS 1181213-14-8); 8-(2-Cyclopropylmethoxy-4-trifluormethylphenoxy)-3-(6-trifluormethylpyridazin-3-yl)-3-azabicyclo[3.2.1]octan (CAS 1253850-56-4), (8-anti)-8-(2-Cyclopropylmethoxy-4-trifluormethylphenoxy)-3-(6-trifluormethylpyridazin-3-yl)-3-azabicyclo[3.2.1]octan (CAS 933798-27-7), (8-syn)-8-(2-Cyclopropylmethoxy-4-trifluormethylphenoxy)-3-(6-trifluormethylpyridazin-3-yl)-3-azabicyclo[3.2.1]octan (bekannt aus WO 2007040280 A1, WO 2007040282 A1) (CAS 934001-66-8), N-[4-(Aminothioxomethyl)-2-methyl-6-[(methylamino)carbonyl] phenyl]-3-brom-1-(3-chlor-2-pyridinyl)-1*H*-pyrazol-5-carboxamid (bekannt aus CN 103265527 A) (CAS 1452877-50-7), 3-(4-Chlor-2,6-dimethylphenyl)-8-methoxy-1-methyl-1,8-diazaspiro[4.5]decan-2,4-dion (bekannt aus WO 2014/187846 A1) (CAS 1638765-58-8), 3-(4-Chlor-2,6-dimethylphenyl)-8-methoxy-1-methyl-2-oxo-1,8-diazaspiro[4.5]dec-3-en-4-yl-carbonsäureethylester (bekannt aus WO 2010/066780 A1, WO 2011151146 A1) (CAS 1229023-00-0), *N*-[1-(2,6-Difluorphenyl)-1*H*-pyrazol-3-yl]-2-(trifluormethyl)benzamid (bekannt aus WO 2014/053450 A1) (CAS 1594624-87-9), N-[2-(2,6-Difluorphenyl)-2H-1,2,3-triazol-4-yl]-2-(trifluormethyl)benzamid (bekannt aus WO 2014/053450 A1) (CAS 1594637-65-6), N-[1-(3,5-Difluor-2-pyridinyl)-1H-pyrazol-3-yl]-2-(trifluormethyl)benzamid (bekannt aus WO 2014/053450 A1) (CAS 1594626-19-3), (3*R*)-3-(2-Chlor-5-thiazolyl)-2,3-dihydro-8-methyl-5,7-dioxo-6-phenyl-5*H*-thiazolo[3,2-a]pyrimidinium inneres Salz (bekannt aus WO 2018/177970 A1) (CAS 2246757-58-2); 3-(2-Chlor-5-thiazolyl)-2,3-dihydro-8-methyl-5,7-dioxo-6-phenyl-5H-thiazolo[3,2-a]pyrimidinium inneres Salz (bekannt aus WO 2018/177970 A1) (CAS 2246757-56-0); *N*-[3-Chlor-1-(3-pyridinyl)-1*H*-pyrazol-4-yl]-2-(methylsulfonyl)-propanamid (bekannt aus WO 2019/236274 A1) (CAS 2396747-83-2), *N*-[2-bromo-4-[1,2,2,2-tetrafluoro-1-(trifluoromethyl)ethyl]-6-(trifluoromethyl)phenyl]-2-fluoro-3-[(4-fluorobenzoyl)amino]-benzamid (known from WO 2019059412 A1) (CAS 1207977-87-4).

### Fungizide

Die hier mit ihrem "Common Name" spezifizierten Wirkstoffe sind bekannt und beispielsweise im "Pesticide Manual" (16. Aufl. British Crop Protection Council) beschrieben oder im Internet recherchierbar (beispielsweise: www.alanwood.net/pesticides) beschrieben.

Alle genannten Mischungspartner der Klassen (1) bis (15) können, wenn sie auf Grund ihrer funktionellen Gruppen dazu imstande sind, gegebenenfalls mit geeigneten Basen oder Säuren Salze bilden. Alle genannten fungiziden Mischungspartner der Klassen (1) bis (15) können gegebenenfalls tautomere Formen einschließen.
1) Inhibitoren der Ergosterolbiosynthese, zum Beispiel (1.001) Cyproconazol, (1.002) Difenoconazol, (1.003) Epoxiconazol, (1.004) Fenhexamid, (1.005) Fenpropidin, (1.006) Fenpropimorph, (1.007) Fenpyrazamin, (1.008) Fluquinconazol, (1.009) Flutriafol, (1.010) Imazalil, (1.011) Imazalilsulfat, (1.012) Ipconazol, (1.013) Metconazol, (1.014) Myclobutanil, (1.015) Paclobutrazol, (1.016) Prochloraz, (1.017) Propiconazol, (1.018) Prothioconazol, (1.019) Pyrisoxazol, (1.020) Spiroxamin, (1.021) Tebuconazol, (1.022) Tetraconazol, (1.023) Triadimenol, (1.024) Tridemorph, (1.025) Triticonazol, (1.026) (1R,2S,5S)-5-(4-Chlorbenzyl)-2-(chlormethyl)-2-methyl-1-(1H-1,2,4-triazol-1-ylmethyl)cyclopentanol, (1.027) (1S,2R,5R)-5-(4-Chlorbenzyl)-2-(chlormethyl)-2-methyl-1-(1H-1,2,4-triazol-1-ylmethyl)cyclopentanol, (1.028) (2R)-2-(1-Chlorcyclopropyl)-4-[(1R)-2,2-dichlorcyclopropyl]-1-(1H-1,2,4-triazol-1-yl)butan-2-ol, (1.029) (2R)-2-(1-Chlorcyclopropyl)-4-[(1S)-2,2-dichlorcyclopropyl]-1-(1H-1,2,4-triazol-1-yl)butan-2-ol, (1.030) (2R)-2-[4-(4-Chlorphenoxy)-2-(trifluormethyl)phenyl]-1-(1H-1,2,4-triazol-1-yl)propan-2-ol, (1.031) (2S)-2-(1-Chlorcyclopropyl)-4-[(1R)-2,2-dichlorcyclopropyl]-1-(1H-1,2,4-triazol-1-yl)butan-2-ol, (1.032) (2S)-2-(1-Chlorcyclopropyl)-4-[(1S)-2,2-dichlorcyclopropyl]-1-(1H-1,2,4-triazol-1-yl)butan-2-ol, (1.033) (2S)-2-[4-(4-Chlorphenoxy)-2-(trifluormethyl)phenyl]-1-(1H-1,2,4-triazol-1-yl)propan-2-ol, (1.034) (R)-[3-(4-Chlor-2-fluorphenyl)-5-(2,4-difluorphenyl)-1,2-oxazol-4-yl](pyridin-3-yl)methanol, (1.035) (S)-[3-(4-Chlor-2-fluorphenyl)-5-(2,4-difluorphenyl)-1,2-oxazol-4-yl](pyridin-3-yl)methanol, (1.036) [3-(4-Chlor-2-fluorphenyl)-5-(2,4-difluorphenyl)-1,2-oxazol-4-yl](pyridin-3-yl)methanol, (1.037) 1-({(2R,4S)-2-[2-Chlor-4-(4-chlorphenoxy)phenyl]-4-methyl-1,3-dioxolan-2-yl}methyl)-1H-1,2,4-triazol, (1.038) 1-({(2S,4S)-2-[2-Chlor-4-(4-chlorphenoxy)phenyl]-4-methyl-1,3-dioxolan-2-yl}methyl)-1H-1,2,4-triazol, (1.039) 1-{[3-(2-Chlorphenyl)-2-(2,4-difluorphenyl)oxiran-2-yl]methyl}-1H-1,2,4-triazol-5-ylthiocyanat, (1.040) 1-{[rel(2R,3R)-3-(2-Chlorphenyl)-2-(2,4-difluorphenyl)oxiran-2-yl]methyl}-1H-1,2,4-triazol-5-ylthiocyanat, (1.041) 1-{[rel(2R,3S)-3-(2-Chlorphenyl)-2-(2,4-difluorphenyl)oxiran-2-yl]methyl}-1H-1,2,4-triazol-5-ylthiocyanat, (1.042) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.043) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.044) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (1.045) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.046) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.047) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.048) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.049) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.050) 2-[1-(2,4-Dichlorphenyl)-5-hydroxy-2,6,6-trimethylheptan-4-yl]-2,4-dihydro-3H-1,2,4-triazol-3-thion, (1.051) 2-[2-Chlor-4-(2,4-dichlorphenoxy)phenyl]-1-(1H-1,2,4-triazol-1-yl)propan-2-ol, (1.052) 2-[2-Chlor-4-(4-chlorphenoxy)phenyl]-1-(1H-1,2,4-triazol-1-yl)butan-2-ol, (1.053) 2-[4-(4-Chlorphenoxy)-2-(trifluormethyl)phenyl]-1-(1H-1,2,4-triazol-1-yl)butan-2-ol, (1.054) 2-[4-(4-Chlorphenoxy)-2-(trifluormethyl)phenyl]-1-(1H-1,2,4-triazol-1-yl)pentan-2-ol, (1.055) Mefentrifluconazol, (1.056) 2-{[3-(2-Chlorphenyl)-2-(2,4-difluorphenyl)oxiran-2-yl]methyl}-2,4-dihydro-3H-1,2,4-triazol-3-thion, (1.057) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.058) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.059) 5-(4-Chlorbenzyl)-2-(chlormethyl)-2-methyl-1-(1H-1,2,4-triazol-1-ylmethyl)cyclopentanol, (1.060) 5-(Allylsulfanyl)-1-{[3-(2-chlorphenyl)-2-(2,4-difluorphenyl)oxiran-2-yl]methyl}-1H-1,2,4-triazol, (1.061) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.062) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.063) N'-(2,5-Dimethyl-4-{[3-(1,1,2,2-tetrafluorethoxy)phenyl]sulfanyl}phenyl)-N-ethyl-N-methylimidoformamid, (1.064) N'-(2,5-Dimethyl-4-{[3-(2,2,2-trifluorethoxy)phenyl]sulfanyl}phenyl)-N-ethyl-N-methylimidoformamid, (1.065) N'-(2,5-Dimethyl-4-{[3-(2,2,3,3-tetrafluorpropoxy)phenyl]sulfanyl}phenyl)-N-ethyl-N-methylimidoformamid, (1.066) N'-(2,5-Dimethyl-4-{[3-(pentafluorethoxy)phenyl]sulfanyl}phenyl)-N-ethyl-N-methylimidoformamid, (1.067) N'-(2,5-Dimethyl-4-{3-[(1,1,2,2-tetrafluorethyl)sulfanyl]phenoxy}phenyl)-N-ethyl-N-methylimidoformamid, (1.068) N'-(2,5-Dimethyl-4-{3-[(2,2,2-trifluorethyl)sulfanyl]phenoxy}phenyl)-N-ethyl-N-methylimidoformamid, (1.069) N'-(2,5-Dimethyl-4-{3-[(2,2,3,3-tetrafluorpropyl)sulfanyl]phenoxy}phenyl)-N-ethyl-N-methylimidoformamid, (1.070) N'-(2,5-Dimethyl-4-{3-[(pentafluorethyl)sulfanyl]phenoxy}phenyl)-N-ethyl-N-methylimidoformamid, (1.071) N'-(2,5-Dimethyl-4-phenoxyphenyl)-N-ethyl-N-methylimidoformamid, (1.072) N'-(4-{[3-(Difluormethoxy)phenyl]sulfanyl}-2,5-dimethylphenyl)-N-ethyl-N-methylimidoformamid, (1.073) N'-(4-{3-[(Difluormethyl)sulfanyl]phenoxy}-2,5-dimethylphenyl)-N-ethyl-N-methylimidoformamid, (1.074) N'-[5-Brom-6-(2,3-dihydro-1H-inden-2-yloxy)-2-methylpyridin-3-yl]-N-ethyl-N-methylimidoformamid, (1.075) N'-{4-[(4,5-Dichlor-1,3-thiazol-2-yl)oxy]-2,5-dimethylphenyl}-N-ethyl-N-methylimidoformamid, (1.076) N'-{5-Brom-6-[(1R)-1-(3,5-difluorphenyl)ethoxy]-2-methylpyridin-3-yl}-N-ethyl-N-methylimidoformamid, (1.077) N'-15-Brom-6-[(1S)-1-(3,5-difluorphenyl)ethoxy]-2-methylpyridin-3-yl}-N-ethyl-N-methylimidoformamid, (1.078) N'-{5-Brom-6-[(cis-4-isopropylcyclohexyl)oxy]-2-methylpyridin-3-yl}-N-ethyl-N-methylimidoformamid, (1.079) N'-{5-Brom-6-[(trans-4-isopropylcyclohexyl)oxy]-2-methylpyridin-3-yl}-N-ethyl-N-methylimidoformamid, (1.080) N'-{5-Brom-6-[1-(3,5-difluorphenyl)ethoxy]-2-methylpyridin-3-yl}-N-ethyl-N-methylimidoformamid, (1.081) Ipfentrifluconazol, (1.082) 2-[4-(4-Chlorphenoxy)-2-(trifluormethyl)phenyl]-1-(1H-1,2,4-triazol-1-yl)propan-2-ol, (1.083) 2-[6-(4-Bromphenoxy)-2-(trifluormethyl)-3-pyridyl]-1-(1,2,4-triazol-1-yl)propan-2-ol, (1.084) 2-[6-(4-Chlorphenoxy)-2-(trifluormethyl)-3-pyridyl]-1-(1,2,4-triazol-1-yl)propan-2-ol, (1.085) 3-[2-(1-Chlorcyclopropyl)-3-(3-chlor-2-fluorphenyl)-2-hydroxy-propyl]imidazol-4-carbonitril und (1.086) 4-[[6-[rac-(2R)-2-(2,4-Difluorphenyl)-1,1-difluor-2-hydroxy-3-(5-thioxo-4H-1,2,4-triazol-1-yl)propyl]-3-pyridyl]oxy]benzonitril.
2) Inhibitoren der Atmungskette an Komplex I oder II, zum Beispiel (2.001) Benzovindiflupyr, (2.002) Bixafen, (2.003) Boscalid, (2.004) Carboxin, (2.005) Fluopyram, (2.006) Flutolanil, (2.007) Fluxapyroxad, (2.008) Furametpyr, (2.009) Isofetamid, (2.010) Isopyrazam (anti-epimeres Enantiomer 1R,4S,9S), (2.011) Isopyrazam (anti-epimeres Enantiomer 1S,4R,9R), (2.012) Isopyrazam (anti-epimeres Racemat 1RS,4SR,9SR), (2.013) Isopyrazam (Mischung von syn-epimerem Racemat 1RS,4SR,9RS und anti-epimerem Racemat 1RS,4SR,9SR), (2.014) Isopyrazam (syn-epimeres Enantiomer 1R,4S,9R), (2.015) Isopyrazam (syn-epimeres Enantiomer 1S,4R,9S), (2.016) Isopyrazam (syn-epimeres Racemat 1RS,4SR,9RS), (2.017) Penflufen, (2.018) Penthiopyrad, (2.019) Pydiflumetofen, (2.020) Pyraziflumid, (2.021) Sedaxan, (2.022) 1,3-Dimethyl-N-(1,1,3-trimethyl-2,3-dihydro-1H-inden-4-yl)-1H-pyrazol-4-carboxamid, (2.023) 1,3-Dimethyl-N-[(3R)-1,1,3-trimethyl-2,3-dihydro-1H-inden-4-yl]-1H-pyrazol-4-carboxamid, (2.024) 1,3-Dimethyl-N-[(3S)-1,1,3-trimethyl-2,3-dihydro-1H-inden-4-yl]-1H-pyrazol-4-carboxamid, (2.025) 1-Methyl-3-(trifluormethyl)-N-[2'-(trifluormethyl)biphenyl-2-yl]-1H-pyrazol-4-carboxamid, (2.026) 2-Fluor-6-(trifluormethyl)-N-(1,1,3-trimethyl-2,3-dihydro-1H-inden-4-yl)benzamid, (2.027) 3-(Difluormethyl)-1-methyl-N-(1,1,3-trimethyl-2,3-dihydro-1H-inden-4-yl)-1H-pyrazol-4-carboxamid, (2.028) Inpyrfluxam, (2.029) 3-(Difluormethyl)-1-methyl-N-[(3S)-1,1,3-trimethyl-2,3-dihydro-1H-inden-4-yl]-1H-pyrazol-4-carboxamid, (2.030) Fluindapyr, (2.031) 3-(Difluormethyl)-N-[(3R)-7-fluor-1,1,3-trimethyl-2,3-dihydro-1H-inden-4-yl]-1-methyl-1H-pyrazol-4-carboxamid, (2.032) 3-(Difluormethyl)-N-[(3S)-7-fluor-1,1,3-trimethyl-2,3-dihydro-1H-inden-4-yl]-1-methyl-1H-pyrazol-4-carboxamid, (2.033) 5,8-Difluor-N-[2-(2-fluor-4-1[4-(trifluormethyl)pyridin-2-yl]oxy}phenyl)ethyl]chinazolin-4-amin, (2.034) N-(2-Cyclopentyl-5-fluorbenzyl)-N-cyclopropyl-3-(difluormethyl)-5-fluor-1-methyl-1H-pyrazol-4-carboxamid, (2.035) N-(2-tert.-Butyl-5-methylbenzyl)-N-cyclopropyl-3-(difluormethyl)-5-fluor-1-methyl-1H-pyrazol-4-carboxamid, (2.036) N-(2-tert.-Butylbenzyl)-N-cyclopropyl-3-(difluormethyl)-5-fluor-1-methyl-1H-pyrazol-4-carboxamid, (2.037) N-(5-Chlor-2-ethylbenzyl)-N-cyclopropyl-3-(difluormethyl)-5-fluor-1-methyl-1H-pyrazol-4-carboxamid, (2.038) N-(5-Chlor-2-isopropylbenzyl)-N-cyclopropyl-3-(difluormethyl)-5-fluor-1-methyl-1H-pyrazol-4-carboxamid, (2.039) N-[(1R,4S)-9-(Dichlormethylen)-1,2,3,4-tetrahydro-1,4-methanonaphthalin-5-yl]-3-(difluormethyl)-1-methyl-1H-pyrazol-4-carboxamid, (2.040) N-[(1S,4R)-9-(Dichlormethylen)-1,2,3,4-tetrahydro-1,4-methanonaphthalin-5-yl]-3-(difluormethyl)-1-methyl-1H-pyrazol-4-carboxamid, (2.041) N-[1-(2,4-Dichlorphenyl)-1-methoxypropan-2-yl]-3-(difluormethyl)-1-methyl-1H-pyrazol-4-carboxamid, (2.042) N-[2-Chlor-6-(trifluormethyl)benzyl]-N-cyclopropyl-3-(difluormethyl)-5-fluor-1-methyl-1H-pyrazol-4-carboxamid, (2.043) N-[3-Chlor-2-fluor-6-(trifluormethyl)benzyl]-N-cyclopropyl-3-(difluormethyl)-5-fluor-1-methyl-1H-pyrazol-4-carboxamid, (2.044) N-[5-Chlor-2-(trifluormethyl)benzyl]-N-cyclopropyl-3-(difluormethyl)-5-fluor-1-methyl-1H-pyrazol-4-carboxamid, (2.045) N-Cyclopropyl-3-(difluormethyl)-5-fluor-1-methyl-N-[5-methyl-2-(trifluormethyl)benzyl]-1H-pyrazol-4-carboxamid, (2.046) N-Cyclopropyl-3-(difluormethyl)-5-fluor-N-(2-fluor-6-isopropylbenzyl)-1-methyl-1H-pyrazol-4-carboxamid, (2.047) N-Cyclopropyl-3-(difluormethyl)-5-fluor-N-(2-isopropyl-5-methylbenzyl)-1-methyl-1H-pyrazol-4-carboxamid, (2.048) N-Cyclopropyl-3-(difluormethyl)-5-fluor-N-(2-isopropylbenzyl)-1-methyl-1H-pyrazol-4-carbothioamid, (2.049) N-Cyclopropyl-3-(difluormethyl)-5-fluor-N-(2-isopropylbenzyl)-1-methyl-1H-pyrazol-4-carboxamid, (2.050) N-Cyclopropyl-3-(difluormethyl)-5-fluor-N-(5-fluor-2-isopropylbenzyl)-1-methyl-1H-pyrazol-4-carboxamid, (2.051) N-Cyclopropyl-3-(difluormethyl)-N-(2-ethyl-4,5-dimethylbenzyl)-5-fluor-1-methyl-1H-pyrazol-4-carboxamid, (2.052) N-Cyclopropyl-3-(difluormethyl)-N-(2-ethyl-5-fluorbenzyl)-5-fluor-1-methyl-1H-pyrazol-4-carboxamid, (2.053) N-Cyclopropyl-3-(difluormethyl)-N-(2-ethyl-5-methylbenzyl)-5-fluor-1-methyl-1H-pyrazol-4-carboxamid, (2.054) N-Cyclopropyl-N-(2-cyclopropyl-5-fluorbenzyl)-3-(difluormethyl)-5-fluor-1-methyl-1H-pyrazol-4-carboxamid, (2.055) N-Cyclopropyl-N-(2-cyclopropyl-5-methylbenzyl)-3-(difluormethyl)-5-fluor-1-methyl-1H-pyrazol-4-carboxamid, (2.056) N-Cyclopropyl-N-(2-cyclopropylbenzyl)-3-(difluormethyl)-5-fluor-1-methyl-1H-pyrazol-4-carboxamid, (2.057) Pyrapropoyn.
3) Inhibitoren der Atmungskette an Komplex III, zum Beispiel (3.001) Ametoctradin, (3.002) Amisulbrom, (3.003) Azoxystrobin, (3.004) Coumethoxystrobin, (3.005) Coumoxystrobin, (3.006) Cyazofamid, (3.007) Dimoxystrobin, (3.008) Enoxastrobin, (3.009) Famoxadon, (3.010) Fenamidon, (3.011) Flufenoxystrobin, (3.012) Fluoxastrobin, (3.013) Kresoxim-methyl, (3.014) Metominostrobin, (3.015) Orysastrobin, (3.016) Picoxystrobin, (3.017) Pyraclostrobin, (3.018) Pyrametostrobin, (3.019) Pyraoxystrobin, (3.020) Trifloxystrobin, (3.021) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.022) (2E,3Z)-5-{[1-(4-Chlorphenyl)-1H-pyrazol-3-yl]oxy}-2-(methoxyimino)-N,3-dimethylpent-3-enamid, (3.023) (2R)-2-{2-[(2,5-Dimethylphenoxy)methyl]phenyl}-2-methoxy-N-methylacetamid, (3.024) (2S)-2-{2-[(2,5-Dimethylphenoxy)methyl]phenyl}-2-methoxy-N-methylacetamid, (3.025) Fenpicoxamid, (3.026) Mandestrobin, (3.027) N-(3-Ethyl-3,5,5-trimethylcyclohexyl)-3-formamido-2-hydroxybenzamid, (3.028) (2E,3Z)-5-{[1-(4-Chlor-2-fluorphenyl)-1H-pyrazol-3-yl]oxy}-2-(methoxyimino)-N,3-dimethylpent-3-enamid, (3.029) {5-[3-(2,4-Dimethylphenyl)-1H-pyrazol-1-yl]-2-methylbenzyl}carbamidsäuremethylester, (3.030) Metyltetraprol, (3.031) Florylpicoxamid.
4) Inhibitoren von Mitose und Zellteilung, zum Beispiel (4.001) Carbendazim, (4.002) Diethofencarb, (4.003) Ethaboxam, (4.004) Fluopicolid, (4.005) Pencycuron, (4.006) Thiabendazol, (4.007) Thiophanatmethyl, (4.008) Zoxamid, (4.009) 3-Chlor-4-(2,6-difluorphenyl)-6-methyl-5-phenylpyridazin, (4.010) 3-Chlor-5-(4-chlorphenyl)-4-(2,6-difluorphenyl)-6-methylpyridazin, (4.011) 3-Chlor-5-(6-chlorpyridin-3-yl)-6-methyl-4-(2,4,6-trifluorphenyl)pyridazin, (4.012) 4-(2-Brom-4-fluorphenyl)-N-(2,6-difluorphenyl)-1,3-dimethyl-1H-pyrazol-5-amin, (4.013) 4-(2-Brom-4-fluorphenyl)-N-(2-brom-6-fluorphenyl)-1,3-dimethyl-1H-pyrazol-5-amin, (4.014) 4-(2-Brom-4-fluorphenyl)-N-(2-bromphenyl)-1,3-dimethyl-1H-pyrazol-5-amin, (4.015) 4-(2-Brom-4-fluorphenyl)-N-(2-chlor-6-fluorphenyl)-1,3-dimethyl-1H-pyrazol-5-amin, (4.016) 4-(2-Brom-4-fluorphenyl)-N-(2-chlorphenyl)-1,3-dimethyl-1H-pyrazol-5-amin, (4.017) 4-(2-Brom-4-fluorphenyl)-N-(2-fluorphenyl)-1,3-dimethyl-1H-pyrazol-5-amin, (4.018) 4-(2-Chlor-4-fluorphenyl)-N-(2,6-difluorphenyl)-1,3-dimethyl-1H-pyrazol-5-amin, (4.019) 4-(2-Chlor-4-fluorphenyl)-N-(2-chlor-6-fluorphenyl)-1,3-dimethyl-1H-pyrazol-5-amin, (4.020) 4-(2-Chlor-4-fluorphenyl)-N-(2-chlorphenyl)-1,3-dimethyl-1H-pyrazol-5-amin, (4.021) 4-(2-Chlor-4-fluorphenyl)-N-(2-fluorphenyl)-1,3-dimethyl-1H-pyrazol-5-amin, (4.022) 4-(4-Chlorphenyl)-5-(2,6-difluorphenyl)-3,6-dimethylpyridazin, (4.023) N-(2-Brom-6-fluorphenyl)-4-(2-chlor-4-fluorphenyl)-1,3-dimethyl-1H-pyrazol-5-amin, (4.024) N-(2-Bromphenyl)-4-(2-chlor-4-fluorphenyl)-1,3-dimethyl-1H-pyrazol-5-amin, (4.025) N-(4-Chlor-2,6-difluorphenyl)-4-(2-chlor-4-fluorphenyl)-1,3-dimethyl-1H-pyrazol-5-amin.
5) Verbindungen, die an mehreren Stellen wirken können ("Multisite Action"), zum Beispiel (5.001) Bordeaux-Mischung, (5.002) Captafol, (5.003) Captan, (5.004) Chlorthalonil, (5.005) Kupferhydroxid, (5.006) Kupfernaphthenat, (5.007) Kupferoxid, (5.008) Kupferoxychlorid, (5.009) Kupfer(2+)-sulfat, (5.010) Dithianon, (5.011) Dodin, (5.012) Folpet, (5.013) Mancozeb, (5.014) Maneb, (5.015) Metiram, (5.016) Metiram-Zink, (5.017) Oxin-Kupfer, (5.018) Propineb, (5.019) Schwefel und Schwefelzubereitungen einschließlich Calciumpolysulfid, (5.020) Thiram, (5.021) Zineb, (5.022) Ziram, (5.023) 6-Ethyl-5,7-dioxo-6,7-dihydro-5H-pyrrolo[3',4':5,6][1,4]dithiino[2,3-c][1,2]thiazol-3-carbonsäurenitril.
6) Verbindungen, die dazu in der Lage sind, Abwehrreaktionen des Wirtes zu induzieren, zum Beispiel (6.001) Acibenzolar-S-methyl, (6.002) Isotianil, (6.003) Probenazol, (6.004) Tiadinil.
7) Inhibitoren von Aminosäure- und/oder Proteinbiosynthese, zum Beispiel (7.001) Cyprodinil, (7.002) Kasugamycin, (7.003) Kasugamycinhydrochlorid-hydrat, (7.004) Oxytetracyclin, (7.005) Pyrimethanil, (7.006) 3-(5-Fluor-3,3,4,4-tetramethyl-3,4-dihydroisochinolin-1-yl)chinolin.
8) Inhibitoren der ATP-Produktion, zum Beispiel (8.001) Silthiofam.
9) Inhibitoren der Zellwandsynthese, zum Beispiel (9.001) Benthiavalicarb, (9.002) Dimethomorph, (9.003) Flumorph, (9.004) Iprovalicarb, (9.005) Mandipropamid, (9.006) Pyrimorph, (9.007) Valifenalat, (9.008) (2E)-3-(4-tert.-Butylphenyl)-3-(2-chlorpyridin-4-yl)-1-(morpholin-4-yl)prop-2-en-1-on, (9.009) (2Z)-3-(4-tert.-Butylphenyl)-3-(2-chlorpyridin-4-yl)-1-(morpholin-4-yl)prop-2-en-1-on.
10) Inhibitoren der Lipid- und Membransynthese, zum Beispiel (10.001) Propamocarb, (10.002) Propamocarb-hydrochlorid, (10.003) Tolclofos-methyl.
11) Inhibitoren der Melaninbiosynthese, zum Beispiel (11.001) Tricyclazol, (11.002) {3-Methyl-1-[(4-methylbenzoyl)amino]butan-2-yl}carbamidsäure-2,2,2-trifluorethylester.
12) Inhibitoren der Nukleinsäuresynthese, zum Beispiel (12.001) Benalaxyl, (12.002) Benalaxyl-M (Kiralaxyl), (12.003) Metalaxyl, (12.004) Metalaxyl-M (Mefenoxam).
13) Inhibitoren der Signalübertragung, zum Beispiel (13.001) Fludioxonil, (13.002) Iprodion, (13.003) Procymidon, (13.004) Proquinazid, (13.005) Quinoxyfen, (13.006) Vinclozolin.
14) Verbindungen, die als Entkoppler wirken können, zum Beispiel (14.001) Fluazinam, (14.002) Meptyldinocap.
15) Weitere Fungizide ausgewählt aus der Gruppe bestehend aus (15.001) Abscisinsäure, (15.002) Benthiazol, (15.003) Bethoxazin, (15.004) Capsimycin, (15.005) Carvon, (15.006) Chinomethionat, (15.007) Cufraneb, (15.008) Cyflufenamid, (15.009) Cymoxanil, (15.010) Cyprosulfamid, (15.011) Flutianil, (15.012) Fosetyl-Aluminium, (15.013) Fosetyl-Calcium, (15.014) Fosetyl-Natrium, (15.015) Methylisothiocyanat, (15.016) Metrafenon, (15.017) Mildiomycin, (15.018) Natamycin, (15.019) Nickeldimethyldithiocarbamat, (15.020) Nitrothal-isopropyl, (15.021) Oxamocarb, (15.022) Oxathiapiprolin, (15.023) Oxyfenthiin, (15.024) Pentachlorphenol und Salze, (15.025) phosphorige Säure und deren Salze, (15.026) Propamocarb-fosetylat, (15.027) Pyriofenon (Chlazafenon), (15.028) Tebufloquin, (15.029) Tecloftalam, (15.030) Tolnifanid, (15.031) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.032) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.033) 2-(6-Benzylpyridin-2-yl)chinazolin, (15.034) Dipymetitron, (15.035) 2-[3,5-Bis(difluormethyl)-1H-pyrazol-1-yl]-1-[4-(4-{5-[2-(prop-2-in-1-y-loxy)phenyl]-4,5-dihydro-1,2-oxazol-3-yl}-1,3-thiazol-2-yl)piperidin-1-yl]ethanon, (15.036) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.037) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)-piperidin-1-yl]ethanon, (15.038) 2-[6-(3-Fluor-4-methoxyphenyl)-5-methylpyridin-2-yl]chinazolin, (15.039) Methansulfonsäure-2-{(5R)-3-[2-(1-{[3,5-bis(difluormethyl)-1H-pyrazol-1-yl]acetyl}piperidin-4-yl)-1,3-thiazol-4-yl]-4,5-dihydro-1,2-oxazol-5-yl}-3-chlorphenylester, (15.040) Methansulfonsäure-2-{(5S)-3-[2-(1-{[3,5-bis(difluormethyl)-1H-pyrazol-1-yl]acetyl}piperidin-4-yl)-1,3-thiazol-4-yl]-4,5-dihydro-1,2-oxazol-5-yl}-3-chlorphenylester, (15.041) Ipflufenoquin, (15.042) 2-{2-Fluor-6-[(8-fluor-2-methylchinolin-3-yl)oxy]phenyl}propan-2-ol, (15.043) Fluoxapiprolin, (15.044) Methansulfonsäure-2-{3-[2-(1-{[3,5-bis(difluormethyl)-1H-pyrazol-1-yl]acetyl}piperidin-4-yl)-1,3-thiazol-4-yl]-4,5-dihydro-1,2-oxazol-5-yl}-3-chlorphenylester, (15.044) Fluoxapiprolin, (15.045) 2-Phenylphenol und Salze, (15.046) 3-(4,4,5-Trifluor-3,3-dimethyl-3,4-dihydroisochinolin-1-yl)chinolin, (15.047) Quinofumelin, (15.048) 4-Amino-5-fluorpyrimidin-2-ol (tautomere Form: 4-Amino-5-fluorpyrimidin-2(1H)-on), (15.049) 4-Oxo-4-[(2-phenylethyl)amino]butansäure, (15.050) 5-Amino-1,3,4-thiadiazol-2-thiol, (15.051) 5-Chlor-N'-phenyl-N'-(prop-2-in-1-yl)thiophen-2-sulfonohydrazid, (15.052) 5-Fluor-2-[(4-fluorbenzyl)oxy]pyrimidin-4-amin, (15.053) 5-Fluor-2-[(4-methylbenzyl)oxy]pyrimidin-4-amin, (15.054) 9-Fluor-2,2-dimethyl-5-(chinolin-3-yl)-2,3-dihydro-1,4-benzoxazepin, (15.055) {6-[({[(Z)-(1-Methyl-1H-tetrazol-5-yl)(phenyl)methylen]amino}oxy)methyl]pyridin-2-yl}carbamidsäurebut-3-in-1-ylester, (15.056) (2Z)-3-Amino-2-cyano-3-phenylacrylsäureethylester, (15.057) Phenazin-1-carbonsäure, (15.058) 3,4,5-Trihydroxybenzoesäurepropylester, (15.059) Chinolin-8-ol, (15.060) Chinolin-8-olsulfat (2:1), (15.061) {6-[({[(1-Methyl-1H-tetrazol-5-yl)(phenyl)methylen]amino}oxy)methyl]pyridin-2-yl}carbamidsäure-tert. -butylester, (15.062) 5-Fluor-4-imino-3-methyl-1- [(4-methylphenyl)sulfonyl] -3,4-dihydropyrimidin-2(1H)-on, (15.063) Aminopyrifen, (15.064) (N'-[2-Chlor-4-(2-fluorphenoxy)-5-methylphenyl]-N-ethyl-N-methylimidoformamid), (15.065) (N'-(2-Chlor-5-methyl-4-phenoxyphenyl)-N-ethyl-N-methylimidoformamid), (15.066) (2-{2-[(7,8-Difluor-2-methylchinolin-3-yl)oxy]-6-fluorphenyl}propan-2-ol), (15.067) (5-Brom-1-(5,6-dimethylpyridin-3-yl)-3,3-dimethyl-3,4-dihydroisochinolin), (15.068) (3-(4,4-Difluor-5,5-dimethyl-4,5-dihydrothieno[2,3-c]pyridin-7-yl)chinolin), (15.069) (1-(4,5-Dimethyl-1H-benzimidazol-1-yl)-4,4-difluor-3,3-dimethyl-3,4-dihydroisochinolin), (15.070) 8-Fluor-3-(5-fluor-3,3-dimethyl-3,4-dihydroisochinolin-1-yl)chinolon, (15.071) 8-Fluor-3-(5-fluor-3,3,4,4-tetramethyl-3,4-dihydroisochinolin-1-yl)chinolon, (15.072) 3-(4,4-Difluor-3,3-dimethyl-3,4-dihydroisochinolin-1-yl)-8-fluorchinolin, (15.073) (N-Methyl-N-phenyl-4-[5-(trifluormethyl)-1,2,4-oxadiazol-3-yl]benzamid), (15.074) (Methyl{4-[5-(trifluormethyl)-1,2,4-oxadiazol-3-yl]phenyl}carbamat), (15.075) (N-{4-[5-(Trifluormethyl)-1,2,4-oxadiazol-3-yl]benzyl}cyclopropancarboxamid), (15.076) N-Methyl-4-(5-(trifluormethyl)-1,2,4-oxadiazol-3-yl]benzamid, (15.077) N-[(E)-Methoxyiminomethyl]-4-[5-(trifluormethyl)-1,2,4-oxadiazol-3-yl]benzamid, (15.078) N-[(Z)-Methoxyiminomethyl]-4-[5-(trifluormethyl)-1,2,4-oxadiazol-3-yl]benzamid, (15.079) N-[4-[5-(Trifluormethyl)-1,2,4-oxadiazol-3-yl]phenyl]-cyclopropancarboxamid, (15.080) N-(2-Fluorphenyl)-4-[5-(trifluormethyl)-1,2,4-oxadiazol-3-yl]benzamid, (15.081) 2,2-Difluor-N-methyl-2-[4-[5-(trifluormethyl)-1,2,4-oxadiazol-3-yl]phenyl]-acetamid, (15.082) N-Allyl-N-[[4-[5-(trifluormethyl)-1,2,4-oxadiazol-3-yl)phenyl]methyl]acetamid, (15.083) N-[(E)-N-Methoxy-C-methyl-carbonimidoyl]-4-(5-(trifluormethyl)-1,2,4-oxadiazol-3-yl]-benzamid, (15.084) N-[(Z)-N-Methoxy-C-methyl-carbonimidoyl]-4-[5-(trifluormethyl)-1,2,4-oxadiazol-3-yl]benzamid, (15.085) N-Allyl-N-[[4-[5-(trifluormethyl)-1,2,4-oxadiazol-3-yl]phenyl]-methyl]propanamid, (15.086) 4,4-Dimethyl-1-[[4-[5-(trifluormethyl)-1,2,4-oxadiazol-3-yl]phenyl]methyl]pyrrolidin-2-on, (15.087) N-Methyl-4-[5-(trifluormethyl)-1,2,4-oxadiazol-3-yl]-benzencarbothioamid, (15.088) 5-Methyl-1-[[4-[5-(trifluormethyl)-1,2,4-oxadiazol-3-yl]phenyl]methyl]pyrrolidin-2-on, (15.089) N-((2,3-Difluor-4-[5-(trifluormethyl)-1,2,4-oxadiazol-3-yl]phenyl]methyl]-3,3,3-trifluor-propanamid, (15.090) 1-Methoxy-1-methyl-3-[[4-[5-(trifluormethyl}-1,2,4-oxadiazol-3-yl]phenyl]methyl]harnstoff, (15.091) 1,1-Diethyl-3-[[4-[5-(trifluormethyl}-1,2,4-oxadiazol-3-yl]phenyl]methyl]harnstoff, (15.092) N-[[4-[5-(Trifluormethyl)-1,2,4-oxadiazol-3-yl]phenyl]methyl]propanamid, (15.093) N-Methoxy-N-[[4-[5-(trifluormethyl)-1,2,4-o-xadiazol-3-yl]phenyl]methyl]cyclopropancarboxamid, (15.094) 1-Methoxy-3-methyl-1-[[4-[5-(trifluormethyl)-1,2,4-oxadiazol-3-yl]phenyl]methyl]harnstoff, (15.095) N-Methoxy-N-[[4-[5-(trifluormethyl)-1,2,4-oxadiazol-3-yl]phenyl]methyl)cyclopropancarboxamid, (15.096) N,2-Dimethoxy-N-[[4-[5-(trifluormethyl}-1,2,4-oxadiazol-3-yl]phenyl]methyl]propanamid, (15.097) N-Ethyl-2-methyl-N-[[4-[5-(trifluormethyl)-1,2,4-oxadiazol-3-yl)phenyl]methyl]propanamid, (15.098) 1-Methoxy-3-methyl-1-[[4-[5-(trifluormethyl)-1,2,4-oxadiazol-3-yl]phenyl]methyl]harnstoff, (15.099) 1,3-Dimethoxy-1-[[4-[5-(trifluormethyl)-1,2,4-oxadiazol-3-yl]phenyl]methyl]harnstoff, (15.100) 3-Ethyl-1-methoxy-1-[[4-[5-(trifluormethyl)-1,2,4-oxadiazol-3-yl]phenyl]methyl]harnstoff, (15.101) 1-[[4-[5-(Trifluormethyl)-1,2,4-oxadiazol-3-yl]phenyl]methyl]piperidin-2-on, (15.102) 4,4-Dimethyl-2-[[4-[5-(trifluormethyl)-1,2,4-oxadiazol-3-yl]phenyl]methyl]isooxazolidin-3-on, (15.103) 5,5-Dimethyl-2-[[4-[5-(trifluormethyl)-1,2,4-oxadiazol-3-yl]phenyl]methyl]isoxazolidin-3-on, (15.104) 3,3-Dimethyl-1-[[4-[5-(trifluormethyl)-1,2,4-oxadiazol-3-yl]phenyl]methyl]piperidin-2-on, (15.105) 1-[[3-Fluor-4-(5-(trifluormethyl)-1,2,4-oxadiazol-3-yl]phenyl]methyl]azepan-2-on, (15.106) 4,4-Dimethyl-2-[[4-(5-(trifluormethyl)-1,2,4-oxadiazol-3-yl]phenyl]methyl]isoxazolidin-3-on, (15.107) 5,5-Dimethyl-2-[[4-[5-(trifluormethyl)-1,2,4-oxadiazol-3-yl]phenyl]methyl]isoxazolidin-3-on, (15.108) (1-{4-[5-(Trifluormethyl)-1,2,4-oxadiazol-3-yl]benzyl}-1H-pyrazol-4-yl)essigsäureethylester, (15.109) N,N-Dimethyl-1-{4-[5-(trifluormethyl)-1,2,4-oxadiazol-3-yl]benzyl}-1H-1,2,4-triazol-3-amin und (15.110) N-{2,3-Difluor-4-[5-(trifluormethyl)-1,2,4-oxadiazol-3-yl]benzyl}butanamid.

### Biologische Schädlingsbekämpfungsmittel als Mischungskomponenten

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421), *Bacillus thuringiensis,* insbesondere *B. thuringiensis* Subspezies *israelensis* (Serotyp H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii* (ehemals bekannt als *Verticillium lecanii),* insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus* (neu: *Isaria fumosorosea*), insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia*), *Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense, Fortune Aza, Fungastop, Heads Up (Chenopodium quinoa-Saponinextrakt), Pyrethrum/Pyrethrine, Quassia amara, Quercus, Quillaja, Regalia, "Requiem^{™} Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver, sowie bioinsektizide/akarizide Wirkstoffe erhalten aus Olivenöl, insbesondere ungesättigte Fett-/Carbonsäuren mit Carbonkettenlängen C₁₆-C₂₀ als Wirkstoffe wie beispielsweise enthalten im Produkt mit dem Handelsnamen FLiP-PER^{®}.

### Safener als Mischungskomponenten

Die Verbindungen der Formel (I) können mit Safenern kombiniert werden, wie zum Beispiel Benoxacor, Cloquintocet (-mexyl), Cyometrinil, Cyprosulfamid, Dichlormid, Fenchlorazol (-ethyl), Fenclorim, Flurazol, Fluxofenim, Furilazol, Isoxadifen (-ethyl), Mefenpyr (-diethyl), Naphthalsäureanhydrid, Oxabetrinil, 2-Methoxy-N-({4-[(methylcarbamoyl)amino]phenyl}sulfonyl)benzamid (CAS 129531-12-0), 4-(Dichloracetyl)-1-oxa-4-azaspiro[4.5]decan (CAS 71526-07-3), 2,2,5-Trimethyl-3-(dichloracetyl)-1,3-o-xazolidin (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenteile verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika, Gurke, Melone, Möhre, Wassermelone, Zwiebel, Salat, Spinat, Porree, Bohnen, *Brassica oleracea* (z. B. Kohl) und andere Gemüsesorten, Baumwolle, Tabak, Raps, sowie Obstpflanzen (mit den Früchten Äpfel, Birnen, Zitrusfrüchte und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien wie Saatgut, Stecklinge, junge (unausgereifte) Pflanzen bis hin zu ausgereiften Pflanzen verstanden werden. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehören auch geerntete Pflanzen oder geerntete Pflanzenteile sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung der Verbindungen auf die Umgebung, den Lebensraum oder den Lagerraum nach den üblichen Behandlungsmethoden, z. B. durch Eintauchen, Spritzen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die durch konventionelle Züchtung, durch Mutagenese oder durch rekombinante DNA-Techniken erhalten worden sind. Dies können Sorten, Rassen, Bio- und Genotypen sein.

### Transgene Pflanze, Saatgutbehandlung und Integrationsereignisse

Erfindungsgemäß können die Verbindungen der Formel (I) vorteilhaft zum Behandeln von transgenen Pflanzen, Pflanzenkultivaren oder Pflanzenteilen eingesetzt werden, die genetisches Material erhalten haben, das diesen Pflanzen, Pflanzenkultivaren bzw. Pflanzenteilen vorteilhafte und/oder brauchbare Eigenschaften (Traits) verleiht. Es wird daher in Betracht gezogen, die vorliegende Erfindung mit einem oder mehreren rekombinanten Traits oder transgenen Events oder einer Kombination davon zu kombinieren. Für die Zwecke der vorliegenden Anmeldung kommt es durch Insertion eines spezifischen rekombinanten DNA-Moleküls in eine spezifische Position (locus) im Chromosom des Pflanzengenoms zu einem transgenen Event. Durch die Insertion wird eine neue DNA-Sequenz geschaffen, die als "Event" bezeichnet wird, und die durch das insertierte rekombinante DNA-Molekül und eine gewisse Menge genomischer DNA unmittelbar benachbart zur insertierten DNA/die insertierte DNA an beiden Enden flankierend gekennzeichnet ist. Solche Traits bzw. transgenen Events schließen, wobei dies nicht einschränkend ist, Resistenz gegenüber Schädlingen, Wasserausnutzungseffizienz, Ertragsleistung, Dürretoleranz, Samenqualität, verbesserte Nährstoffqualität, Hybridsamenproduktion und Herbizidtoleranz ein, wobei der Trait in Bezug auf eine Pflanze, der ein solcher Trait bzw. ein solches transgenes Event fehlt, gemessen wird. Konkrete Beispiele für solche vorteilhaften und/oder brauchbaren Eigenschaften (Traits) sind besseres Pflanzenwachstum, Lebenskraft, Stresstoleranz, Standfähigkeit, Resistenz gegenüber Lagern, Nährstoffaufnahme, Pflanzenernährung und/oder Ertrag, insbesondere verbessertes Wachstum, erhöhte Toleranz gegenüber hohen oder niedrigen Temperaturen, erhöhte Toleranz gegenüber Dürre oder Wasser- oder Bodensalzgehalt, erhöhte Blühleistung, erleichterte Ernte, Beschleunigung der Reife, höhere Erträge, höhere Qualität und/oder höherer Nährwert der Ernteprodukte, bessere Haltbarkeit und/oder Bearbeitbarkeit der Ernteprodukte und erhöhte Resistenz bzw. Toleranz gegenüber tierischen und mikrobiellen Schädlingen wie gegen Insekten, Spinnentiere, Milben und Schnecken.

Von den für Proteine, die Resistenz- oder Toleranzeigenschaften gegenüber solchen tierischen und mikrobiellen Schädlingen, insbesondere Insekten, verleihen, codierenden DNA-Sequenzen soll insbesondere das genetische Material von Bacillus thuringiensis erwähnt werden, das für die Bt-Proteine codiert, die in der Literatur ausführlich beschrieben und dem Fachmann gut bekannt sind. Erwähnt werden sollen auch von Bakterien wie *Photorhabdus* (WO97/17432 und WO98/08932) extrahierte Proteine. Insbesondere sollen Bt-Cry- oder VIP-Proteine Erwähnung finden, die CrylA-, CryIAb-, CryIAc-, CryIIA-, CryIIIA-, CryIIIB2-, Cry9c-, Cry2Ab-, Cry3Bb- und CryIF-Proteine oder toxische Fragmente davon einschließen, und außerdem Hybride oder Kombinationen davon, insbesondere das Cry1F-Protein oder von einem Cry1F-Protein abgeleitete Hybride (z.B. Hybrid-CrylA-CrylF-Proteine oder toxische Fragmente davon), die Proteine vom Cry1A-Typ oder toxische Fragmente davon, vorzugsweise das Cry1Ac-Protein oder vom Cry1Ac-Protein abgeleitete Hybride (z.B. Hybrid-CrylAb-CrylAc-Proteine) oder das Cry1Ab- oder Bt2-Protein oder toxische Fragmente davon, die Cry2Ae-, Cry2Af- oder Cry2Ag-Proteine oder toxische Fragmente davon, das CrylA. 105-Protein oder ein toxisches Fragment davon, das VIP3Aa19-Protein, das VIP3Aa20-Protein, die VIP3A-Proteine, die bei den COT202- oder COT203-Baumwoll-Events produziert werden, das VIP3Aa-Protein oder ein toxisches Fragment davon, wie in Estruch et al. (1996), Proc Natl Acad Sci US A. 28;93(11):5389-94 beschrieben, die wie in WO2001/47952 beschriebenen Cry-Proteine, die insektiziden Proteine aus *Xenorhabdus* (wie in WO98/50427 beschrieben), *Serratia* (insbesondere aus *S*. *entomophila*) oder Strängen der *Photorhabdus*-Art, wie Tc-Proteine aus Photorhabdus, wie in WO98/08932 beschrieben. Dies schließt auch alle Varianten bzw. Mutanten eines dieser Proteine ein, die sich in einigen Aminosäuren (1-10, vorzugsweise 1-5) von beliebigen der oben angeführten Sequenzen, insbesondere der Sequenz ihres toxischen Fragments, unterscheiden, oder die an ein Transitpeptid wie ein Plastidtransitpeptid oder ein anderes Protein oder Peptid fusioniert sind, ein.

Ein anderes und besonders hervorgehobenes Beispiel für solche Eigenschaften ist eine verliehene Toleranz gegenüber einem oder mehreren Herbiziden, zum Beispiel Imidazolinonen, Sulphonylharnstoffen, Glyphosat oder Phospinothricin. Von den für Proteine, die den transformierten Pflanzenzellen und Pflanzen Toleranzeigenschaften gegenüber bestimmten Herbiziden verleihen, codierenden DNA-Sequenzen sollte insbesondere das bar- bzw. PAT-Gen oder das Streptomyces-coelicolor-Gen, das in WO2009/152359 beschrieben ist und das Toleranz gegenüber Glufonsinatherbiziden verleiht, ein Gen, das für eine geeignete EPSPS (5-Enolpyruvylshikimat-3-phosphat-Synthase) codiert, die Toleranz gegenüber Herbiziden mit EPSPS als Target, insbesondere Herbiziden wie Glyphosat und dessen Salzen, verleiht, ein für Glyphosat-N-Acetyltransferase codierendes Gen oder ein für Glyphosatoxoreduktase codierendes Gen erwähnt werden. Weitere geeignete Herbizidtoleranz-Traits schließen mindestens einen ALS(Acetolactatsynthase)-Inhibitor (z.B. WO2007/024782), ein mutiertes Arabidopsis ALS/AHAS-Gen (z.B. US-Patentschrift 6,855,533), für 2,4-D-Monooxygenasen codierende Gene, die Toleranz gegenüber 2,4-D (2,4-Dichlorphenoxyessigsäure) verleihen, und für Dicamba-Monooxygenasen codierende Gene, die Toleranz gegenüber Dicamba (3,6-Dichlor-2-methoxybenzoesäure) verleihen, ein.

Weitere und besonders hervorgehobene Beispiele für solche Eigenschaften sind eine erhöhte Resistenz gegenüber phytopathogenen Pilzen, Bakterien und/oder Viren, die zum Beispiel auf systemische erworbene Resistenz (Systemic Acquired Resistance, SAR) zurückgeht, Systemin, Phytoalexine, Elizitoren und außerdem Resistenzgene und die entsprechend exprimierten Proteine und Toxine.

Besonders brauchbare transgene Events in transgenen Pflanzen oder Pflanzenkultivaren, die vorzugsweise erfindungsgemäß behandelt werden können, schließen Event 531/ PV-GHBK04 (Baumwolle, Insektenbekämpfung, beschrieben in WO2002/040677), Event 1143-14A (Baumwolle, Insektenbekämpfung, nicht hinterlegt, beschrieben in WO2006/128569); Event 1143-51B (Baumwolle, Insektenbekämpfung, nicht hinterlegt, beschrieben in WO2006/128570); Event 1445 (Baumwolle, Herbizidtoleranz, nicht hinterlegt, beschrieben in US-A 2002-120964 oder WO2002/034946); Event 17053 (Reis, Herbizidtoleranz, hinterlegt als PTA-9843, beschrieben in WO2010/117737); Event 17314 (Reis, Herbizidtoleranz, hinterlegt als PTA-9844, beschrieben in WO2010/117735); Event 281-24-236 (Baumwolle, Insektenbekämpfung - Herbizidtoleranz, hinterlegt als PTA-6233, beschrieben in WO2005/103266 oder US-A 2005-216969); Event 3006-210-23 (Baumwolle, Insektenbekämpfung - Herbizidtoleranz, hinterlegt als PTA-6233, beschrieben in US-A 2007-143876 oder WO2005/103266); Event 3272 (Mais, Qualitätsmerkmal, hinterlegt als PTA-9972, beschrieben in WO2006/098952 oder US-A 2006-230473); Event 33391 (Weizen, Herbizidtoleranz, hinterlegt als PTA-2347, beschrieben in WO2002/027004), Event 40416 (Mais, Insektenbekämpfung - Herbizidtoleranz, hinterlegt als ATCC PTA-11508, beschrieben in WO 11/075593); Event 43A47 (Mais, Insektenbekämpfung - Herbizidtoleranz, hinterlegt als ATCC PTA-11509, beschrieben in WO2011/075595); Event 5307 (Mais, Insektenbekämpfung, hinterlegt als ATCC PTA-9561, beschrieben in WO2010/077816); Event ASR-368 (Bentgras, Herbizidtoleranz, hinterlegt als ATCC PTA-4816, beschrieben in US-A 2006-162007 oder WO2004/053062); Event B16 (Mais, Herbizidtoleranz, nicht hinterlegt, beschrieben in US-A 2003-126634); Event BPS-CV127- 9 (Sojabohne, Herbizidtoleranz, hinterlegt als NCIMB Nr. 41603, beschrieben in WO2010/080829); Event BLR1 (Raps, Restauration von Pollensterilität, hinterlegt als NCIMB 41193, beschrieben in WO2005/074671), Event CE43-67B (Baumwolle, Insektenbekämpfung, hinterlegt als DSM ACC2724, beschrieben in US-A 2009-217423 oder WO2006/128573); Event CE44-69D (Baumwolle, Insektenbekämpfung, nicht hinterlegt, beschrieben in US-A 2010- 0024077); Event CE44-69D (Baumwolle, Insektenbekämpfung, nicht hinterlegt, beschrieben in WO2006/128571); Event CE46-02A (Baumwolle, Insektenbekämpfung, nicht hinterlegt, beschrieben in WO2006/128572); Event COT102 (Baumwolle, Insektenbekämpfung, nicht hinterlegt, beschrieben in US-A 2006-130175 oder WO2004/039986); Event COT202 (Baumwolle, Insektenbekämpfung, nicht hinterlegt, beschrieben in US-A 2007-067868 oder WO2005/054479); Event COT203 (Baumwolle, Insektenbekämpfung, nicht hinterlegt, beschrieben in WO2005/054480); ); Event DAS21606-3 / 1606 (Sojabohne, Herbizidtoleranz, hinterlegt als PTA-11028, beschrieben in WO2012/033794), Event DAS40278 (Mais, Herbizidtoleranz, hinterlegt als ATCC PTA-10244, beschrieben in WO2011/022469); Event DAS-44406-6 / pDAB8264.44.06.1 (Sojabohne, Herbizidtoleranz, hinterlegt als PTA-11336, beschrieben in WO2012/075426), Event DAS-14536-7 /pDAB8291.45.36.2 (Sojabohne, Herbizidtoleranz, hinterlegt als PTA-11335, beschrieben in WO2012/075429), Event DAS-59122-7 (Mais, Insektenbekämpfung - Herbizidtoleranz, hinterlegt als ATCC PTA 11384, beschrieben in US-A 2006-070139); Event DAS-59132 (Mais, Insektenbekämpfung - Herbizidtoleranz, nicht hinterlegt, beschrieben in WO2009/100188); Event DAS68416 (Sojabohne, Herbizidtoleranz, hinterlegt als ATCC PTA-10442, beschrieben in WO2011/066384 oder WO2011/066360); Event DP-098140-6 (Mais, Herbizidtoleranz, hinterlegt als ATCC PTA-8296, beschrieben in US-A 2009- 137395 oder WO 08/112019); Event DP-305423-1 (Sojabohne, Qualitätsmerkmal, nicht hinterlegt, beschrieben in US-A 2008-312082 oder WO2008/054747); Event DP-32138-1 (Mais, Hybridisierungssystem, hinterlegt als ATCC PTA-9158, beschrieben in US-A 2009-0210970 oder WO2009/103049); Event DP-356043-5 (Sojabohne, Herbizidtoleranz, hinterlegt als ATCC PTA-8287, beschrieben in US-A 2010-0184079 oder WO2008/002872); Event EE-I (Aubergine, Insektenbekämpfung, nicht hinterlegt, beschrieben in WO 07/091277); Event Fil 17 (Mais, Herbizidtoleranz, hinterlegt als ATCC 209031, beschrieben in US-A 2006-059581 oder WO 98/044140); Event FG72 (Sojabohne, Herbizidtoleranz, hinterlegt als PTA-11041, beschrieben in WO2011/063413), Event GA21 (Mais, Herbizidtoleranz, hinterlegt als ATCC 209033, beschrieben in USA 2005-086719 oder WO 98/044140); Event GG25 (Mais, Herbizidtoleranz, hinterlegt als ATCC 209032, beschrieben in US-A 2005-188434 oder WO98/044140); Event GHB119 (Baumwolle, Insektenbekämpfung - Herbizidtoleranz, hinterlegt als ATCC PTA-8398, beschrieben in WO2008/151780); Event GHB614 (Baumwolle, Herbizidtoleranz, hinterlegt als ATCC PTA-6878, beschrieben in US-A 2010-050282 oder WO2007/017186); Event GJ11 (Mais, Herbizidtoleranz, hinterlegt als ATCC 209030, beschrieben in US-A 2005-188434 oder WO98/044140); Event GM RZ13 (Zuckerrübe, Virusresistenz, hinterlegt als NCIMB-41601, beschrieben in WO2010/076212); Event H7-1 (Zuckerrübe, Herbizidtoleranz, hinterlegt als NCIMB 41158 oder NCIMB 41159, beschrieben in US-A 2004-172669 oder WO 2004/074492); Event JOPLIN1 (Weizen, Krankheitstoleranz, nicht hinterlegt, beschrieben in US-A 2008-064032); Event LL27 (Sojabohne, Herbizidtoleranz, hinterlegt als NCIMB41658, beschrieben in WO2006/108674 oder US-A 2008-320616); Event LL55 (Sojabohne, Herbizidtoleranz, hinterlegt als NCIMB 41660, beschrieben in WO 2006/108675 oder US-A 2008-196127); Event LLcotton25 (Baumwolle, Herbizidtoleranz, hinterlegt als ATCC PTA-3343, beschrieben in WO2003/013224 oder US- A 2003-097687); Event LLRICE06 (Reis, Herbizidtoleranz, hinterlegt als ATCC 203353, beschrieben in US 6,468,747 oder WO2000/026345); Event LLRice62 (Reis, Herbizidtoleranz, hinterlegt als ATCC 203352, beschrieben in WO2000/026345), Event LLRICE601 (Reis, Herbizidtoleranz, hinterlegt als ATCC PTA-2600, beschrieben in US-A 2008-2289060 oder WO2000/026356); Event LY038 (Mais, Qualitätsmerkmal, hinterlegt als ATCC PTA-5623, beschrieben in US-A 2007-028322 oder WO2005/061720); Event MIR162 (Mais, Insektenbekämpfung, hinterlegt als PTA-8166, beschrieben in US-A 2009-300784 oder WO2007/142840); Event MIR604 (Mais, Insektenbekämpfung, nicht hinterlegt, beschrieben in US-A 2008-167456 oder WO2005/103301); Event MON15985 (Baumwolle, Insektenbekämpfung, hinterlegt als ATCC PTA-2516, beschrieben in US-A 2004-250317 oder WO2002/100163); Event MON810 (Mais, Insektenbekämpfung, nicht hinterlegt, beschrieben in US-A 2002-102582); Event MON863 (Mais, Insektenbekämpfung, hinterlegt als ATCC PTA-2605, beschrieben in WO2004/011601 oder US-A 2006-095986); Event MON87427 (Mais, Bestäubungskontrolle, hinterlegt als ATCC PTA-7899, beschrieben in WO2011/062904); Event MON87460 (Mais, Stresstoleranz, hinterlegt als ATCC PTA-8910, beschrieben in WO2009/111263 oder US-A 2011-0138504); Event MON87701 (Sojabohne, Insektenbekämpfung, hinterlegt als ATCC PTA- 8194, beschrieben in US-A 2009-130071 oder WO2009/064652); Event MON87705 (Sojabohne, Qualitätsmerkmal - Herbizidtoleranz, hinterlegt als ATCC PTA-9241, beschrieben in US-A 2010-0080887 oder WO201O/037016); Event MON87708 (Sojabohne, Herbizidtoleranz, hinterlegt als ATCC PTA-9670, beschrieben in WO2011/034704); Event MON87712 (Sojabohne, Ertrag, hinterlegt als PTA-10296, beschrieben in WO2012/051199), Event MON87754 (Sojabohne, Qualitätsmerkmal, hinterlegt als ATCC PTA-9385, beschrieben in WO2010/024976); Event MON87769 (Sojabohne, Qualitätsmerkmal, hinterlegt als ATCC PTA- 8911, beschrieben in US-A 2011-0067141 oder WO2009/102873); Event MON88017 (Mais, Insektenbekämpfung - Herbizidtoleranz, hinterlegt als ATCC PTA-5582, beschrieben in US-A 2008-028482 oder WO2005/059103); Event MON88913 (Baumwolle, Herbizidtoleranz, hinterlegt als ATCC PTA-4854, beschrieben in WO2004/072235 oder US-A 2006-059590); Event MON88302 (Raps, Herbizidtoleranz, hinterlegt als PTA-10955, beschrieben in WO2011/153186), Event MON88701 (Baumwolle, Herbizidtoleranz, hinterlegt als PTA-11754, beschrieben in WO2012/134808), Event MON89034 (Mais, Insektenbekämpfung, hinterlegt als ATCC PTA-7455, beschrieben in WO 07/140256 oder US-A 2008-260932); Event MON89788 (Sojabohne, Herbizidtoleranz, hinterlegt als ATCC PTA-6708, beschrieben in US-A 2006-282915 oder WO2006/130436); Event MS1 1 (Raps, Bestäubungskontrolle - Herbizidtoleranz, hinterlegt als ATCC PTA-850 oder PTA-2485, beschrieben in WO2001/031042); Event MS8 (Raps, Bestäubungskontrolle - Herbizidtoleranz, hinterlegt als ATCC PTA-730, beschrieben in WO2001/041558 oder US-A 2003-188347); Event NK603 (Mais, Herbizidtoleranz, hinterlegt als ATCC PTA-2478, beschrieben in US-A 2007-292854); Event PE-7 (Reis, Insektenbekämpfung, nicht hinterlegt, beschrieben in WO2008/114282); Event RF3 (Raps, Bestäubungskontrolle - Herbizidtoleranz, hinterlegt als ATCC PTA-730, beschrieben in WO2001/041558 oder US-A 2003-188347); Event RT73 (Raps, Herbizidtoleranz, nicht hinterlegt, beschrieben in WO2002/036831 oder US-A 2008-070260); Event SYHT0H2 / SYN-000H2-5 (Sojabohne, Herbizidtoleranz, hinterlegt als PTA-11226, beschrieben in WO2012/082548), Event T227-1 (Zuckerrübe, Herbizidtoleranz, nicht hinterlegt, beschrieben in WO2002/44407 oder US-A 2009-265817); Event T25 (Mais, Herbizidtoleranz, nicht hinterlegt, beschrieben in US-A 2001-029014 oder WO2001/051654); Event T304-40 (Baumwolle, Insektenbekämpfung - Herbizidtoleranz, hinterlegt als ATCC PTA-8171, beschrieben in US-A 2010-077501 oder WO2008/122406); Event T342-142 (Baumwolle, Insektenbekämpfung, nicht hinterlegt, beschrieben in WO2006/128568); Event TC1507 (Mais, Insektenbekämpfung - Herbizidtoleranz, nicht hinterlegt, beschrieben in US-A 2005-039226 oder WO2004/099447); Event VIP1034 (Mais, Insektenbekämpfung - Herbizidtoleranz, hinterlegt als ATCC PTA-3925, beschrieben in WO2003/052073), Event 32316 (Mais, Insektenbekämpfung-Herbizidtoleranz, hinterlegt als PTA-11507, beschrieben in WO2011/084632), Event 4114 (Mais, Insektenbekämpfung-Herbizidtoleranz, hinterlegt als PTA-11506, beschrieben in WO2011/084621), Event EE-GM3 / FG72 (Sojabohne, Herbizidtoleranz, ATCC-Zugangsnr. PTA-11041) gegebenenfalls gestapelt mit Event EE-GM1/LL27 oder Event EE-GM2/LL55 (WO2011/063413A2), Event DAS-68416-4 (Sojabohne, Herbizidtoleranz, ATCC-Zugangsnr. PTA-10442, WO2011/066360A1), Event DAS-68416-4 (Sojabohne, Herbizidtoleranz, ATCC-Zugangsnr. PTA-10442, WO2011/066384A1), Event DP-040416-8 (Mais, Insektenbekämpfung, ATCC-Zugangsnr. PTA-11508, WO2011/075593A1), Event DP-043A47-3 (Mais, Insektenbekämpfung, ATCC-Zugangsnr. PTA-11509, WO2011/075595A1), Event DP- 004114-3 (Mais, Insektenbekämpfung, ATCC-Zugangsnr. PTA-11506, WO2011/084621A1), Event DP-032316-8 (Mais, Insektenbekämpfung, ATCC-Zugangsnr. PTA-11507, WO2011/084632A1), Event MON-88302-9 (Raps, Herbizidtoleranz, ATCC-Zugangsnr. PTA-10955, WO2011/153186A1), Event DAS-21606-3 (Sojabohne, Herbizidtoleranz, ATCC-Zugangsnr. PTA-11028, WO2012/033794A2), Event MON-87712-4 (Sojabohne, Qualitätsmerkmal, ATCC-Zugangsnr. PTA-10296, WO2012/051199A2), Event DAS-44406-6 (Sojabohne, gestapelte Herbizidtoleranz, ATCC-Zugangsnr. PTA-11336, WO2012/075426A1), Event DAS-14536-7 (Sojabohne, gestapelte Herbizidtoleranz, ATCC-Zugangsnr. PTA-11335, WO2012/075429A1), Event SYN-000H2-5 (Sojabohne, Herbizidtoleranz, ATCC-Zugangsnr. PTA-11226, WO2012/082548A2), Event DP-061061-7 (Raps, Herbizidtoleranz, keine Hinterlegungsnr. verfügbar, WO2012071039A1), Event DP-073496-4 (Raps, Herbizidtoleranz, keine Hinterlegungsnr. verfügbar, US2012131692), Event 8264.44.06.1 (Sojabohne, gestapelte Herbizidtoleranz, Zugangsnr. PTA-11336, WO2012075426A2), Event 8291.45.36.2 (Sojabohne, gestapelte Herbizidtoleranz, Zugangsnr. PTA-11335, WO2012075429A2), Event SYHT0H2 (Sojabohne, ATCC-Zugangsnr. PTA-11226, WO2012/082548A2), Event MON88701 (Baumwolle, ATCC-Zugangsnr. PTA-11754, WO2012/134808A1), Event KK179-2 (Luzerne, ATCC-Zugangsnr. PTA-11833, WO2013/003558A1), Event pDAB8264.42.32.1 (Sojabohne, gestapelte Herbizidtoleranz, ATCC-Zugangsnr. PTA-11993, WO2013/010094A1), Event MZDT09Y (Mais, ATCC-Zugangsnr. PTA-13025, WO2013/012775A1) ein.

Weiterhin wird eine solche Liste transgener Events vom United States Department of Agriculture's (USDA) Animal and Plant Health Inspection Service (APHIS) bereitgestellt und findet sich auf deren Webseite auf dem World Wide Web bei aphis.usda.gov. Für die vorliegende Anmeldung ist der Status dieser Liste, wie er am Anmeldetag der vorliegenden Anmeldung war, von Relevanz.

Die Gene/Events, die die betreffenden gewünschten Merkmale verleihen, können in den transgenen Pflanzen auch in Kombinationen miteinander vorliegen. Beispiele für transgene Pflanzen, die erwähnt werden können, sind wichtige Kulturpflanzen wie Getreide (Weizen, Reis, Triticale, Gerste, Roggen, Hafer), Mais, Sojabohnen, Kartoffeln, Zuckerrübe, Zuckerrohr, Tomaten, Erbsen und andere Arten von Gemüse, Baumwolle, Tabak, Raps und außerdem Obstpflanzen (mit den Früchten Äpfeln, Birnen, Zitrusfrüchte und Weintrauben), wobei Mais, Sojabohnen, Weizen, Reis, Kartoffeln, Baumwolle, Zuckerrohr, Tabak und Raps besonders hervorgehoben sind. Traits, die besonders hervorgehoben werden, sind die erhöhte Resistenz der Pflanzen gegenüber Insekten, Spinnentieren und Schnecken sowie die erhöhte Resistenz der Pflanzen gegenüber einem oder mehreren Herbiziden.

Im Handel erhältliche Beispiele für solche Pflanzen, Pflanzenteile oder Pflanzensamen, die vorzugsweise erfindungsgemäß behandelt werden können, schließen im Handel erhältliche Produkte wie Pflanzensamen ein, die unter den GENUITY^{®}-, DROUGHTGARD^{®}-, SMARTSTAX^{®}-, RIB COMPLETE^{®}-, ROUNDUP READY^{®}-, VT DOUBLE PRO^{®}-, VT TRIPLE PRO^{®}-, BOLLGARD II^{®}-, ROUNDUP READY 2 YIELD^{®}-, YIELDGARD^{®}-, ROUNDUP READY^{®} 2 XTEN^{DTM}-, INTACTA RR2 PRO^{®}-, VISTIVE GOLD^{®}- und/oder XTENDFLEX^{™}-Handelsnamen verkauft bzw. vertrieben werden.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z. B. durch Tauchen, Spritzen, Sprühen, Berieseln, Verdampfen, Zerstäuben, Vernebeln, Verstreuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d. h. die Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Wirkstoffen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d. h. der Standort der Pflanze (z. B. Boden oder hydroponische Systeme) wird mit einer flüssigen Form der Verbindungen der Formel (I) getränkt, oder durch die Bodenapplikation, d. h. die erfindungsgemäßen Verbindungen der Formel (I) werden in fester Form (z. B. in Form eines Granulats) in den Standort der Pflanzen eingebracht, oder durch Tropfapplikation (oftmals auch als "Chemigation" bezeichnet), d.h. die erfindungsgemäßen Verbindungen der Formel (I) werden mittels Oberflächen- oder Untergrund-Tropfrohren über bestimmte Zeiträume zusammen mit variierenden Mengen an Wasser an definierten Stellen in der Nähe der Pflanzen eingebracht. Bei Wasserreiskulturen kann das auch durch Zudosieren der Verbindung der Formel (I) in einer festen Anwendungsform (z. B. als Granulat) in ein überflutetes Reisfeld sein.

### Digitale Technologien

Die erfindungsgemäßen Verbindungen können in Kombination mit z.B. in Computerprogrammen für ortsspezifisches Kulturpflanzenmanagement eingebetteten Modellen, Satelliten-Ackerbau, Präzisionsackerbau bzw. Präzisionslandwirtschaft eingesetzt werden. Solche Modelle unterstützen das ortsspezifische Management landwirtschaftlicher Anlagen mit Daten aus verschiedenen Quellen wie Böden, Wetter, Kulturpflanzen (z.B. Typ, Wachstumsstadium, Pflanzengesundheit), Unkräuter (z.B. Typ, Wachstumsstadium), Krankheiten, Schädlingen, Nährstoffen, Wasser, Feuchtigkeit, Biomasse, Satellitendaten, Ertrag usw., mit dem Ziel, Rentabilität, Nachhaltigkeit und Umweltschutz zu optimieren. Insbesondere können solche Modelle helfen, agronomische Entscheidungen zu optimieren, die Präzision von Pestizidanwendungen zu steuern und die durchgeführten Arbeiten aufzuzeichnen.

Beispielsweise kann man die erfindungsgemäßen Verbindungen gemäß einem entsprechenden Anwendungsprotokoll auf eine Kulturpflanze aufbringen, wenn das Modell das Auftreten eines Schädlings moduliert und berechnet, dass eine Schwelle erreicht wurde, bei der es empfohlen wird, die erfindungsgemäße Verbindung auf die Kulturpflanze aufzubringen.

Im Handel erhältliche Systeme, die agronomische Modelle einschließen, sind z.B. FieldScriptsTM von The Climate Corporation, XarvioTM von BASF, AGLogicTM von John Deere usw.

Die erfindungsgemäßen Verbindungen können außerdem in Kombination mit smartem Sprühgerät wie z.B. Gerät zum punktuellen Sprühen oder Präzisionssprühen, das an einem Farmvehikel wie einem Traktor, einem Roboter, einem Helikopter, einem Flugzeug, einem unbemannten Luftfahrzeug (Unmanned Aerial Vehicle, UAV) wie einer Drohne an - bzw. untergebracht ist, eingesetzt werden. Solches Gerät umfasst gewöhnlich Input-Sensoren (wie z.B. eine Kamera) und eine Bearbeitungseinheit, die für die Analyse der Input-Daten und die Bereitstellung einer Entscheidung, die auf der Analyse der Input-Daten basiert, zur Anwendung der erfindungsgemäßen Verbindung auf den Kulturpflanzen (beziehungsweise den Unkräutern) in spezifischer und präziser Weise konfiguriert ist. Der Einsatz solcher smarten Sprühgeräte erfordert gewöhnlich Positionssysteme (z.B. GPS-Empfänger), mit denen die aufgenommenen Daten lokalisiert und Farmvehikel gesteuert bzw. kontrolliert werden, geografische Informationssysteme (GIS), mit denen die Informationen auf verständlichen Karten dargestellt werden, und entsprechende Farmvehikel zum Durchführen der erforderlichen landwirtschaftlichen Maßnahme wie dem Sprühen.

Bei einem Beispiel können Schädlinge aus von einer Kamera aufgenommenen Bildern nachgewiesen werden. Bei einem Beispiel können die Schädlinge auf Basis dieser Bilder identifiziert und/oder klassifiziert werden. Bei einer solchen Identifikation und/oder Klassifikation kann man sich Algorithmen zur Bildverarbeitung bedienen. Solche Algorithmen zur Bildverarbeitung können Algorithmen zum maschinellen Lernen wie künstliche neuronale Netze, Entscheidungsbäume, und Künstliche-Intelligenz-Algorithmen nutzen. Auf diese Weise ist es möglich, die hier beschriebenen Verbindungen nur dort anzuwenden, wo sie benötigt werden.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Be-handlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden Eigenschaften schädlingsresistenter bzw. -toleranter transgener Pflanzen einbeziehen, um einen optimalen Schutz des Saatguts und auch der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und einer Mischungskomponente behandelt wird. Es umfasst ferner auch ein Verfahren, in dem das Saatgut zu unterschiedlichen Zeiten mit einer Verbindung der Formel (I) und einer Mischungskomponente behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer erfindungsgemäßen Verbindung der Formel (I) behandelt wurde. Die Erfindung bezieht sich auch auf Saatgut, welches zur gleichen Zeit mit einer Verbindung der Formel (I) und einer Mischungskomponente behandelt wurde. Die Erfindung bezieht sich weiterhin auf Saatgut, welches zu unterschiedlichen Zeiten mit einer Verbindung der Formel (I) und einer Mischungskomponente behandelt wurde. Bei Saatgut, welches zu unterschiedlichen Zeiten mit einer Verbindung der Formel (I) und einer Mischungskomponente behandelt wurde, können die einzelnen Substanzen in unterschiedlichen Schichten auf dem Saatgut vorhanden sein. Dabei können die Schichten, die eine Verbindung der Formel (I) und Mischungskomponenten enthalten, gegebenenfalls durch eine Zwischenschicht getrennt sein. Die Erfindung bezieht sich auch auf Saatgut, bei dem eine Verbindung der Formel (I) und eine Mischungskomponente als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating-Verfahren unterzogen wird, um Staubabrieb am Saatgut zu vermeiden.

Einer der auftretenden Vorteile, wenn eine Verbindung der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Zusammensetzungen oder Verbindungen der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z. B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hüllen, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z. B. mit Wasser behandelt und dann erneut getrocknet wurde, zum Beispiel Priming. Im Fall von Reis-Saatgut ist es auch möglich, Saatgut zu verwenden, das getränkt wurde, zum Beispiel in Wasser bis zu einem bestimmten Stadium des Reisembryos ("Pigeon Breast Stage"), wodurch die Keimung und ein einheitlicheres Auflaufen stimuliert wird.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalinsulfonate, wie Diisopropyl- oder Diisobutylnaphthalinsulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tri-stryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formu-lierungen enthalten sein können, kommen alle für derartige Zwecke in agrochemischen Mitteln ein-setzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zu-bereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder den daraus durch Zugabe von Wasser hergestellten Anwendungsformen kommen alle üblicherweise für die Beizung einsetzbaren Mischgeräte in Betracht. Im Einzelnen geht man bei der Beizung so vor, dass man das Saatgut in einen Mischer im diskontinuierlichen oder kontinuierlichen Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d. h. dem Gebiet der Tiermedizin, sind die Verbindungen der Formel (I) gegen Tierparasiten, insbesondere Ektoparasiten oder Endoparasiten, wirksam. Der Begriff Endoparasit umfasst insbesondere Helminthen und Protozoen wie Kokzidien. Ektoparasiten sind typischerweise und bevorzugt Arthropoden, insbesondere Insekten oder Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z. B. in der Aquakultur, oder gegebenenfalls Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel; Reptilien, Amphibien oder Aquariumfische.

Gemäß einer bestimmten Ausführungsform werden die Verbindungen der Formel (I) an Säugetiere verabreicht.

Gemäß einer weiteren bestimmten Ausführungsform werden die Verbindungen der Formel (I) an Vögel, nämlich Stubenvögel oder insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen" im vorliegenden Zusammenhang, dass durch die Verbindungen der Formel (I) wirksam das Auftreten des jeweiligen Parasiten in einem Tier, das mit solchen Parasiten in einem harmlosen Ausmaß infiziert ist, reduziert wird. Genauer gesagt bedeutet "bekämpfen" im vorliegenden Zusammenhang, dass die Verbindungen der Formel (I) den jeweiligen Parasiten abtöten, sein Wachstum verhindern oder seine Vermehrung verhindern.

Zu den Arthropoden zählen beispielsweise, ohne hierauf beschränkt zu sein,
aus der Ordnung Anoplurida zum Beispiel Haematopinus spp., Linognathus spp., Pediculus spp., Phtirus spp., Solenopotes spp.;
aus der Ordnung Mallophagida und den Unterordnungen Amblycerina und Ischnocerina, zum Beispiel Bovicola spp., Damalina spp., Felicola spp.; Lepikentron spp., Menopon spp., Trichodectes spp., Trimenopon spp., Trinoton spp., Werneckiella spp;
aus der Ordnung Diptera und den Unterordnungen Nematocerina und Brachycerina, zum Beispiel Aedes spp., Anopheles spp., Atylotus spp., Braula spp., Calliphora spp., Chrysomyia spp., Chrysops spp., Culex spp., Culicoides spp., Eusimulium spp., Fannia spp., Gasterophilus spp., Glossina spp., Haematobia spp., Haematopota spp., Hippobosca spp., Hybomitra spp., Hydrotaea spp., Hypoderma spp., Lipoptena spp., Lucilia spp., Lutzomyia spp., Melophagus spp., Morellia spp., Musca spp., Odagmia spp., Oestrus spp., Philipomyia spp., Phlebotomus spp., Rhinoestrus spp., Sarcophaga spp., Simulium spp., Stomoxys spp., Tabanus spp., Tipula spp., Wilhelmia spp., Wohlfahrtia spp.;
aus der Ordnung Siphonapterida, zum Beispiel Ceratophyllus spp., Ctenocephalides spp., Pulex spp., Tunga spp., Xenopsylla spp.;
aus der Ordnung Heteropterida, zum Beispiel Cimex spp., Panstrongylus spp., Rhodnius spp., Triatoma spp.; sowie Lästlinge und Hygieneschädlinge aus der Ordnung Blattarida.

Weiterhin sind bei den Arthropoden beispielhaft, ohne hierauf beschränkt zu sein, die folgenden Akari zu nennen:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Amblyomma spp., Dermacentor spp., Haemaphysalis spp., Hyalomma spp., Ixodes spp., Rhipicephalus (Boophilus) spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Sternostoma spp., Tropilaelaps spp., Varroa spp.; aus der Ordnung Actinedida (Prostigmata), zum Beispiel Acarapis spp., Cheyletiella spp., Demodex spp., Listrophorus spp., Myobia spp., Neotrombicula spp., Ornithocheyletia spp., Psorergates spp., Trombicula spp.; und aus der Ordung der Acaridida (Astigmata), zum Beispiel Acarus spp., Caloglyphus spp., Chorioptes spp., Cytodites spp., Hypodectes spp., Knemidocoptes spp., Laminosioptes spp., Notoedres spp., Otodectes spp., Psoroptes spp., Pterolichus spp., Sarcoptes spp., Trixacarus spp., Tyrophagus spp.

Zu Beispielen für parasitäre Protozoen zählen, ohne hierauf beschränkt zu sein:
Mastigophora (Flagellata), wie:
Metamonada: aus der Ordnung Diplomonadida zum Beispiel Giardia spp., Spironucleus spp.
Parabasala: aus der Ordnung Trichomonadida zum Beispiel Histomonas spp., Pentatrichomonas spp., Tetratrichomonas spp., Trichomonas spp., Tritrichomonas spp.
Euglenozoa: aus der Ordnung Trypanosomatida zum Beispiel Leishmania spp., Trypanosoma spp.

Sarcomastigophora (Rhizopoda), wie Entamoebidae, zum Beispiel Entamoeba spp., Centramoebidae, zum Beispiel Acanthamoeba sp., Euamoebidae, z. B. Hartmanella sp.

Alveolata wie Apicomplexa (Sporozoa): z. B. Cryptosporidium spp.; aus der Ordnung Eimeriida zum Beispiel Besnoitia spp., Cystoisospora spp., Eimeria spp., Hammondia spp., Isospora spp., Neospora spp., Sarcocystis spp., Toxoplasma spp.; aus der Ordnung Adeleida z. B. Hepatozoon spp., Klossiella spp.; aus der Ordnung Haemosporida z. B. Leucocytozoon spp., Plasmodium spp.; aus der Ordnung Piroplasmida z. B. Babesia spp., Ciliophora spp., Echinozoon spp., Theileria spp.; aus der Ordnung Vesibuliferida z. B. Balantidium spp., Buxtonella spp.

Microspora wie Encephalitozoon spp., Enterocytozoon spp., Globidium spp., Nosema spp., und außerdem z. B. Myxozoa spp.

Zu den für Menschen oder Tiere pathogenen Helminthen zählen zum Beispiel Acanthocephala, Nematoden, Pentastoma und Platyhelminthen (z.B. Monogenea, Cestodes und Trematodes).

Zu beispielhaften Helminthen zählen, ohne hierauf beschränkt zu sein:
Monogenea: z. B.: Dactylogyrus spp., Gyrodactylus spp., Microbothrium spp., Polystoma spp., Troglecephalus spp.;
Cestodes: aus der Ordnung Pseudophyllidea zum Beispiel: Bothridium spp., Diphyllobothrium spp., Diplogonoporus spp. Ichthyobothrium spp., Ligula spp., Schistocephalus spp., Spirometra spp.
Aus der Ordnung Cyclophyllida zum Beispiel: Andyra spp., Anoplocephala spp., Avitellina spp., Bertiella spp., Cittotaenia spp., Davainea spp., Diorchis spp., Diplopylidium spp., Dipylidium spp., Echinococcus spp., Echinocotyle spp., Echinolepis spp., Hydatigera spp., Hymenolepis spp., Joyeuxiella spp., Mesocestoides spp., Moniezia spp., Paranoplocephala spp., Raillietina spp., Stilesia spp., Taenia spp., Thysaniezia spp., Thysanosoma spp.
Trematodes: aus der Klasse Digenea zum Beispiel: Austrobilharzia spp., Brachylaima spp., Calicophoron spp., Catatropis spp., Clonorchis spp. Collyriclum spp., Cotylophoron spp., Cyclocoelum spp., Dicrocoelium spp., Diplostomum spp., Echinochasmus spp., Echinoparyphium spp., Echinostoma spp., Eurytrema spp., Fasciola spp., Fasciolides spp., Fasciolopsis spp., Fischoederius spp., Gastrothylacus spp., Gigantobilharzia spp., Gigantocotyle spp., Heterophyes spp., Hypoderaeum spp., Leucochloridium spp., Metagonimus spp., Metorchis spp., Nanophyetus spp., Notocotylus spp., Opisthorchis spp., Ornithobilharzia spp., Paragonimus spp., Paramphistomum spp., Plagiorchis spp., Posthodiplostomum spp., Prosthogonimus spp., Schistosoma spp., Trichobilharzia spp., Troglotrema spp., Typhlocoelum spp.
Acanthocephala: aus der Ordnung Oligacanthorhynchida z.B: Macracanthorhynchus spp., Prosthenorchis spp.; aus der Ordnung Moniliformida zum Beispiel: Moniliformis spp.,
Aus der Ordnung Polymorphida zum Beispiel: Filicollis spp.; aus der Ordnung Echinorhynchida zum Beispiel Acanthocephalus spp., Echinorhynchus spp., Leptorhynchoides spp.
Pentastoma: aus der Ordnung Porocephalida zum Beispiel Linguatula spp.

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch; metaphylaktisch oder therapeutisch erfolgen.

So bezieht sich eine Ausführungsform der vorliegenden Erfindung auf die Verbindungen der Formel (I) zur Verwendung als Arzneimittel.

Ein weiterer Aspekt bezieht sich auf die Verbindungen der Formel (I) zur Verwendung als Antiendoparasitikum.

Ein weiterer spezieller Aspekt betrifft die Verbindungen der Formel (I) zur Verwendung als Antihelminthikum, insbesondere zur Verwendung als Platymelminthizid, Acanthocephalizid oder Pentastomizid.

Ein weiterer spezieller Aspekt betrifft die Verbindungen der Formel (I) zur Verwendung als Antiprotozoikum.

Ein weiterer Aspekt betrifft die Verbindungen der Formel (I) zur Verwendung als Antiektoparasitikum, insbesondere ein Arthropodizid, ganz besonders ein Insektizid oder ein Akarizid.

Weitere Aspekte der Erfindung sind veterinärmedizinische Formulierungen, die eine wirksame Menge mindestens einer Verbindung der Formel (I) und mindestens einen der folgenden umfassen: einen pharmazeutisch unbedenklichen Exzipienten (z.B. feste oder flüssige Verdünnungsmittel), ein pharmazeutisch unbedenkliches Hilfsmittel (z.B. Tenside), insbesondere einen herkömmlicherweise in veterinärmedizinischen Formulierungen verwendeten pharmazeutisch unbedenklichen Exzipienten und/oder ein herkömmlicherweise in veterinärmedizinischen Formulierungen verwendetes pharmazeutisch unbedenkliches Hilfsmittel.

Ein verwandter Aspekt der Erfindung ist ein Verfahren zur Herstellung einer wie hier beschriebenen veterinärmedizinischen Formulierung, welches den Schritt des Mischens mindestens einer Verbindung der Formel (I) mit pharmazeutisch unbedenklichen Exzipienten und/oder Hilfsmitteln, insbesondere mit herkömmlicherweise in veterinärmedizinischen Formulierungen verwendeten pharmazeutisch unbedenklichen Exzipienten und/oder Hilfsmitteln umfasst.

Ein anderer spezieller Aspekt der Erfindung sind veterinärmedizinische Formulierungen ausgewählt aus der Gruppe ektoparasitizider und endoparasitizider Formulierungen, insbesondere ausgewählt aus der Gruppe anthelmintischer, antiprotozolischer und arthropodizider Formulierungen, ganz besonders ausgewählt aus der Gruppe platyhelminthizider, acanthocephalizider, pentastomizider, insektizider und akkarizider Formulierungen, gemäß den erwähnten Aspekten, sowie Verfahren zu ihrer Herstellung.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wirksamen Menge einer Verbindung der Formel (I) bei einem Tier, insbesondere einem nichthumanen Tier, das dessen bedarf.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wie hier definierten veterinärmedizinischen Formulierung bei einem Tier, insbesondere einem nichthumanen Tier, das dessen bedarf.

Ein anderer Aspekt bezieht sich auf die Verwendung der Verbindungen der Formel (I) bei der Behandlung einer Parasiteninfektion, insbesondere einer Infektion durch einen Parasiten ausgewählt aus der Gruppe der hier erwähnten Ektoparasiten und Endoparasiten, bei einem Tier, insbesondere einem nichthumanen Tier.

Im vorliegenden tiergesundheitlichen oder veterinärmedizinischen Zusammenhang schließt der Begriff "Behandlung" die prophylaktische, die metaphylaktische und die therapeutische Behandlung ein.

Bei einer bestimmten Ausführungsform werden hiermit Mischungen mindestens einer Verbindung der Formel (I) mit anderen Wirkstoffen, insbesondere mit Endo- und Ektoparasitiziden, für das veterinärmedizinische Gebiet bereitgestellt.

Auf dem Gebiet der Tiergesundheit bedeutet "Mischung" nicht nur, dass zwei (oder mehr) verschiedene Wirkstoffe in einer gemeinsamen Formulierung formuliert werden und entsprechend zusammen angewendet werden, sondern bezieht sich auch auf Produkte, die für jeden Wirkstoff getrennte Formulierungen umfassen. Dementsprechend können, wenn mehr als zwei Wirkstoffe angewendet werden sollen, alle Wirkstoffe in einer gemeinsamen Formulierung formuliert werden oder alle Wirkstoffe in getrennten Formulierungen formuliert werden; ebenfalls denkbar sind gemischte Formen, bei denen einige der Wirkstoffe gemeinsam formuliert und einige der Wirkstoffe getrennt formuliert sind. Getrennte Formulierungen erlauben die getrennte oder aufeinanderfolgende Anwendung der in Rede stehenden Wirkstoffe.

Die hier mit ihrem "Common Name" spezifizierten Wirkstoffe sind bekannt und beispielsweise im "Pesticide Manual" (siehe oben) beschrieben oder im Internet recherchierbar (z.B. http://www.alan-wood.net/pesticides).

Beispielhafte Wirkstoffe aus der Gruppe der Ektoparasitizide als Mischungspartner schließen, ohne dass dies eine Einschränkung darstellen soll, die oben ausführlich aufgelisteten Insektizide und Akarizide ein. Weitere verwendbare Wirkstoffe sind unten gemäß der oben erwähnten Klassifikation, die auf dem aktuellen IRAC Mode of Action Classification Scheme beruht, aufgeführt: (1) Acetylcholinesterase (AChE)-Inhibitoren; (2) GABA-gesteuerte Chlorid-Kanal-Blocker; (3) Natrium-Kanal-Modulatoren; (4) kompetitive Modulatoren des nicotinischen Acetylcholin-Rezeptors (nAChR); (5) allosterische Modulatoren des nicotinischen Acetylcholin-Rezeptors (nAChR); (6) allosterische Modulatoren des Glutamat-abhängigen Chloridkanals (GluCl); (7) Juvenilhormon-Mimetika; (8) verschiedene nichtspezifische (Multi-Site) Inhibitoren; (9) Modulatoren Chordotonaler Organe; (10) Milbenwachstumsinhibitoren; (12) Inhibitoren der mitochondrialen ATP-Synthase, wie ATP-Disruptoren; (13) Entkoppler der oxidativen Phosphorylierung durch Störung des Protonengradienten; (14) Blocker des nicotinischen Acetylcholinrezeptorkanals; (15) Inhibitoren der Chitinbiosynthese, Typ 0; (16) Inhibitoren der Chitinbiosynthese, Typ 1; (17) Häutungsdisruptor (insbesondere bei Dipteren, d.h. Zweiflüglern); (18) Ecdyson-Rezeptor-Agonisten; (19) Octopamin-Rezeptor-Agonisten; (21) mitochondriale Komplex-I-Elektronentransportinhibitoren; (25) mitochondriale Komplex-II-Elektronentransportinhibitoren; (20) mitochondriale Komplex-III-Elektronentransportinhibitoren; (22) Blocker des spannungsabhängigen Natriumkanals; (23) Inhibitoren der Acetyl-CoA-Carboxylase; (28) Ryanodinrezeptor-Modulatoren; (30) allosterische Modulatoren des GABA-abhängigen Chlorid-Kanals.

Wirkstoffe mit unbekannten oder nicht spezifischen Wirkmechanismen, z. B. Fentrifanil, Fenoxacrim, Cyclopren, Chlorobenzilat, Chlordimeform, Flubenzimin, Dicyclanil, Amidoflumet, Quinomethionat, Triarathen, Clothiazoben, Tetrasul, Kaliumoleat, Petroleum, Metoxadiazon, Gossyplur, Flutenzin, Brompropylat, Cryolit;
Verbindungen aus anderen Klassen, z.B. Butacarb, Dimetilan, Cloethocarb, Phosphocarb, Pirimiphos(-ethyl), Parathion(-ethyl), Methacrifos, Isopropyl-o-salicylat, Trichlorfon, Tigolaner, Sulprofos, Propaphos, Sebufos, Pyridathion, Prothoat, Dichlofenthion, Demeton-S-methylsulfon, Isazofos, Cyanofenphos, Dialifos, Carbophenothion, Autathiofos, Aromfenvinfos(-methyl), Azinphos(-ethyl), Chlorpyrifos(-ethyl), Fosmethilan, Iodofenphos, Dioxabenzofos, Formothion, Fonofos, Flupyrazofos, Fensulfothion, Etrimfos;
Organochlorverbindungen, z. B. Camphechlor, Lindan, Heptachlor; oder Phenylpyrazole, z. B. Acetoprol, Pyrafluprol, Pyriprol, Vaniliprol, Sisapronil; oder Isoxazoline, z. B. Sarolaner, Afoxolaner, Lotilaner, Fluralaner;
Pyrethroide, z. B. (cis-, trans-)Metofluthrin, Profluthrin, Flufenprox, Flubrocythrinat, Fubfenprox, Fenfluthrin, Protrifenbut, Pyresmethrin, RU15525, Terallethrin, cis-Resmethrin, Heptafluthrin, Bioethanomethrin, Biopermethrin, Fenpyrithrin, cis-Cypermethrin, cis-Permethrin, Clocythrin, Cyhalothrin (lambda-), Chlovaporthrin, oder halogenierte Kohlenwasserstoffverbindungen (HCHs),
Neonicotinoide, z. B. Nithiazin
Dicloromezotiaz, Triflumezopyrim
makrocyclische Lactone, z. B. Nemadectin, Ivermectin, Latidectin, Moxidectin, Selamectin, Eprinomectin, Doramectin, Emamectinbenzoat; Milbemycinoxim
Tripren, Epofenonan, Diofenolan;
Biologicals, Hormone oder Pheromone, zum Beispiel natürliche Produkte, z.B. Thuringiensin, Codlemon oder Neem-Komponenten
Dinitrophenole, z. B. Dinocap, Dinobuton, Binapacryl;
Benzoylharnstoffe, z. B. Fluazuron, Penfluron,
Amidinderivate, z. B. Chlormebuform, Cymiazol, Demiditraz
Bienenstockvarroa-Akarizide, zum Beispiel organische Säuren, z.B. Ameisensäure, Oxalsäure.

Zu beispielhaften Wirkstoffen aus der Gruppe der Endoparasitizide, als Mischungspartner, zählen, ohne hierauf beschränkt zu sein, anthelmintische Wirkstoffe und antiprotozoische Wirkstoffe.

Zu den anthelmintischen Wirkstoffen zählen, ohne hierauf beschränkt zu sein, die folgenden nematiziden, trematiziden und/oder cestoziden Wirkstoffe:
aus der Klasse der makrocyclischen Lactone zum Beispiel: Eprinomectin, Abamectin, Nemadectin, Moxidectin, Doramectin, Selamectin, Lepimectin, Latidectin, Milbemectin, Ivermectin, Emamectin, Milbemycin;
aus der Klasse der Benzimidazole und Probenzimidazole zum Beispiel: Oxibendazol, Mebendazol, Triclabendazol, Thiophanat, Parbendazol, Oxfendazol, Netobimin, Fenbendazol, Febantel, Thiabendazol, Cyclobendazol, Cambendazol, Albendazol-sulfoxid, Albendazol, Flubendazol;
aus der Klasse der Depsipeptide, vorzugsweise cyclischen Depsipetide, insbesondere 24-gliedrigen cyclischen Depsipeptide, zum Beispiel: Emodepsid, PF1022A;
aus der Klasse der Tetrahydropyrimidine zum Beispiel: Morantel, Pyrantel, Oxantel;
aus der Klasse der Imidazothiazole zum Beispiel: Butamisol, Levamisol, Tetramisol;
aus der Klasse der Aminophenylamidine zum Beispiel: Amidantel, deacyliertes Amidantel (dAMD), Tribendimidin;
aus der Klasse der Aminoacetonitrile zum Beispiel: Monepantel;
aus der Klasse der Paraherquamide zum Beispiel: Paraherquamid, Derquantel;
aus der Klasse der Salicylanilide zum Beispiel: Tribromsalan, Bromoxanid, Brotianid, Clioxanid, Closantel, Niclosamid, Oxyclozanid, Rafoxanid;
aus der Klasse der substituierten Phenole zum Beispiel: Nitroxynil, Bithionol, Disophenol, Hexachlorophen, Niclofolan, Meniclopholan;
aus der Klasse der Organophosphate zum Beispiel: Trichlorfon, Naphthalofos, Dichlorvos/DDVP, Crufomat, Coumaphos, Haloxon;
aus der Klasse der Piperazinone/Chinoline zum Beispiel: Praziquantel, Epsiprantel;
aus der Klasse der Piperazine zum Beispiel: Piperazin, Hydroxyzin;
aus der Klasse der Tetracycline zum Beispiel: Tetracyclin, Chlorotetracyclin, Doxycyclin, Oxytetracyclin, Rolitetracyclin;
aus diversen anderen Klassen zum Beispiel: Bunamidin, Niridazol, Resorantel, Omphalotin, Oltipraz, Nitroscanat, Nitroxynil, Oxamniquin, Mirasan, Miracil, Lucanthon, Hycanthon, Hetolin, Emetin, Diethylcarbamazin, Dichlorophen, Diamfenetid, Clonazepam, Bephenium, Amoscanat, Clorsulon.

Antiprotozoische Wirkstoffe, darunter, ohne hierauf beschränkt zu sein, die folgenden Wirkstoffe:
aus der Klasse der Triazine zum Beispiel: Diclazuril, Ponazuril, Letrazuril, Toltrazuril;
aus der Klasse Polyletherionophor zum Beispiel: Monensin, Salinomycin, Maduramicin, Narasin;
aus der Klasse der makrocyclischen Lactone zum Beispiel: Milbemycin, Erythromycin;
aus der Klasse der Chinolone zum Beispiel: Enrofloxacin, Pradofloxacin;
aus der Klasse der Chinine zum Beispiel: Chloroquin;
aus der Klasse der Pyrimidine zum Beispiel: Pyrimethamin;
aus der Klasse der Sulfonamide zum Beispiel: Sulfachinoxalin, Trimethoprim, Sulfaclozin;
aus der Klasse der Thiamine zum Beispiel: Amprolium;
aus der Klasse der Lincosamide zum Beispiel: Clindamycin;
aus der Klasse der Carbanilide zum Beispiel: Imidocarb;
aus der Klasse der Nitrofurane zum Beispiel: Nifurtimox;
aus der Klasse der Chinazolinonalkaloide zum Beispiel: Halofuginon;
aus diversen anderen Klassen zum Beispiel: Oxamniquin, Paromomycin;
aus der Klasse der Vakzine oder Antigene aus Mikroorganismen zum Beispiel: Babesia canis rossi, Eimeria tenella, Eimeria praecox, Eimeria necatrix, Eimeria mitis, Eimeria maxima, Eimeria brunetti, Eimeria acervulina, Babesia canis vogeli, Leishmania infantum, Babesia canis canis, Dictyocaulus viviparus.

Alle genannten Mischungspartner können außerdem, wenn sie auf Grund ihrer funktionellen Gruppen dazu imstande sind, gegebenenfalls mit geeigneten Basen oder Säuren Salze bilden.

### Vektorbekämpfung

Die Verbindungen der Formel (I) können auch in der Vektorbekämpfung eingesetzt werden. Ein Vektor im Sinne der vorliegenden Erfindung ist ein Arthropode, insbesondere ein Insekt oder Arachnid, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z. B. Trachoma durch nicht-stechende Fliegen) auf einen Wirt, oder nach Injektion (z. B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, weitere virale Erkrankungen, Filariasis, Übertragung von anderen Würmern;
   - Aedes: Gelbfieber, Dengue-Fieber, weitere virale Erkrankungen, Filariasis;
   - Simulien: Übertragung von Würmern, insbesondere Onchocerca volvulus;
   - Psychodidae: Übertragung von Leishmaniose
2) Läuse: Hautinfektionen, epidemisches Fleckfieber;
3) Flöhe: Pest, endemisches Fleckfieber, Bandwürmer;
4) Fliegen: Schlafkrankheit (Trypanosomiasis); Cholera, weitere bakterielle Erkrankungen;
5) Milben: Acariose, epidemisches Fleckfieber, Rickettsipocken, Tularämie, Saint-Louis-Enzephalitis, Frühsommer-Meningoenzephalitis (FSME), hämorrhagisches Krim-Kongo-Fieber, Borreliose;
6) Zecken: Borelliosen wie Borrelia bungdorferi sensu lato., Borrelia duttoni, Frühsommer-Meningoenzephalitis, Q-Fieber (Coxiella burnetii), Babesien (Babesia canis canis), Ehrlichiose.

Beispiele für Vektoren im Sinne der vorliegenden Erfindung sind Insekten, zum Beispiel Aphiden, Fliegen, Zikaden oder Thripse, die Pflanzenviren auf Pflanzen übertragen können. Weitere Vektoren, die Pflanzenviren übertragen können, sind Spinnmilben, Läuse und Käfer.

Weitere Beispiele für Vektoren im Sinne der vorliegenden Erfindung sind Insekten und Arachniden wie Mücken, insbesondere der Gattungen Aedes, Anopheles, z. B. A. gambiae, A. arabiensis, A. funestus, A. dirus (Malaria) und Culex, Psychodide wie Phlebotomus, Lutzomyia, Läuse, Flöhe, Fliegen, Milben und Zecken, die Krankheitserreger auf Tiere und/oder Menschen übertragen können.

Eine Vektorbekämpfung ist auch möglich, wenn die Verbindungen der Formel (I) Resistenz-brechend sind.

Verbindungen der Formel (I) sind zur Verwendung in der Prävention von Krankheiten und/oder Krankheitserregern, die durch Vektoren übertragen werden, geeignet. Somit ist ein weiterer Aspekt der vorliegenden Erfindung die Verwendung von Verbindungen der Formel (I) zur Vektorbekämpfung, z. B. in der Landwirtschaft, im Gartenbau, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z. B. aus den Ordnungen Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d. h., sie können ohne weitere Änderungen auf das entsprechende Material aufgebracht werden. Als weitere Insektizide oder Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Kontakt kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren, Zecken und Milben, die in geschlossenen Räumen, wie beispielsweise Wohnungen, Fabrikhallen, Büros, Fahrzeugkabinen, Tierzuchtanlagen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z. B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

Die folgenden Herstellungs- und Verwendungsbeispiele illustrieren die Erfindung ohne sie zu beschränken.

### Herstellungsbeispiele

### Synthesebeispiel 1: 3-(2-Isopropylphenyl)-2-[[4-[1-methyl-5-[4-(trifluoromethoxy)anilino]-1,2,4-triazol-3-yl]phenyl]methylenehydrazono]thiazolidin-4-on (I-002)

### Herstellung von 3,5-Dibromo-1-methyl-1,2,4-triazol (IM-1-01) (Verfahren J)

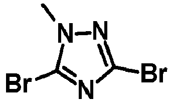

3,5-Dibrom-1H-1,2,4-triazol (10,00 g, 44,08 mmol), Kaliumcarbonat (12,18 g, 88,16 mmol) wurden in DMF(100 mL) vorgelegt und Methyljodid (8,13 g 57,3 mmol) zugesetzt. Anschließend wurde 2 h bei Raumtemperatur gerührt. Das Reaktionsgemisch auf Wasser gegossen , mehrfach mit Essigsäureethylester extrahiert und die organische Phase nach Trocknen über Magnesiumsulfat unter vermindertem Druck eingedampt. Dieser Rückstand wurde mit Wasser versetzt und der ausgefallenene Feststoff abfiltiert. Es wurden 6,65 g 3,5-Dibromo-1-methyl-1,2,4-triazol erhalten.
¹H-NMR siehe Tabelle 2

Weitere in Analogie zu (IM-1-01) hergestellte Verbindungen sind in Tabelle 2 aufgeführt.

### Herstellung von 5-Bromo-2-methyl-N-[4-(trifluoromethoxy)phenyl]-1,2,4-triazol-3-amin (IM-2-01) (Verfahren I)

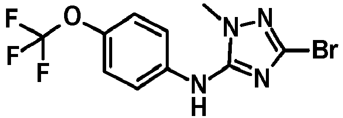

Eine Mischung aus 3,5-Dibromo-1-methyl-1,2,4-triazole (400 mg 1,661mmol) und 4-(Trifluoromethoxy)anilin (747 mg 4,218 mmol) in THF (5ml) vorgelegt. Bei 0°C mit Lithium trimethyl-N-(trimethylsilyl)silanaminide (322 mg 3,354 mmol) versetzt. Anschließend wurde 16h bei Raumtemperatur gerührt. Das Reaktionsgemisch wurde in Ammoniumchlorid-Lösung gegossen und mit Dichlormethan verrührt, über eine Chromabond^{™} PTS Trennsäule filtriert. Das Rohprodukt wurde an Kieselgel chromatographisch gereinigt (Gradient: Hexan/Essigsäureethylester). Es wurden 175 mg 5-Bromo-2-methyl-N-[4-(trifluoromethoxy)phenyl]-1,2,4-triazol-3-amin erhalten.
¹H-NMR siehe Tabelle 3

Weitere in Analogie zu (IM-2-01) hergestellte Verbindungen sind in Tabelle 3 aufgeführt.

### Herstellung von 4-[1-Methyl-5-[4-(trifluoromethoxy)anilino]-1,2,4-triazol-3-yl]benzaldehyde(IM-3-01) (Verfahren G)

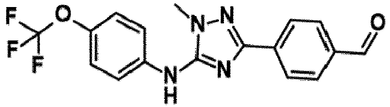

Eine Mischung aus 5-Bromo-2-methyl-N-[4-(trifluoromethoxy)phenyl]-1,2,4-triazol-3-amin (200 mg, 0,593 mmol), 4-Formylphenylboronsäurepinakolester (206,5 mg, 0,89 mmol), Cäsiumcarbonat (580 mg, 1,78 mmol), Dioxan (6 mL) und Wasser (2 mL) wurde unter Argon entgast und mit 1,1'-Bis(diphenylphosphino)ferrocen-palladium(II)dichlorid (29 mg, 0,036 mmol) versetzt. Anschließend wurde 3 h bei 90 °C gerührt. Das Reaktionsgemisch wurde über Kieselgur filtriert, mit Dichlormethan nachgewaschen und das Filtrat mit Natriumchlorid-Lösung verrührt, über eine Chromabond^{™} PTS Trennsäule filtriert unter vermindertem Druck eingedampft. Das Rohprodukt wurde an Kieselgel chromatographisch gereinigt (Gradient: Hexan/Essigsäureethylester). Es wurden 193 mg 4-[1-Methyl-5-[4-(trifluoromethoxy)anilino]-1,2,4-triazol-3-yl]benzaldehyd erhalten.
¹H-NMR siehe Tabelle 4

Weitere in Analogie zu (IM-3-01) hergestellte Verbindungen sind in Tabelle 4 aufgeführt.

### Herstellung von 1-(2-Isopropylphenyl)-3-[[4-[1-methyl-5-[4-(trifluoromethoxy)anilino]-1,2,4-triazol-3-yl]phenyl]methyleneamino]thioharnstoff (I-003) (Verfahren A)

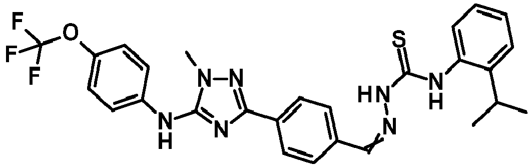

Eine Mischung aus 4-[1-Methyl-5-[4-(trifluoromethoxy)anilino]-1,2,4-triazol-3-yl]benzaldehyd (350 mg, 0,966 mmol) und N-(2-Isopropylphenyl)hydrazincarbothioamid (202 mg, 0,966 mmol) (bekannt aus WO 2010/062559) in Ethanol (30 mL) wurde bei 75°C 16 h gerührt. Das Reaktionsgemisch wurde auf Wasser geschüttet und der ausgefallenene Feststoff abfiltiert. Das Rohprodukt wurde an Kieselgel chromatographisch gereinigt (Gradient: Hexan/Essigsäureethylester). Es wurden 296 mg 1-(2-Isopropylphenyl)-3-[[rac-(1S)-4-[5-(methylamino)-1-[4-(trifluoromethoxy)phenyl]-1,2,4-triazol-3-yl]cyclohex-3-en-1 -yl]methyleneamino]thioharnstoff erhalten.
¹H-NMR siehe Tabelle 1

### Herstellung von 3-(2-Isopropylphenyl)-2-[[4-[1-methyl-5-[4-(trifluoromethoxy)anilino]-1,2,4-triazol-3-yl]phenyl]methylenehydrazono]thiazolidin-4-on (I-002) (Verfahren C)

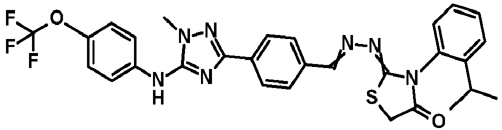

1-(2-Isopropylphenyl)-3-[[4-[1-methyl-5-[4-(trifluoromethoxy)anilino]-1,2,4-triazol-3-yl]phenyl]methyleneamino]thioharnstoff (150 mg, 0,271 mmol) wurde in Ethanol (10 mL) vorgelegt, Natriumacetat (89 mg, 1,084 mmol) zugegeben, mit Bromessigsäuremethylester (50 mg, 0,325 mmol) versetzt und anschließend 3 h bei 75 °C gerührt. Das Reaktionsgemisch wurde auf Wasser geschüttet und der ausgefallenene Feststoff abfiltiert. Es wurden 138 mg 3-(2-Isopropylphenyl)-2-[[4-[1-methyl-5-[4-(trifluoromethoxy)anilino]-1,2,4-triazol-3-yl]phenyl]methylenehydrazono]thiazolidin-4-on erhalten.
¹H-NMR siehe Tabelle 1

### Synthesebeispiel 2: 3-(2-Isopropylphenyl)-2-[[4-[1-methyl-5-[4-(trifluoromethyl)-1-piperidyl]-1,2,4-triazol-3-yl]phenyl]methylenehydrazono]thiazolidin-4-on (I-045)

### Herstellung von 1-(5-Bromo-2-methyl-1,2,4-triazol-3-yl)-4-(trifluoromethyl)piperidin (IM-2-06) (Verfahren I)

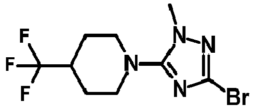

3,5-Dibromo-1-methyl-1,2,4-triazole (500 mg, 2,076 mmol) wurde in Dimethylacetamid (4 mL) gelöst und mit 4-(Trifluoromethyl)piperidine (954 mg, 6,227 mmol) versetzt. Anschließend wurde 16 h bei 120°C gerührt. Das Reaktionsgemisch wurde mit Acetonitril verdünnt und an Reverse Phase (RP)-chromatographiert (Gradient: Wasser/Acetonitril).Es wurden 537 mg 1-(5-Bromo-2-methyl-1,2,4-triazol-3-yl)-4-(trifluoromethyl)piperidine erhalten.
¹H-NMR siehe Tabelle 3

Weitere in Analogie zu (IM-2-02) hergestellte Verbindungen sind in Tabelle 3 aufgeführt.

### Herstellung von 4-[1-Methyl-5-[4-(trifluoromethyl)-1-piperidyl]-1,2,4-triazol-3-yl]benzaldehyd (IM-3-14) (Verfahren G)

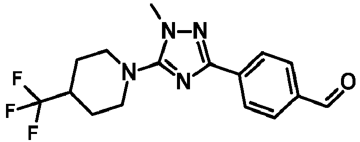

Eine Mischung aus 1-(5-Bromo-2-methyl-1,2,4-triazol-3-yl)-4-(trifluoromethyl)piperidine (500 mg, 1,597 mmol), 4-Formylphenylboronsäurepinakolester (556 mg, 2,395 mmol), Cäsiumcarbonat (1,56 g, 4,791 mmol), Dioxan (12 mL) und Wasser (4 mL) wurde unter Argon entgast und mit 1,1'-Bis(diphenylphosphino)ferrocen-palladium(II)dichlorid (78,2mg, 0,096 mmol) versetzt. Anschließend wurde 3 h bei 90 °C gerührt. Das Reaktionsgemisch wurde über Kieselgur filtriert, mit Dichlormethan nachgewaschen und das Filtrat mit Natriumchlorid-Lösung verrührt, über eine Chromabond^{™} PTS Trennsäule filtriert unter vermindertem Druck eingedampft. Das Rohprodukt wurde an Kieselgel chromatographisch gereinigt (Gradient: Hexan/Essigsäureethylester). Es wurden 522 mg 4-[1-Methyl-5-[4-(trifluoromethyl)-1-piperidyl]-1,2,4-triazol-3-yl]benzaldehyd erhalten.
¹H-NMR siehe Tabelle 4

Weitere in Analogie zu (IM-3-01) hergestellte Verbindungen sind in Tabelle 4 aufgeführt.

### Herstellung von 1-(2-Isopropylphenyl)-3-[[4-[1-methyl-5-[4-(trifluoromethyl)-1-piperidyl]-1,2,4-triazol-3-yl]phenyl]methyleneamino]thioharnstoff (I-042) (Verfahren A)

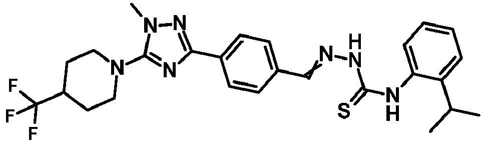

Eine Mischung aus 4-[1-Methyl-5-[4-(trifluoromethyl)-1-piperidyl]-1,2,4-triazol-3-yl]benzaldehyde (240mg, 0,709 mmol) und N-(2-Isopropylphenyl)hydrazincarbothioamid (148,5 mg, 0,709 mmol) (bekannt aus WO 2010/062559) in Ethanol (8 mL) wurde bei 75°C 4 h gerührt. Der Feststoff wurde abfiltiert, wobei 260 mg 1-(2-Isopropylphenyl)-3-[[4-[1-methyl-5-[4-(trifluoromethyl)-1-piperidyl]-1,2,4-triazol-3-yl]phenyl]methyleneamino]thioharnstoff erhalten wurden.
¹H-NMR siehe Tabelle 1

### Herstellung von 3-(2-Isopropylphenyl)-2-[[4-[1-methyl-5-[4-(trifluoromethyl)-1-piperidyl]-1,2,4-triazol-3-yl]phenyl]methylenehydrazono]thiazolidin-4-on (I-045) (Verfahren C)

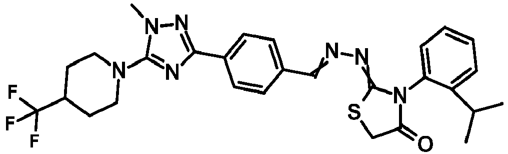

1-(2-Isopropylphenyl)-3-[[4-[1-methyl-5-[4-(trifluoromethyl)-1-piperidyl]-1,2,4-triazol-3-yl]phenyl]methyleneamino]thioharnstoff (195 mg, 0,368 mmol) wurde in Ethanol (7 mL) vorgelegt, Natriumacetat (121 mg, 1,473 mmol) zugegeben, mit Bromessigsäuremethylester (68 mg, 0,442 mmol) versetzt und anschließend 3 h bei 75 °C gerührt. Der Feststoff wurde abfiltiert, wobei 166 mg 3-(2-Isopropylphenyl)-2-[[4-[1-methyl-5-[4-(trifluoromethyl)-1-piperidyl]-1,2,4-triazol-3-yl]phenyl]methylenehydrazono]thiazolidin-4-on erhalten wurden.
¹H-NMR siehe Tabelle 1

### Synthesebeispiel 3: 1-[3-(2-isopropylphenyl)-4-oxo-thiazolidin-2-ylidenel-3-[4-[1-methyl-5-[4-(trifluoromethyl)-1-piperidyl]-1,2,4-triazol-3-yl]phenyl]harnstoff (I-001)

### 4-[1-Methyl-5-[4-(trifluoromethyl)-1-piperidyl]-1,2,4-triazol-3-yl]anilin (IM-4-01) (Verfahren H)

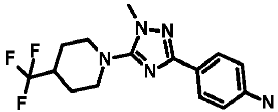

Eine Mischung aus 1-(5-Bromo-2-methyl-1,2,4-triazol-3-yl)-4-(trifluoromethyl)piperidine (IM-2-06) (1000 mg, 3,190 mmol), 4-Aminobenzolboronsäurepinakolester (1049 mg, 4,790 mmol), Cäsiumcarbonat (3,12 g, 9,58 mmol), Dioxan (24 mL) und Wasser (8 mL) wurde unter Argon entgast und mit 1,1'-Bis(diphenylphosphino)ferrocen-palladium(II)dichlorid (156 mg, 0,19 mmol) versetzt. Anschließend wurde 3 h bei 90 °C gerührt. Das Reaktionsgemisch wurde über Kieselgur filtriert, mit Dichlormethan nachgewaschen, das Filtrat mit Natriumchlorid-Lösung verrührt, über eine Chromabond^{™} PTS Trennsäule filtriert und unter vermindertem Druck eingedampft. Das Rohprodukt wurde an Kieselgel chromatographisch gereinigt (Gradient: Hexan/Essigsäureethylester), wobei 1040 mg 4-[1-Methyl-5-[4-(trifluoromethyl)-1-piperidyl]-1,2,4-triazol-3-yl]anilin erhalten wurden.
¹H-NMR siehe Tabelle 5

Weitere in Analogie zu (IM-4-01) hergestellte Verbindungen sind in Tabelle 4 aufgeführt.

### Herstellung von 1-[3-(2-isopropylphenyl)-4-oxo-thiazolidin-2-ylidene]-3-[4-[1-methyl-5-[4-(trifluoromethyl)-1-piperidyl]-1,2,4-triazol-3-yl]phenyl]harnstoff (I-001) (Verfahren E)

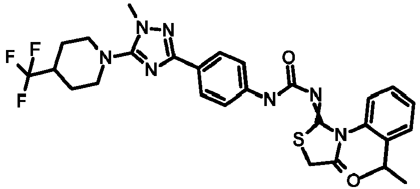

### Stufe 1:

4-[1-methyl-5-[4-(trifluoromethyl)-1-piperidyl]-1,2,4-triazol-3-yl]anilin (IM-4-01) (1000 mg, 3,07 mmol) wurde in THF (2,5 mL) vorgelegt und mit einer Lösung von Chlorameisensäure-4-nitrophenylester (681 mg, 3,38 mmol) in THF (20 mL) versetzt. Nach 16 h Rühren bei Raumtemperatur wurde Hexan (5 mL) zugesetzt und der ausgefallene Feststoff abgesaugt, wobei 1510 mg (4-Nitrophenyl) N-[4-[1-methyl-5-[4-(trifluoromethyl)-1-piperidyl]-1,2,4-triazol-3-yl]phenyl]carbamat erhalten wurden, das ohne weitere Reinigung in Stufe 2 eingesetzt wurde.

### Stufe 2:

2-Imino-3-(2-isopropylphenyl)thiazolidin-4-on (83,6 mg, 0,35 mmol) (bekannt aus WO2016/033025) wurde in 6 mL Acetonitril vorgelegt und anschließen zunächst Cäsiumcarbonat (174 mg, 0,53 mmol) und dann (4-Nitrophenyl) N-[4-[1-methyl-5-[4-(trifluoromethyl)-1-piperidyl]-1,2,4-triazol-3-yl]phenyl]carbamat aus Stufe 1 (175 mg, 0,35 mmol) zugesetzt. Nach 4 h Rühren bei 50 °C wurde das das Reaktionsgemisch über Celite filtriert und mit Dichlormethan nachgewaschen. Der Rückstand wurde an Kieselgel chromatographiert (Gradient: Dichlormethan/Essigsäureethylester). Es wurden 82 mg 1-[3-(2-Isopropylphenyl)-4-oxo-thiazolidin-2-ylidene]-3-[4-[1-methyl-5-[4-(trifluoromethyl)-1-piperidyl]-1,2,4-triazol-3-yl]phenyl]harnstoff erhalten.
¹H-NMR siehe Tabelle 1

### Synthesebeispiel 4: 3-(2-Isopropylphenyl)-2-[[4-[1-methyl-5-(3,3,3-trifluoropropylamino)-1,2,4-triazol-3-yl]phenyl]methylenehydrazono]thiazolidin-4-on (I-048)

### Herstellung von 5-Bromo-2-methyl-N-(3,3,3-trifluoropropyl)-1,2,4-triazol-3-amin (IM-2-13) (Verfahren I)

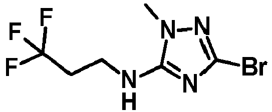

3,5-Dibromo-1-methyl-1,2,4-triazole (400 mg, 1,661 mmol) wurde in Dimethylacetamid (3 mL) gelöst und mit 3,3,3-Trifluoropropan-1-amine (488 mg, 4,317 mmol) versetzt. Anschließend wurde 16 h bei 120 °C gerührt. Das Reaktionsgemisch wurde mit Acetonitril verdünnt und an Reverse Phase (RP)-chromatographiert (Gradient: Wasser/Acetonitril). Es wurden 384 mg 5-Bromo-2-methyl-N-(3,3,3-trifluoropropyl)-1,2,4-triazol-3-amin erhalten.
¹H-NMR siehe Tabelle 3

Weitere in Analogie zu (IM-2-13) hergestellte Verbindungen sind in Tabelle 3 aufgeführt.

### Herstellung von 4-[1-Methyl-5-(3,3,3-trifluoropropylamino)-1,2,4-triazol-3-yl]benzaldehyde(IM-3-12) (Verfahren G)

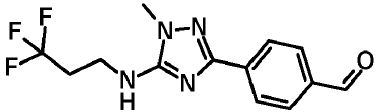

Eine Mischung aus 5-Bromo-2-methyl-N-(3,3,3-trifluoropropyl)-1,2,4-triazol-3-amine (350 mg, 1,282 mmol), 4-Formylphenylboronsäurepinakolester (446 mg, 1,923mmol), Cäsiumcarbonat (1,25 g, 3,845 mmol), Dioxan (9 mL) und Wasser (3 mL) wurde unter Argon entgast und mit 1,1'-Bis(diphenylphosphino)ferrocen-palladium(II)dichlorid (63mg, 0,077 mmol) versetzt. Anschließend wurde 3 h bei 90 °C gerührt. Das Reaktionsgemisch wurde über Kieselgur filtriert, mit Dichlormethan nachgewaschen und das Filtrat mit Natriumchlorid-Lösung verrührt, über eine Chromabond^{™} PTS Trennsäule filtriert unter vermindertem Druck eingedampft. Das Rohprodukt wurde an Kieselgel chromatographisch gereinigt (Gradient: Hexan/Essigsäureethylester). Es wurden 275 mg 4-[1-Methyl-5-(3,3,3-trifluoropropylamino)-1,2,4-triazol-3-yl]benzaldehyd erhalten.
¹H-NMR siehe Tabelle 4

Weitere in Analogie zu (IM-3-12) hergestellte Verbindungen sind in Tabelle 4 aufgeführt.

### Herstellung von 1-(2-Isopropylphenyl)-3-[[4-[1-methyl-5-(3,3,3-trifluoropropylamino)-1,2,4-triazol-3-yl]phenyl]methyleneamino]thioharnstoff (I-040, Verfahren A)

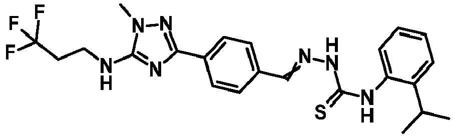

Eine Mischung aus 4-[1-Methyl-5-(3,3,3-trifluoropropylamino)-1,2,4-triazol-3-yl]benzaldehyd (245mg, 0,821 mmol) und N-(2-Isopropylphenyl)hydrazincarbothioamid (172 mg, 0,821 mmol) (bekannt aus WO 2010/062559) in Ethanol (8 mL) wurde bei 75°C 4 h gerührt. Der Feststoff wurde abfiltiert, wobei 260 mg 1-(2-Isopropylphenyl)-3-[[4-[1-methyl-5-[4-(trifluoromethyl)-1-piperidyl]-1,2,4-triazol-3-yl]phenyl]methylenamino]thioharnstoff erhalten wurden.
¹H-NMR siehe Tabelle 1

### Herstellung von 3-(2-Isopropylphenyl)-2-[[4-[1-methyl-5-(3,3,3-trifluoropropylamino)-1,2,4-triazol-3-yl]phenyl]methylenehydrazono]thiazolidin-4-on (I-048) (Verfahren C)

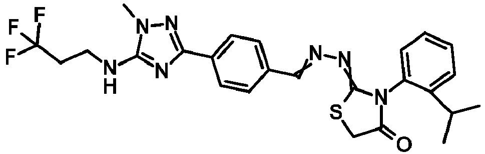

1-(2-Isopropylphenyl)-3-[[4-[1-methyl-5-[4-(trifluoromethyl)-1-piperidyl]-1,2,4-triazol-3-yl]phenyl]methyleneamino]thioharnstoff (224 mg, 0,457 mmol) wurde in Ethanol (9,2 mL) vorgelegt, Natriumacetat (150 mg, 1,473 mmol) zugegeben, mit Bromessigsäuremethylester (84 mg, 0,442 mmol) versetzt und anschließend 4 h bei 75 °C gerührt. Der Feststoff wurde abfiltiert, wobei 178mg 3-(2-Isopropylphenyl)-2-[[4-[1-methyl-5-(3,3,3-trifluoropropylamino)-1,2,4-triazol-3-yl]phenyl]methylenehydrazono]thiazolidin-4-on erhalten wurden.
¹H-NMR siehe Tabelle 1

### Synthesebeispiel 5: 3-(2-Isopropylphenyl)-2-[[4-[1-methyl-5-(3,3,3-trifluoropropylamino)-1,2,4-triazol-3-yl]phenyl]methylenehydrazono]thiazolidin-4-on (I-083)

### Herstellung von N-(5-Bromo-2-methyl-1,2,4-triazol-3-yl)-N-[4-(trifluoromethoxy)phenyl]acetamid

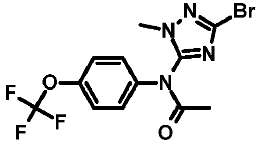

5-Bromo-2-methyl-N-[4-(trifluoromethoxy)phenyl]-1,2,4-triazol-3-amin (IM-2-01) (337 mg, 1,00 mmol) wurde in Acetanhydrid (1,5 mL, 15,8 mmol)) gelöst und anschließend 16 h bei 130°C gerührt. Das Reaktionsgemisch wurde in 10%ige Natriumhydrogencarbonatlösung (25 mL) gegossen, mit Dichlormethan extrahiert (2x 25 mL), die vereinigten organischen Phasen über eine Chromabond^{™} PTS Trennsäule filtriert und unter vermindertem Druck eingedampft. Man erhielt 390 mg N-(5-Bromo-2-methyl-1,2,4-triazol-3-yl)-N-[4-(trifluoromethoxy)phenyl]acetamid.
¹H-NMR (400 MHz, d₆-DMSO): δ 2.50 (s, 3H), 3.83 (s, 3H), 7.49 (m, 2H), 7.68 (m, 2H).

### Herstellung von N-[5-(4-Formylphenyl)-2-methyl-1,2,4-triazol-3-yl]-N-[4-(trifluoromethoxy)phenyl]acetamid (IM-3-07)

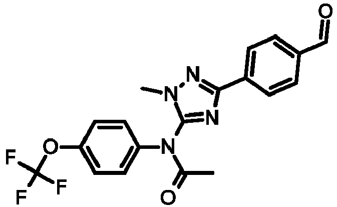

Eine Mischung aus N-(5-Bromo-2-methyl-1,2,4-triazol-3-yl)-N-[4-(trifluoromethoxy)phenyl]acetamid (350 mg, 0,920 mmol), 4-Formylphenylboronsäurepinakolester (385 mg, 1,66 mmol), Kaliumcarbonat (391 mg, 1,84 mmol), Dioxan (15 mL) undWasser(5 mL) wurde unter Argon entgast und mit 1,1'-Bis(diphenylphosphino)ferrocen-palladium(II)dichlorid (29 mg, 0,04 mmol) versetzt. Anschließend wurde 5 h bei 90 °C gerührt. Das Reaktionsgemisch wurde über Kieselgur filtriert, mit Dichlormethan nachgewaschen, das Filtrat mit Natriumchlorid-Lösung verrührt, über eine Chromabond^{™} PTS Trennsäule filtriert und unter vermindertem Druck eingedampft. Das Rohprodukt wurde an Kieselgel chromatographiert (Gradient: Hexan/Essigsäureethylester), wobei 233 mg N-[5-(4-Formylphenyl)-2-methyl-1,2,4-triazol-3-yl]-N-[4-(trifluoromethoxy)phenyl]acetamid erhalten wurden.
¹H-NMR siehe Tabelle 4

### Herstellung von N-[5-[4-[[(2-Isopropylphenyl)carbamothioylhydrazono]methyl]phenyl]-2-methyl-1,2,4-triazol-3-yl]-N-[4-(trifluoromethoxy)phenyl]acetamid (I-080)

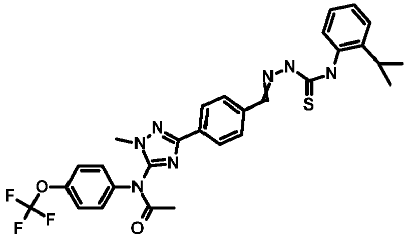

Eine Mischung aus N-[5-(4-Formylphenyl)-2-methyl-1,2,4-triazol-3-yl]-N-[4-(trifluoromethoxy)phenyl]acetamid (200 mg, 0,4900 mmol) und N-(2-Isopropylphenyl)hydrazincarbothioamid (103 mg, 0,490 mmol) (bekannt aus WO 2010/062559) in Ethanol (8 mL) wurde bei 75°C 4 h gerührt. Das Reaktionsgemisch wurde auf Wasser geschüttet und der ausgefallenene Feststoff abfiltiert. Es wurden 216 mg N-[5-[4-[[(2-Isopropylphenyl)carbamothioylhydrazono]methyl]phenyl]-2-methyl-1,2,4-triazol-3-yl]-N-[4-(trifluoromethoxy)phenyl] acetamid erhalten.
1H-NMR siehe Tabelle 1

### Herstellung von N-[5-[4-[[[3-(2-Isopropylphenyl)-4-oxo-thiazolidin-2-ylidene]hydrazono]methyl]phenyl]-2-methyl-1,2,4-triazol-3-yl]-N-[4-(trifluoromethoxy)phenyl]acetamid (I-083)

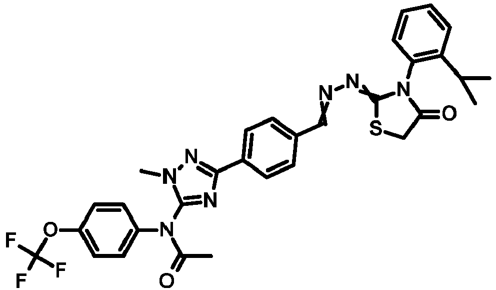

N-[5-[4-[[(2-Isopropylphenyl)carbamothioylhydrazono]methyl]phenyl]-2-methyl-1,2,4-triazol-3-yl]-N-[4-(trifluoromethoxy)phenyl]acetamid (140 mg, 0,230 mmol) wurde in Ethanol (9 mL) vorgelegt, Natriumacetat (77 mg, 0,940 mmol) zugegeben, mit Bromessigsäuremethylester (43 mg, 0,280 mmol) versetzt und anschließend 4 h bei 75 °C gerührt. Das Reaktionsgemisch wurde auf Wasser geschüttet und der ausgefallenene Feststoff abfiltiert, mit Wasser gewaschen und getrocknet. Es wurden 103 mg N-[5-[4-[[[3-(2-Isopropylphenyl)-4-oxo-thiazolidin-2-ylidene]hydrazono]methyl]phenyl]-2-methyl-1,2,4-triazol-3-yl]-N-[4-(trifluoromethoxy)phenyl]acetamid erhalten.
¹H-NMR siehe Tabelle 1

### Synthesebeispiel 6: 3-(2-Isopropylphenyl)-2-[[4-[1-methyl-5-(3,3,3-trifluoropropylamino)-1,2,4-triazol-3-yl]phenyl]methylenehydrazono]thiazolidin-4-on (I-020)

### Herstellung von Methyl 4-(5-amino-1-methyl-pyrazol-3-yl)benzoat (Verfahren P)

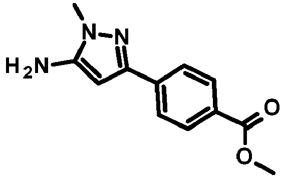

Ein Gemisch aus 4-(4,4,4,5,5-Pentamethyl-1,3,2-dioxaborolan-2-yl)benzoat (3,50 g, 1,.63 mmol), 5-Brom-2-methylpyrazol-3-amin (3,33 g, 1,.94 mmol), Pd(PPh₃)₂Cl₂ (886,40 mg, 1.26 mmol) und Cs₂CO₃ (8,23 g, 25,26 mmol) in Dioxan (40.00 mL) wurde über Nacht bei 90°C gerührt. Das Reaktionsgemisch wurde mit Wasser (200 mL) verdünnt und mit Ethylacetat extrahiert (2 x 100 mL). Die vereinigten organischen Phasen wurden mit gesättigter Kochsalzlösung gewaschen (2 x 100 mL) und über Natriumsulfat getrocknet. Nach Abdestillieren des Lösungsmittels unter vermindertem Druck wurde der Rückstand an Kieselgel chromatographiert (Petrolether/Ethylacetat 1:1) wobei 2,40 g Methyl 4-(5-amino-1-methylpyrazol-3-yl)benzoat erhalten wurden.
¹H-NMR (400 MHz, d₆-DMSO): δ 3.60 (s, 3H), 3.85 (s, 3H), 5.35 (s, 2H), 7.79 (d, 2H), 7.92 (d, 2H).

### Herstellung von Methyl 4-[1-methyl-5-[4-(trifluoromethoxy)anilino]pyrazol-3-yl]benzoat (IM-5-01, Verfahren P)

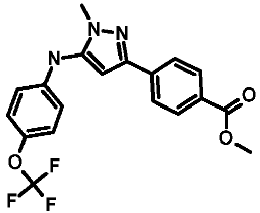

Eine Mischung aus Methyl 4-(5-amino-1-methylpyrazol-3-yl)benzoat (700,00 mg, 3,03 mmol), 1-Iodo-4-(trifluoromethoxy)benzol (1,75 g, 6,05 mmol), Pd₂(dba)₃.CHCl₃ (156,7 mg, 0,15 mmol), XantPhos (175,15 mg, 0.30 mmol) and Cs₂CO₃ (2,00 g, 6,05 mmol) in Dioxane (15,00 mL) wurde unter Stickstoffatmosphäre 4 Stunden bei 20 °C gerührt Das Reaktionsgemisch wurde mit Wasser verdünnt (100 mL), anschließend mit Ethylacetat extrahiert (2 x 50 mL) und die vereinigten organischen Phasen über Natriumsulfat getrocknet. Nach Abdestillieren des Lösungsmittels unter vermindertem Druck wurde der Rückstand an Kieselgel chromatographiert (Petrolether/Ethylacetat 2:1) wobei 2,05 g Methyl 4-[1-methyl-5-[4-(trifluoromethoxy)anilino]pyrazol-3-yl]benzoat erhalten wurden.
¹H-NMR siehe Tabelle 5

Weitere in Analogie zu (IM-5-01) hergestellte Verbindungen sind in Tabelle 5 aufgeführt.

### Herstellung von 4-[1-Methyl-5-[4-(trifluoromethoxy)anilino]pyrazol-3-yl]benzaldehyd (IM-6-01, Verfahren P)

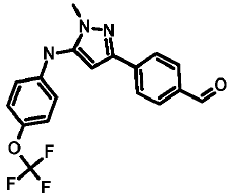

### Stufe 1:

Methyl 4-(1-methyl-5-[[3-(trifluoromethoxy)phenyl]amino]pyrazol-3-yl)benzoat (1.2 g, 3.04 mmol) wurde in THF (50.00 mL) unter Stickstoffathosphäre vorgelegt und bei 65°C DIBAL-H (1.31 g, 9.20 mmol) tropfenweise hinzugefügt. Das Reaktionsgemisch wurde 1,5h bei 65°C gerührt und anschließend durch Zusatz von gesättigter wässriger NH₄Cl Lösung (100 mL) gequencht. Das Gemisch wurde mit Ethylacetat extrahiert(2 x 80 mL), mit gesättigter Kochsallösung gewaschen (2x 50 mL) und die vereinigten organischen Phasen über Natriumsulfat getrocknet. Nach Abdestillieren des Lösungsmittels unter vermindertem Druck wurde der Rückstand an Kieselgel chromatographiert (Petrolether/Ethylacetat 1:1) wobei 960 mg [4-(1-Methyl-5-[[4-(trifluoromethoxy)phenyl]amino]pyrazol-3-yl)phenyl]methanol erhalten wurden.

### Stufe 2:

[4-(1-Methyl-5-[[4-(trifluoromethoxy)phenyl]amino]pyrazol-3-yl)phenyl]methanol (550,00 mg, 1,51 mmol) wurde in Dichlormethan (20,00 mL) vorgelegt und bei 25 °C Dess-Martin Reagenz (1,28 g, 3.03 mmol) hinzugefügt. Das Reaktionsgemisch wurde 1 Stunde bei 25°C gerührt und anschließend unter vermindertem Druck eingeengt. Der Rückstand wurde an Kieselgel chromatographiert (Petrolether/Ethylacetat 1:1) wobei 400 mg 4-[1-Methyl-5-[4-(trifluoromethoxy)anilino]pyrazol-3-yl]benzaldehyd erhalten wurden.
¹H-NMR siehe Tabelle 5

Weitere in Analogie zu (IM-6-01) hergestellte Verbindungen sind in Tabelle 5 aufgeführt.

### Herstellung von 1-(2-Isopropylphenyl)-3-[[4-[1-methyl-5-[4-(trifluoromethoxy)anilino]pyrazol-3-yl]phenyl]methyleneamino]thioharnstoff (I-015, Verfahren A)

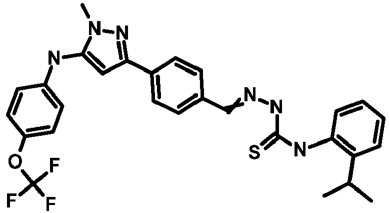

Eine Mischung aus 4-(1-Methyl-5-[[4-(trifluoromethoxy)phenyl]amino]pyrazol-3-yl)benzaldehyd (180,00 mg, 0,50 mmol) und N-(2-Isopropylphenyl)hydrazincarbothioamid (114 mg, 0,55 mmol) (bekannt aus WO 2010/062559) in Ethanol (6,0 mL) wurde bei 80°C 3 h lang gerührt. Das Reaktionsgemisch wurde auf Wasser geschüttet und der ausgefallenene Feststoff abfiltiert. Das Rohprodukt wurde durch preparative HPLC gereinigt (Bedingungen: YMC-Actus Triart C18, 30*250,5um; Mobile Phase A: Wasser(10MMOL/L NH₄HCO₃+0.1%NH₃.H2O), Mobile Phase B: Acetonitril; Fluss rate:45 mL/min). Es wurden 160mg 1-(2-Isopropylphenyl)-3-[[4-[1-methyl-5-[4-(trifluoromethoxy)anilino]pyrazol-3-yl]phenyl]methyleneamino]thioharnstoff erhalten.
¹H-NMR siehe Tabelle 1

### Herstellung von 3-(2-Isopropylphenyl)-2-[[4-[1-methyl-5-[4-(trifluoromethoxy)anilino]pyrazol-3-yl]phenyl]methylenehydrazono]thiazolidin-4-on (I-020, Verfahren C)

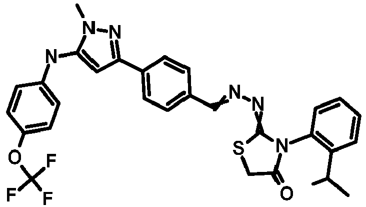

1-(2-Isopropylphenyl)-3-[[4-[1-methyl-5-[4-(trifluoromethoxy)anilino]-1,2,4-triazol-3-yl]phenyl]methyleneamino]thioharnstoff (90 mg, 0,16 mmol) wurde in Ethanol (10 mL) vorgelegt, Natriumacetat (40,08 mg, 0,49 mmol) zugegeben, mit Bromessigsäuremethylester (124,5 mg, 0,81 mmol) versetzt und anschließend 2 h bei 80 °C gerührt. Das Reaktionsgemisch wurde auf Wasser geschüttet und der ausgefallenene Feststoff abfiltiert. Nach Reinigung durch präparative HPLC (Bedingungen: YMC-Actus Triart C18, 30*250,5um; Mobile Phase A: Water (10MMOL/L NH₄HCO₃ + 0.1% NH₃.H₂O; Mobile Phase B: Acetonitril; Fluss Rate: 45 mL/min) wurden 51,80 mg 3-(2-Isopropylphenyl)-2-[[4-[1-methyl-5-[4-(trifluoromethoxy)anilino]pyrazol-3-yl]phenyl]methylenehydrazono]thiazolidin-4-on erhalten.
¹H-NMR siehe Tabelle 1

### Synthesebeispiel 7: N-[5-[4-[[[3-(2-Isopropylphenyl)-4-oxo-thiazolidin-2-ylidene]hydrazono]methyl]phenyl]-2-methyl-pyrazol-3-yl]-4-(trifluoromethyl)cyclohexancarboxamid (I-092)

### Herstellung von 5-Bromo-2-methyl-1,2,4-triazol-3-amin

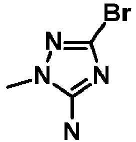

### Stufe 1:

Eine Mischung aus 3,5-Dibromo-1-methyl-1,2,4-triazole (250 mg, 1,038 mmol), und Natriumazide (76mg 1,173mol) in DMF (2ml) wurde bei 95°C 4h gerührt Das Reaktionsgemisch wurde auf Wasser geschüttet und mehrfach mit Essigsäureethylester extrahiert und die organische Phase nach waschen mit ges.-Natriumchloridlösung unter vermindertem Druck eingedampt, wobei 260 mg 5-Azido-3-bromo-1-methyl-1,2,4-triazole erhalten wurden, das ohne Reinigung in Stufe 2 weiter umgesetzt wurde.

### Stufe 2:

5-Azido-3-bromo-1-methyl-1,2,4-triazole (260 mg, 1,281 mmol) aus Stufe 1 wurde mit einer Lösung von Natriumsulfid (670 mg, 8,59 mmol) in Wasser (7 mL) versetzt. Nach 0,5 h Rühren bei 95 °C wurde das Reaktionsgemisch auf Wasser gegossen, mehrfach mit Essigsäuremethylester extrahiert und die organische Phase nach Trocknen über Magnesiumsulfat unter vermindertem Druck eingedampt. Es wurden 135 mg 5-Bromo-2-methyl-1,2,4-triazol-3-amin erhalten.
¹H-NMR (400 MHz, d₆-DMSO): δ 3.47 (s, 3H), 6.51 (s, 2H).

### Herstellung von 4-(5-Amino-1-methyl-1,2,4-triazol-3-yl)benzaldehyd (Verfahren H)

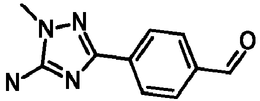

Eine Mischung aus 5-Bromo-2-methyl-1,2,4-triazol-3-amin (700 mg, 3,96 mmol), 4-Formylphenylboronsäurepinakolester (1377 mg, 5,93 mmol) und Cäsiumcarbonat (3,87 g,11,86 mmol), Dioxan (24 mL) und Wasser (8 mL) wurde unter Argon entgast und mit 1,1'-Bis(diphenylphosphino)ferrocen-palladium(II)dichlorid (194 mg, 0,24 mmol) versetzt. Anschließend wurde 3 h bei 90 °C gerührt. Das Reaktionsgemisch wurde auf wässrige Natriumchloridlösung geschüttet und der ausgefallenene Feststoff abfiltiert. Dieser Rückstand wurde mit Dimethylforamid (10ml) warm verrührt, der Feststoff wieder abfiltiert undanschließend das Filtrat unter vermindertem Druck eingedampft. Es wurden 370 mg 4-(5-Amino-1-methyl-1,2,4-triazol-3-yl)benzaldehyd erhalten.
¹H-NMR (400 MHz, d₆-DMSO): δ 3.61 (s, 3H), 6.41 (s, 2H), 7.95 (m, 2H), 8.06 (m, 2H), 10 (s, 1H).

### Herstellung von 1-[[4-(5-Amino-1-methyl-1,2,4-triazol-3-yl)phenyl]methyleneamino]-3-(2-isopropylphenyl)thioharnstoff (Verfahren A)

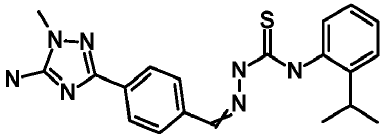

Eine Mischung aus 4-(5-Amino-1-methyl-1,2,4-triazol-3-yl)benzaldehyd (350 mg, 1,731 mmol) und N-(2-Isopropylphenyl)hydrazincarbothioamid (362 mg, 1,731 mmol) (bekannt aus WO 2010/062559) in Dimethylforamid (10 mL) wurde bei 75°C 16 h gerührt. Das Reaktionsgemisch wurde auf Wasser geschüttet und der ausgefallenene Feststoff abfiltiert. Es wurden 513 mg 1-[[4-(5-Amino-1-methyl-1,2,4-triazol-3-yl)phenyl]methyleneamino]-3-(2-isopropylphenyl)thioharnstoff erhalten.
¹H-NMR (400 MHz, d₆-DMSO): δ 1.19 (m, 6H), 3.13 (m,1H), 3.58 (s, 3H), 6.32 (s, 2H), 7.22 (m,2H), 7.32 (m, 2H), 7.89 (m, 4H), 8.15 (s, 1H), 10.02 (s, 1H), 11.78 (s, 1H).

### Herstellung von 2-[[4-(5-Amino-1-methyl-1,2,4-triazol-3-yl)phenyl]methylenehydrazono]-3-(2-isopropylphenyl)thiazolidin-4-on (Verfahren C)

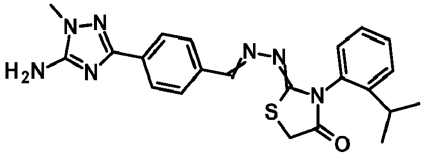

1-[[4-(5-Amino-1-methyl-1,2,4-triazol-3-yl)phenyl]methyleneamino]-3-(2-isopropylphenyl)thiourea (510 mg, 1,296 mmol) wurde in Ethanol (25 mL) vorgelegt, Natriumacetat (425 mg, 5,186 mmol) zugegeben, mit Bromessigsäuremethylester (238 mg, 1,555 mmol) versetzt und anschließend 4 h bei 75 °C gerührt. Der Feststoff wurde abfiltiert, wobei 410 mg 2-[[4-(5-Amino-1-methyl-1,2,4-triazol-3-yl)phenyl]methylenehydrazono]-3-(2-isopropylphenyl)thiazolidin-4-on erhalten wurden.
¹H-NMR (400 MHz, d₆-DMSO): δ 1.14 (m, 6H), 2.78 (m, 1H), 3.59 (s, 3H), 4.19 (dd, 2H), 6.34 (s, 2H), 7.33 (m,2H), 7.46 (m, 2H), 7.74 (m, 2H), 7.90 (m, 2H), 8.32 (s, 1H).

### Herstellung von N-[5-[4-[[[3-(2-Isopropylphenyl)-4-oxo-thiazolidin-2-ylidene]hydrazono]methyl]phenyl]-2-methyl-pyrazol-3-yl]-4-(trifluoromethoxy)cyclohexanecarboxamid (I-092, Verfahren F-1)

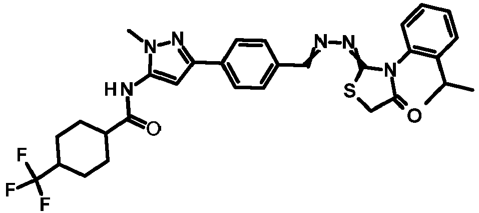

2-[[4-(5-Amino-1-methyl-1,2,4-triazol-3-yl)phenyl]methylenehydrazono]-3-(2-isopropylphenyl)thiazolidin-4-on (100 mg, 0,231 mmol), wurde in Dioxan (3,0 mL) und Dimethylacetamid (1,5mL) vorgelegt und zunächst mit Pyridin (27,3 mg, 0,34 mmol) und anschließend mit 4-(Trifluoromethyl)cyclohexanoyl chlorid (74 mg, 0,34 mmol) versetzt. Das Reaktionsgemisch wurde 15 h bei 90°C gerührt, anschließend auf Wasser geschüttet, der ausgefallenene Feststoff abfiltiert und an Kieselgel chromatographiert (Gradient: Cyclohexan/Ethylacetat). Es wurden 58 mg N-[5-[4-[[[3-(2-Isopropylphenyl)-4-oxo-thiazolidin-2-ylidene]hydrazono]methyl]phenyl]-2-methyl-pyrazol-3-yl]-4-(trifluoromethoxy)cyclohexanecarboxamid erhalten.
¹H-NMR siehe Tabelle 1

### NMR-Daten ausgewählter Beispiele

### NMR-Peak-Listenverfahren

Die ¹H-NMR-Daten ausgewählter Beispiele werden in Form von ¹H-NMR-Peaklisten notiert. Zu jedem Signalpeak wird erst der δ-Wert in ppm und dann die Signalintensität in runden Klammern aufgeführt. Die δ-Wert - Signalintensitäts-Zahlenpaare von verschiedenen Signalpeaks werden durch Semikolons voneinander getrennt aufgelistet.

Die Peakliste eines Beispiels hat daher die Form:
δ ₁ (Intensität₁); δ₂ (Intensität₂); ......; δᵢ (Intensitätᵢ); ......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besonders im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in d₆-DMSO und der Peak von Wasser, gezeigt, die gewöhnlich im Durchschnitt eine hohe Intensität aufweisen.

Die Peaks von Stereoisomeren der Zielverbindungen und/oder Peaks von Verunreinigungen haben gewöhnlich im Durchschnitt eine geringere Intensität als die Peaks der Zielverbindungen (zum Beispiel mit einer Reinheit von > 90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von *"Nebenprodukt-Fingerabdrücken"* zu erkennen.

Ein Experte, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

Weitere Details zu ¹H-NMR-Peaklisten können der Research Disclosure Database Number 564025 entnommen werden.

In Analogie zu den Beispielen und gemäß den oben beschriebenen Herstellverfahren lassen sich die in Tabelle 1 genannten Verbindungen der Formel (I) erhalten:

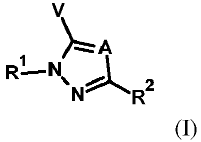

**Tabelle 1**

| **Bsp.-Nr.** | **Struktur** | **NMR-Daten** |
|---|---|---|
| I-001 | 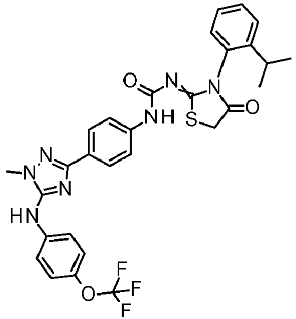 | I-001: ¹H-NMR(400.2 MHz, d₆-DMSO): |
| | | δ= 9.8753 (2.4); 9.1766 (3.6); 8.3147 (0.4); 7.8515 (2.4); 7.8298 (3.4); 7.8094 (0.5); 7.8007 (4.6); 7.7954 (1.6); 7.7834 (1.5); 7.7779 (5.1); 7.7691 (0.5); 7.7227 (3.2); 7.7009 (2.4); 7.5286 (1.0); 7.5115 (1.8); 7.4825 (0.9); 7.4644 (1.3); 7.4466 (0.6); 7.3599 (0.7); 7.3570 (0.7); 7.3386 (1.5); 7.3222 (3.6); 7.3005 (2.6); 7.2648 (1.8); 7.2469 (1.2); 4.2377 (1.3); 4.1926 (2.2); 4.1044 (2.5); 4.0594 (1.4); 3.7924 (0.6); 3.7767 (16.0); 3.3213 (40.2); 2.7516 (0.3); 2.7352 (0.9); 2.7180 (1.2); 2.7009 (0.9); 2.6797 (0.4); 2.6750 (0.7); 2.6705 (0.9); 2.6660 (0.7); 2.5238 (3.2); 2.5104 (54.2); 2.5060 (107.4); 2.5015 (140.9); 2.4969 (102.4); 2.4925 (50.0); 2.3326 (0.6); 2.3284 (0.9); 2.3238 (0.6); 1.1978 (4.2); 1.1808 (4.2); 1.1255 (5.9); 1.1084 (5.8); 0.0079 (2.5); -0.0002 (70.5); -0.0085 (2.4) |
| I-002 | 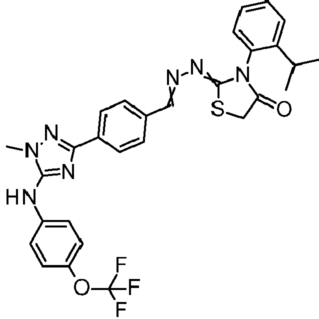 | I-002: ¹H-NMR(400.2 MHz, d₆-DMSO): |
| | | δ= 9.2600 (3.3); 8.3418 (5.1); 8.0400 (3.6); 8.0190 (4.7); 7.8344 (0.4); 7.8258 (4.6); 7.8201 (2.3); 7.8160 (4.6); 7.8088 (2.0); 7.8030 (5.4); 7.7948 (4.0); 7.5240 (0.7); 7.5198 (0.9); 7.5042 (2.1); 7.5002 (2.0); 7.4897 (1.2); 7.4865 (1.2); 7.4721 (1.4); 7.4691 (1.5); 7.4526 (0.6); 7.4493 (0.6); 7.3564 (0.8); 7.3519 (0.9); 7.3369 (4.2); 7.3188 (2.7); 7.3150 (3.5); 7.2751 (2.1); 7.2721 (2.1); 7.2556 (1.4); 7.2524 (1.2); 4.2798 (1.9); 4.2365 (3.1); 4.1616 (3.3); 4.1183 (1.9); 3.8217 (16.0); 3.3231 (28.1); 2.8083 (0.8); 2.7913 (1.2); 2.7741 (0.8); 2.6754 (0.4); 2.6709 (0.6); 2.6662 (0.4); 2.5244 (1.9); 2.5197 (2.8); 2.5110 (36.2); 2.5065 (74.2); 2.5019 (97.5); 2.4973 (69.7); 2.4928 (33.2); 2.3333 (0.4); 2.3287 (0.6); 2.3242 (0.4); 1.1696 (5.9); 1.1524 (6.2); 1.1438 (6.2); 1.1267 (5.7); 0.0080 (1.5); -0.0002 (49.6); -0.0085 (1.7) |
| I-003 | | I-003: ¹H-NMR(400.2 MHz, d₆-DMSO): |
| | 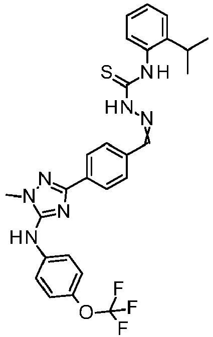 | δ= 11.8287 (2.9); 10.0222 (2.7); 9.2513 (3.2); 8.1712 (3.7); 8.0069 (1.2); 8.0015 (0.6); 7.9850 (8.3); 7.9775 (6.6); 7.9554 (1.0); 7.8337 (0.4); 7.8249 (4.4); 7.8194 (1.4); 7.8076 (1.4); 7.8020 (5.1); 7.7933 (0.5); 7.3728 (1.1); 7.3540 (2.5); 7.3435 (2.8); 7.3223 (3.3); 7.3142 (1.2); 7.3088 (1.0); 7.3006 (1.2); 7.2937 (0.6); 7.2903 (0.6); 7.2811 (0.7); 7.2308 (2.0); 7.2270 (2.9); 7.2173 (3.4); 7.2150 (3.3); 7.2056 (0.3); 3.8199 (16.0); 3.3248 (7.5); 3.1538 (0.8); 3.1366 (1.1); 3.1194 (0.8); 2.6713 (0.3); 2.5249 (0.9); 2.5202 (1.3); 2.5115 (18.9); 2.5070 (39.6); 2.5024 (52.4); 2.4978 (37.8); 2.4932 (18.3); 2.3292 (0.3); 1.9888 (0.7); 1.2028 (12.4); 1.1856 (12.2); 1.1755 (1.0); 1.0704 (0.4); 0.0080 (2.0); -0.0002 (64.9); -0.0086 (2.3) |
| I-004 | 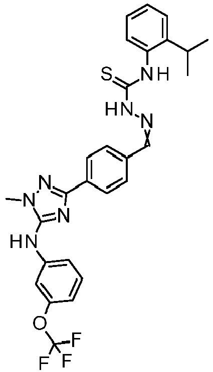 | I-004: ¹H-NMR(400.2 MHz, d₆-DMSO): |
| | | δ= 11.8208 (3.0); 10.0409 (2.7); 9.4015 (3.3); 8.1727 (3.9); 8.0085 (0.5); 7.9854 (16.0); 7.9699 (0.5); 7.9625 (0.6); 7.6319 (1.1); 7.6285 (1.0); 7.6111 (1.3); 7.6078 (1.3); 7.4569 (1.7); 7.4364 (3.0); 7.4158 (1.6); 7.3769 (0.9); 7.3737 (1.0); 7.3554 (2.0); 7.3245 (0.8); 7.3192 (0.8); 7.3081 (1.0); 7.3032 (1.2); 7.2894 (0.6); 7.2836 (0.7); 7.2491 (0.5); 7.2453 (0.5); 7.2296 (1.6); 7.2259 (1.7); 7.2137 (3.4); 7.2098 (3.2); 7.1947 (0.6); 6.9124 (1.1); 6.8940 (1.0); 6.8905 (1.0); 6.8886 (1.0); 3.8471 (0.8); 3.8304 (15.4); 3.3258 (19.1); 3.1564 (0.8); 3.1391 (1.1); 3.1220 (0.8); 3.1046 (0.3); 2.6717 (0.3); 2.5252 (0.9); 2.5204 (1.4); 2.5118 (20.2); 2.5074 (41.5); 2.5028 (54.5); 2.4982 (39.1); 2.4936 (18.7); 2.3296 (0.3); 2.0747 (6.7); 1.2029 (12.0); 1.1857 (11.9); 0.0080 (0.6); -0.0002 (19.2); -0.0085 (0.7) |
| I-005 | 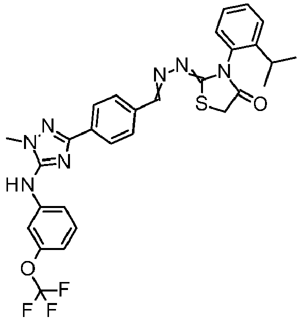 | I-005: ¹H-NMR(400.2 MHz, d₆-DMSO): |
| | | δ= 9.3931 (3.8); 8.3412 (5.5); 8.0259 (4.1); 8.0050 (5.2); 7.9002 (2.6); 7.8278 (4.9); 7.8069 (4.1); 7.6826 (1.5); 7.6791 (1.4); 7.6619 (1.8); 7.6583 (1.7); 7.5204 (1.1); 7.5041 (2.6); 7.5007 (2.6); 7.4898 (1.6); 7.4698 (2.0); 7.4575 (2.0); 7.4370 (3.0); 7.4164 (1.5); 7.3568 (0.9); 7.3527 (0.9); 7.3370 (1.9); 7.3196 (1.2); 7.3158 (1.2); 7.2743 (2.6); 7.2571 (1.6); 6.9168 (1.5); 6.8963 (1.4); 4.2808 (1.9); 4.2375 (3.4); 4.1632 (3.6); 4.1199 (1.8); 3.8308 (16.0); 3.3245 (104.8); 2.8262 (0.4); 2.8094 (1.0); 2.7921 (1.4); 2.7750 (1.1); 2.7582 (0.4); 2.6752 (0.5); 2.6712 (0.7); 2.6666 (0.6); 2.5063 (92.3); 2.5021 (116.2); 2.4978 (88.5); 2.3289 (0.7); 1.1697 (7.0); 1.1525 (7.8); 1.1443 (7.8); 1.1272 (6.9); 0.1457 (0.5); -0.0002 (96.6); -0.1498 (0.5) |
| I-006 | 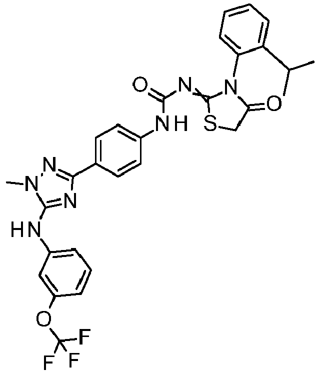 | I-006: ¹H-NMR(400.2 MHz, d₆-DMSO): |
| | | δ= 9.8935 (1.9); 9.3139 (2.6); 7.8824 (1.4); 7.8396 (1.8); 7.8180 (2.6); 7.7296 (2.6); 7.7079 (1.9); 7.6565 (0.8); 7.6384 (1.0); 7.5295 (0.9); 7.5124 (1.5); 7.4821 (0.7); 7.4645 (1.1); 7.4423 (1.4); 7.4215 (1.9); 7.4009 (1.0); 7.3574 (0.6); 7.3408 (1.1); 7.3226 (0.7); 7.2680 (1.4); 7.2490 (0.9); 6.8960 (0.9); 6.8744 (0.8); 4.2380 (1.0); 4.1930 (1.7); 4.1051 (1.8); 4.0599 (1.1); 4.0558 (1.5); 4.0380 (3.7); 4.0202 (3.7); 4.0024 (1.2); 3.7858 (12.0); 3.3212 (42.4); 2.7367 (0.7); 2.7196 (0.9); 2.7024 (0.7); 2.6853 (0.3); 2.6799 (0.4); 2.6752 (0.7); 2.6706 (0.9); 2.6663 (0.7); 2.5105 (56.3); 2.5062 (106.3); 2.5017 (136.6); 2.4972 (99.8); 2.4930 (50.6); 2.3330 (0.6); 2.3286 (0.8); 2.3240 (0.6); 1.9886 (16.0); 1.1988 (3.6); 1.1930 (5.7); 1.1818 (3.7); 1.1752 (9.4); 1.1574 (4.3); 1.1254 (4.6); 1.1084 (4.4); 0.1460 (0.7); 0.0079 (8.4); -0.0002 (158.2); -0.0083 (8.5); -0.1496 (0.7) |
| I-007 | 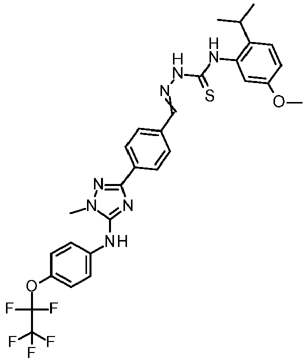 | I-007: ¹H-NMR(400.2 MHz, d₆-DMSO): |
| | | δ= 11.8435 (2.6); 9.9951 (2.5); 9.2696 (3.0); 8.1672 (3.4); 8.0068 (1.4); 7.9851 (6.8); 7.9744 (5.6); 7.9528 (1.2); 7.8326 (3.8); 7.8099 (4.1); 7.3328 (3.2); 7.3103 (3.0); 7.2636 (2.1); 7.2419 (2.3); 6.9076 (1.2); 6.9009 (1.4); 6.8860 (1.0); 6.8793 (1.3); 6.8270 (2.7); 6.8204 (2.3); 3.8217 (12.9); 3.7460 (16.0); 3.7094 (0.8); 3.3338 (155.6); 3.0696 (0.7); 3.0526 (1.0); 3.0354 (0.8); 2.6759 (0.6); 2.6716 (0.8); 2.6671 (0.6); 2.5246 (2.9); 2.5070 (106.6); 2.5026 (136.0); 2.4982 (100.7); 2.3340 (0.6); 2.3294 (0.9); 2.3251 (0.6); 1.2683 (0.3); 1.2128 (0.4); 1.1729 (10.6); 1.1557 (10.5); 1.1264 (0.7); 1.1079 (0.5); 0.0077 (2.4); -0.0002 (62.1); -0.0084 (2.9) |
| I-008 | 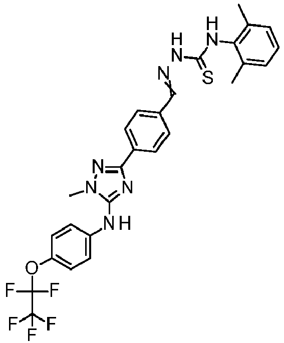 | I-008: ¹H-NMR(400.2 MHz, d₆-DMSO): |
| | | δ= 11.8039 (2.0); 9.9228 (1.8); 9.2671 (2.3); 8.1585 (2.8); 7.9832 (15.1); 7.8297 (2.9); 7.8245 (1.0); 7.8124 (1.0); 7.8069 (3.2); 7.7984 (0.3); 7.3310 (2.4); 7.3084 (2.2); 7.1368 (0.9); 7.1269 (3.4); 7.1223 (4.8); 7.1123 (0.8); 7.0982 (0.4); 4.3673 (1.0); 4.3546 (2.0); 4.3419 (1.0); 3.8218 (10.1); 3.4740 (0.6); 3.4613 (0.7); 3.4565 (2.0); 3.4438 (1.9); 3.4391 (2.0); 3.4263 (1.9); 3.4216 (0.7); 3.4089 (0.6); 3.3326 (21.4); 2.5253 (0.8); 2.5118 (16.1); 2.5075 (32.3); 2.5029 (42.3); 2.4984 (31.1); 2.4939 (15.4); 2.2032 (16.0); 2.1419 (0.6); 1.0742 (4.0); 1.0567 (7.8); 1.0392 (3.8); 0.0079 (0.9); -0.0002 (24.5); -0.0085 (0.9) |
| I-009 | 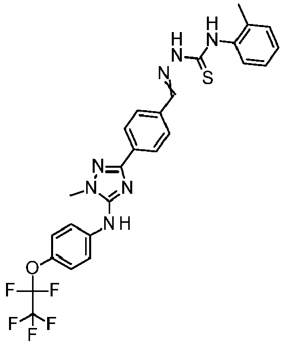 | I-009: ¹H-NMR(400.2 MHz, d₆-DMSO): |
| | | δ= 11.8520 (3.3); 10.0262 (2.9); 9.2698 (3.7); 8.1728 (4.2); 8.0095 (1.3); 7.9875 (9.2); 7.9804 (7.8); 7.9584 (1.2); 7.8424 (0.5); 7.8338 (4.6); 7.8285 (1.6); 7.8163 (1.6); 7.8110 (5.1); 7.8024 (0.6); 7.3340 (3.7); 7.3116 (4.4); 7.3003 (1.6); 7.2938 (2.1); 7.2895 (2.5); 7.2776 (1.8); 7.2678 (0.3); 7.2590 (0.4); 7.2528 (0.6); 7.2405 (2.0); 7.2339 (3.0); 7.2257 (3.4); 7.2166 (2.1); 7.2120 (1.3); 7.1986 (0.4); 4.3697 (0.8); 4.3570 (1.7); 4.3443 (0.9); 3.8239 (16.0); 3.4754 (0.6); 3.4627 (0.6); 3.4579 (1.7); 3.4452 (1.7); 3.4405 (1.8); 3.4278 (1.7); 3.4230 (0.6); 3.4103 (0.6); 3.3373 (25.2); 2.5262 (0.8); 2.5126 (14.4); 2.5083 (29.1); 2.5037 (38.0); 2.4992 (27.9); 2.4948 (13.9); 2.2515 (13.3); 1.0753 (3.5); 1.0579 (6.9); 1.0404 (3.4); 0.0080 (0.7); -0.0002 (19.7); -0.0085 (0.8) |
| I-010 | 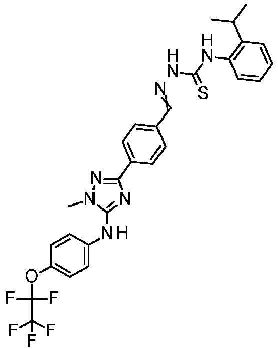 | I-010: ¹H-NMR(400.2 MHz, d₆-DMSO): |
| | | δ= 11.8331 (3.2); 10.0293 (3.0); 9.2682 (3.6); 8.1708 (4.2); 8.0078 (1.2); 7.9858 (9.8); 7.9801 (7.9); 7.9580 (1.0); 7.8424 (0.5); 7.8338 (4.5); 7.8286 (1.6); 7.8163 (1.7); 7.8110 (5.1); 7.8024 (0.6); 7.3732 (1.2); 7.3546 (2.5); 7.3314 (3.8); 7.3147 (2.2); 7.3088 (4.4); 7.3016 (1.8); 7.2956 (0.8); 7.2902 (0.7); 7.2819 (0.8); 7.2505 (0.4); 7.2469 (0.4); 7.2307 (2.1); 7.2273 (2.6); 7.2234 (2.9); 7.2165 (3.8); 7.2146 (3.7); 7.2045 (0.4); 3.8222 (16.0); 3.3333 (62.9); 3.1699 (0.4); 3.1527 (0.9); 3.1356 (1.2); 3.1184 (0.9); 3.1009 (0.4); 2.6720 (0.4); 2.5254 (1.5); 2.5118 (26.4); 2.5075 (51.6); 2.5030 (66.6); 2.4985 (48.9); 2.4941 (24.2); 2.3297 (0.4); 1.2022 (13.1); 1.1850 (12.9); 0.0079 (2.4); - 0.0002 (59.4); -0.0085 (2.3) |
| I-011 | 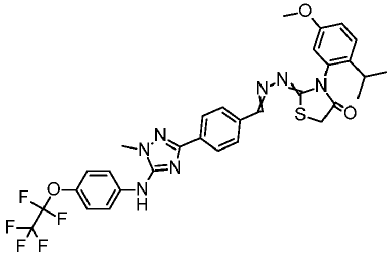 | I-011: ¹H-NMR(400.2 MHz, d₆-DMSO): |
| | | δ= 9.2769 (3.2); 8.3635 (4.5); 8.0435 (3.4); 8.0227 (4.2); 7.8350 (3.9); 7.8298 (1.8); 7.8209 (4.6); 7.8124 (4.8); 7.8002 (3.5); 7.4088 (2.2); 7.3869 (2.5); 7.3255 (3.3); 7.3030 (3.1); 7.0697 (1.4); 7.0629 (1.5); 7.0479 (1.2); 7.0411 (1.3); 6.8877 (3.0); 6.8809 (2.8); 4.2650 (1.6); 4.2217 (2.5); 4.1573 (0.9); 4.1265 (2.7); 4.0833 (1.6); 3.8247 (13.0); 3.7563 (16.0); 3.6925 (1.3); 3.3316 (100.7); 2.7250 (0.8); 2.7081 (1.1); 2.6910 (0.8); 2.6718 (0.8); 2.6673 (0.6); 2.5070 (76.7); 2.5026 (96.8); 2.4982 (71.6); 2.3338 (0.5); 2.3293 (0.6); 1.1370 (5.3); 1.1199 (5.5); 1.1081 (5.6); 1.0910 (5.2); 1.0739 (0.3); 1.0562 (0.3); 0.0076 (4.1); - 0.0002 (68.6); -0.0083 (3.5); -0.1496 (0.3) |
| I-012 | 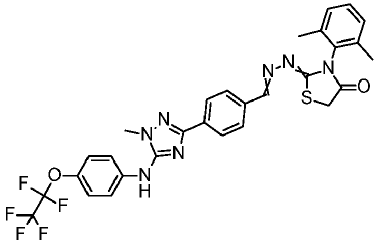 | I-012: ¹H-NMR(400.2 MHz, d₆-DMSO): |
| | | δ= 9.2990 (1.8); 8.3531 (3.1); 8.3182 (0.7); 8.0462 (2.4); 8.0255 (2.9); 7.8393 (2.6); 7.8173 (4.7); 7.7989 (2.4); 7.3241 (2.5); 7.3014 (2.6); 7.2765 (1.3); 7.2284 (3.2); 7.2096 (1.8); 4.2811 (4.9); 3.8272 (8.8); 3.3356 (16.7); 2.5072 (24.8); 2.5034 (30.2); 2.1155 (16.0); 2.0991 (0.8); -0.0002 (17.7) |
| I-013 | 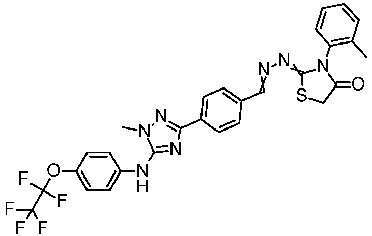 | I-013: ¹H-NMR(400.2 MHz, d₆-DMSO): |
| | | δ= 9.2807 (3.5); 8.3480 (5.6); 8.3166 (0.3); 8.0454 (4.1); 8.0245 (5.1); 7.8366 (4.7); 7.8315 (1.8); 7.8177 (6.6); 7.8139 (7.2); 7.7969 (4.1); 7.4027 (3.4); 7.3935 (5.0); 7.3693 (1.0); 7.3572 (0.8); 7.3495 (1.5); 7.3370 (1.6); 7.3244 (4.4); 7.3147 (3.6); 7.3017 (4.0); 4.6486 (0.8); 4.2603 (1.8); 4.2171 (3.7); 4.1608 (3.9); 4.1175 (1.8); 3.8258 (16.0); 3.6713 (1.2); 3.3408 (123.0); 2.6724 (0.4); 2.5079 (45.8); 2.5035 (58.3); 2.4991 (42.9); 2.3304 (0.4); 2.1432 (15.2); 2.0984 (1.4); -0.0002 (46.4); -0.0085 (2.2) |
| I-014 | 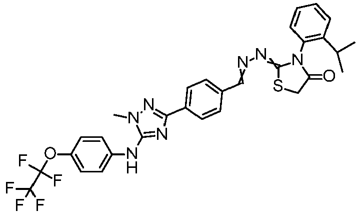 | I-014: ¹H-NMR(400.2 MHz, d₆-DMSO): |
| | | δ= 9.2796 (3.7); 8.3430 (5.6); 8.3157 (0.3); 8.0423 (4.1); 8.0215 (5.2); 7.8353 (4.8); 7.8181 (6.7); 7.8128 (6.9); 7.7974 (4.3); 7.5207 (1.1); 7.5046 (2.6); 7.5012 (2.6); 7.4904 (1.5); 7.4878 (1.5); 7.4705 (1.9); 7.4533 (0.7); 7.4504 (0.7); 7.3572 (0.9); 7.3531 (0.9); 7.3338 (2.3); 7.3248 (4.4); 7.3024 (3.9); 7.2735 (2.6); 7.2563 (1.6); 7.2538 (1.5); 4.2796 (1.9); 4.2362 (3.4); 4.1622 (3.6); 4.1189 (1.9); 3.8243 (16.0); 3.3850 (0.4); 3.3442 (205.2); 2.8256 (0.4); 2.8085 (1.0); 2.7915 (1.4); 2.7743 (1.0); 2.7572 (0.4); 2.6726 (0.4); 2.5079 (55.0); 2.5036 (70.9); 2.4993 (54.2); 2.3303 (0.4); 2.3264 (0.4); 1.1699 (6.8); 1.1527 (7.4); 1.1442 (7.5); 1.1270 (6.7); 1.0573 (0.4); 0.0077 (2.4); -0.0002 (53.3) |
| I-015 | 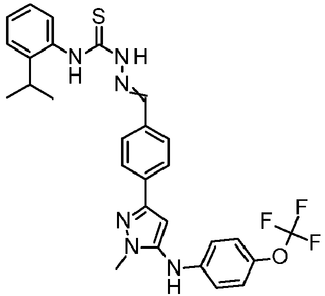 | I-015: ¹H-NMR(400.2 MHz, d₆-DMSO): |
| | | δ= 11.7707 (3.1); 10.0029 (3.0); 8.3336 (3.4); 8.1469 (4.1); 7.9113 (2.8); 7.8901 (5.1); 7.8392 (5.4); 7.8181 (3.2); 7.3682 (1.3); 7.3495 (2.6); 7.3170 (0.8); 7.3084 (1.0); 7.3038 (1.0); 7.2956 (1.4); 7.2878 (0.8); 7.2759 (0.9); 7.2418 (0.7); 7.2308 (3.3); 7.2260 (3.7); 7.2222 (4.7); 7.2105 (7.1); 6.9958 (0.8); 6.9873 (4.8); 6.9819 (1.5); 6.9687 (2.0); 6.9647 (4.0); 6.9562 (0.4); 6.6340 (5.5); 3.7370 (0.8); 3.7211 (16.0); 3.3252 (33.4); 3.1668 (0.4); 3.1493 (0.9); 3.1321 (1.3); 3.1150 (1.0); 3.0974 (0.4); 2.5242 (0.6); 2.5108 (15.9); 2.5063 (34.0); 2.5018 (46.7); 2.4972 (33.6); 2.4927 (15.8); 2.0745 (2.0); 1.1992 (14.0); 1.1820 (14.0); 1.1710 (1.7); 1.1538 (1.1); 0.0081 (0.3); 0.0000 (10.6); - 0.0083 (0.4) |
| I-016 | 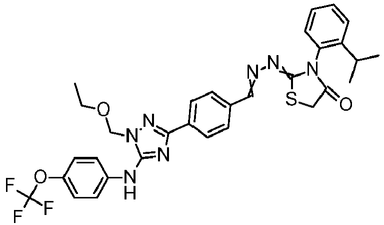 | I-016: ¹H-NMR(400.2 MHz, d₆-DMSO): |
| | | δ= 9.5077 (4.8); 8.3559 (8.4); 8.3175 (0.5); 8.0772 (6.2); 8.0563 (7.7); 7.9249 (0.8); 7.9163 (7.1); 7.9111 (2.5); 7.8988 (2.7); 7.8935 (7.9); 7.8850 (0.9); 7.8380 (7.1); 7.8171 (6.2); 7.5260 (1.3); 7.5222 (1.6); 7.5062 (3.7); 7.5026 (3.6); 7.4916 (2.1); 7.4887 (2.1); 7.4736 (2.6); 7.4713 (2.7); 7.4546 (1.1); 7.4515 (1.1); 7.3579 (6.1); 7.3350 (7.1); 7.3212 (2.0); 7.3170 (1.8); 7.2768 (3.7); 7.2743 (3.7); 7.2573 (2.4); 7.2545 (2.1); 5.5871 (9.6); 4.6487 (0.4); 4.2842 (3.0); 4.2409 (5.0); 4.1648 (5.3); 4.1215 (2.9); 3.6710 (0.7); 3.6457 (2.2); 3.6282 (7.2); 3.6106 (7.3); 3.5931 (2.3); 3.3300 (67.3); 2.8276 (0.5); 2.8109 (1.4); 2.7938 (2.0); 2.7768 (1.5); 2.7597 (0.6); 2.6764 (0.6); 2.6719 (0.7); 2.6673 (0.6); 2.5250 (2.6); 2.5115 (49.1); 2.5073 (95.2); 2.5028 (122.6); 2.4983 (89.7); 2.4941 (44.8); 2.3341 (0.6); 2.3295 (0.8); 2.3252 (0.6); 2.0984 (0.8); 1.1723 (10.1); 1.1551 (10.6); 1.1453 (10.8); 1.1351 (9.7); 1.1282 (10.7); 1.1176 (16.0); 1.1000 (7.4); 0.0079 (1.6); -0.0002 (36.6); -0.0084 (1.5) |
| I-017 | 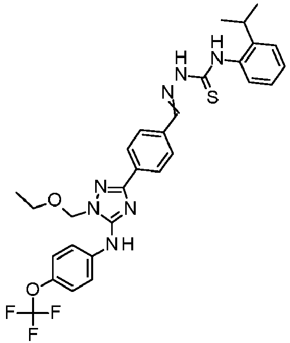 | I-017: ¹H-NMR(400.2 MHz, d₆-DMSO): |
| | | δ= 11.8534 (3.9); 10.0341 (3.8); 9.4981 (4.0); 8.1818 (4.9); 8.0410 (2.6); 8.0197 (8.4); 8.0002 (6.6); 7.9789 (2.2); 7.9237 (0.7); 7.9151 (5.5); 7.9098 (1.9); 7.8977 (2.0); 7.8923 (6.1); 7.8838 (0.6); 7.3754 (2.0); 7.3643 (4.2); 7.3574 (4.1); 7.3430 (3.7); 7.3250 (1.0); 7.3155 (1.4); 7.3037 (1.6); 7.2950 (1.1); 7.2840 (0.9); 7.2316 (5.5); 7.2222 (4.6); 5.5864 (7.2); 3.6478 (1.8); 3.6303 (5.8); 3.6127 (5.8); 3.5952 (1.8); 3.3346 (37.5); 3.1713 (0.4); 3.1540 (1.1); 3.1368 (1.5); 3.1197 (1.2); 3.1024 (0.5); 2.5126 (20.8); 2.5083 (39.8); 2.5039 (50.6); 2.4993 (36.2); 2.4950 (17.4); 1.2045 (16.0); 1.1873 (15.7); 1.1379 (6.0); 1.1204 (12.2); 1.1028 (5.7); -0.0002 (4.2) |
| I-017 | 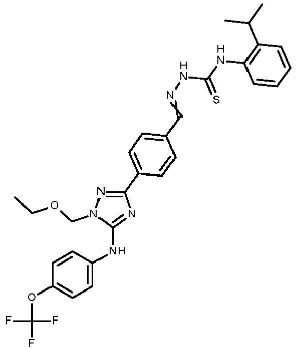 | I-017: ¹H-NMR(400.2 MHz, d₆-DMSO): |
| | | δ= 11.8389 (4.0); 10.0231 (3.8); 9.1848 (4.2); 8.1754 (4.9); 8.0165 (2.0); 7.9949 (9.4); 7.9825 (7.5); 7.9609 (1.9); 7.8547 (0.6); 7.8459 (5.6); 7.8406 (2.0); 7.8285 (2.0); 7.8231 (6.3); 7.8146 (0.7); 7.3747 (1.6); 7.3555 (3.3); 7.3433 (4.0); 7.3225 (4.3); 7.3139 (2.0); 7.3020 (1.6); 7.2934 (1.2); 7.2823 (1.0); 7.2300 (5.5); 7.2206 (4.5); 4.3963 (2.0); 4.3829 (3.9); 4.3692 (2.1); 3.7552 (2.5); 3.7417 (4.9); 3.7279 (2.3); 3.3301 (47.4); 3.2597 (29.1); 3.1702 (0.4); 3.1533 (1.1); 3.1362 (1.5); 3.1190 (1.1); 3.1019 (0.4); 2.6717 (0.4); 2.6672 (0.3); 2.5250 (1.5); 2.5116 (27.9); 2.5072 (55.6); 2.5027 (72.5); 2.4982 (52.4); 2.4938 (25.6); 2.3296 (0.4); 2.3247 (0.3); 1.2038 (16.0); 1.1866 (15.8); 0.0080 (0.4); -0.0002 (11.7); -0.0084 (0.4); -0.0400 (0.6) |
| I-018 | 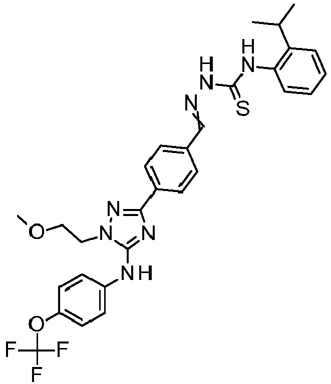 | I-018: ¹H-NMR(400.2 MHz, d₆-DMSO): |
| | | δ= 11.8501 (3.9); 10.0317 (3.7); 9.4948 (4.0); 8.1798 (4.8); 8.0390 (2.6); 8.0175 (8.5); 7.9983 (6.6); 7.9770 (2.2); 7.9214 (0.6); 7.9128 (5.6); 7.9074 (1.9); 7.8954 (2.0); 7.8900 (6.3); 7.8814 (0.6); 7.3748 (1.9); 7.3629 (4.3); 7.3569 (4.2); 7.3420 (3.6); 7.3246 (1.0); 7.3154 (1.3); 7.3121 (1.2); 7.3032 (1.6); 7.2948 (1.1); 7.2835 (0.9); 7.2536 (0.3); 7.2305 (5.2); 7.2211 (4.5); 5.5847 (7.2); 3.6466 (1.7); 3.6291 (5.8); 3.6115 (5.9); 3.5939 (1.8); 3.3307 (79.8); 3.1693 (0.4); 3.1524 (1.1); 3.1352 (1.5); 3.1180 (1.1); 3.1007 (0.4); 2.6762 (0.4); 2.6719 (0.5); 2.6676 (0.4); 2.5253 (1.6); 2.5119 (34.6); 2.5075 (69.2); 2.5030 (90.0); 2.4984 (65.2); 2.4940 (31.8); 2.3344 (0.4); 2.3298 (0.6); 2.3254 (0.4); 1.2035 (16.0); 1.1863 (15.8); 1.1370 (6.0); 1.1194 (12.5); 1.1018 (5.8); 0.0080 (0.5); -0.0002 (14.5); -0.0083 (0.6) |
| I-019 | 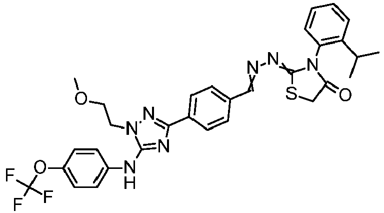 | I-019: ¹H-NMR(400.2 MHz, d₆-DMSO): |
| | | δ= 9.1964 (7.3); 8.3471 (11.4); 8.3170 (0.5); 8.0512 (8.4); 8.0303 (10.5); 7.8577 (1.3); 7.8491 (9.8); 7.8440 (3.9); 7.8258 (16.0); 7.8028 (8.4); 7.5251 (1.8); 7.5216 (2.2); 7.5056 (5.1); 7.5020 (5.1); 7.4910 (2.9); 7.4882 (2.9); 7.4707 (3.8); 7.4539 (1.5); 7.4508 (1.5); 7.3578 (2.0); 7.3534 (2.2); 7.3378 (10.6); 7.3164 (8.8); 7.2756 (5.1); 7.2731 (5.2); 7.2562 (3.2); 7.2534 (3.0); 4.3987 (3.8); 4.3852 (7.3); 4.3717 (4.0); 4.3540 (1.1); 4.3414 (0.6); 4.2819 (4.0); 4.2386 (6.9); 4.1635 (7.2); 4.1202 (3.9); 3.7540 (4.6); 3.7407 (8.6); 3.7268 (4.2); 3.4608 (0.3); 3.4561 (0.8); 3.4433 (0.8); 3.4386 (0.9); 3.4313 (0.4); 3.4259 (0.8); 3.4212 (0.4); 3.3313 (156.1); 3.2572 (46.9); 2.8264 (0.8); 2.8094 (2.0); 2.7922 (2.7); 2.7752 (2.0); 2.7580 (0.8); 2.6759 (0.9); 2.6715 (1.2); 2.6671 (0.9); 2.5246 (4.1); 2.5070 (146.5); 2.5025 |
| | | (189.3); 2.4981 (140.9); 2.3339 (0.9); 2.3294 (1.2); 2.3250 (0.9); 1.1714 (13.7); 1.1543 (14.6); 1.1445 (14.7); 1.1274 (13.4); 1.0738 (1.7); 1.0563 (3.2); 1.0388 (1.6); 0.0078 (2.3); -0.0003 (56.1); - 0.0084 (2.8) |
| I-020 | 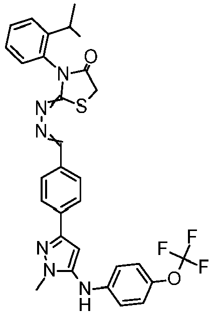 | I-020: ¹H-NMR(400.2 MHz, d₆-DMSO): |
| | | δ= 8.3515 (4.3); 8.3345 (0.4); 8.3092 (5.6); 7.8721 (3.8); 7.8514 (5.3); 7.7500 (5.2); 7.7291 (3.9); 7.5828 (0.4); 7.5200 (1.2); 7.5036 (2.8); 7.4893 (1.6); 7.4695 (2.0); 7.4521 (0.8); 7.3558 (1.0); 7.3521 (1.0); 7.3362 (2.0); 7.3187 (1.4); 7.3149 (1.2); 7.2704 (2.8); 7.2513 (1.9); 7.2342 (3.5); 7.2128 (4.1); 6.9867 (5.2); 6.9644 (4.4); 6.6165 (5.6); 4.2726 (1.9); 4.2293 (3.4); 4.1556 (3.6); 4.1122 (1.9); 3.7236 (16.0); 3.3600 (402.1); 3.3385 (17.0); 2.8212 (0.5); 2.8040 (1.1); 2.7866 (1.4); 2.7694 (1.1); 2.7534 (0.5); 2.6735 (0.3); 2.5080 (42.8); 2.5041 (54.1); 2.5002 (42.0); 2.3310 (0.4); 1.2349 (0.5); 1.1663 (7.0); 1.1489 (8.5); 1.1423 (8.5); 1.1250 (7.2); 1.0910 (0.5); 0.0001 (2.9) |
| I-021 | 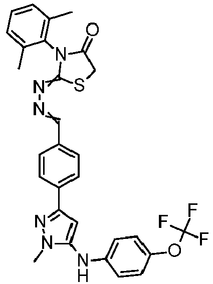 | I-021: ¹H-NMR(400.2 MHz, d₆-DMSO): |
| | | δ= 8.3506 (2.1); 8.3194 (3.3); 7.8761 (2.0); 7.8552 (3.0); 7.7507 (2.9); 7.7296 (2.2); 7.6785 (0.4); 7.6644 (0.4); 7.3122 (0.6); 7.2956 (0.9); 7.2911 (1.0); 7.2746 (1.5); 7.2332 (1.9); 7.2269 (3.5); 7.2106 (2.9); 6.9948 (0.4); 6.9860 (2.9); 6.9805 (0.9); 6.9634 (2.5); 6.6213 (3.2); 4.2740 (5.2); 4.2587 (0.3); 4.2384 (0.3); 4.2220 (0.6); 3.7446 (0.4); 3.7250 (9.7); 3.6971 (0.4); 3.3361 (57.3); 3.3125 (6.7); 3.1752 (0.5); 3.1620 (0.5); 2.5115 (10.0); 2.5070 (20.9); 2.5025 (28.2); 2.4980 (20.1); 2.4935 (9.3); 2.1347 (0.6); 2.1107 (16.0); 2.0892 (1.2); 1.2336 (0.5); 0.9303 (0.5); 0.9119 (1.1); 0.8934 (0.5); 0.0000 (3.0) |
| I-022 | 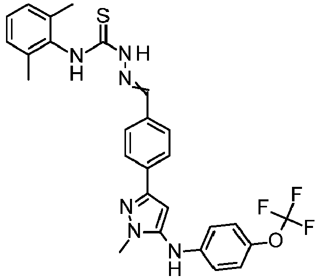 | I-022: ¹H-NMR(400.2 MHz, d₆-DMSO): |
| | | δ= 11.7472 (2.2); 9.8988 (2.1); 8.3408 (2.4); 8.1363 (2.7); 7.9177 (3.0); 7.9010 (3.7); 7.8339 (3.8); 7.8172 (2.8); 7.2339 (3.2); 7.2148 (3.4); 7.1209 (6.7); 6.9893 (3.5); 6.9703 (2.9); 6.6368 (2.8); 3.7243 (8.1); 3.3404 (29.3); 2.6698 (0.3); 2.5051 (27.7); 2.3293 (0.4); 2.3010 (0.4); 2.2008 (16.0); 2.0767 (0.5); 1.9116 (0.5); 1.2366 (0.7); 0.0021 (0.9) |
| I-023 | 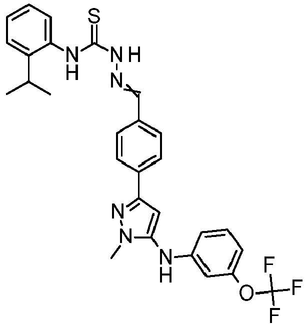 | I-023: ¹H-NMR(400.2 MHz, d₆-DMSO): |
| | | δ= 11.7898 (3.0); 10.0175 (2.6); 8.4848 (3.7); 8.1508 (3.8); 7.9253 (2.7); 7.9042 (4.5); 7.8453 (5.1); 7.8241 (3.2); 7.3702 (1.2); 7.3551 (2.5); 7.3516 (2.6); 7.3352 (3.1); 7.3194 (1.0); 7.3146 (2.2); 7.3052 (1.0); 7.2983 (1.3); 7.2920 (0.6); 7.2866 (0.6); 7.2787 (0.8); 7.2476 (0.4); 7.2441 (0.4); 7.2280 (2.3); 7.2245 (2.4); 7.2199 (2.9); 7.2136 (3.4); 7.2115 (3.3); 7.2010 (0.4); 6.9225 (1.4); 6.9185 (1.3); 6.9019 (1.2); 6.8980 (1.3); 6.8069 (2.1); 6.8052 (2.1); 6.7614 (1.2); 6.7591 (1.2); 6.7410 (1.1); 6.7386 (1.1); 6.6664 (5.5); 3.7243 (16.0); 3.3463 (48.5); 3.3226 (3.3); 3.1688 (0.3); 3.1518 (0.9); 3.1346 (1.2); 3.1174 (0.9); 3.1003 (0.4); 2.5127 (5.4); 2.5083 (11.2); 2.5037 (15.2); 2.4992 (10.9); 2.4948 (5.1); 1.2314 (0.5); 1.2005 (12.8); 1.1833 (12.7); 0.0000 (2.3) |
| I-024 | 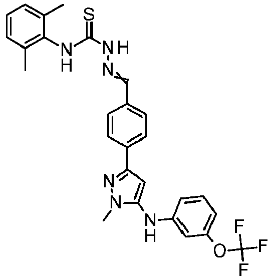 | I-024: ¹H-NMR(400.2 MHz, d₆-DMSO): |
| | | δ= 11.7552 (1.8); 9.9014 (1.6); 8.4806 (2.0); 8.1363 (2.4); 7.9286 (1.9); 7.9075 (3.0); 7.8342 (2.9); 7.8133 (2.0); 7.3555 (0.9); 7.3350 (1.8); 7.3145 (1.1); 7.1331 (0.9); 7.1233 (3.5); 7.1187 (4.9); 7.1082 (0.9); 7.0947 (0.4); 6.9200 (0.9); 6.9158 (0.8); 6.8993 (0.8); 6.8954 (0.7); 6.8050 (1.3); 6.7604 (0.8); 6.7580 (0.8); 6.7399 (0.7); 6.7377 (0.7); 6.6616 (3.1); 3.7228 (9.1); 3.3379 (20.7); 3.3153 (2.4); 2.5118 (8.9); 2.5073 (19.0); 2.5028 (26.1); 2.4983 (18.7); 2.4938 (8.7); 2.1992 (16.0); 2.0767 (1.4); 0.0000 (5.4) |
| I-025 | 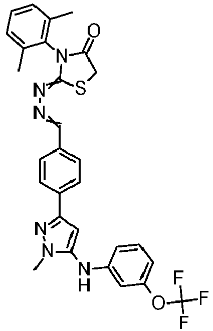 | I-025: ¹H-NMR(400.2 MHz, d₆-DMSO): |
| | | δ= 8.4928 (2.2); 8.3264 (3.2); 7.8849 (2.0); 7.8641 (2.9); 7.7625 (2.8); 7.7415 (2.0); 7.3566 (1.0); 7.3360 (1.9); 7.3153 (1.4); 7.2964 (0.9); 7.2921 (1.0); 7.2756 (1.4); 7.2280 (3.1); 7.2089 (1.6); 6.9219 (1.0); 6.9181 (0.9); 6.9014 (0.8); 6.8973 (0.8); 6.8080 (1.4); 6.7647 (0.9); 6.7444 (0.8); 6.6520 (3.2); 4.2769 (5.1); 4.1949 (0.4); 4.1262 (0.5); 3.7487 (0.9); 3.7299 (9.0); 3.6949 (0.7); 3.3443 (11.3); 3.3425 (10.8); 3.3203 (1.6); 2.5087 (6.6); 2.5043 (8.6); 2.4999 (6.2); 2.1373 (0.4); 2.1148 (16.0); 2.0941 (1.5); 1.9686 (1.2); 1.1999 (0.5); 0.0002 (0.5) |
| I-026 | 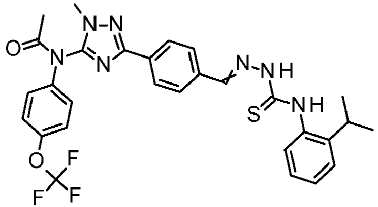 | I-026: ¹H-NMR(400.2 MHz, d₆-DMSO): |
| | | δ= 11.8354 (3.2); 10.0529 (2.8); 8.1686 (3.8); 7.9824 (5.3); 7.6893 (0.3); 7.5018 (2.0); 7.4820 (1.6); 7.3712 (1.2); 7.3526 (2.3); 7.3226 (0.9); 7.3181 (1.0); 7.3056 (1.4); 7.3016 (1.5); 7.2866 (0.7); 7.2818 (0.8); 7.2453 (0.6); 7.2417 (0.6); 7.2257 (1.8); 7.2222 (1.8); 7.2057 (3.9); 7.2014 (2.7); 7.1863 (0.8); 3.8428 (6.7); 3.3788 (0.4); 3.3472 (187.3); 3.1618 (0.4); 3.1446 (0.9); 3.1274 (1.3); 3.1104 (1.0); 3.0936 (0.4); 2.6733 (0.4); 2.5268 (1.1); 2.5132 (23.9); 2.5090 (47.8); 2.5045 (62.3); 2.5000 (46.0); 2.4958 (23.3); 2.3313 (0.4); 2.0951 (16.0); 1.1960 (13.2); 1.1788 (13.0); -0.0002 (5.8) |
| I-027 | 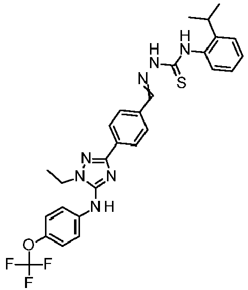 | I-027: ¹H-NMR(400.2 MHz, d₆-DMSO): |
| | | δ= 11.8361 (4.1); 10.0245 (3.9); 9.2112 (4.4); 8.1723 (5.1); 8.0132 (2.3); 7.9918 (8.9); 7.9772 (7.3); 7.9558 (2.1); 7.8345 (5.2); 7.8118 (5.9); 7.3745 (1.6); 7.3552 (3.5); 7.3429 (4.2); 7.3220 (4.6); 7.3019 (1.7); 7.2935 (1.1); 7.2823 (0.9); 7.2519 (0.4); 7.2287 (5.1); 7.2190 (4.7); 4.2362 (1.1); 4.2184 (3.4); 4.2005 (3.4); 4.1827 (1.2); 3.3326 (38.9); 3.1694 (0.4); 3.1523 (1.1); 3.1351 (1.6); 3.1178 (1.2); 3.1009 (0.4); 2.6718 (0.5); 2.5074 (62.6); 2.5031 (79.4); 2.4987 (59.7); 2.3299 (0.5); 1.3973 (4.7); 1.3795 (9.9); 1.3616 (4.7); 1.2031 (16.0); 1.1859 (15.7); 1.0567 (0.4); -0.0002 (6.4) |
| I-028 | 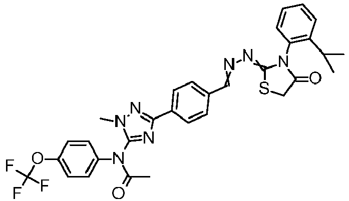 | I-028: ¹H-NMR(400.2 MHz, d₆-DMSO): |
| | | δ= 8.3539 (4.2); 8.0179 (1.4); 8.0021 (1.5); 7.8287 (3.1); 7.8082 (2.7); 7.6880 (0.4); 7.5178 (1.5); 7.5020 (4.5); 7.4987 (4.4); 7.4844 (3.0); 7.4699 (2.3); 7.4674 (2.3); 7.4503 (0.8); 7.4474 (0.8); 7.3542 (0.9); 7.3500 (0.9); 7.3345 (1.8); 7.3307 (1.7); 7.3171 (1.2); 7.3131 (1.2); 7.2737 (2.5); 7.2714 (2.5); 7.2544 (1.6); 7.2516 (1.4); 4.2816 (1.9); 4.2382 (3.2); 4.1613 (3.5); 4.1179 (1.9); 3.8464 (6.6); 3.3372 (23.6); 2.8160 (0.4); 2.7993 (1.0); 2.7822 (1.3); 2.7653 (1.0); 2.7485 (0.4); 2.6725 (0.4); 2.5082 (52.2); 2.5038 (67.3); 2.4994 (51.6); 2.3348 (0.3); 2.3307 |
| | | (0.4); 2.3259 (0.3); 2.0931 (16.0); 1.1620 (6.6); 1.1446 (7.9); 1.1395 (8.0); 1.1222 (6.5) |
| I-029 | 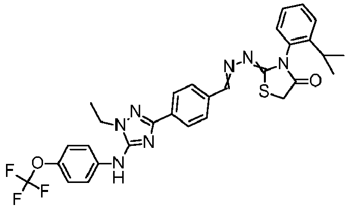 | I-029: ¹H-NMR(400.2 MHz, d₆-DMSO): |
| | | δ= 9.2264 (6.4); 8.3463 (9.4); 8.3200 (0.3); 8.0494 (7.4); 8.0287 (9.0); 7.8395 (8.1); 7.8175 (16.0); 7.7979 (7.5); 7.5219 (2.1); 7.5050 (4.9); 7.4900 (2.9); 7.4711 (3.6); 7.4536 (1.4); 7.3576 (1.8); 7.3538 (2.0); 7.3382 (10.1); 7.3169 (8.2); 7.2757 (4.9); 7.2572 (2.9); 4.2825 (3.2); 4.2392 (7.6); 4.2214 (5.7); 4.2035 (5.6); 4.1855 (2.0); 4.1633 (6.1); 4.1200 (3.2); 3.3382 (36.8); 2.8251 (0.7); 2.8085 (1.8); 2.7915 (2.5); 2.7744 (1.9); 2.7572 (0.8); 2.6725 (0.7); 2.5036 (115.9); 2.3305 (0.8); 1.3970 (7.1); 1.3792 (14.4); 1.3613 (7.0); 1.1707 (12.1); 1.1534 (13.4); 1.1444 (13.4); 1.1272 (11.8); 1.0745 (0.6); 1.0570 (0.9); 1.0395 (0.5); - 0.0001 (0.3) |
| I-030 | 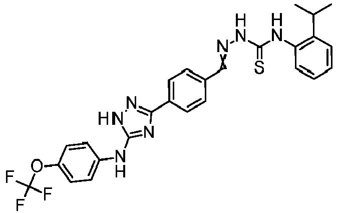 | I-030: ¹H-NMR(400.2 MHz, d₆-DMSO): |
| | | δ= 13.8812 (1.8); 12.8931 (0.4); 11.8959 (1.8); 11.8370 (0.4); 10.0858 (1.7); 10.0207 (0.4); 9.7313 (0.4); 9.5374 (2.0); 8.1790 (3.5); 8.0958 (1.5); 8.0749 (2.3); 8.0351 (0.4); 8.0040 (2.8); 7.9835 (2.1); 7.9668 (0.4); 7.9535 (2.3); 7.7114 (0.4); 7.6828 (2.4); 7.6601 (2.4); 7.3793 (1.0); 7.3601 (1.8); 7.3275 (1.0); 7.3102 (1.4); 7.2949 (0.8); 7.2909 (0.7); 7.2563 (2.0); 7.2348 (3.3); 7.2314 (2.7); 7.2181 (1.5); 7.2149 (1.4); 7.2077 (1.6); 7.1881 (0.5); 3.3358 (68.4); 3.1538 (0.8); 3.1367 (1.0); 3.1194 (0.8); 2.8914 (16.0); 2.7331 (13.9); 2.7318 (13.4); 2.5262 (1.0); 2.5214 (1.6); 2.5128 (20.2); 2.5084 (41.0); 2.5038 (53.3); 2.4993 (38.2); 2.4948 (18.3); 1.2036 (11.4); 1.1864 (11.2) |
| I-031 | 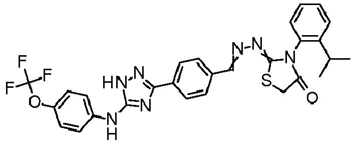 | I-031: ¹H-NMR(400.2 MHz, d₆-DMSO): |
| | | δ= 13.9245 (0.7); 9.5711 (0.9); 8.3716 (5.0); 8.3163 (0.6); 8.0452 (4.0); 8.0250 (4.9); 7.8753 (2.6); 7.7576 (0.5); 7.6868 (4.4); 7.6646 (5.0); 7.6387 (0.6); 7.5938 (0.5); 7.5267 (1.8); 7.5229 (2.1); 7.5069 (5.2); 7.5032 (5.0); 7.4924 (3.0); 7.4893 (2.8); 7.4747 (3.6); 7.4719 (3.8); 7.4552 (1.5); 7.4520 (1.5); 7.3589 (2.1); 7.3547 (1.9); 7.3393 (4.0); 7.3352 (3.6); 7.3218 (2.8); 7.3177 (2.7); 7.2792 (7.0); 7.2765 (7.2); 7.2599 (5.9); 7.2568 (5.4); 5.7563 (0.5); 5.2873 (0.5); 4.2925 (3.3); 4.2492 (5.7); 4.1912 (0.4); 4.1712 (6.7); 4.1381 (0.5); 4.1278 (3.6); 4.0557 (0.7); 4.0379 (2.2); 4.0202 (2.2); 4.0023 (0.7); 3.3311 (172.7); 2.8276 (0.9); 2.8108 (2.1); 2.7937 (2.8); 2.7767 (2.1); 2.7596 (0.8); 2.6763 (1.0); 2.6718 (1.3); 2.6674 (0.9); 2.5251 (4.1); 2.5116 (84.2); 2.5073 (160.8); 2.5028 (205.4); 2.4983 (150.4); 2.4939 (73.8); 2.3341 (0.9); 2.3297 (1.2); 2.3252 (0.9); 2.0116 (0.4); 1.9891 (9.6); 1.2332 (0.4); 1.1931 (2.9); 1.1751 (9.8); 1.1709 (14.4); 1.1536 (16.0); 1.1467 (16.0); 1.1295 (14.1); 1.1004 (0.8); 1.0833 (0.7); 0.8884 (0.4); 0.8716 (0.5); 0.0127 (6.7); 0.0080 (1.6); -0.0002 (28.3); -0.0085 (1.1) |
| I-032 | 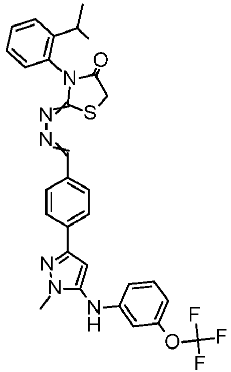 | I-032: ¹H-NMR(400.2 MHz, d₆-DMSO): |
| | | δ= 8.4896 (3.4); 8.3160 (5.8); 7.8784 (3.6); 7.8574 (5.3); 7.7583 (5.1); 7.7372 (3.7); 7.7266 (0.3); 7.5235 (0.8); 7.5195 (1.0); 7.5037 (2.4); 7.4998 (2.4); 7.4886 (1.4); 7.4855 (1.4); 7.4709 (1.7); 7.4681 (1.8); 7.4516 (0.7); 7.4482 (0.7); 7.3554 (2.2); 7.3517 (1.2); 7.3346 (4.5); 7.3185 (1.5); 7.3143 (2.9); 7.2734 (2.5); 7.2706 (2.5); 7.2540 (1.6); 7.2508 (1.4); 6.9190 (1.5); 6.9152 (1.4); 6.8984 (1.4); 6.8945 (1.3); 6.8055 (2.2); 6.7631 (1.3); 6.7607 (1.3); 6.7426 (1.2); 6.7402 (1.2); 6.6462 (5.4); 4.2758 (2.1); 4.2325 (3.5); 4.2225 (0.8); 4.2061 (0.4); 4.1566 (3.7); 4.1133 (2.0); 3.7445 (0.4); 3.7260 (16.0); 3.3363 (45.5); 3.3126 (6.0); 2.8245 (0.4); 2.8073 (1.0); 2.7903 (1.3); 2.7731 (1.0); 2.7562 (0.4); 2.5118 (10.4); 2.5074 (22.0); 2.5029 (30.2); 2.4984 (22.0); 2.4940 (10.8); 1.2332 (0.4); 1.1679 (6.7); 1.1507 (7.4); 1.1431 (7.5); 1.1259 (6.7); 1.1075 (0.4); 0.9305 (0.6); 0.9121 (1.1); 0.8936 (0.5); 0.0000 (2.5) |
| I-033 | 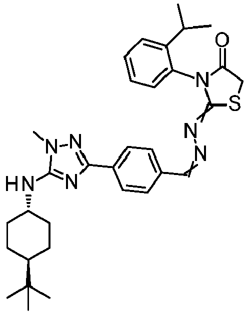 | I-033: ¹H-NMR(400.2 MHz, d₆-DMSO): |
| | | δ= 8.3072 (2.1); 7.9330 (1.6); 7.9122 (2.0); 7.7543 (1.9); 7.7334 (1.6); 7.5158 (0.4); 7.4993 (1.0); 7.4962 (1.0); 7.4849 (0.6); 7.4659 (0.8); 7.3520 (0.3); 7.3482 (0.3); 7.3320 (0.7); 7.3149 (0.5); 7.3111 (0.4); 7.2675 (1.0); 7.2489 (0.6); 6.2708 (0.8); 6.2517 (0.8); 4.2682 (0.7); 4.2249 (1.3); 4.1528 (1.4); 4.1094 (0.7); 3.5747 (5.9); 3.3256 (14.0); 2.8013 (0.4); 2.7841 (0.6); 2.7670 (0.4); 2.5058 (20.2); 2.5017 (24.7); 2.4977 (18.8); 2.0976 (0.9); 2.0764 (0.7); 1.7988 (0.7); 1.7710 (0.7); 1.2583 (0.6); 1.2276 (0.7); 1.1969 (0.4); 1.1642 (2.8); 1.1469 (3.4); 1.1395 (3.2); 1.1221 (3.2); 1.0840 (0.6); 1.0219 (0.5); 0.9933 (0.4); 0.8639 (16.0); -0.0002 (0.4) |
| I-034 | 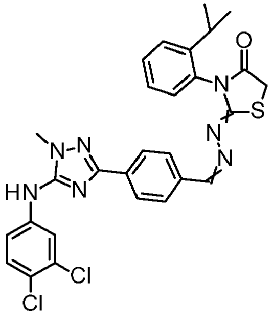 | I-034: ¹H-NMR(400.2 MHz, d₆-DMSO): |
| | | δ= 9.4039 (3.7); 8.3452 (5.3); 8.0878 (3.6); 8.0814 (3.7); 8.0309 (3.8); 8.0100 (5.0); 7.8310 (4.6); 7.8099 (3.8); 7.7123 (1.7); 7.7058 (1.5); 7.6900 (2.1); 7.6836 (2.1); 7.5686 (4.2); 7.5464 (3.2); 7.5238 (0.8); 7.5197 (1.0); 7.5039 (2.4); 7.5000 (2.4); 7.4902 (1.4); 7.4871 (1.4); 7.4726 (1.6); 7.4697 (1.7); 7.4531 (0.7); 7.4498 (0.7); 7.3577 (0.9); 7.3533 (0.9); 7.3380 (1.7); 7.3339 (1.6); 7.3206 (1.2); 7.3164 (1.2); 7.2778 (2.4); 7.2750 (2.4); 7.2584 (1.5); 7.2552 (1.4); 4.2828 (2.0); 4.2396 (3.3); 4.1644 (3.5); 4.1211 (1.9); 3.8458 (0.7); 3.8222 (16.0); 3.4526 (0.6); 3.4351 (0.6); 3.3347 (14.0); 2.8289 (0.4); 2.8119 (0.9); 2.7948 (1.3); 2.7777 (1.0); 2.7608 (0.4); 2.5127 (11.8); 2.5083 (22.8); 2.5038 (29.2); 2.4992 (21.3); 2.4948 (10.6); 1.1713 (6.6); 1.1541 (7.1); 1.1455 (7.0); 1.1284 (6.3); 1.0763 (1.4); 1.0588 (2.7); 1.0414 (1.4); -0.0002 (0.6) |
| I-035 | 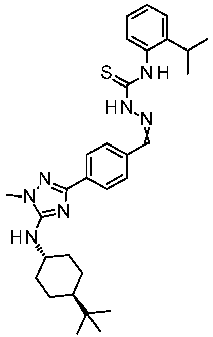 | I-035: ¹H-NMR(400.2 MHz, d₆-DMSO): |
| | | δ= 11.7930 (0.7); 9.9927 (0.9); 8.1498 (2.1); 7.8947 (7.7); 7.3677 (0.6); 7.3486 (1.0); 7.3163 (0.4); 7.3064 (0.5); 7.2948 (0.6); 7.2861 (0.4); 7.2753 (0.3); 7.2227 (1.9); 7.2134 (1.5); 6.2557 (0.8); 6.2365 (0.8); 3.5713 (6.4); 3.3250 (17.6); 3.1460 (0.4); 3.1287 (0.5); 3.1116 (0.4); 2.5237 (0.5); 2.5105 (8.2); 2.5062 (15.8); 2.5016 (20.4); 2.4971 (15.0); 2.4927 (7.5); 2.1048 (0.5); 2.0790 (0.6); 1.8007 (0.5); 1.7704 (0.6); 1.2593 (0.5); 1.2300 (0.6); 1.1978 (5.8); 1.1806 (5.5); 1.1180 (0.5); 1.0913 (0.5); 1.0858 (0.5); 1.0217 (0.4); 0.9930 (0.4); 0.8628 (16.0); 0.0078 (0.4); -0.0002 (9.8); -0.0084 (0.4) |
| I-036 | 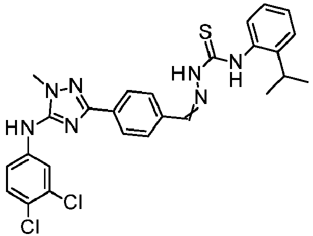 | I-036: ¹H-NMR(600.1 MHz, d₆-DMSO): |
| | | δ= 11.8295 (3.3); 10.0280 (2.9); 9.4000 (3.6); 8.1753 (4.1); 8.1299 (3.2); 8.1255 (3.3); 8.0064 (0.8); 7.9915 (7.3); 7.9885 (8.6); 7.9776 (0.5); 7.9737 (0.9); 7.6819 (1.5); 7.6775 (1.4); 7.6671 (1.9); 7.6628 (1.9); 7.5683 (3.6); 7.5536 (2.7); 7.3695 (1.2); 7.3571 (2.0); 7.3177 (0.8); 7.3143 (0.8); 7.3064 (1.0); 7.3036 (1.3); 7.2940 (0.6); 7.2903 (0.8); 7.2427 (0.5); 7.2403 (0.5); 7.2296 (1.6); 7.2274 (1.6); 7.2186 (3.6); 7.2164 (3.0); 7.2055 (0.6); 4.3536 (0.4); 4.3452 (0.9); 4.3367 (0.5); 3.8466 (0.7); 3.8211 (16.0); 3.4550 (0.7); 3.4466 (0.7); 3.4433 (0.7); 3.4349 (0.7); 3.3286 (12.0); 3.1652 (0.3); 3.1538 (0.9); 3.1423 (1.2); 3.1308 (0.9); 3.1193 (0.4); 2.5101 (5.4); 2.5071 (11.8); 2.5041 (16.4); 2.5011 (11.8); 2.4981 (5.4); 1.2023 (13.1); 1.1908 (13.2); 1.0715 (1.9); 1.0599 (3.7); 1.0482 (1.8); 0.0053 (0.4); - 0.0001 (14.2); -0.0056 (0.4) |
| I-037 | 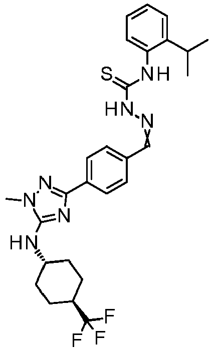 | I-037: ¹H-NMR(400.2 MHz, d₆-DMSO): |
| | | δ= 11.8003 (3.3); 10.0012 (3.2); 8.1533 (4.0); 7.9045 (16.0); 7.3721 (1.4); 7.3532 (2.5); 7.3212 (0.8); 7.3088 (1.1); 7.3002 (1.3); 7.2919 (0.9); 7.2806 (0.7); 7.2474 (0.3); 7.2253 (4.0); 7.2159 (3.9); 6.3876 (1.8); 6.3688 (1.8); 4.3652 (0.7); 4.3526 (1.4); 4.3399 (0.7); 3.5858 (13.3); 3.5551 (0.9); 3.4777 (0.4); 3.4604 (1.2); 3.4471 (1.3); 3.4433 (1.4); 3.4300 (1.1); 3.4125 (0.4); 3.3309 (34.1); 3.1668 (0.4); 3.1493 (0.9); 3.1321 (1.2); 3.1151 (1.0); 3.0979 (0.4); 2.6748 (0.4); 2.5062 (55.7); 2.3325 (0.5); 2.2962 (0.6); 2.2750 (0.6); 2.1531 (2.0); 2.1267 (1.6); 1.9514 (1.6); 1.9225 (2.2); 1.4577 (0.4); 1.4282 (1.4); 1.3935 (2.5); 1.3619 (2.6); 1.3293 (1.2); 1.2983 (0.4); 1.2014 (12.2); 1.1843 (12.0); 1.0780 (2.2); 1.0605 (4.2); 1.0431 (2.2) |
| I-038 | 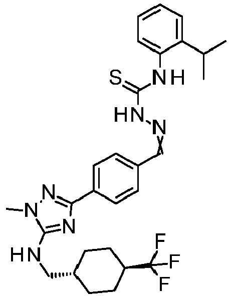 | I-038: ¹H-NMR(400.2 MHz, d₆-DMSO): |
| | | δ= 11.7951 (3.0); 10.0064 (2.6); 9.9975 (1.8); 8.1535 (5.0); 7.9212 (1.1); 7.8998 (13.4); 7.8773 (0.9); 7.8722 (0.9); 7.3676 (1.6); 7.3485 (3.1); 7.3168 (1.0); 7.3095 (1.2); 7.3022 (1.3); 7.2957 (1.7); 7.2896 (0.9); 7.2841 (0.8); 7.2759 (1.0); 7.2433 (0.5); 7.2199 (3.4); 7.2163 (3.7); 7.2089 (4.8); 6.6450 (0.6); 3.5930 (10.9); 3.5898 (12.6); 3.4128 (0.3); 3.3464 (13.3); 3.1967 (0.9); 3.1804 (1.5); 3.1647 (1.2); 3.1467 (1.2); 3.1294 (1.6); 3.1123 (1.2); 3.0953 (0.5); 2.6766 (0.4); 2.6722 (0.5); 2.5076 (60.1); 2.5033 (74.7); 2.4990 (55.0); 2.3300 (0.6); 2.2846 (0.5); 2.2751 (0.5); 2.2435 (0.4); 1.9840 (0.7); 1.9104 (1.7); 1.8822 (1.6); 1.6638 (1.0); 1.6542 (1.2); 1.6329 (3.7); 1.6132 (3.3); 1.5869 (1.7); 1.5825 (1.8); 1.5567 (1.3); 1.5378 (1.0); 1.5271 (0.9); 1.5025 (0.4); 1.2390 (1.0); 1.2215 (1.5); 1.1969 (16.0); 1.1797 (15.8); 1.1489 (0.4); 1.1388 (0.4); 1.0736 (0.5); 1.0556 (0.5); 1.0397 (0.8); 1.0169 (0.7); 0.1457 (0.4); -0.0002 (92.6); - 0.0084 (5.0); -0.1498 (0.4) |
| I-039 | 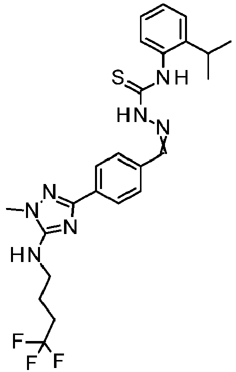 | I-039: ¹H-NMR(400.2 MHz, d₆-DMSO): |
| | | δ= 11.7943 (3.6); 10.0026 (3.3); 8.1510 (4.5); 7.9298 (0.7); 7.9062 (14.6); 7.8902 (0.6); 7.8833 (0.8); 7.3681 (1.3); 7.3495 (2.6); 7.3181 (0.9); 7.3114 (1.0); 7.3033 (1.1); 7.2969 (1.4); 7.2917 (0.7); 7.2848 (0.7); 7.2773 (0.8); 7.2444 (0.4); 7.2411 (0.4); 7.2246 (2.1); 7.2214 (2.4); 7.2158 (3.1); 7.2094 (4.0); 7.1965 (0.5); 6.6270 (1.0); 6.6131 (2.0); 6.5990 (1.0); 4.3619 (0.4); 4.3492 (0.8); 4.3365 (0.4); 3.5879 (16.0); 3.4571 (0.7); 3.4443 (0.7); 3.4396 (0.7); 3.4269 (0.7); 3.4093 (0.5); 3.4010 (0.9); 3.3846 (2.4); 3.3694 (2.4); 3.3530 (1.0); 3.3281 (19.9); 3.1648 (0.4); 3.1478 (1.0); 3.1307 (1.3); 3.1135 (1.0); 3.0963 (0.4); 2.5069 (27.9); 2.5024 (35.7); 2.4980 (26.3); 2.4096 (0.4); 2.4003 (1.0); 2.3807 (1.1); 2.3713 (1.2); 2.3601 (1.3); 2.3518 (1.1); 2.3424 (0.7); 2.3311 (1.2); 2.3020 (0.3); 1.8693 (0.6); 1.8518 (1.6); 1.8316 (1.9); 1.8126 (1.5); 1.7951 (0.5); 1.1982 (13.8); 1.1809 (13.5); 1.0746 (1.3); 1.0571 (2.6); 1.0396 (1.4); 0.0075 (2.7); -0.0002 (46.7); -0.0083 (2.3) |
| I-040 | 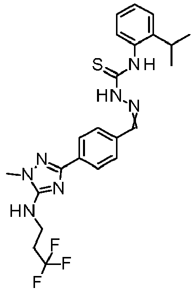 | I-040: ¹H-NMR(400.2 MHz, d₆-DMSO): |
| | | δ= 11.8015 (3.1); 10.0046 (2.8); 8.1534 (4.1); 7.9381 (0.5); 7.9166 (16.0); 7.8945 (0.5); 7.3684 (1.2); 7.3496 (2.3); 7.3181 (0.7); 7.3111 (0.8); 7.3036 (1.0); 7.2970 (1.2); 7.2851 (0.6); 7.2773 (0.7); 7.2450 (0.4); 7.2221 (2.4); 7.2174 (2.8); 7.2099 (3.6); 7.1985 (0.4); 6.8355 (0.9); 6.8216 (1.7); 6.8076 (0.9); 3.5847 (13.6); 3.5640 (1.1); 3.5471 (2.2); 3.5316 (2.2); 3.5148 (1.0); 3.3300 (14.4); 3.1657 (0.3); 3.1487 (0.8); 3.1315 (1.2); 3.1143 (0.9); 3.0974 (0.4); 2.6839 (0.6); 2.6731 (1.1); 2.6555 (1.5); 2.6446 (1.1); 2.6381 (1.0); 2.6269 (1.5); 2.6156 (0.5); 2.6094 (0.9); 2.5982 (0.6); 2.5066 (19.4); 2.5026 (24.4); 2.4985 (18.5); 1.1986 (12.0); 1.1815 (11.7); -0.0002 (27.9) |
| I-041 | 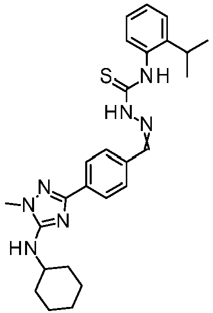 | I-041: ¹H-NMR(400.2 MHz, d₆-DMSO): |
| | | δ= 11.7947 (3.2); 9.9937 (3.0); 8.1487 (4.1); 7.9186 (0.5); 7.8956 (16.0); 7.8753 (0.5); 7.8728 (0.5); 7.3679 (1.2); 7.3489 (2.4); 7.3170 (0.8); 7.3082 (1.0); 7.3043 (0.9); 7.2956 (1.2); 7.2873 (0.8); 7.2761 (0.7); 7.2218 (3.9); 7.2124 (3.6); 6.2507 (1.7); 6.2315 (1.8); 3.5776 (15.6); 3.5567 (0.7); 3.5470 (0.6); 3.3257 (30.8); 3.1635 (0.3); 3.1464 (0.9); 3.1292 (1.2); 3.1119 (0.9); 3.0952 (0.4); 2.5105 (20.3); 2.5064 (38.9); 2.5019 (49.4); 2.4974 (35.7); 2.4930 (17.6); 2.0042 (1.5); 1.9796 (1.2); 1.7584 (1.0); 1.7533 (1.0); 1.7282 (1.6); 1.6322 (0.6); 1.6003 (0.7); 1.3455 (0.9); 1.3121 (1.8); 1.2829 (2.4); 1.2580 (1.0); 1.2511 (1.0); 1.2208 (0.5); 1.1979 (13.0); 1.1807 (12.8); 1.1347 (0.5); 0.0077 (3.4); -0.0002 (71.2); -0.0086 (3.1); -0.1496 (0.3) |
| I-042 | 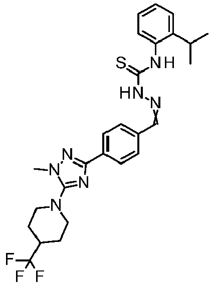 | I-042: ¹H-NMR(400.2 MHz, d₆-DMSO): |
| | | δ= 11.8068 (3.0); 10.0203 (2.7); 8.1603 (4.0); 7.9610 (0.4); 7.9382 (16.0); 7.9156 (0.5); 7.3692 (1.1); 7.3509 (2.1); 7.3197 (0.8); 7.3140 (0.8); 7.3040 (1.0); 7.2984 (1.2); 7.2852 (0.6); 7.2789 (0.7); 7.2445 (0.5); 7.2408 (0.4); 7.2249 (1.7); 7.2214 (1.8); 7.2119 (2.8); 7.2065 (3.5); 7.1933 (0.5); 3.7230 (14.7); 3.5741 (1.4); 3.5437 (1.6); 3.3243 (13.6); 3.1481 (0.8); 3.1309 (1.2); 3.1138 (0.9); 3.0968 (0.3); 2.9828 (1.1); 2.9562 (2.0); 2.9517 (2.0); 2.9253 (1.0); 2.5906 (0.4); 2.5817 (0.5); 2.5695 (0.4); 2.5601 (0.5); 2.5511 (0.5); 2.5377 (0.4); 2.5108 (16.4); 2.5065 (31.2); 2.5021 (39.4); 2.4975 (28.4); 2.4932 (14.0); 1.9130 (1.2); 1.8854 (1.5); 1.7302 (0.5); 1.7199 (0.6); 1.6985 (1.2); 1.6887 (1.3); 1.6671 (1.2); 1.6577 (1.1); 1.6360 (0.4); 1.1982 (12.1); 1.1810 (11.9); 0.0078 (3.1); -0.0002 (60.9); -0.0085 (2.8) |
| I-043 | 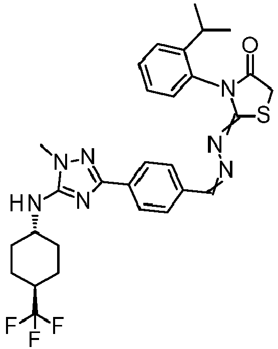 | I-043: ¹H-NMR(400.2 MHz, d₆-DMSO): |
| | | δ= 8.3092 (5.6); 7.9398 (4.2); 7.9191 (5.3); 7.7574 (5.1); 7.7366 (4.2); 7.5158 (1.1); 7.4991 (2.8); 7.4842 (1.6); 7.4663 (2.0); 7.4481 (0.8); 7.3518 (0.9); 7.3482 (0.9); 7.3319 (1.9); 7.3149 (1.2); 7.3113 (1.2); 7.2680 (2.8); 7.2489 (1.7); 6.3980 (2.0); 6.3792 (2.0); 4.2688 (1.9); 4.2255 (3.5); 4.1532 (3.6); 4.1099 (1.8); 3.5850 (16.0); 3.5575 (0.9); 3.5477 (0.9); 3.3258 (28.3); 3.3035 (0.4); 2.8182 (0.4); 2.8016 (1.1); 2.7843 (1.5); 2.7675 (1.1); 2.7504 (0.4); 2.6713 (0.4); 2.5056 (45.9); 2.5019 (55.1); 2.3276 (0.6); 2.2940 (0.6); 2.2722 (0.6); 2.2438 (0.4); 2.1461 (2.2); 2.1204 (1.7); 2.0984 (0.5); 1.9470 (1.6); 1.9196 (2.4); 1.4509 (0.5); 1.4222 (1.4); 1.3884 (3.1); 1.3592 (3.2); 1.3255 (1.3); 1.2983 (0.5); 1.1639 (7.3); 1.1466 (8.4); 1.1399 (8.2); 1.1226 (7.0); -0.0002 (55.5) |
| I-044 | 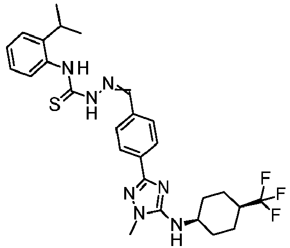 | I-044: ¹H-NMR(400.2 MHz, d₆-DMSO): |
| | | δ= 11.8003 (3.3); 10.0012 (3.2); 8.1533 (4.0); 7.9045 (16.0); 7.3721 (1.4); 7.3532 (2.5); 7.3212 (0.8); 7.3088 (1.1); 7.3002 (1.3); 7.2919 (0.9); 7.2806 (0.7); 7.2474 (0.3); 7.2253 (4.0); 7.2159 (3.9); 6.3876 (1.8); 6.3688 (1.8); 4.3652 (0.7); 4.3526 (1.4); 4.3399 (0.7); 3.5858 (13.3); 3.5551 (0.9); 3.4777 (0.4); 3.4604 (1.2); 3.4471 (1.3); 3.4433 (1.4); 3.4300 (1.1); 3.4125 (0.4); 3.3309 (34.1); 3.1668 (0.4); 3.1493 (0.9); 3.1321 (1.2); 3.1151 (1.0); 3.0979 (0.4); 2.6748 (0.4); 2.5062 (55.7); 2.3325 (0.5); 2.2962 (0.6); 2.2750 (0.6); 2.1531 (2.0); 2.1267 (1.6); 1.9514 (1.6); 1.9225 (2.2); 1.4577 (0.4); 1.4282 (1.4); 1.3935 (2.5); 1.3619 (2.6); 1.3293 (1.2); 1.2983 (0.4); 1.2014 (12.2); 1.1843 (12.0); 1.0780 (2.2); 1.0605 (4.2); 1.0431 (2.2) |
| I-045 | 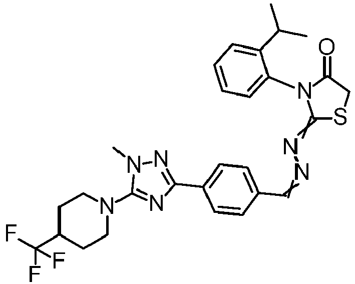 | I-045: ¹H-NMR(400.2 MHz, d₆-DMSO): |
| | | δ= 8.3292 (5.5); 7.9793 (4.2); 7.9587 (5.3); 7.7912 (5.1); 7.7705 (4.2); 7.5165 (1.2); 7.4975 (2.9); 7.4848 (1.7); 7.4667 (2.1); 7.4478 (0.8); 7.3527 (0.9); 7.3493 (0.9); 7.3329 (2.0); 7.3157 (1.2); 7.3126 (1.2); 7.2702 (2.8); 7.2518 (1.7); 4.3629 (1.0); 4.3503 (2.0); 4.3376 (1.1); 4.2738 (1.9); 4.2305 (3.5); 4.1576 (3.6); 4.1143 (1.8); 3.7256 (16.0); 3.5729 (2.1); 3.5417 (2.3); 3.4746 (0.6); 3.4573 (1.7); 3.4444 (1.9); 3.4400 (1.9); 3.4271 (1.7); 3.4096 (0.6); 3.3279 (20.5); 2.9822 (1.5); 2.9518 (2.9); 2.9241 (1.5); 2.8178 (0.4); 2.8009 (1.1); 2.7839 (1.5); 2.7669 (1.1); 2.7496 (0.5); 2.6097 (0.4); 2.5884 (0.6); 2.5802 (0.8); 2.5689 (0.7); 2.5587 (0.8); 2.5492 (0.8); 2.5023 (37.8); 1.9124 (1.8); 1.8831 (2.4); 1.7261 (0.7); 1.7171 (0.8); 1.6949 (1.8); 1.6859 (1.9); 1.6640 (1.6); 1.6545 (1.6); 1.6326 (0.6); 1.6231 (0.5); 1.1634 (7.4); 1.1458 (9.0); 1.1406 (9.0); 1.1231 (7.1); 1.0747 (3.4); 1.0572 (6.5); 1.0397 (3.2); -0.0002 (30.5) |
| I-046 | 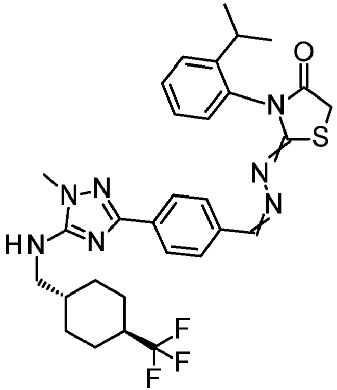 | I-046: ¹H-NMR(400.2 MHz, d₆-DMSO): |
| | | δ= 8.3168 (1.5); 8.3079 (7.3); 7.9338 (2.8); 7.9264 (3.6); 7.9128 (3.6); 7.9055 (4.5); 7.7551 (6.5); 7.7341 (5.3); 7.5154 (1.4); 7.4995 (3.4); 7.4959 (3.4); 7.4855 (2.0); 7.4827 (2.0); 7.4654 (2.5); 7.4485 (1.0); 7.4455 (1.0); 7.3523 (1.2); 7.3480 (1.2); 7.3326 (2.4); 7.3286 (2.2); 7.3151 (1.6); 7.3110 (1.6); 7.2692 (3.4); 7.2668 (3.4); 7.2498 (2.1); 7.2469 (2.0); 6.5906 (0.8); 6.5766 (1.5); 6.5621 (0.8); 6.5498 (0.6); 6.5357 (1.1); 6.5215 (0.5); 4.6480 (1.0); 4.2696 (2.6); 4.2263 (4.6); 4.1536 (4.8); 4.1420 (0.4); 4.1103 (2.6); 3.6708 (1.6); 3.5881 (13.9); 3.5855 (16.0); 3.3280 (40.0); 3.1905 (1.1); 3.1747 (1.8); 3.1594 (1.1); 2.8184 (0.5); 2.8012 (1.3); 2.7841 (1.8); 2.7670 (1.4); 2.7500 (0.5); 2.6756 (0.4); 2.6714 (0.5); 2.6667 (0.4); 2.5067 (69.0); 2.5023 (88.5); 2.4978 (64.7); 2.3335 (0.6); 2.3292 (0.8); 2.3246 (0.7); 2.2904 (0.5); 2.2767 (0.5); 2.2679 (0.5); 2.2437 (0.4); 2.2359 (0.4); 2.2125 (0.4); 2.0978 (1.8); 1.9852 (0.7); 1.9079 (1.9); 1.8806 (2.0); 1.7982 (0.4); 1.6561 (1.6); 1.6293 (4.3); 1.5981 (2.0); 1.5757 (2.1); 1.5507 (1.7); 1.5413 (1.1); 1.5306 (1.1); 1.5046 (0.4); 1.2782 (0.4); 1.2456 (0.9); 1.2388 (0.9); 1.2215 (1.4); 1.2150 (1.0); 1.1972 (0.6); 1.1898 (0.6); 1.1796 (0.7); 1.1633 (9.4); 1.1460 (10.7); 1.1395 (10.7); 1.1223 (9.3); 1.0976 (0.4); 1.0740 (1.2); 1.0565 (1.8); 1.0392 (1.6); 1.0149 (0.8); 0.9846 (0.3); 0.1459 (0.5); 0.0078 (5.5); -0.0002 (110.2); - 0.0084 (5.0); -0.1496 (0.5) |
| I-047 | 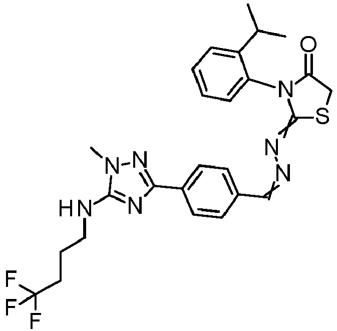 | I-047: ¹H-NMR(400.2 MHz, d₆-DMSO): |
| | | δ= 8.3110 (5.5); 7.9414 (4.0); 7.9205 (5.1); 7.7619 (4.9); 7.7411 (4.0); 7.5157 (1.0); 7.4997 (2.5); 7.4962 (2.5); 7.4855 (1.4); 7.4830 (1.4); 7.4658 (1.8); 7.4484 (0.7); 7.4458 (0.7); 7.3524 (0.8); 7.3483 (0.8); 7.3327 (1.7); 7.3290 (1.6); 7.3153 (1.2); 7.3113 (1.1); 7.2699 (2.5); 7.2680 (2.5); 7.2506 (1.6); 6.6334 (1.0); 6.6196 (2.0); 6.6055 (1.0); 4.2709 (1.9); 4.2275 (3.5); 4.1546 (3.6); 4.1113 (1.9); 3.5909 (16.0); 3.3991 (1.0); 3.3827 (2.5); 3.3676 (2.5); 3.3511 (1.1); 3.3271 (30.9); 2.8181 (0.4); 2.8012 (1.0); 2.7840 (1.4); 2.7669 (1.0); 2.7498 (0.4); 2.6712 (0.3); 2.5065 (41.6); 2.5022 (52.0); 2.4978 (38.0); 2.4285 (0.3); 2.4086 (0.4); 2.3990 (1.0); 2.3794 (1.1); 2.3702 (1.3); 2.3588 (1.3); 2.3504 (1.1); 2.3297 (1.4); 2.3006 (0.4); 1.8650 (0.6); 1.8474 (1.7); 1.8276 (2.0); 1.8083 (1.6); 1.7910 (0.5); 1.1632 (6.9); 1.1460 (7.9); 1.1397 (7.7); 1.1225 (6.6); 1.0741 (0.4); 1.0566 (0.7); 1.0391 (0.4); -0.0002 (54.5); -0.0083 (2.6) |
| I-048 | 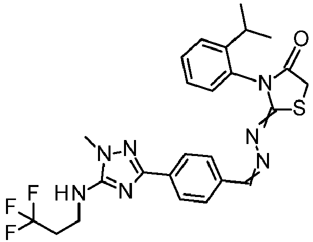 | I-048: ¹H-NMR(400.2 MHz, d₆-DMSO): |
| | | δ= 8.3162 (5.9); 7.9550 (4.2); 7.9343 (5.2); 7.7700 (5.0); 7.7491 (4.2); 7.5162 (1.1); 7.4996 (2.8); 7.4969 (2.7); 7.4857 (1.6); 7.4662 (2.0); 7.4486 (0.8); 7.3527 (0.9); 7.3488 (0.9); 7.3330 (1.9); 7.3157 (1.2); 7.3120 (1.2); 7.2699 (2.7); 7.2510 (1.7); 6.8454 (1.1); 6.8317 (2.1); 6.8178 (1.1); 4.2718 (1.9); 4.2284 (3.5); 4.1553 (3.6); 4.1120 (1.8); 3.5873 (16.0); 3.5614 (1.3); 3.5449 (2.6); 3.5294 (2.7); 3.5124 (1.3); 3.3285 (22.1); 3.3063 (0.4); 2.8202 (0.4); 2.8031 (1.0); 2.7860 (1.4); 2.7689 (1.1); 2.7521 (0.4); 2.7009 (0.3); 2.6827 (0.7); 2.6720 (1.4); 2.6544 (1.8); 2.6432 (1.4); 2.6371 (1.3); 2.6258 (1.8); 2.6083 (1.1); 2.5974 (0.7); 2.5795 (0.4); 2.5063 (32.9); 2.5024 (39.8); 1.1647 (7.2); 1.1474 (8.3); 1.1406 (8.1); 1.1233 (6.9); -0.0002 (36.3) |
| I-049 | 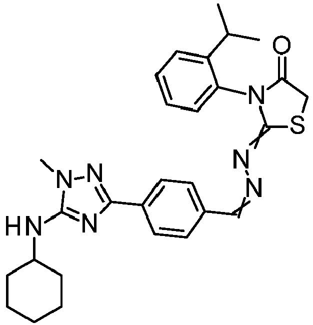 | I-049: ¹H-NMR(400.2 MHz, d₆-DMSO): |
| | | δ= 8.3154 (0.4); 8.3066 (5.1); 7.9322 (3.6); 7.9113 (4.8); 7.7551 (4.5); 7.7341 (3.6); 7.5197 (0.7); 7.5157 (0.9); 7.5001 (2.2); 7.4960 (2.2); 7.4860 (1.2); 7.4828 (1.2); 7.4684 (1.5); 7.4655 (1.6); 7.4489 (0.6); 7.4457 (0.6); 7.3524 (0.8); 7.3481 (0.8); 7.3328 (1.6); 7.3286 (1.4); 7.3154 (1.1); 7.3111 (1.1); 7.2691 (2.2); 7.2663 (2.2); 7.2496 (1.4); 7.2465 (1.2); 6.2623 (1.7); 6.2431 (1.7); 4.6477 (0.4); 4.2691 (1.9); 4.2258 (3.2); 4.1535 (3.4); 4.1101 (1.8); 3.6705 (0.6); 3.5807 (16.0); 3.5527 (0.6); 3.5441 (0.6); 3.3274 (65.0); 2.8183 (0.3); 2.8014 (0.9); 2.7842 (1.2); 2.7671 (0.9); 2.7498 (0.4); 2.6709 (0.4); 2.5241 (1.5); 2.5109 (24.7); 2.5065 (47.9); 2.5019 (61.3); 2.4974 (43.5); 2.4929 (20.6); 2.3288 (0.4); 2.0975 (0.7); 2.0024 (1.4); 1.9776 (1.2); 1.7588 (1.0); 1.7507 (0.9); 1.7274 (1.5); 1.6329 (0.6); 1.6005 (0.6); 1.3430 (0.9); 1.3092 (1.7); 1.2806 (2.3); 1.2557 (0.9); 1.2487 (1.0); 1.2251 (0.3); 1.2185 (0.4); 1.1641 (6.7); 1.1469 (7.0); 1.1394 (6.9); 1.1223 (6.2); 1.0737 (0.4); 1.0562 (0.6); 0.1459 (0.3); 0.0079 (3.8); -0.0002 (79.3); -0.0085 (2.9); -0.1497 (0.3) |
| I-050 | 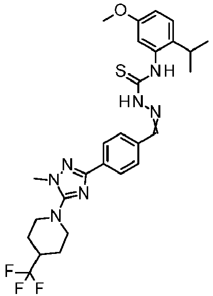 | I-050: ¹H-NMR(400.2 MHz, d₆-DMSO): |
| | | δ= 11.8222 (2.5); 9.9888 (2.3); 8.1610 (3.5); 7.9536 (0.4); 7.9388 (16.0); 7.2600 (2.0); 7.2384 (2.2); 6.9050 (1.1); 6.8983 (1.3); 6.8835 (1.0); 6.8767 (1.2); 6.8211 (2.5); 6.8144 (2.2); 4.3519 (0.5); 3.7445 (14.7); 3.7239 (11.9); 3.5743 (1.2); 3.5436 (1.4); 3.4582 (0.5); 3.4455 (0.5); 3.4408 (0.5); 3.4280 (0.5); 3.3322 (11.2); 3.0687 (0.7); 3.0516 (1.0); 3.0344 (0.7); 2.9822 (1.0); 2.9555 (1.7); 2.9511 (1.7); 2.9245 (0.9); 2.5894 (0.3); 2.5806 (0.4); 2.5692 (0.4); 2.5590 (0.4); 2.5498 (0.4); 2.5256 (0.5); 2.5075 (14.2); 2.5031 (18.2); 2.4987 (13.4); 1.9129 (1.0); 1.8854 (1.4); 1.7299 (0.4); 1.7201 (0.5); 1.6986 (1.1); 1.6891 (1.2); 1.6674 (1.0); 1.6578 (1.0); 1.6362 (0.3); 1.1711 (9.9); 1.1539 (9.7); 1.0756 (1.0); 1.0582 (1.9); 1.0406 (1.0) |
| I-051 | 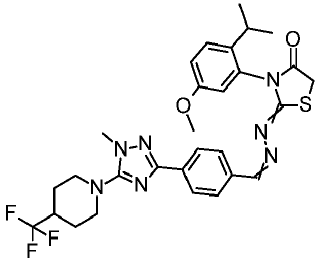 | I-051: ¹H-NMR(600.1 MHz, d₆-DMSO): |
| | | δ= 8.3470 (4.3); 7.9770 (3.1); 7.9630 (3.8); 7.7907 (3.6); 7.7768 (3.1); 7.4004 (2.2); 7.3858 (2.4); 7.0617 (1.3); 7.0572 (1.4); 7.0472 (1.2); 7.0426 (1.3); 6.8806 (2.7); 6.8760 (2.6); 4.2476 (1.9); 4.2189 (2.5); 4.1166 (2.7); 4.0879 (1.8); 3.7541 (16.0); 3.7265 (13.9); 3.5691 (1.2); 3.5485 (1.3); 3.3205 (62.6); 2.9746 (1.0); 2.9571 (1.8); 2.9538 (1.8); 2.9363 (0.9); 2.7120 (0.8); 2.7008 (1.0); 2.6894 (0.8); 2.6137 (0.3); 2.5834 (0.3); 2.5776 (0.4); 2.5697 (0.3); 2.5630 (0.4); 2.5228 (0.7); 2.5198 (0.9); 2.5166 (0.9); 2.5075 (15.6); 2.5047 (32.6); 2.5018 (44.7); 2.4988 (32.7); 2.4961 (15.7); 1.9085 (1.1); 1.8894 (1.3); 1.7118 (0.4); 1.7053 (0.5); 1.6908 (1.1); 1.6843 (1.2); 1.6699 (1.1); 1.6636 (1.0); 1.6490 (0.4); 1.1281 (5.3); 1.1167 (5.3); 1.1018 (5.4); 1.0904 (5.3); 0.0053 (1.3); -0.0001 (36.9); -0.0056 (1.5) |
| I-052 | 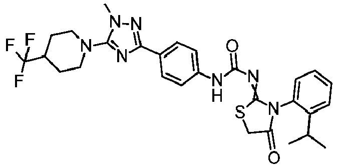 | I-052: ¹H-NMR(400.2 MHz, d₆-DMSO): |
| | | δ= 9.8869 (2.4); 8.1528 (0.4); 7.7946 (2.2); 7.7727 (3.6); 7.7021 (3.4); 7.6802 (2.3); 7.5251 (1.0); 7.5082 (1.8); 7.5057 (1.8); 7.4776 (0.9); 7.4603 (1.3); 7.4414 (0.6); 7.3557 (0.7); 7.3524 (0.7); 7.3359 (1.3); 7.3180 (0.8); 7.3149 (0.8); 7.2636 (1.8); 7.2613 (1.8); 7.2441 (1.2); 4.2362 (1.3); 4.1911 (2.2); 4.1021 (2.5); 4.0571 (1.4); 3.6787 (16.0); 3.5353 (1.4); 3.5041 (1.6); 3.3355 (8.6); 2.9533 (1.1); 2.9264 (2.0); 2.9221 (2.0); 2.8959 (1.1); 2.7283 (0.8); 2.7112 (1.2); 2.6941 (0.9); 2.6764 (0.4); 2.5752 (0.4); 2.5660 (0.5); 2.5538 (0.4); 2.5442 (0.5); 2.5353 (0.4); 2.5240 (0.8); 2.5191 (1.2); 2.5105 (12.1); 2.5062 (23.8); 2.5016 (30.6); 2.4971 (21.7); 2.4927 (10.4); 2.0751 (5.8); 1.9029 (1.2); 1.8722 (1.5); 1.7127 (0.5); 1.7026 (0.6); 1.6810 (1.2); 1.6714 (1.3); 1.6498 (1.2); 1.6402 (1.1); 1.6186 (0.4); 1.6087 |
| | | (0.3); 1.1923 (4.2); 1.1753 (4.1); 1.1208 (5.7); 1.1037 (5.6); 0.0079 (0.3); -0.0002 (8.6) |
| I-053 | 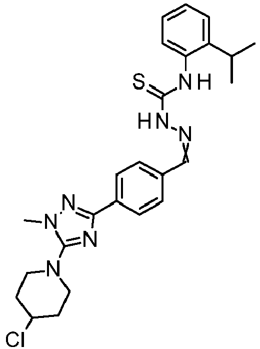 | I-053: ¹H-NMR(400.2 MHz, d₆-DMSO): |
| | | δ= 11.8071 (2.5); 10.0212 (2.2); 8.1602 (3.6); 7.9384 (16.0); 7.3692 (0.9); 7.3507 (1.8); 7.3197 (0.6); 7.3138 (0.7); 7.3039 (0.8); 7.2984 (1.0); 7.2851 (0.5); 7.2788 (0.6); 7.2446 (0.4); 7.2409 (0.4); 7.2250 (1.4); 7.2215 (1.6); 7.2124 (2.3); 7.2065 (3.0); 7.1934 (0.4); 4.4600 (0.4); 4.4489 (0.6); 4.4387 (0.9); 4.4284 (0.6); 4.4175 (0.5); 3.7200 (13.5); 3.4486 (0.6); 3.4388 (0.9); 3.4177 (0.8); 3.4031 (1.0); 3.3933 (0.7); 3.3289 (27.6); 3.1644 (0.4); 3.1554 (1.0); 3.1477 (1.7); 3.1308 (1.7); 3.1251 (1.7); 3.1145 (1.3); 3.1015 (0.9); 3.0945 (0.9); 2.5245 (0.9); 2.5197 (1.4); 2.5111 (15.5); 2.5067 (31.1); 2.5022 (40.1); 2.4977 (27.9); 2.4932 (12.9); 2.2344 (0.4); 2.2261 (0.7); 2.2178 (0.7); 2.2113 (0.7); 2.2015 (0.8); 2.1927 (0.9); 2.1857 (0.9); 2.1779 (0.8); 1.9805 (0.5); 1.9717 (0.5); 1.9590 (0.9); 1.9496 (1.2); 1.9380 (0.8); 1.9266 (1.0); 1.9170 (0.7); 1.9044 (0.4); 1.8953 (0.3); 1.1980 (10.7); 1.1808 (10.5); 0.0079 (1.7); -0.0002 (41.8); - 0.0086 (1.3) |
| I-054 | 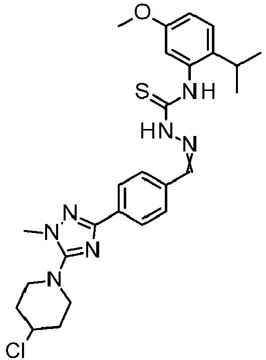 | I-054: ¹H-NMR(400.2 MHz, d₆-DMSO): |
| | | δ= 11.8190 (2.4); 9.9868 (2.1); 8.1594 (3.4); 7.9523 (0.4); 7.9370 (16.0); 7.2595 (1.8); 7.2379 (2.1); 6.9043 (1.0); 6.8975 (1.2); 6.8828 (0.9); 6.8760 (1.1); 6.8203 (2.2); 6.8137 (1.8); 4.4603 (0.4); 4.4492 (0.6); 4.4389 (0.8); 4.4286 (0.6); 4.4179 (0.4); 3.7436 (14.2); 3.7206 (11.5); 3.4489 (0.5); 3.4396 (0.8); 3.4177 (0.8); 3.4040 (0.9); 3.3933 (0.7); 3.3296 (23.5); 3.1557 (0.8); 3.1481 (0.9); 3.1338 (0.9); 3.1249 (1.3); 3.1161 (0.8); 3.1016 (0.8); 3.0940 (0.7); 3.0671 (0.6); 3.0498 (0.9); 3.0327 (0.6); 2.5247 (0.6); 2.5112 (10.9); 2.5070 (21.9); 2.5025 (28.5); 2.4980 (20.2); 2.4937 (9.6); 2.2263 (0.6); 2.2182 (0.7); 2.2112 (0.6); 2.2019 (0.7); 2.1931 (0.8); 2.1860 (0.9); 2.1785 (0.8); 1.9808 (0.4); 1.9721 (0.5); 1.9594 (0.8); 1.9500 (1.1); 1.9382 (0.8); 1.9270 (1.0); 1.9174 (0.7); 1.9045 (0.4); 1.1703 (9.2); 1.1531 (9.0); 0.0078 (1.2); -0.0002 (30.0); -0.0085 (1.1) |
| I-055 | 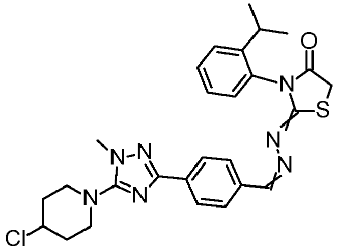 | I-055: ¹H-NMR(400.2 MHz, d₆-DMSO): |
| | | δ= 8.3280 (5.0); 8.3152 (0.4); 7.9787 (3.5); 7.9578 (4.6); 7.7896 (4.3); 7.7685 (3.5); 7.5211 (0.7); 7.5169 (0.8); 7.5013 (2.0); 7.4973 (2.0); 7.4871 (1.1); 7.4838 (1.1); 7.4694 (1.4); 7.4665 (1.4); 7.4499 (0.6); 7.4465 (0.6); 7.3535 (0.8); 7.3491 (0.8); 7.3339 (1.4); 7.3297 (1.4); 7.3164 (1.0); 7.3121 (1.0); 7.2712 (2.1); 7.2682 (2.0); 7.2517 (1.3); 7.2484 (1.2); 4.6475 (0.6); 4.4601 (0.5); 4.4486 (0.7); 4.4385 (1.0); 4.4282 (0.7); 4.4173 (0.5); 4.2737 (1.9); 4.2303 (3.2); 4.1572 (3.4); 4.1138 (1.8); 3.7233 (16.0); 3.6704 (1.0); 3.4488 (0.6); 3.4383 (0.9); 3.4169 (0.9); 3.4034 (1.1); 3.3928 (0.8); 3.3254 (147.6); 3.1558 (0.9); 3.1482 (1.1); 3.1340 (1.1); 3.1251 (1.6); 3.1161 (0.9); 3.1017 (0.9); 3.0942 (0.8); 2.7994 (0.8); 2.7821 (1.1); 2.7651 (0.8); 2.6754 (0.6); 2.6709 (0.9); 2.6662 (0.6); 2.5244 (2.3); 2.5196 (3.3); 2.5109 (50.2); 2.5065 (103.1); 2.5019 (135.9); 2.4973 (96.5); 2.4927 (45.5); 2.3332 (0.6); 2.3287 (0.9); 2.3241 (0.6); 2.2333 (0.4); 2.2243 (0.7); 2.2168 (0.8); 2.2090 (0.7); 2.2000 (0.8); 2.1917 (1.0); 2.1843 (1.0); 2.1768 (0.9); 2.1678 (0.5); 2.0974 (1.2); 1.9789 (0.5); 1.9700 (0.6); 1.9572 (1.0); 1.9478 (1.3); 1.9359 (0.9); 1.9246 (1.2); 1.9151 (0.8); 1.9023 (0.5); 1.8935 (0.4); 1.1630 (5.8); 1.1458 (6.3); 1.1396 (6.3); 1.1224 (5.7); -0.0001 (8.8) |
| I-056 | 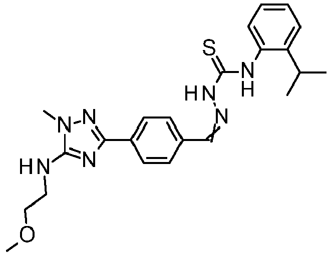 | I-056: ¹H-NMR(400.2 MHz, d₆-DMSO): |
| | | δ= 11.7930 (3.2); 10.0044 (2.9); 8.1520 (3.9); 7.9266 (0.5); 7.9043 (16.0); 7.8844 (0.5); 7.8820 (0.5); 7.3677 (1.1); 7.3491 (2.3); 7.3176 (0.8); 7.3105 (0.9); 7.3031 (0.9); 7.2964 (1.2); 7.2906 (0.6); 7.2846 (0.6); 7.2767 (0.7); 7.2442 (0.4); 7.2411 (0.4); 7.2244 (1.9); 7.2214 (2.3); 7.2167 (2.7); 7.2098 (3.4); 7.1976 (0.4); 6.6400 (0.9); 6.6263 (1.8); 6.6128 (0.9); 3.5848 (13.6); 3.5359 (1.2); 3.5222 (3.8); 3.5100 (3.2); 3.4859 (1.6); 3.4739 (2.8); 3.4603 (2.0); 3.4448 (0.6); 3.3337 (35.6); 3.2898 (19.2); 3.1651 (0.3); 3.1479 (0.8); 3.1307 (1.1); 3.1135 (0.9); 3.0966 (0.4); 2.5068 (19.4); 2.5025 (24.7); 2.4981 (18.4); 1.1981 (11.6); 1.1809 (11.4); 1.0573 (0.4); -0.0002 (0.5) |
| I-057 | 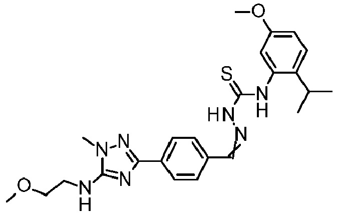 | I-057: ¹H-NMR(400.2 MHz, d₆-DMSO): |
| | | δ= 11.8030 (2.5); 9.9683 (2.3); 8.1487 (3.3); 7.9005 (16.0); 7.2578 (1.9); 7.2362 (2.1); 6.9018 (1.1); 6.8951 (1.3); 6.8803 (0.9); 6.8735 (1.2); 6.8253 (2.3); 6.8187 (2.0); 6.6405 (0.7); 6.6268 (1.4); 6.6133 (0.7); 3.7428 (14.0); 3.5843 (11.4); 3.5354 (0.9); 3.5217 (3.1); 3.5097 (2.6); 3.4850 (1.3); 3.4729 (2.2); 3.4594 (1.6); 3.4457 (0.5); 3.3291 (43.6); 3.2898 (16.7); 3.0655 (0.7); 3.0484 (0.9); 3.0312 (0.7); 2.5066 (31.5); 2.5023 (40.0); 2.4979 (29.5); 1.1696 (9.6); 1.1524 (9.4); -0.0002 (0.5) |
| I-058 | 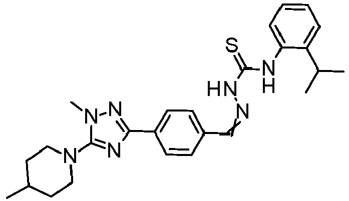 | I-058: ¹H-NMR(400.2 MHz, d₆-DMSO): |
| | | δ= 11.8024 (2.6); 10.0181 (2.3); 8.1589 (3.4); 7.9314 (16.0); 7.3715 (0.8); 7.3686 (0.9); 7.3505 (1.8); 7.3193 (0.6); 7.3133 (0.7); 7.3037 (0.8); 7.2979 (1.0); 7.2935 (0.5); 7.2848 (0.4); 7.2783 (0.6); 7.2443 (0.4); 7.2406 (0.4); 7.2248 (1.4); 7.2211 (1.6); 7.2126 (2.0); 7.2062 (2.9); 7.1934 (0.4); 3.6979 (13.7); 3.4584 (1.2); 3.4272 (1.3); 3.3297 (45.9); 3.1475 (0.7); 3.1304 (0.9); 3.1131 (0.7); 2.8948 (0.7); 2.8891 (0.9); 2.8641 (1.5); 2.8584 (1.6); 2.8335 (0.8); 2.8279 (0.7); 2.5248 (0.5); 2.5201 (0.7); 2.5114 (10.4); 2.5069 (21.8); 2.5023 (29.1); 2.4976 (20.8); 2.4931 (9.8); 1.7181 (0.9); 1.6913 (1.0); 1.6864 (1.0); 1.5641 (0.4); 1.5568 (0.3); 1.5478 (0.4); 1.5371 (0.3); 1.3659 (0.4); 1.3563 (0.5); 1.3347 (0.9); 1.3256 (1.0); 1.3043 (0.8); 1.2968 (0.7); 1.1979 (10.2); 1.1807 (10.0); 0.9697 (5.4); 0.9534 (5.2); - 0.0002 (1.0) |
| I-059 | 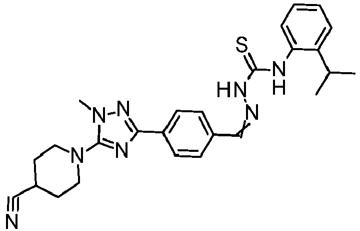 | I-059: ¹H-NMR(400.2 MHz, d₆-DMSO): |
| | | δ= 11.8076 (2.6); 10.0227 (2.4); 8.1615 (3.5); 7.9544 (0.4); 7.9404 (16.0); 7.3721 (0.8); 7.3691 (0.9); 7.3509 (1.8); 7.3201 (0.6); 7.3142 (0.7); 7.3043 (0.8); 7.2988 (1.0); 7.2946 (0.5); 7.2855 (0.5); 7.2791 (0.6); 7.2450 (0.4); 7.2413 (0.4); 7.2255 (1.4); 7.2218 (1.6); 7.2128 (2.2); 7.2068 (3.0); 7.1937 (0.4); 3.7160 (13.5); 3.3468 (0.8); 3.3326 (34.7); 3.3152 (1.0); 3.3058 (1.0); 3.2992 (1.0); 3.2905 (0.8); 3.1476 (1.1); 3.1419 (1.1); 3.1319 (1.7); 3.1261 (1.2); 3.1201 (1.4); 3.1136 (2.4); 3.1044 (1.3); 3.0945 (0.9); 3.0883 (0.9); 3.0811 (0.8); 2.5251 (0.4); 2.5204 (0.6); 2.5117 (7.8); 2.5072 (16.1); 2.5026 (21.3); 2.4980 (15.3); 2.4935 (7.2); 2.0573 (0.6); 2.0489 (0.6); 2.0409 (0.6); 2.0325 (0.8); 2.0238 (0.9); 2.0164 (1.0); 2.0081 (0.9); 1.9992 (0.4); 1.9377 (0.5); 1.9290 (0.6); 1.9163 (1.0); 1.9072 (1.2); 1.8953 (0.8); 1.8838 (1.0); 1.8744 (0.6); 1.8616 (0.4); 1.1984 (10.5); 1.1811 (10.3); -0.0002 (0.7) |
| I-060 | 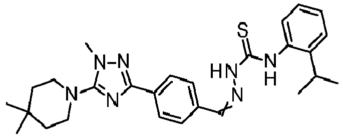 | I-060: ¹H-NMR(400.2 MHz, d₆-DMSO): |
| | | δ= 11.8027 (1.8); 10.0185 (1.6); 8.1600 (2.4); 7.9343 (11.5); 7.3688 (0.6); 7.3506 (1.2); 7.3195 (0.4); 7.3135 (0.5); 7.3038 (0.6); 7.2981 (0.7); 7.2939 (0.3); 7.2850 (0.3); 7.2784 (0.4); 7.2250 (1.0); 7.2214 (1.1); 7.2127 (1.5); 7.2065 (2.0); 3.6974 (9.1); 3.3284 (20.5); 3.1695 (2.0); 3.1557 (2.4); 3.1504 (2.0); 3.1414 (2.1); 3.1311 (0.8); 3.1137 (0.5); 2.5111 (7.1); 2.5066 (14.2); 2.5021 (18.6); 2.4975 (13.3); 2.4930 (6.4); 1.4973 (2.0); |
| | | 1.4833 (2.4); 1.4690 (1.9); 1.1983 (7.0); 1.1811 (6.9); 0.9903 (16.0); -0.0002 (0.6) |
| I-061 | 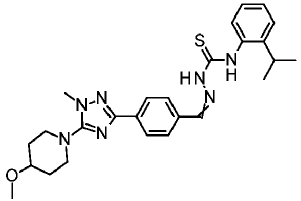 | I-061: ¹H-NMR(400.2 MHz, d₆-DMSO): |
| | | δ= 11.8043 (2.8); 10.0188 (2.5); 8.1591 (3.6); 7.9557 (0.3); 7.9330 (16.0); 7.9104 (0.3); 7.3688 (1.0); 7.3506 (1.9); 7.3194 (0.7); 7.3134 (0.8); 7.3038 (0.9); 7.2981 (1.1); 7.2937 (0.5); 7.2850 (0.5); 7.2785 (0.7); 7.2444 (0.4); 7.2408 (0.4); 7.2249 (1.5); 7.2213 (1.6); 7.2128 (2.3); 7.2065 (3.1); 7.1937 (0.4); 4.3497 (0.5); 3.7091 (14.2); 3.4563 (0.6); 3.4437 (0.5); 3.4389 (0.5); 3.4262 (0.6); 3.4216 (0.4); 3.4123 (0.6); 3.4086 (0.6); 3.4018 (1.2); 3.3916 (1.9); 3.3809 (1.3); 3.3709 (1.3); 3.3602 (1.3); 3.3484 (0.8); 3.3280 (35.6); 3.2824 (21.0); 3.1474 (0.7); 3.1302 (1.0); 3.1131 (0.8); 3.0287 (0.8); 3.0211 (1.0); 3.0051 (1.0); 2.9973 (1.7); 2.9898 (0.9); 2.9737 (0.9); 2.9661 (0.7); 2.5245 (0.6); 2.5199 (0.8); 2.5112 (12.8); 2.5068 (26.3); 2.5022 (34.6); 2.4976 (24.7); 2.4931 (11.8); 1.9904 (0.7); 1.9840 (0.8); 1.9775 (0.7); 1.9672 (0.8); 1.9526 (0.9); 1.9455 (0.8); 1.6559 (0.4); 1.6467 (0.5); 1.6332 (0.8); 1.6239 (1.2); 1.6125 (0.7); 1.6014 (1.1); 1.5927 (0.7); 1.5790 (0.4); 1.5696 (0.3); 1.1980 (11.0); 1.1808 (10.8); 1.1674 (0.5); 1.0741 (1.0); 1.0566 (2.0); 1.0391 (1.1); -0.0002 (0.7) |
| I-062 | 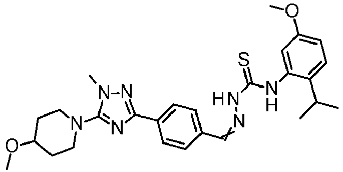 | I-062: ¹H-NMR(400.2 MHz, d₆-DMSO): |
| | | δ= 11.8166 (2.3); 9.9846 (2.1); 8.1583 (3.1); 7.9473 (0.4); 7.9317 (16.0); 7.2595 (1.8); 7.2378 (2.1); 6.9041 (1.0); 6.8973 (1.2); 6.8826 (0.9); 6.8757 (1.1); 6.8212 (2.2); 6.8144 (1.8); 3.7436 (14.4); 3.7320 (0.4); 3.7097 (11.5); 3.4125 (0.5); 3.4020 (1.0); 3.3918 (1.6); 3.3809 (1.2); 3.3710 (1.1); 3.3607 (1.1); 3.3486 (0.6); 3.3289 (21.8); 3.2827 (16.8); 3.0670 (0.6); 3.0498 (0.8); 3.0310 (1.0); 3.0213 (0.9); 3.0053 (0.9); 2.9975 (1.5); 2.9900 (0.7); 2.9738 (0.7); 2.9664 (0.6); 2.5249 (0.4); 2.5115 (8.6); 2.5071 (17.1); 2.5025 (22.2); 2.4980 (15.7); 2.4935 (7.4); 1.9911 (0.6); 1.9838 (0.6); 1.9784 (0.6); 1.9673 (0.7); 1.9580 (0.8); 1.9532 (0.8); 1.9458 (0.7); 1.6562 (0.4); 1.6471 (0.4); 1.6336 (0.7); 1.6244 (1.0); 1.6127 (0.6); 1.6019 (1.0); 1.5930 (0.6); 1.5792 (0.4); 1.1703 (8.8); 1.1531 (8.7); -0.0002 (0.4) |
| I-063 | 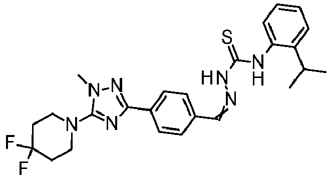 | I-063: ¹H-NMR(400.2 MHz, d₆-DMSO): |
| | | δ= 11.8116 (2.9); 10.0214 (2.6); 8.1615 (3.7); 7.9654 (0.4); 7.9431 (16.0); 7.9210 (0.3); 7.3696 (1.0); 7.3513 (2.0); 7.3202 (0.7); 7.3143 (0.8); 7.3045 (0.9); 7.2990 (1.1); 7.2858 (0.5); 7.2794 (0.6); 7.2453 (0.4); 7.2418 (0.4); 7.2257 (1.6); 7.2223 (1.7); 7.2134 (2.5); 7.2076 (3.2); 7.1945 (0.5); 4.3625 (0.3); 4.3498 (0.7); 4.3371 (0.4); 3.7740 (0.3); 3.7514 (13.3); 3.4570 (0.6); 3.4443 (0.6); 3.4396 (0.6); 3.4268 (0.6); 3.3469 (3.0); 3.3290 (25.6); 3.3194 (3.4); 3.1475 (0.8); 3.1304 (1.0); 3.1132 (0.8); 2.5109 (10.3); 2.5067 (19.4); 2.5023 (24.7); 2.4978 (18.0); 2.4935 (9.0); 2.2147 (0.7); 2.2005 (1.1); 2.1797 (1.6); 2.1653 (2.1); 2.1507 (1.6); 2.1302 (1.1); 2.1158 (0.7); 1.1983 (10.9); 1.1811 (10.7); 1.0746 (1.2); 1.0571 (2.4); 1.0397 (1.2); -0.0002 (0.4) |
| I-064 | 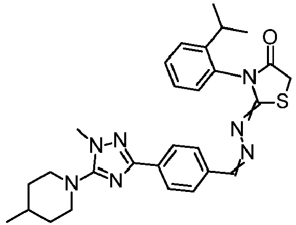 | I-064: ¹H-NMR(400.2 MHz, d₆-DMSO): |
| | | δ= 8.3269 (0.4); 7.9534 (0.4); 7.7939 (0.4); 3.7106 (1.3); 3.5715 (16.0); 2.5141 (1.9); 2.5099 (3.6); 2.5054 (4.6); 2.5008 (3.3); 2.4966 (1.6); 1.1628 (0.5); 1.1456 (0.6); 1.1400 (0.6); 1.1227 (0.5); 0.9696 (0.6); 0.9533 (0.5) |
| I-065 | 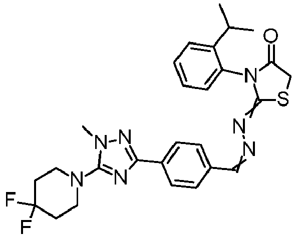 | I-065: ¹H-NMR(400.2 MHz, d₆-DMSO): |
| | | δ= 8.3307 (5.6); 7.9854 (4.2); 7.9647 (5.3); 7.7949 (5.1); 7.7741 (4.2); 7.5164 (1.1); 7.4997 (2.8); 7.4859 (1.6); 7.4667 (2.0); 7.4488 (0.8); 7.3531 (0.9); 7.3493 (0.9); 7.3332 (2.0); 7.3159 (1.2); 7.3125 (1.2); 7.2710 (2.8); 7.2519 (1.7); 4.2740 (1.9); 4.2307 (3.6); 4.1577 (3.6); 4.1143 (1.8); 3.7728 (0.5); 3.7540 (16.0); 3.3465 (4.4); 3.3288 (23.4); 2.8186 (0.4); 2.8012 (1.0); 2.7839 (1.4); 2.7672 (1.1); 2.7500 (0.4); 2.5023 (32.6); 2.2128 (1.1); 2.1986 (1.7); 2.1777 (2.4); 2.1635 (3.2); 2.1489 (2.4); 2.1280 (1.6); 2.1144 (1.0); 1.1637 (7.1); 1.1461 (8.7); 1.1409 (8.4); 1.1233 (6.8) |
| I-066 | 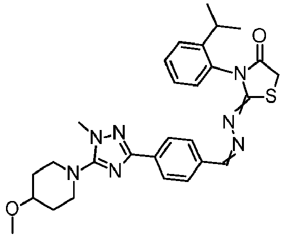 | I-066: ¹H-NMR(400.2 MHz, d₆-DMSO): |
| | | δ= 8.3260 (5.5); 7.9743 (4.0); 7.9535 (5.1); 7.7863 (4.8); 7.7654 (4.0); 7.5160 (1.0); 7.5000 (2.5); 7.4965 (2.5); 7.4860 (1.4); 7.4835 (1.4); 7.4661 (1.8); 7.4490 (0.7); 7.4461 (0.7); 7.3532 (0.8); 7.3489 (0.8); 7.3334 (1.7); 7.3298 (1.6); 7.3160 (1.1); 7.3121 (1.1); 7.2712 (2.5); 7.2690 (2.5); 7.2518 (1.5); 7.2493 (1.4); 4.3641 (0.9); 4.3514 (1.9); 4.3388 (1.0); 4.2727 (1.9); 4.2293 (3.4); 4.1568 (3.6); 4.1135 (1.8); 3.7116 (16.0); 3.4750 (0.5); 3.4621 (0.7); 3.4575 (1.7); 3.4448 (1.7); 3.4401 (1.8); 3.4273 (1.7); 3.4226 (1.0); 3.4099 (1.4); 3.4002 (1.7); 3.3901 (2.6); 3.3793 (2.1); 3.3696 (1.9); 3.3593 (2.0); 3.3467 (1.4); 3.3304 (34.7); 3.2819 (21.0); 3.0279 (1.1); 3.0206 (1.3); 3.0042 (1.4); 2.9966 (2.3); 2.9728 (1.2); 2.9657 (1.0); 2.8178 (0.4); 2.8007 (1.0); 2.7836 (1.3); 2.7665 (1.0); 2.7492 (0.4); 2.5068 (28.0); 2.5025 (35.6); 2.4981 (26.6); 1.9883 (1.1); 1.9817 (1.1); 1.9765 (1.1); 1.9661 (1.2); 1.9510 (1.4); 1.6533 (0.6); 1.6443 (0.7); 1.6303 (1.2); 1.6215 (1.6); 1.6103 (1.1); 1.5989 (1.6); 1.5900 (1.0); 1.5765 (0.6); 1.5676 (0.5); 1.1634 (6.7); 1.1461 (7.7); 1.1401 (7.6); 1.1228 (6.5); 1.0750 (3.4); 1.0575 (6.6); 1.0400 (3.3) |
| I-067 | 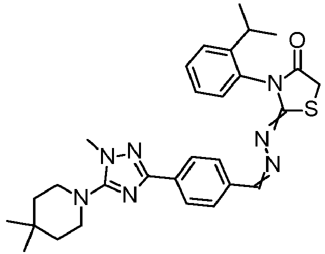 | I-067: ¹H-NMR(400.2 MHz, d₆-DMSO): |
| | | δ= 8.3252 (2.9); 7.9759 (2.3); 7.9555 (2.8); 7.7853 (2.8); 7.7648 (2.4); 7.5166 (0.7); 7.4976 (1.7); 7.4849 (1.0); 7.4665 (1.2); 7.4482 (0.5); 7.3499 (0.6); 7.3317 (1.2); 7.3152 (0.7); 7.2697 (1.6); 7.2506 (1.0); 4.3646 (0.4); 4.3523 (0.6); 4.3397 (0.4); 4.2722 (1.0); 4.2287 (1.9); 4.1567 (1.9); 4.1134 (0.9); 3.6998 (8.4); 3.4571 (0.6); 3.4402 (0.7); 3.4269 (0.6); 3.3317 (20.8); 3.1684 (2.8); 3.1553 (3.7); 3.1416 (2.8); 2.7999 (0.6); 2.7831 (0.8); 2.7664 (0.6); 2.5023 (22.5); 1.4944 (2.9); 1.4809 (3.8); 1.4674 (2.8); 1.1630 (4.1); 1.1450 (5.3); 1.1407 (5.3); 1.1227 (3.9); 1.0746 (1.2); 1.0572 (2.3); 1.0397 (1.2); 0.9888 (16.0) |
| I-068 | 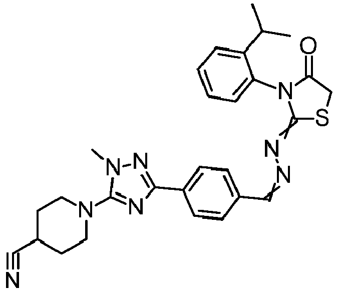 | I-068: ¹H-NMR(400.2 MHz, d₆-DMSO): |
| | | δ= 8.3267 (5.4); 7.9810 (3.9); 7.9601 (5.0); 7.7913 (4.7); 7.7703 (3.9); 7.5208 (0.8); 7.5172 (1.0); 7.5013 (2.4); 7.4977 (2.4); 7.4878 (1.4); 7.4853 (1.4); 7.4677 (1.7); 7.4510 (0.7); 7.4478 (0.7); 7.3544 (0.8); 7.3502 (0.8); 7.3347 (1.6); 7.3309 (1.5); 7.3173 (1.1); 7.3132 (1.0); 7.2706 (2.4); 7.2682 (2.4); 7.2511 (1.5); 7.2485 (1.4); 4.2720 (1.8); 4.2286 (3.3); 4.1577 (3.5); 4.1144 (1.8); 3.7182 (16.0); 3.4574 (0.4); 3.4447 (0.5); 3.4399 (0.5); 3.4273 (0.6); 3.4225 (0.5); 3.4098 (0.7); 3.3588 (258.1); 3.3155 (2.0); 3.3061 (1.7); 3.2998 (1.7); 3.2913 (1.3); 3.1407 (1.4); 3.1338 (1.8); 3.1190 (1.9); 3.1122 (2.6); 3.1028 (1.9); 3.0874 (1.3); 3.0810 (1.1); 2.8163 (0.4); 2.7992 (0.9); 2.7820 (1.3); 2.7649 (1.0); 2.7477 (0.4); 2.6731 (0.3); 2.5086 (42.3); 2.5042 (53.4); 2.4998 (38.7); 2.3308 (0.3); 2.0651 (0.5); 2.0561 (0.8); 2.0484 (0.9); 2.0406 (0.9); 2.0314 (1.2); 2.0228 (1.4); 2.0156 (1.4); 2.0079 (1.2); 1.9361 (0.7); 1.9272 (0.8); 1.9145 (1.4); 1.9054 (1.7); 1.8931 (1.1); 1.8825 (1.3); 1.8727 (0.8); 1.8597 (0.5); 1.8511 (0.4); 1.1631 (6.6); 1.1458 (7.5); 1.1400 |
| | | (7.3); 1.1227 (6.3); 1.0750 (0.6); 1.0576 (1.1); 1.0400 (0.6); - 0.0002 (0.6) |
| I-069 | 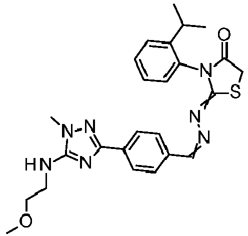 | I-069: ¹H-NMR(400.2 MHz, d₆-DMSO): |
| | | δ= 8.3127 (6.3); 7.9432 (4.4); 7.9226 (5.4); 7.7605 (5.3); 7.7398 (4.3); 7.5157 (1.2); 7.4984 (3.1); 7.4848 (1.8); 7.4663 (2.2); 7.4473 (0.9); 7.3520 (1.0); 7.3486 (0.9); 7.3323 (2.1); 7.3150 (1.3); 7.2698 (3.0); 7.2506 (1.8); 6.6530 (1.2); 6.6394 (2.2); 6.6261 (1.1); 4.2700 (1.9); 4.2267 (3.6); 4.1541 (3.6); 4.1107 (1.8); 3.5885 (16.0); 3.5343 (1.7); 3.5208 (4.8); 3.5085 (4.3); 3.4842 (2.2); 3.4721 (3.7); 3.4587 (2.9); 3.4453 (1.0); 3.3344 (30.8); 3.3108 (1.6); 3.2886 (20.5); 2.8194 (0.4); 2.8023 (1.1); 2.7852 (1.5); 2.7681 (1.2); 2.7513 (0.5); 2.5025 (33.5); 1.1639 (7.4); 1.1464 (9.0); 1.1404 (8.6); 1.1228 (7.2) |
| I-070 | 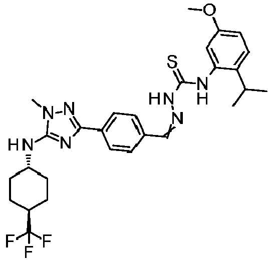 | I-070: ¹H-NMR(400.2 MHz, d₆-DMSO): |
| | | δ= 11.8087 (2.1); 9.9621 (2.1); 8.1488 (3.2); 7.9148 (0.3); 7.8998 (16.0); 7.2580 (1.9); 7.2364 (2.1); 6.9019 (1.0); 6.8951 (1.2); 6.8804 (0.9); 6.8736 (1.2); 6.8334 (2.3); 6.8267 (1.8); 6.3865 (1.2); 6.3677 (1.3); 4.3703 (1.0); 4.3576 (2.1); 4.3449 (1.1); 3.7435 (14.4); 3.5826 (11.5); 3.5618 (0.5); 3.5526 (0.5); 3.5437 (0.3); 3.4750 (0.7); 3.4623 (0.8); 3.4575 (2.0); 3.4448 (2.1); 3.4400 (2.1); 3.4274 (2.0); 3.4226 (0.8); 3.4099 (0.7); 3.3429 (85.6); 3.0648 (0.6); 3.0476 (0.8); 3.0305 (0.6); 2.5256 (0.8); 2.5207 (1.2); 2.5121 (13.8); 2.5078 (27.1); 2.5033 (34.5); 2.4987 (24.4); 2.4944 (11.7); 2.2905 (0.3); 2.1507 (1.1); 2.1241 (0.9); 1.9483 (0.8); 1.9193 (1.2); 1.4262 (0.8); 1.4190 (0.7); 1.3907 (1.4); 1.3756 (0.8); 1.3594 (1.4); 1.3314 (0.6); 1.3254 (0.7); 1.1701 (9.1); 1.1529 (9.0); 1.0750 (4.4); 1.0575 (8.7); 1.0400 (4.3); 0.0079 (0.6); -0.0002 (18.6); -0.0085 (0.6) |
| I-071 | 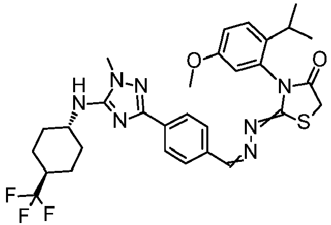 | I-071: ¹H-NMR(400.2 MHz, d₆-DMSO): |
| | | δ= 8.3294 (4.6); 7.9430 (3.3); 7.9222 (4.3); 7.7619 (4.0); 7.7409 (3.3); 7.4037 (2.2); 7.3818 (2.5); 7.0656 (1.4); 7.0588 (1.5); 7.0438 (1.2); 7.0370 (1.3); 6.8810 (3.0); 6.8742 (2.8); 6.3997 (1.6); 6.3810 (1.6); 4.6479 (1.2); 4.6242 (0.3); 4.2536 (1.7); 4.2103 (2.6); 4.1182 (2.7); 4.0749 (1.6); 3.7535 (16.0); 3.6709 (1.9); 3.5861 (13.3); 3.5579 (0.6); 3.5489 (0.6); 3.5414 (0.4); 3.3328 (54.0); 2.7186 (0.8); 2.7017 (1.1); 2.6845 (0.8); 2.6763 (0.4); 2.6673 (0.5); 2.5069 (33.7); 2.5025 (43.0); 2.4981 (31.6); 2.3292 (0.4); 2.3249 (0.4); 2.2940 (0.4); 2.2725 (0.4); 2.1468 (1.5); 2.1216 (1.2); 2.0979 (2.3); 1.9469 (1.1); 1.9194 (1.7); 1.4242 (1.0); 1.4174 (0.9); 1.3888 (2.1); 1.3598 (2.2); 1.3324 (0.8); 1.3262 (0.9); 1.1313 (5.3); 1.1142 (5.6); 1.1042 (5.7); 1.0871 (5.2) |
| I-072 | 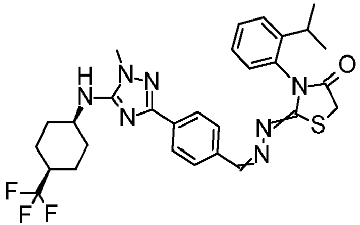 | I-072: ¹H-NMR(400.2 MHz, d₆-DMSO): |
| | | δ= 8.3092 (5.6); 7.9398 (4.2); 7.9191 (5.3); 7.7574 (5.1); 7.7366 (4.2); 7.5158 (1.1); 7.4991 (2.8); 7.4842 (1.6); 7.4663 (2.0); 7.4481 (0.8); 7.3518 (0.9); 7.3482 (0.9); 7.3319 (1.9); 7.3149 (1.2); 7.3113 (1.2); 7.2680 (2.8); 7.2489 (1.7); 6.3980 (2.0); 6.3792 (2.0); 4.2688 (1.9); 4.2255 (3.5); 4.1532 (3.6); 4.1099 (1.8); 3.5850 (16.0); 3.5575 (0.9); 3.5477 (0.9); 3.3258 (28.3); 3.3035 (0.4); 2.8182 (0.4); 2.8016 (1.1); 2.7843 (1.5); 2.7675 (1.1); 2.7504 (0.4); 2.6713 (0.4); 2.5056 (45.9); 2.5019 (55.1); 2.3276 (0.6); 2.2940 (0.6); 2.2722 (0.6); 2.2438 (0.4); 2.1461 (2.2); 2.1204 (1.7); 2.0984 (0.5); 1.9470 (1.6); 1.9196 (2.4); 1.4509 (0.5); 1.4222 (1.4); 1.3884 (3.1); 1.3592 (3.2); 1.3255 (1.3); 1.2983 (0.5); 1.1639 (7.3); 1.1466 (8.4); 1.1399 (8.2); 1.1226 (7.0); -0.0002 (55.5) |
| I-073 | 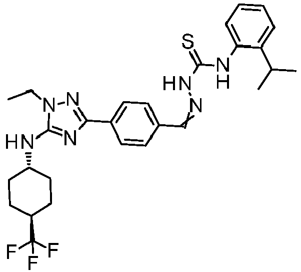 | I-073: ¹H-NMR(400.2 MHz, d₆-DMSO): |
| | | δ= 11.7989 (2.6); 9.9926 (2.4); 8.1533 (3.4); 7.9038 (16.0); 7.3687 (1.0); 7.3496 (1.8); 7.3174 (0.6); 7.3067 (1.2); 7.2961 (1.0); 7.2866 (0.8); 7.2764 (0.6); 7.2258 (3.7); 7.2165 (2.8); 6.3693 (1.4); 6.3503 (1.4); 5.7539 (1.0); 4.0561 (0.6); 4.0383 (2.0); 4.0205 (2.0); 4.0027 (0.7); 3.9933 (0.6); 3.9756 (2.0); 3.9577 (2.1); 3.9398 (0.7); 3.5926 (0.4); 3.5835 (0.4); 3.5739 (0.4); 3.5649 (0.4); 3.5557 (0.3); 3.3304 (27.8); 3.1475 (0.7); 3.1303 (1.0); 3.1132 (0.7); 2.5250 (0.6); 2.5202 (0.9); 2.5116 (11.0); 2.5071 (22.0); 2.5026 (29.1); 2.4980 (21.7); 2.4936 (10.8); 2.2877 (0.3); 2.2664 (0.3); 2.1437 (1.2); 2.1184 (0.9); 2.0109 (0.7); 1.9887 (8.4); 1.9462 (0.9); 1.9181 (1.3); 1.4234 (0.8); 1.4168 (0.7); 1.3884 (1.6); 1.3589 (1.8); 1.3324 (0.7); 1.3254 (0.7); 1.2987 (0.3); 1.2772 (3.0); 1.2594 (6.6); 1.2415 (3.0); 1.1995 (10.1); 1.1935 (4.0); 1.1823 (10.0); 1.1757 (5.6); 1.1578 (2.3); 0.8888 (0.8); 0.8720 (0.7); 0.0080 (1.1); -0.0002 (32.7); - 0.0085 (1.2) |
| I-074 | 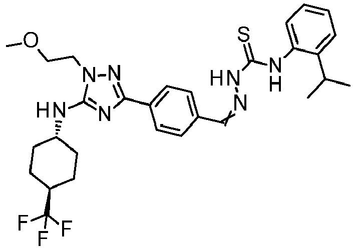 | I-074: ¹H-NMR(400.2 MHz, d₆-DMSO): |
| | | δ= 11.8019 (2.5); 9.9928 (2.4); 8.1541 (3.3); 7.9053 (16.0); 7.3688 (1.0); 7.3496 (1.8); 7.3174 (0.6); 7.3068 (1.2); 7.2961 (1.0); 7.2866 (0.7); 7.2764 (0.6); 7.2256 (3.6); 7.2163 (2.7); 6.3172 (1.3); 6.2981 (1.3); 4.1337 (1.2); 4.1198 (2.4); 4.1059 (1.3); 4.0379 (0.6); 4.0201 (0.6); 3.6543 (1.6); 3.6405 (3.2); 3.6263 (1.4); 3.5912 (0.4); 3.5815 (0.4); 3.5728 (0.4); 3.5632 (0.4); 3.5546 (0.3); 3.3278 (37.5); 3.2507 (17.8); 3.1468 (0.7); 3.1297 (0.9); 3.1124 (0.7); 2.5245 (0.8); 2.5198 (1.2); 2.5111 (14.8); 2.5066 (29.8); 2.5021 (39.6); 2.4975 (29.7); 2.4930 (14.8); 2.3289 (0.3); 2.2925 (0.3); 2.2706 (0.4); 2.1341 (1.0); 2.1069 (0.9); 2.0107 (0.7); 1.9884 (2.5); 1.9440 (0.8); 1.9144 (1.2); 1.4226 (0.8); 1.4157 (0.7); 1.3874 (1.2); 1.3680 (1.0); 1.3486 (1.2); 1.3142 (0.7); 1.1992 (9.7); 1.1820 (9.6); 1.1757 (2.4); 1.1577 (0.8); 0.8887 (0.7); 0.8719 (0.7); 0.0080 (1.4); -0.0002 (40.9); -0.0085 (1.4) |
| I-075 | 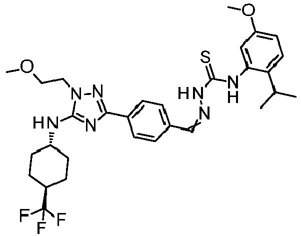 | I-075: ¹H-NMR(400.2 MHz, d₆-DMSO): |
| | | δ= 11.8157 (2.6); 9.9587 (2.4); 8.1532 (3.4); 7.9037 (16.0); 7.2587 (2.0); 7.2371 (2.3); 6.9021 (1.1); 6.8952 (1.4); 6.8805 (0.9); 6.8737 (1.3); 6.8426 (2.5); 6.8359 (2.0); 6.3204 (1.3); 6.3014 (1.4); 4.1345 (1.2); 4.1207 (2.6); 4.1068 (1.3); 4.0380 (0.9); 4.0203 (0.9); 3.7440 (15.0); 3.6548 (1.7); 3.6410 (3.3); 3.6269 (1.5); 3.5917 (0.4); 3.5824 (0.4); 3.5683 (2.6); 3.5554 (0.4); 3.3298 (44.4); 3.2508 (17.0); 3.0658 (0.7); 3.0486 (1.0); 3.0314 (0.7); 2.5247 (1.0); 2.5109 (18.1); 2.5068 (34.2); 2.5023 (43.2); 2.4979 (31.2); 2.4937 (15.3); 2.3290 (0.4); 2.3247 (0.3); 2.2950 (0.4); 2.2730 (0.4); 2.1344 (1.2); 2.1056 (1.0); 2.0110 (0.6); 1.9887 (3.9); 1.9453 (0.9); 1.9151 (1.3); 1.4231 (0.9); 1.4163 (0.8); 1.3882 (1.3); 1.3683 (1.1); 1.3494 (1.3); 1.3214 (0.7); 1.3150 (0.8); 1.1931 (1.1); 1.1718 (10.0); 1.1546 (9.8); 0.8886 (0.7); 0.8718 (0.6); 0.0079 (1.4); -0.0002 (38.6); -0.0084 (1.4) |
| I-076 | 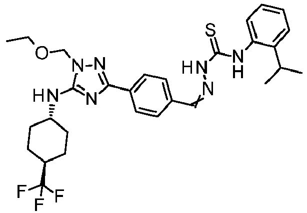 | I-076: ¹H-NMR(400.2 MHz, d₆-DMSO): |
| | | δ= 11.8154 (2.5); 10.0051 (2.5); 8.1605 (3.4); 7.9272 (16.0); 7.3698 (1.0); 7.3507 (1.9); 7.3189 (0.6); 7.3086 (1.1); 7.2976 (1.0); 7.2886 (0.8); 7.2779 (0.6); 7.2263 (3.5); 7.2166 (2.7); 6.6970 (1.0); 6.6778 (1.1); 5.4433 (1.0); 5.3440 (3.8); 4.0561 (0.4); 4.0383 (1.1); 4.0205 (1.2); 4.0027 (0.4); 3.9312 (0.9); 3.6290 (0.3); 3.6201 (0.3); 3.6108 (0.4); 3.5826 (0.4); 3.5779 (1.0); 3.5686 (2.7); 3.5656 (1.2); 3.5603 (3.2); 3.5476 (1.0); 3.5427 (3.2); 3.5298 (0.4); 3.5252 (1.0); 3.3308 (34.3); 3.1474 (0.7); 3.1301 (1.0); 3.1129 (0.8); 2.5252 (0.7); 2.5204 (1.1); 2.5117 (13.1); 2.5073 (25.7); 2.5028 (33.0); 2.4982 (24.0); 2.4938 (11.7); 2.3297 (0.4); 2.3252 (0.3); 2.1319 (1.0); 2.1079 (0.7); |
| | | 2.0110 (0.6); 1.9888 (5.1); 1.9461 (0.7); 1.9385 (0.7); 1.9220 (1.2); 1.6819 (0.7); 1.6676 (0.9); 1.4263 (0.6); 1.3961 (1.8); 1.3711 (1.8); 1.3485 (0.6); 1.3434 (0.6); 1.1995 (10.4); 1.1938 (3.2); 1.1823 (10.2); 1.1758 (3.8); 1.1579 (1.5); 1.1135 (3.4); 1.0960 (7.0); 1.0784 (3.4); 1.0707 (6.0); 0.8888 (0.5); 0.8720 (0.5); 0.0080 (1.3); -0.0002 (34.5); -0.0085 (1.2) |
| I-077 | 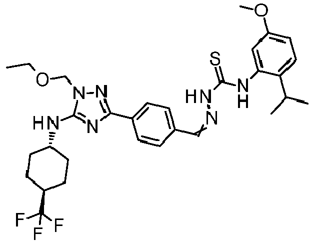 | I-077: ¹H-NMR(400.2 MHz, d₆-DMSO): |
| | | δ= 11.8292 (2.2); 9.9698 (2.2); 8.1600 (3.0); 7.9498 (0.6); 7.9433 (0.4); 7.9274 (9.1); 7.9250 (7.9); 7.9020 (0.6); 7.2591 (2.0); 7.2376 (2.3); 6.9031 (1.1); 6.8963 (1.4); 6.8816 (0.9); 6.8748 (1.3); 6.8425 (2.0); 6.8360 (1.8); 6.7055 (0.8); 6.6864 (0.8); 5.7541 (0.8); 5.4445 (0.8); 5.3452 (3.5); 4.0385 (1.0); 4.0207 (1.0); 4.0029 (0.3); 3.7449 (16.0); 3.6120 (0.3); 3.5836 (0.4); 3.5789 (1.1); 3.5689 (3.9); 3.5614 (3.4); 3.5486 (0.9); 3.5437 (3.3); 3.5308 (0.3); 3.5262 (1.0); 3.3555 (3.6); 3.0668 (0.6); 3.0498 (0.9); 3.0326 (0.7); 2.5257 (0.6); 2.5210 (0.9); 2.5123 (10.3); 2.5078 (20.8); 2.5032 (27.2); 2.4985 (19.7); 2.4940 (9.4); 2.1321 (0.9); 2.1088 (0.6); 2.0112 (0.4); 1.9890 (4.4); 1.9482 (0.6); 1.9224 (1.1); 1.6823 (0.5); 1.6684 (0.7); 1.4269 (0.6); 1.4208 (0.6); 1.4137 (0.4); 1.3967 (1.7); 1.3721 (1.7); 1.3496 (0.5); 1.3439 (0.5); 1.1937 (1.3); 1.1756 (5.0); 1.1722 (9.5); 1.1550 (9.3); 1.1144 (3.5); 1.0969 (7.2); 1.0793 (3.4); 1.0711 (7.1); 0.8889 (0.4); 0.8721 (0.4); 0.0080 (1.2); -0.0002 (35.4); - 0.0086 (1.2) |
| I-078 | 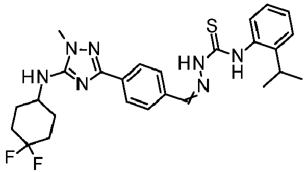 | I-078: ¹H-NMR(400.2 MHz, d₆-DMSO): |
| | | δ= 11.8002 (3.0); 9.9954 (2.8); 8.1497 (4.0); 7.9283 (0.5); 7.9052 (15.8); 7.8900 (0.3); 7.8849 (0.4); 7.8823 (0.5); 7.3685 (1.1); 7.3498 (2.3); 7.3182 (0.8); 7.3103 (0.9); 7.3045 (0.8); 7.2967 (1.2); 7.2899 (0.6); 7.2860 (0.6); 7.2771 (0.7); 7.2457 (0.3); 7.2261 (2.0); 7.2224 (2.7); 7.2199 (2.7); 7.2124 (3.4); 7.2102 (3.2); 6.3793 (1.7); 6.3610 (1.8); 3.7984 (0.4); 3.7798 (0.5); 3.5950 (16.0); 3.3304 (53.8); 3.1454 (0.8); 3.1283 (1.1); 3.1110 (0.8); 2.6712 (0.3); 2.5248 (1.1); 2.5201 (1.7); 2.5114 (18.9); 2.5069 (37.8); 2.5023 (49.6); 2.4976 (36.1); 2.4931 (17.3); 2.0963 (0.6); 2.0824 (0.7); 2.0766 (0.7); 2.0551 (1.6); 2.0403 (1.6); 2.0159 (1.5); 1.9886 (0.7); 1.9759 (0.4); 1.9629 (0.4); 1.9514 (0.3); 1.9373 (0.4); 1.9226 (0.4); 1.6854 (0.4); 1.6632 (1.0); 1.6366 (0.8); 1.1978 (12.2); 1.1805 (12.1); 0.0080 (2.0); -0.0002 (57.1); -0.0085 (1.8) |
| I-079 | 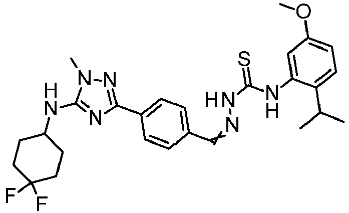 | I-079: ¹H-NMR(400.2 MHz, d₆-DMSO): |
| | | δ= 11.8118 (2.6); 9.9591 (2.3); 8.1484 (3.4); 7.9032 (16.0); 7.2582 (1.9); 7.2366 (2.2); 6.9022 (1.1); 6.8954 (1.3); 6.8807 (0.9); 6.8739 (1.2); 6.8321 (2.3); 6.8255 (1.9); 6.3795 (1.4); 6.3612 (1.4); 4.3623 (0.5); 4.3496 (1.0); 4.3369 (0.5); 3.7997 (0.4); 3.7814 (0.4); 3.7435 (14.0); 3.5957 (11.2); 3.4568 (0.8); 3.4441 (0.8); 3.4393 (0.9); 3.4266 (0.8); 3.4219 (0.4); 3.3295 (35.0); 3.0647 (0.6); 3.0476 (0.9); 3.0304 (0.7); 2.5244 (0.9); 2.5109 (13.9); 2.5068 (26.8); 2.5024 (35.1); 2.4979 (26.6); 2.4938 (13.7); 2.0971 (0.6); 2.0553 (1.4); 2.0406 (1.4); 2.0167 (1.4); 1.9760 (0.4); 1.9630 (0.4); 1.9376 (0.4); 1.9239 (0.4); 1.6879 (0.4); 1.6640 (0.9); 1.6371 (0.8); 1.1701 (9.3); 1.1529 (9.2); 1.0744 (1.8); 1.0569 (3.6); 1.0394 (1.8); 0.0079 (1.2); -0.0002 (30.2); -0.0083 (1.3) |
| I-080 | 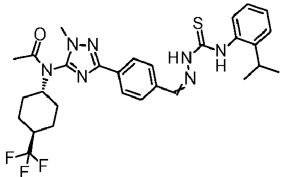 | I-080: ¹H-NMR(600.1 MHz, d₆-DMSO): |
| | | δ= 11.8371 (0.3); 10.0454 (0.2); 8.1797 (0.5); 8.0079 (0.7); 7.3692 (0.2); 7.3565 (0.3); 7.3172 (0.1); 7.3142 (0.1); 7.3030 (0.2); 7.2930 (0.1); 7.2898 (0.1); 7.2270 (0.2); 7.2248 (0.2); 7.2132 (0.5); 7.2097 (0.3); 5.7514 (0.3); 4.0355 (0.2); 4.0237 (0.2); 3.8512 (0.6); 3.3293 (16.0); 3.1459 (0.1); 3.1344 (0.2); 3.1230 (0.1); 2.5086 (1.6); 2.5056 (3.6); 2.5026 (5.0); 2.4996 (3.7); 2.4966 (1.7); 2.0107 (0.2); 1.9884 (0.7); 1.8907 (0.1); |
| | | 1.7521 (0.6); 1.1990 (1.9); 1.1875 (2.1); 1.1759 (0.4); 1.1641 (0.2); 1.1542 (0.1); 0.8863 (0.2); 0.8751 (0.2); 0.0053 (0.2); - 0.0001 (6.3); -0.0056 (0.2) |
| I-081 | 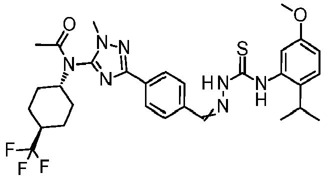 | I-081: ¹H-NMR(400.2 MHz, d₆-DMSO): |
| | | δ= 11.8539 (2.4); 10.0179 (2.0); 8.1765 (3.5); 8.0056 (6.3); 7.2643 (2.1); 7.2426 (2.4); 6.9094 (1.2); 6.9028 (1.4); 6.8879 (1.1); 6.8812 (1.3); 6.8145 (2.6); 6.8079 (2.3); 4.3641 (0.6); 4.3516 (1.0); 4.3391 (0.6); 3.8524 (4.4); 3.7457 (16.0); 3.6594 (0.6); 3.4572 (0.6); 3.4444 (0.6); 3.4397 (0.6); 3.4270 (0.6); 3.3317 (38.9); 3.0698 (0.8); 3.0525 (1.1); 3.0352 (0.8); 3.0177 (0.3); 2.5073 (30.2); 2.5030 (38.5); 2.4987 (29.1); 2.3341 (0.8); 2.3305 (0.8); 2.1932 (0.4); 2.1708 (0.5); 2.0231 (0.4); 2.0136 (0.4); 2.0004 (0.4); 1.9961 (0.4); 1.9666 (0.3); 1.8847 (1.2); 1.7525 (4.5); 1.4306 (0.5); 1.4034 (0.8); 1.3729 (0.7); 1.1729 (11.2); 1.1557 (10.9); 1.0747 (1.3); 1.0573 (2.5); 1.0398 (1.4); 1.0131 (0.3); 0.9971 (0.3) |
| I-082 | 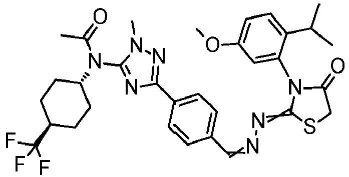 | I-082: ¹H-NMR(400.2 MHz, d₆-DMSO): |
| | | δ= 8.3819 (3.5); 8.0628 (1.7); 8.0431 (2.2); 7.8492 (2.6); 7.8291 (2.4); 7.4085 (2.3); 7.3866 (2.6); 7.0695 (1.5); 7.0629 (1.6); 7.0478 (1.3); 7.0411 (1.4); 6.8838 (3.2); 6.8771 (2.9); 4.3559 (0.5); 4.2681 (1.7); 4.2248 (2.7); 4.1274 (2.8); 4.0841 (1.7); 3.8554 (4.9); 3.7551 (16.0); 3.6626 (0.7); 3.3262 (62.1); 2.7401 (0.4); 2.7238 (0.9); 2.7065 (1.2); 2.6895 (1.0); 2.6717 (0.9); 2.5061 (76.3); 2.5022 (92.1); 2.3294 (1.2); 2.1908 (0.5); 2.1668 (0.6); 2.0006 (0.5); 1.8814 (1.4); 1.7506 (5.0); 1.4295 (0.5); 1.4009 (0.9); 1.3706 (0.8); 1.1353 (5.9); 1.1181 (6.4); 1.1085 (6.4); 1.0913 (5.8); 1.0109 (0.3); 0.9874 (0.3); 0.1462 (0.4); - 0.0002 (65.2); -0.1498 (0.4) |
| I-083 | 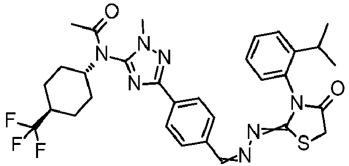 | I-083: ¹H-NMR(400.2 MHz, d₆-DMSO): |
| | | δ= 8.3615 (8.5); 8.3157 (0.4); 8.0611 (3.5); 8.0409 (4.6); 7.8463 (5.5); 7.8260 (5.0); 7.5241 (1.8); 7.5203 (2.2); 7.5045 (5.4); 7.5006 (5.2); 7.4900 (3.0); 7.4870 (2.9); 7.4723 (3.7); 7.4696 (3.9); 7.4529 (1.6); 7.4497 (1.6); 7.3565 (1.9); 7.3522 (1.9); 7.3368 (3.8); 7.3328 (3.5); 7.3194 (2.6); 7.3152 (2.5); 7.2722 (5.4); 7.2695 (5.3); 7.2528 (3.5); 7.2497 (3.1); 4.3832 (0.6); 4.3546 (1.0); 4.3254 (0.6); 4.2831 (4.4); 4.2398 (7.5); 4.1632 (8.0); 4.1198 (4.4); 3.8548 (10.5); 3.6614 (1.4); 3.3275 (125.4); 2.8235 (0.8); 2.8066 (2.1); 2.7895 (2.9); 2.7724 (2.2); 2.7552 (0.8); 2.6758 (0.8); 2.6713 (1.1); 2.6668 (0.8); 2.5247 (3.6); 2.5198 (5.9); 2.5113 (66.1); 2.5069 (128.1); 2.5023 (165.8); 2.4978 (121.7); 2.4934 (59.9); 2.4498 (0.4); 2.3337 (2.1); 2.3294 (2.3); 2.3251 (1.7); 2.1904 (1.0); 2.1678 (1.1); 1.9961 (1.0); 1.8794 (2.7); 1.7501 (10.8); 1.5875 (0.6); 1.5673 (0.6); 1.5224 (0.4); 1.4623 (0.5); 1.4270 (1.0); 1.3993 (1.7); 1.3696 (1.5); 1.3413 (0.6); 1.1684 (14.8); 1.1512 (16.0); 1.1440 (15.8); 1.1268 (14.3); 1.0738 (0.3); 1.0564 (0.5); 1.0388 (0.5); 1.0085 (0.6); 0.9914 (0.6); 0.1460 (0.7); 0.0079 (6.0); -0.0002 (151.0); -0.0085 (5.3); -0.1495 (0.7) |
| I-084 | 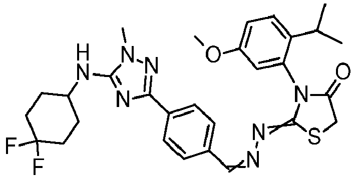 | I-084: ¹H-NMR(400.2 MHz, d₆-DMSO): |
| | | δ= 8.3281 (4.8); 8.3155 (1.2); 7.9453 (3.4); 7.9245 (4.4); 7.7673 (4.2); 7.7464 (3.4); 7.4046 (2.3); 7.3828 (2.6); 7.0672 (1.4); 7.0604 (1.5); 7.0454 (1.3); 7.0387 (1.4); 6.8802 (3.1); 6.8735 (2.9); 6.3985 (1.7); 6.3802 (1.7); 4.2540 (1.7); 4.2107 (2.7); 4.1210 (2.8); 4.0777 (1.6); 3.7918 (0.6); 3.7731 (0.8); 3.7540 (16.0); 3.6008 (13.2); 3.4231 (0.4); 3.3552 (153.1); 2.7175 (0.8); 2.7004 (1.1); 2.6832 (1.0); 2.6737 (0.5); 2.6679 (0.5); 2.5090 (37.0); 2.5046 (46.4); 2.5003 (35.6); 2.0984 (0.8); 2.0789 (1.0); 2.0567 (1.9); 2.0399 (2.0); 2.0155 (1.8); 1.9731 (0.5); 1.9602 (0.5); 1.9482 (0.4); 1.9347 (0.6); 1.9202 (0.5); 1.6845 (0.5); |
| | | 1.6612 (1.2); 1.6343 (1.0); 1.6101 (0.4); 1.1310 (5.5); 1.1138 (5.9); 1.1046 (6.0); 1.0874 (5.4); -0.0002 (36.4) |
| I-085 | 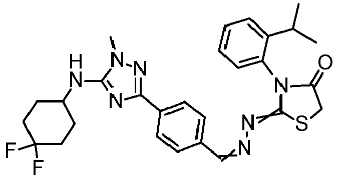 | I-085: ¹H-NMR(400.2 MHz, d₆-DMSO): |
| | | δ= 8.3144 (8.0); 8.3086 (5.4); 7.9413 (3.8); 7.9203 (4.9); 7.7625 (4.6); 7.7414 (3.7); 7.5203 (0.7); 7.5161 (0.9); 7.5005 (2.2); 7.4965 (2.1); 7.4867 (1.3); 7.4836 (1.2); 7.4692 (1.5); 7.4662 (1.6); 7.4498 (0.6); 7.4463 (0.6); 7.3530 (0.8); 7.3486 (0.8); 7.3333 (1.6); 7.3291 (1.4); 7.3159 (1.1); 7.3116 (1.1); 7.2687 (2.2); 7.2658 (2.2); 7.2492 (1.4); 7.2460 (1.3); 6.3917 (1.6); 6.3735 (1.7); 4.6475 (0.7); 4.2699 (1.9); 4.2266 (3.3); 4.1548 (3.4); 4.1115 (1.9); 3.7938 (0.5); 3.7719 (0.5); 3.6707 (1.2); 3.5983 (16.0); 3.5852 (0.4); 3.4559 (0.4); 3.4440 (0.4); 3.4387 (0.4); 3.4267 (0.4); 3.4213 (0.3); 3.3378 (106.5); 3.3153 (2.6); 2.8170 (0.3); 2.7999 (0.8); 2.7828 (1.2); 2.7657 (0.9); 2.7486 (0.3); 2.6764 (0.4); 2.6717 (0.5); 2.6673 (0.4); 2.5252 (1.8); 2.5204 (2.8); 2.5118 (32.8); 2.5073 (64.2); 2.5027 (83.4); 2.4981 (60.7); 2.4936 (29.1); 2.3344 (0.4); 2.3296 (0.5); 2.3249 (0.4); 2.0976 (2.0); 2.0952 (1.2); 2.0837 (0.8); 2.0559 (1.7); 2.0391 (1.7); 2.0152 (1.6); 1.9727 (0.4); 1.9597 (0.4); 1.9486 (0.4); 1.9344 (0.5); 1.9189 (0.4); 1.6834 (0.4); 1.6587 (1.0); 1.6322 (0.9); 1.1633 (6.1); 1.1460 (6.8); 1.1398 (6.6); 1.1226 (6.0); 1.0740 (1.5); 1.0565 (3.0); 1.0391 (1.5); 0.8603 (0.4); 0.1459 (0.3); 0.0137 (0.4); 0.0080 (3.0); -0.0002 (80.4); -0.0086 (2.5); - 0.1495 (0.3) |
| I-086 | 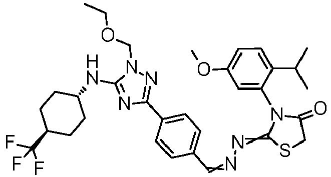 | I-086: ¹H-NMR(400.2 MHz, d₆-DMSO): |
| | | δ= 8.3420 (4.9); 8.3159 (2.5); 7.9743 (3.3); 7.9537 (4.3); 7.7822 (4.2); 7.7613 (3.5); 7.4054 (2.3); 7.3835 (2.7); 7.0671 (1.4); 7.0603 (1.6); 7.0453 (1.3); 7.0385 (1.4); 6.8814 (3.1); 6.8746 (2.9); 6.7144 (1.2); 6.6953 (1.3); 5.4466 (1.0); 5.3471 (4.8); 4.6479 (0.6); 4.2571 (1.7); 4.2139 (2.7); 4.1209 (2.7); 4.0776 (1.6); 3.7744 (0.4); 3.7543 (16.0); 3.6710 (0.9); 3.6336 (0.4); 3.6236 (0.4); 3.6153 (0.5); 3.6056 (0.5); 3.5777 (1.3); 3.5602 (3.5); 3.5426 (3.5); 3.5251 (1.2); 3.3277 (28.5); 3.3041 (1.0); 2.7208 (0.8); 2.7038 (1.1); 2.6864 (0.8); 2.6709 (0.7); 2.5066 (53.1); 2.5024 (67.7); 2.4983 (51.8); 2.3294 (0.7); 2.3004 (0.4); 2.2788 (0.4); 2.1278 (1.3); 2.0978 (1.8); 1.9474 (0.9); 1.9215 (1.5); 1.6805 (0.8); 1.6663 (1.0); 1.4233 (0.9); 1.3943 (2.4); 1.3720 (2.4); 1.3443 (0.8); 1.1336 (5.6); 1.1159 (6.8); 1.1126 (6.6); 1.1057 (6.5); 1.0943 (9.1); 1.0888 (6.4); 1.0769 (4.1); - 0.0002 (48.2); -0.0077 (2.4) |
| I-087 | 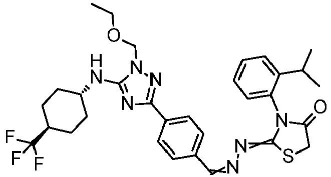 | I-087: ¹H-NMR(400.2 MHz, d₆-DMSO): |
| | | δ= 8.3197 (9.9); 7.9725 (7.1); 7.9520 (9.0); 7.7788 (8.9); 7.7581 (7.6); 7.5176 (2.0); 7.4982 (5.1); 7.4866 (3.0); 7.4685 (3.7); 7.4487 (1.5); 7.3541 (1.6); 7.3505 (1.6); 7.3342 (3.4); 7.3169 (2.1); 7.3136 (2.1); 7.2672 (5.1); 7.2475 (3.1); 6.7124 (2.8); 6.6934 (2.9); 6.4995 (0.6); 6.4870 (0.6); 5.4451 (2.2); 5.3453 (10.7); 4.2705 (3.3); 4.2271 (6.1); 4.1563 (6.4); 4.1129 (3.2); 3.9453 (0.4); 3.6319 (1.0); 3.6234 (1.1); 3.6133 (1.2); 3.6048 (1.2); 3.5771 (2.8); 3.5595 (7.3); 3.5419 (7.3); 3.5244 (2.4); 3.3521 (482.1); 2.8195 (0.8); 2.8029 (1.9); 2.7858 (2.6); 2.7687 (2.0); 2.7514 (0.8); 2.6728 (0.8); 2.5039 (130.0); 2.3304 (1.4); 2.2987 (1.1); 2.2756 (1.0); 2.1269 (3.2); 2.1047 (2.5); 2.0406 (0.6); 2.0200 (0.5); 1.9453 (2.3); 1.9212 (3.6); 1.8871 (0.3); 1.6788 (1.8); 1.6655 (2.2); 1.4535 (0.7); 1.4227 (2.1); 1.3938 (5.8); 1.3713 (5.5); 1.3442 (1.9); 1.3109 (0.6); 1.2328 (0.4); 1.1663 (12.7); 1.1490 (14.4); 1.1412 (14.1); 1.1239 (12.6); 1.1116 (8.8); 1.0939 (16.0); 1.0764 (7.8); 1.0582 (0.4); 0.1458 (0.5); - 0.0004 (93.6); -0.1499 (0.5) |
| I-088 | 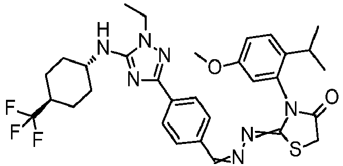 | I-088: ¹H-NMR(400.2 MHz, d₆-DMSO): |
| | | δ= 8.3264 (4.8); 7.9503 (3.3); 7.9295 (4.3); 7.7629 (4.0); 7.7419 (3.4); 7.4062 (2.3); 7.3843 (2.6); 7.0688 (1.4); 7.0620 (1.5); 7.0470 (1.2); 7.0401 (1.4); 6.8781 (3.1); 6.8713 (2.9); 6.3914 (1.6); 6.3724 (1.6); 4.6468 (0.6); 4.2506 (1.7); 4.2073 (2.7); 4.1205 (2.8); 4.0771 (1.6); 3.9944 (0.8); 3.9767 (2.4); 3.9589 (2.4); 3.9409 (0.8); 3.7755 (0.4); 3.7545 (16.0); 3.6714 (0.9); 3.5889 (0.6); 3.5803 (0.6); 3.5711 (0.7); 3.5615 (0.7); 3.4465 (1.2); 3.3799 (351.7); 2.7181 (0.8); 2.7010 (1.1); 2.6838 (0.9); 2.6748 (0.6); 2.6699 (0.5); 2.5278 (1.5); 2.5102 (49.2); 2.5058 (64.6); 2.5014 (49.6); 2.3371 (0.4); 2.3325 (0.5); 2.3279 (0.4); 2.2874 (0.4); 2.2641 (0.4); 2.2589 (0.4); 2.1384 (1.5); 2.1137 (1.1); 2.0981 (1.4); 1.9470 (1.1); 1.9194 (1.6); 1.4218 (1.0); 1.3883 (2.4); 1.3597 (2.4); 1.3279 (0.8); 1.2974 (0.4); 1.2777 (3.3); 1.2600 (7.0); 1.2421 (3.3); 1.1326 (5.3); 1.1155 (5.6); 1.1051 (5.7); 1.0879 (5.3); 1.0760 (1.8); 1.0584 (3.0); 1.0409 (1.4); 0.0077 (2.2); -0.0002 (59.9); -0.0082 (2.7) |
| I-089 | 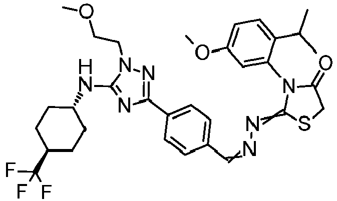 | I-089: ¹H-NMR(400.2 MHz, d₆-DMSO): |
| | | δ= 8.3312 (4.1); 8.3125 (2.3); 7.9493 (2.8); 7.9284 (3.6); 7.7674 (3.4); 7.7464 (2.8); 7.4057 (2.0); 7.3838 (2.2); 7.0676 (1.2); 7.0608 (1.3); 7.0458 (1.1); 7.0389 (1.2); 6.8794 (2.7); 6.8725 (2.5); 6.3351 (1.3); 6.3161 (1.3); 4.6472 (0.7); 4.2536 (1.5); 4.2103 (2.3); 4.1367 (1.3); 4.1207 (4.1); 4.1090 (1.4); 4.0767 (1.4); 3.7542 (13.8); 3.6709 (1.1); 3.6552 (1.6); 3.6415 (3.1); 3.6274 (1.4); 3.5883 (0.4); 3.5769 (0.4); 3.5702 (0.5); 3.5597 (0.5); 3.5516 (0.4); 3.3468 (179.4); 3.3236 (2.1); 3.2491 (16.0); 2.7191 (0.6); 2.7020 (0.9); 2.6848 (0.7); 2.6770 (0.4); 2.6722 (0.5); 2.6679 (0.6); 2.5255 (1.5); 2.5078 (49.0); 2.5034 (63.0); 2.4989 (46.9); 2.3345 (0.4); 2.3302 (0.5); 2.3257 (0.4); 2.2956 (0.4); 2.2733 (0.3); 2.1303 (1.2); 2.0977 (2.0); 1.9454 (0.9); 1.9159 (1.3); 1.4207 (0.8); 1.4141 (0.7); 1.3864 (1.3); 1.3681 (1.0); 1.3504 (1.4); 1.3165 (0.8); 1.1331 (4.4); 1.1159 (4.6); 1.1051 (4.7); 1.0880 (4.4); 1.0746 (0.4); 1.0570 (0.5); 0.0079 (2.3); -0.0002 (52.4); -0.0084 (2.0) |
| I-090 | 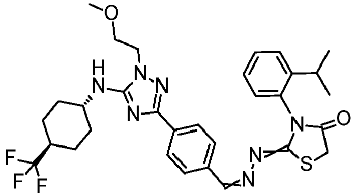 | I-090: ¹H-NMR(400.2 MHz, d₆-DMSO): |
| | | δ= 8.3123 (4.1); 7.9465 (2.9); 7.9257 (3.7); 7.7632 (3.5); 7.7423 (2.9); 7.5168 (0.7); 7.5008 (1.8); 7.4972 (1.7); 7.4863 (1.0); 7.4668 (1.3); 7.4497 (0.5); 7.4468 (0.5); 7.3534 (0.6); 7.3491 (0.6); 7.3335 (1.2); 7.3309 (1.1); 7.3161 (0.8); 7.3121 (0.8); 7.2684 (1.8); 7.2662 (1.8); 7.2491 (1.2); 7.2466 (1.0); 6.3346 (1.3); 6.3156 (1.4); 4.6476 (0.5); 4.6238 (0.3); 4.2699 (1.4); 4.2266 (2.5); 4.1547 (2.6); 4.1364 (1.3); 4.1227 (2.7); 4.1110 (2.4); 3.6706 (0.9); 3.6543 (1.6); 3.6404 (3.1); 3.6264 (1.4); 3.5871 (0.4); 3.5776 (0.4); 3.5679 (0.4); 3.5588 (0.5); 3.5502 (0.4); 3.3274 (37.1); 3.2485 (16.0); 2.8024 (0.7); 2.7853 (1.0); 2.7680 (0.7); 2.6710 (0.3); 2.5241 (1.2); 2.5063 (42.8); 2.5019 (55.3); 2.4975 (41.5); 2.3285 (0.5); 2.3243 (0.4); 2.2999 (0.4); 2.2886 (0.3); 2.2732 (0.4); 2.1301 (1.2); 2.0974 (1.8); 1.9438 (0.9); 1.9149 (1.4); 1.4203 (0.8); 1.3858 (1.4); 1.3676 (1.0); 1.3496 (1.4); 1.3157 (0.8); 1.2897 (0.4); 1.2820 (0.4); 1.2410 (0.7); 1.1973 (0.4); 1.1789 (0.4); 1.1657 (4.7); 1.1485 (5.1); 1.1404 (5.1); 1.1233 (4.7); 0.8772 (0.4); 0.8605 (1.0); 0.8432 (0.4); 0.0079 (1.6); -0.0002 (42.4); -0.0084 (1.7) |
| I-091 | 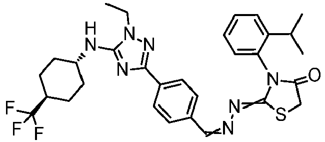 | I-091: ¹H-NMR(400.2 MHz, d₆-DMSO): |
| | | δ= 8.3100 (10.5); 7.9471 (7.6); 7.9263 (9.7); 7.7581 (9.2); 7.7372 (7.6); 7.5198 (1.7); 7.5162 (2.0); 7.5001 (4.9); 7.4967 (4.7); 7.4862 (2.8); 7.4837 (2.7); 7.4664 (3.5); 7.4492 (1.4); 7.4463 (1.4); 7.3530 (1.6); 7.3488 (1.6); 7.3333 (3.4); 7.3159 (2.2); 7.3119 (2.1); 7.2685 (5.0); 7.2664 (4.8); 7.2491 (3.1); 6.3886 (3.6); 6.3698 (3.7); 4.6481 (1.9); 4.6243 (0.7); 4.2694 (3.6); 4.2261 (6.5); 4.1542 (6.8); 4.1421 (0.6); 4.1242 (0.4); 4.1109 |
| | | (3.5); 3.9956 (1.8); 3.9779 (5.6); 3.9601 (5.6); 3.9423 (1.9); 3.6708 (3.0); 3.5871 (1.1); 3.5778 (1.2); 3.5686 (1.2); 3.5597 (1.3); 3.5517 (1.0); 3.5333 (0.5); 3.3288 (13.9); 2.8192 (0.7); 2.8021 (1.8); 2.7851 (2.6); 2.7680 (1.9); 2.7508 (0.8); 2.6711 (0.6); 2.5065 (83.3); 2.5022 (102.0); 2.4978 (74.6); 2.3288 (0.9); 2.3244 (0.8); 2.2900 (1.0); 2.2835 (1.0); 2.2681 (1.0); 2.1393 (3.6); 2.1146 (2.8); 2.0978 (4.6); 1.9449 (2.6); 1.9185 (4.0); 1.7348 (0.6); 1.4524 (0.7); 1.4205 (2.3); 1.3872 (5.8); 1.3589 (5.9); 1.3276 (2.1); 1.2982 (1.0); 1.2766 (7.7); 1.2589 (16.0); 1.2410 (7.5); 1.2150 (0.5); 1.1972 (0.8); 1.1788 (1.0); 1.1652 (12.9); 1.1480 (14.3); 1.1402 (14.0); 1.1230 (12.5); 0.1459 (0.4); - 0.0002 (80.4); -0.0084 (3.9); -0.1497 (0.4) |
| I-092 | 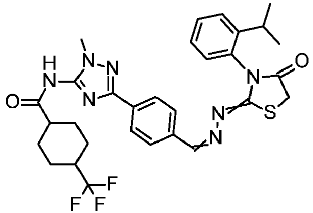 | I-092: ¹H-NMR(400.2 MHz, d₆-DMSO): |
| | | δ= 10.6597 (0.4); 8.3513 (5.5); 8.0081 (3.8); 7.9871 (5.0); 7.8237 (4.6); 7.8027 (3.8); 7.5218 (0.8); 7.5178 (1.0); 7.5021 (2.4); 7.4981 (2.2); 7.4877 (1.3); 7.4845 (1.3); 7.4701 (1.6); 7.4671 (1.7); 7.4506 (0.7); 7.4472 (0.6); 7.3547 (0.9); 7.3503 (0.8); 7.3349 (1.7); 7.3307 (1.6); 7.3175 (1.2); 7.3133 (1.2); 7.2760 (2.4); 7.2730 (2.3); 7.2565 (1.5); 7.2533 (1.4); 5.7550 (0.5); 4.2796 (2.1); 4.2362 (3.4); 4.1612 (3.6); 4.1178 (2.0); 4.0205 (0.4); 3.6914 (16.0); 3.6775 (0.9); 3.6749 (1.0); 3.5683 (12.2); 3.3298 (84.5); 2.8215 (0.5); 2.8044 (1.6); 2.7874 (2.0); 2.7702 (1.1); 2.7530 (0.4); 2.6761 (0.4); 2.6714 (0.6); 2.6668 (0.4); 2.5390 (1.3); 2.5248 (2.0); 2.5201 (3.1); 2.5114 (34.6); 2.5070 (67.6); 2.5024 (87.0); 2.4978 (63.0); 2.4933 (30.4); 2.3383 (0.6); 2.3337 (0.8); 2.3291 (1.0); 2.3249 (0.8); 2.3203 (0.6); 2.3006 (0.8); 2.0556 (1.6); 2.0113 (0.9); 1.9888 (1.1); 1.7585 (0.5); 1.7361 (1.5); 1.7278 (1.4); 1.6956 (1.3); 1.6663 (2.8); 1.6442 (3.2); 1.6252 (0.9); 1.5155 (0.3); 1.2345 (0.8); 1.1930 (0.4); 1.1752 (1.0); 1.1651 (6.5); 1.1575 (1.5); 1.1478 (7.3); 1.1418 (7.1); 1.1246 (6.3); 1.0697 (0.5); 0.8886 (0.6); 0.8718 (0.6); 0.0080 (2.1); -0.0002 (59.5); -0.0085 (2.0) |

In Analogie zu den Beispielen und gemäß den oben beschriebenen Herstellverfahren lassen sich die in Tabelle 2 genannten Intermediate (IM-1) erhalten:

**Tabelle 2**

| **Nr.** | **Struktur** | **NMR-Daten** |
|---|---|---|
| IM-1-01 | 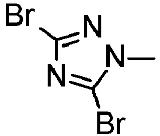 | IM-1-01: ¹H-NMR(400.2 MHz, d₆-DMSO): |
| | | δ= 3.8364 (16.0); 3.3607 (20.0); 2.8971 (1.2); 2.7375 (1.0); 2.7367 (1.0); 2.5182 (1.5); 2.5137 (3.0); 2.5092 (3.8); 2.5046 (2.8); 2.5002 (1.3); -0.0002 (1.0) |
| IM-1-02 | 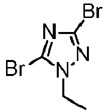 | IM-1-02: ¹H-NMR(400.2 MHz, d₆-DMSO): |
| | | δ= 4.2052 (2.4); 4.1871 (7.5); 4.1689 (7.6); 4.1508 (2.5); 3.3297 (15.2); 2.5126 (8.4); 2.5083 (16.5); 2.5039 (21.4); 2.4994 (15.6); 2.4952 (7.7); 1.3683 (8.0); 1.3502 (16.0); 1.3321 (7.7); -0.0002 (1.3) |
| IM-1-03 | 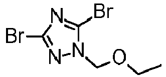 | IM-1-03: ¹H-NMR(400.2 MHz, CD3CN): |
| | | δ= 5.4387 (16.0); 3.6262 (2.1); 3.6086 (6.2); 3.5911 (6.2); 3.5735 (2.1); 2.1431 (4.9); 1.9542 (2.5); 1.9480 (4.7); 1.9419 (6.5); 1.9357 (4.5); 1.9295 (2.4); 1.1560 (6.6); 1.1385 (12.6); 1.1209 (6.4); -0.0002 (5.2) |
| IM-1-04 | 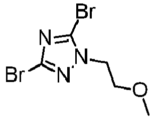 | IM-1-04: ¹H-NMR(400.2 MHz, CDCl3): |
| | | δ= 7.2788 (0.8); 4.3244 (3.3); 4.3110 (5.6); 4.2976 (3.7); 3.8855 (0.5); 3.7896 (3.6); 3.7759 (5.7); 3.7627 (3.4); 3.3405 (16.0); 1.7138 (0.8); - 0.0002 (1.4) |

In Analogie zu den Beispielen und gemäß den oben beschriebenen Herstellverfahren lassen sich die in Tabelle 3 genannten Intermediate (IM-2) erhalten:

**Tabelle 3**

| **Nr.** | **Struktur** | **NMR-Daten** |
|---|---|---|
| IM-2-01 | 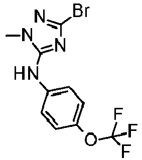 | IM-2-01: ¹H-NMR(400.2 MHz, d₆-DMSO): |
| | | δ= 9.3614 (2.1); 7.6359 (3.5); 7.6302 (1.1); 7.6187 (1.2); 7.6129 (4.2); 7.6042 (0.4); 7.3286 (2.1); 7.3078 (1.8); 3.7170 (16.0); 3.3279 (4.7); 2.8916 (0.3); 2.5211 (0.5); 2.5124 (7.0); 2.5079 (14.6); 2.5034 (19.3); 2.4987 (13.7); 2.4942 (6.4); -0.0002 (1.6) |
| IM-2-02 | 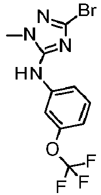 | IM-2-02: ¹H-NMR(400.2 MHz, d₆-DMSO): |
| | | δ= 9.4769 (2.2); 7.6142 (1.5); 7.6121 (1.5); 7.5724 (0.8); 7.5706 (1.0); 7.5674 (0.8); 7.5654 (0.7); 7.5517 (1.1); 7.5498 (1.2); 7.5466 (1.1); 7.5446 (1.0); 7.4498 (1.6); 7.4293 (2.6); 7.4086 (1.2); 6.9371 (0.8); 6.9344 (0.9); 6.9316 (0.8); 6.9167 (0.7); 6.9139 (0.8); 6.9112 (0.7); 3.7314 (16.0); 3.3402 (1.8); 2.5173 (2.5); 2.5128 (5.3); 2.5082 (7.0); 2.5036 (5.0); 2.4990 (2.4); -0.0002 (4.3) |
| IM-2-03 | 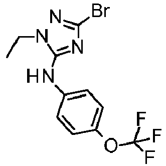 | IM-2-03: ¹H-NMR(400.2 MHz, d₆-DMSO): |
| | | δ= 9.3255 (5.0); 7.6515 (0.7); 7.6429 (7.4); 7.6375 (2.4); 7.6254 (2.8); 7.6201 (8.5); 7.6114 (0.9); 7.3285 (5.2); 7.3066 (4.6); 4.1364 (2.0); 4.1185 (6.3); 4.1006 (6.4); 4.0827 (2.1); 3.3321 (20.5); 2.5085 (36.3); 2.5041 (46.0); 2.4996 (33.2); 1.3329 (7.3); 1.3150 (16.0); 1.2971 (7.1); 0.0079 (0.5); -0.0002 (12.3); -0.0083 (0.5) |
| IM-2-04 | 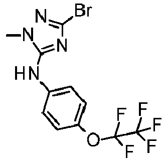 | IM-2-04: ¹H-NMR(400.2 MHz, d₆-DMSO): |
| | | δ= 9.3784 (2.6); 7.6563 (0.4); 7.6476 (3.7); 7.6421 (1.2); 7.6303 (1.3); 7.6247 (4.4); 7.6160 (0.4); 7.3195 (3.0); 7.2969 (2.7); 3.7202 (16.0); 3.3357 (14.8); 2.5266 (0.4); 2.5130 (8.8); 2.5088 (17.0); 2.5043 (21.9); 2.4997 (15.9); 2.4954 (7.8); 0.0079 (0.7); -0.0002 (18.4); -0.0085 (0.7) |
| IM-2-05 | 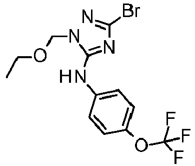 | IM-2-05: ¹H-NMR(400.2 MHz, CD3CN): |
| | | δ= 7.7928 (1.4); 7.6586 (0.6); 7.6500 (5.5); 7.6444 (1.9); 7.6326 (2.2); 7.6271 (6.0); 7.6184 (0.7); 7.2737 (3.7); 7.2528 (3.3); 5.3828 (16.0); 3.6190 (2.1); 3.6014 (6.5); 3.5838 (6.5); 3.5663 (2.2); 2.1825 (58.5); 1.9657 (1.5); 1.9595 (2.1); 1.9538 (12.7); 1.9476 (23.4); 1.9414 (31.5); 1.9352 (21.7); 1.9291 (11.0); 1.1677 (6.8); 1.1501 (13.6); 1.1326 (6.6); 0.0078 (1.1); -0.0002 (21.6); -0.0085 (0.9) |
| IM-2-06 | 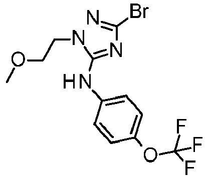 | IM-2-06: ¹H-NMR(400.2 MHz, d₆-DMSO): |
| | | δ= 9.3108 (1.4); 7.6507 (2.9); 7.6451 (0.9); 7.6335 (1.0); 7.6278 (3.4); 7.6190 (0.3); 7.3315 (1.8); 7.3104 (1.6); 4.2991 (1.2); 4.2857 (2.6); 4.2722 (1.3); 3.6786 (1.4); 3.6652 (2.9); 3.6517 (1.3); 3.3310 (38.4); 3.2402 (16.0); 2.5248 (0.7); 2.5199 (1.1); 2.5113 (14.4); 2.5069 (29.4); 2.5023 (38.7); 2.4977 (28.0); 2.4932 (13.6) |
| IM-2-07 | 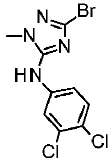 | IM-2-07: ¹H-NMR(400.2 MHz, d₆-DMSO): |
| | | δ= 9.4930 (2.5); 7.8691 (2.6); 7.8632 (2.6); 7.5670 (1.2); 7.5449 (4.0); 7.5312 (2.6); 7.5252 (2.4); 7.5091 (0.7); 7.5030 (0.8); 4.0392 (0.9); 4.0214 (0.9); 3.7179 (16.0); 3.3304 (6.8); 2.5138 (4.4); 2.5094 (8.6); 2.5048 (11.6); 2.5003 (8.6); 2.4958 (4.2); 1.9903 (3.9); 1.1946 (1.1); 1.1768 (2.1); 1.1590 (1.0); 0.8892 (0.3); 0.8724 (0.3); -0.0002 (0.6) |
| IM-2-08 | 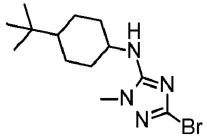 | IM-2-08: ¹H-NMR(400.2 MHz, d₆-DMSO): |
| | | δ= 6.5290 (0.6); 6.5099 (0.6); 4.0384 (0.4); 4.0206 (0.4); 3.5414 (1.8); 3.4658 (6.8); 3.3265 (4.1); 2.5118 (2.7); 2.5074 (5.5); 2.5029 (7.5); 2.4983 (5.7); 2.4939 (2.8); 2.0109 (0.5); 2.0002 (0.5); 1.9890 (1.9); 1.9695 (0.6); 1.7655 (0.5); 1.7541 (0.9); 1.7382 (0.6); 1.2089 (0.9); 1.1937 (0.8); 1.1859 (0.5); 1.1812 (0.5); 1.1760 (1.1); 1.1582 (0.7); 1.0737 (0.4); 1.0668 (0.5); 1.0351 (0.6); 1.0109 (0.4); 1.0029 (0.4); 0.9957 (0.5); 0.9908 (0.5); 0.9620 (0.4); 0.9221 (1.0); 0.9128 (0.4); 0.9054 (0.3); 0.8981 (0.3); 0.8889 (0.4); 0.8840 (0.9); 0.8721 (0.5); 0.8596 (1.0); 0.8425 (16.0); 0.8242 (2.7) |
| IM-2-09 | 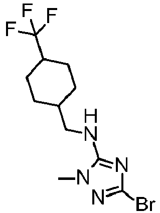 | IM-2-09: ¹H-NMR(400.2 MHz, d₆-DMSO): |
| | | δ= 8.1535 (1.3); 6.8084 (2.5); 6.7942 (5.0); 6.7803 (2.5); 5.7545 (16.0); 3.4748 (62.1); 3.3243 (23.0); 3.1982 (4.8); 3.1835 (5.7); 3.1791 (5.8); 3.1645 (4.8); 3.0587 (3.0); 3.0426 (4.7); 3.0269 (3.1); 2.6758 (0.5); 2.6712 (0.7); 2.6668 (0.5); 2.5246 (2.0); 2.5112 (43.7); 2.5068 (85.4); 2.5023 (111.4); 2.4977 (82.4); 2.4933 (41.2); 2.3336 (0.7); 2.3290 (1.0); 2.3245 (0.8); 2.3200 (0.6); 2.3017 (0.8); 2.2914 (1.0); 2.2794 (1.1); 2.2679 (1.1); 2.2568 (0.9); 2.2481 (0.9); 2.2248 (0.8); 2.2178 (0.8); 2.2096 (0.6); 2.2021 (0.6); 2.1956 (0.8); 2.1867 (0.6); 2.1796 (0.4); 2.1734 (0.4); 2.1646 (0.4); 1.9127 (1.5); 1.9033 (2.3); 1.8943 (3.1); 1.8874 (2.8); 1.8533 (4.0); 1.8185 (2.0); 1.7934 (0.4); 1.6403 (1.1); 1.6195 (3.7); 1.6117 (3.6); 1.5819 (4.4); 1.5760 (4.4); 1.5430 (4.3); 1.5238 (7.3); 1.4985 (6.8); 1.4817 (3.2); 1.4544 (0.5); 1.4466 (0.4); 1.2676 (0.7); 1.2600 (0.7); 1.2354 (1.8); 1.2276 (1.9); 1.2026 (1.8); 1.1966 (1.9); 1.1718 (0.9); 1.0153 (1.0); 0.9900 (2.0); 0.9591 (1.7); 0.9530 (1.5); 0.9276 (0.6); 0.9202 (0.5); 0.0079 (2.2); -0.0002 (52.0); -0.0085 (1.9) |
| IM-2-10 | 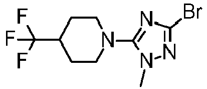 | IM-2-10: ¹H-NMR(400.2 MHz, d₆-DMSO): |
| | | δ= 3.6330 (16.0); 3.5184 (1.0); 3.4865 (1.1); 3.3222 (9.2); 2.9350 (0.8); 2.9293 (0.8); 2.9035 (1.6); 2.8979 (1.5); 2.8720 (0.8); 2.8663 (0.7); 2.5724 (0.3); 2.5633 (0.4); 2.5415 (0.4); 2.5324 (0.4); 2.5248 (0.4); 2.5199 (0.6); 2.5115 (4.6); 2.5070 (8.7); 2.5024 (11.4); 2.4978 (8.4); 2.4933 (4.1); 1.8678 (0.9); 1.8643 (0.9); 1.8366 (1.1); 1.6607 (0.4); 1.6505 (0.4); 1.6293 (1.0); 1.6192 (1.0); 1.5975 (0.9); 1.5876 (0.9); -0.0002 (7.4) |
| IM-2-11 | 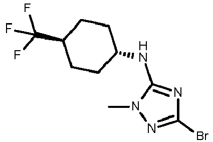 | IM-2-11: ¹H-NMR(400.2 MHz, d₆-DMSO): |
| | | δ= 8.1473 (1.2); 6.6351 (4.5); 6.6164 (4.6); 6.4317 (1.1); 6.4182 (1.2); 5.7548 (3.0); 3.7428 (0.4); 3.7320 (0.8); 3.7220 (0.8); 3.7117 (0.5); 3.5380 (16.0); 3.5087 (0.3); 3.4769 (54.1); 3.4638 (2.0); 3.4355 (0.5); 3.4250 (0.7); 3.4072 (1.6); 3.3975 (1.8); 3.3886 (1.8); 3.3793 (2.2); 3.3698 (1.8); 3.3604 (1.7); 3.3278 (5.2); 3.2991 (1.3); 2.5253 (0.8); 2.5119 (16.0); 2.5077 (32.3); 2.5032 (43.0); 2.4986 (32.8); 2.4943 (17.2); 2.3302 (0.6); 2.3251 (0.5); 2.3204 (0.6); 2.3087 (0.7); 2.2963 (1.0); 2.2850 (1.0); 2.2689 (1.5); 2.2612 (1.3); 2.2468 (1.4); 2.2394 (1.2); 2.2257 (0.7); 2.2174 (0.7); 2.0748 (0.6); 2.0502 (4.7); 2.0241 (3.6); 1.9126 (3.7); 1.8844 (6.0); 1.6997 (0.7); 1.6717 (1.4); 1.6634 (1.5); 1.6419 (3.6); 1.6203 (0.8); 1.6128 (1.0); 1.6045 (1.2); 1.5863 (0.4); 1.5758 (0.4); 1.4076 (1.1); 1.4014 (1.0); 1.3752 (3.3); 1.3680 (2.8); 1.3402 (6.3); 1.3263 (2.9); 1.3094 (6.6); 1.2830 (2.4); 1.2758 (3.0); 1.2499 (0.9); 1.2431 (0.9); 0.8815 (0.4); 0.8652 (0.4); 0.0080 (1.0); -0.0002 (27.7); -0.0084 (1.2) |
| IM-2-12 | 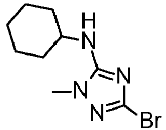 | IM-2-12: ¹H-NMR(400.2 MHz, CDCl3): |
| | | δ= 7.2726 (1.3); 3.8094 (0.6); 3.7916 (0.7); 3.6841 (0.3); 3.6741 (0.3); 3.6665 (0.5); 3.6575 (0.7); 3.6478 (0.6); 3.6382 (0.6); 3.6304 (0.5); 3.6214 (0.3); 3.5230 (16.0); 2.1000 (1.2); 2.0918 (1.2); 2.0804 (0.9); 2.0688 (1.2); 2.0610 (1.2); 1.7645 (0.9); 1.7555 (1.5); 1.7451 (1.9); 1.7311 (1.1); 1.7220 (1.4); 1.7136 (1.1); 1.6699 (0.5); 1.6610 (0.6); 1.6515 (0.5); 1.6378 (0.6); 1.6285 (0.7); 1.6195 (0.5); 1.4398 (0.5); 1.4177 (0.7); 1.4095 (1.3); 1.3766 (1.3); 1.3540 (0.4); 1.3458 (0.7); 1.3376 (0.4); 1.2211 (1.0); 1.2125 (0.8); 1.1903 (2.0); 1.1833 (1.6); 1.1583 (1.7); 1.1341 (0.5); 1.1266 (0.5); -0.0002 (1.8) |
| IM-2-13 | 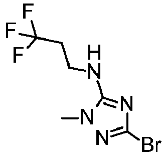 | IM-2-13: ¹H-NMR(400.2 MHz, CDCl3): |
| | | δ= 7.2654 (3.6); 4.2356 (0.5); 3.7152 (1.1); 3.6996 (2.7); 3.6841 (2.7); 3.6684 (1.1); 3.5508 (16.0); 3.5483 (8.3); 2.5346 (0.5); 2.5236 (1.0); 2.5205 (0.8); 2.5078 (1.4); 2.4965 (1.2); 2.4927 (1.3); 2.4808 (1.4); 2.4655 (1.0); 2.4538 (0.5); 1.8574 (0.5); -0.0002 (4.4); -0.0029 (2.2) |
| IM-2-14 | 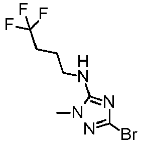 | IM-2-14: ¹H-NMR(400.2 MHz, CDCl3): |
| | | δ= 7.2643 (3.7); 5.3018 (0.7); 4.0206 (0.6); 3.5545 (16.0); 3.5122 (0.9); 3.4952 (2.1); 3.4786 (2.1); 3.4618 (0.9); 2.9652 (1.4); 2.8852 (1.3); 2.2315 (0.3); 2.2233 (0.8); 2.2047 (0.9); 2.1965 (1.0); 2.1838 (1.1); 2.1780 (1.0); 2.1709 (0.6); 2.1570 (1.0); 1.9542 (0.6); 1.9354 (1.4); 1.9163 (1.8); 1.8978 (1.2); 1.8796 (0.4); -0.0002 (4.9) |
| IM-2-15 | 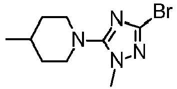 | IM-2-15: ¹H-NMR(400.2 MHz, d₆-DMSO): |
| | | δ= 3.6098 (16.0); 3.4062 (1.2); 3.3747 (1.4); 3.3277 (1.0); 2.8405 (1.0); 2.8343 (1.0); 2.8095 (1.8); 2.8036 (1.7); 2.7786 (0.9); 2.7725 (0.8); 2.5134 (2.1); 2.5091 (4.1); 2.5045 (5.3); 2.5000 (3.8); 2.4955 (1.8); 1.6740 (1.0); 1.6687 (1.0); 1.6419 (1.1); 1.6372 (1.1); 1.5351 (0.4); 1.5275 (0.4); 1.5185 (0.4); 1.5077 (0.4); 1.3015 (0.5); 1.2917 (0.5); 1.2701 (1.0); 1.2608 (1.0); 1.2401 (0.9); 1.2305 (0.8); 1.2098 (0.3); 0.9434 (6.5); 0.9270 (6.1); -0.0002 (5.3) |
| IM-2-16 | 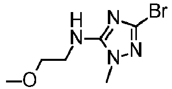 | IM-2-16: ¹H-NMR(400.2 MHz, CDCl3): |
| | | δ= 8.0810 (2.1); 7.2805 (1.3); 4.5921 (0.5); 3.5855 (0.6); 3.5701 (7.7); 3.5573 (16.0); 3.5412 (0.7); 3.5346 (0.3); 3.3884 (16.0); 2.0484 (0.6); 1.2610 (0.4); - 0.0002 (1.7) |
| IM-2-17 | 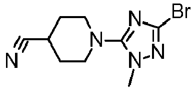 | IM-2-17: ¹H-NMR(400.2 MHz, CDCl3): |
| | | δ= 7.2795 (1.7); 3.6754 (16.0); 3.4231 (0.7); 3.4143 (0.8); 3.4058 (0.8); 3.3966 (0.8); 3.3907 (0.9); 3.3816 (0.9); 3.3733 (1.0); 3.3645 (0.9); 3.1560 (1.0); 3.1478 (1.0); 3.1354 (1.0); 3.1263 (1.2); 3.1151 (0.8); 3.1026 (0.8); 3.0945 (0.8); 2.8671 (0.5); 2.8569 (0.7); 2.8466 (0.9); 2.8363 (0.7); 2.8261 (0.5); 2.1402 (0.3); 2.1307 (0.6); 2.1221 (0.6); 2.1135 (0.6); 2.1056 (0.8); |
| | | 2.0972 (1.0); 2.0887 (1.1); 2.0801 (1.0); 2.0703 (0.5); 2.0355 (0.7); 2.0267 (0.7); 2.0150 (1.3); 2.0062 (1.3); 1.9939 (0.9); 1.9853 (0.7); 1.9814 (0.8); 1.9723 (0.7); 1.9605 (0.4); 1.9518 (0.3); 1.8172 (4.5); -0.0002 (2.1) |
| IM-2-18 | 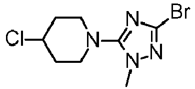 | IM-2-18: ¹H-NMR(400.2 MHz, d₆-DMSO): |
| | | δ= 4.4336 (0.4); 4.4227 (0.6); 4.4125 (0.9); 4.4023 (0.6); 4.3914 (0.4); 3.6301 (16.0); 3.6214 (0.4); 3.3891 (0.6); 3.3794 (0.7); 3.3753 (0.6); 3.3582 (0.7); 3.3429 (0.9); 3.3288 (10.8); 3.1073 (0.9); 3.0994 (0.9); 3.0855 (1.0); 3.0765 (1.3); 3.0670 (0.8); 3.0530 (0.8); 3.0452 (0.8); 2.5254 (0.4); 2.5206 (0.5); 2.5119 (7.4); 2.5075 (15.2); 2.5030 (19.7); 2.4984 (13.8); 2.4939 (6.5); 2.1803 (0.3); 2.1717 (0.6); 2.1637 (0.6); 2.1561 (0.6); 2.1471 (0.7); 2.1382 (0.8); 2.1310 (0.8); 2.1232 (0.8); 2.1145 (0.4); 2.0754 (0.6); 1.9128 (0.5); 1.9038 (0.5); 1.8914 (0.9); 1.8821 (1.0); 1.8701 (0.8); 1.8586 (0.9); 1.8490 (0.7); 1.8365 (0.4); 1.8275 (0.4); 0.0078 (0.6); -0.0002 (19.4); -0.0086 (0.7) |
| IM-2-19 | 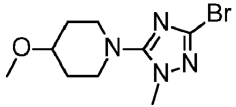 | IM-2-19: ¹H-NMR(400.2 MHz, CDCl3): |
| | | δ= 7.2726 (1.6); 3.6652 (14.9); 3.4134 (1.0); 3.4029 (1.4); 3.3935 (1.5); 3.3833 (1.7); 3.3727 (16.0); 3.3562 (0.7); 3.0432 (0.9); 3.0352 (0.9); 3.0203 (1.0); 3.0121 (1.6); 3.0037 (0.8); 2.9888 (0.8); 2.9808 (0.8); 2.0395 (0.3); 2.0311 (0.6); 2.0234 (0.6); 2.0163 (0.6); 2.0075 (0.7); 1.9986 (0.7); 1.9917 (0.8); 1.9841 (0.7); 1.9756 (0.4); 1.7517 (0.5); 1.7424 (0.5); 1.7296 (0.9); 1.7195 (1.6); 1.7134 (1.4); 1.6983 (1.0); 1.6881 (0.6); 1.6756 (0.4); 1.6662 (0.4); -0.0002 (1.9) |
| IM-2-20 | 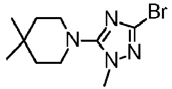 | IM-2-20: ¹H-NMR(400.2 MHz, d₆-DMSO): |
| | | δ= 3.6098 (16.0); 3.4062 (1.2); 3.3747 (1.4); 3.3277 (1.0); 2.8405 (1.0); 2.8343 (1.0); 2.8095 (1.8); 2.8036 (1.7); 2.7786 (0.9); 2.7725 (0.8); 2.5134 (2.1); 2.5091 (4.1); 2.5045 (5.3); 2.5000 (3.8); 2.4955 (1.8); 1.6740 (1.0); 1.6687 (1.0); 1.6419 (1.1); 1.6372 (1.1); 1.5351 (0.4); 1.5275 (0.4); 1.5185 (0.4); 1.5077 (0.4); 1.3015 (0.5); 1.2917 (0.5); 1.2701 (1.0); 1.2608 (1.0); 1.2401 (0.9); 1.2305 (0.8); 1.2098 (0.3); 0.9434 (6.5); 0.9270 (6.1); -0.0002 (5.3) |
| IM-2-21 | 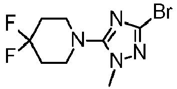 | IM-2-21: ¹H-NMR(400.2 MHz, CDCl3): |
| | | δ= 7.2643 (3.7); 3.6910 (16.0); 3.3534 (2.6); 3.3393 (3.0); 3.3246 (2.7); 2.1857 (0.7); 2.1710 (0.9); 2.1520 (1.5); 2.1370 (1.7); 2.1223 (1.4); 2.1033 (0.9); 2.0886 (0.7); 1.5918 (2.0); -0.0002 (4.7) |
| IM-2-22 | 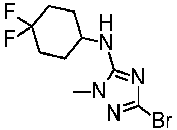 | IM-2-23: ¹H-NMR(400.2 MHz, d₆-DMSO): |
| | | δ= 8.3133 (0.5); 6.6231 (14.2); 6.6047 (14.4); 3.6677 (2.1); 3.6610 (3.2); 3.6425 (5.3); 3.6339 (4.7); 3.6245 (5.3); 3.4877 (202.3); 3.4684 (0.9); 3.3838 (0.4); 3.3635 (1.2); 3.3360 (375.6); 3.3099 (1.8); 3.2980 (0.6); 2.6772 (0.8); 2.6725 (1.1); 2.6682 (0.8); 2.5260 (3.6); 2.5125 (71.7); 2.5081 (142.0); 2.5037 (183.4); 2.4991 (130.9); 2.4947 (62.8); 2.3348 (0.9); 2.3304 (1.1); 2.3258 (0.8); 2.0842 (2.3); 2.0740 (3.2); 2.0588 (7.6); 2.0516 (8.2); 2.0330 (9.7); 2.0266 (10.0); 2.0145 (9.4); 2.0031 (8.5); 1.9912 (7.2); 1.9621 (14.5); 1.9288 (16.0); 1.9229 (15.6); 1.8931 (6.5); 1.8831 (3.9); 1.8612 (3.2); 1.8508 (1.9); 1.6156 (4.5); 1.6037 (3.3); 1.5832 (10.7); 1.5497 (10.2); 1.5273 (4.1); - 0.0002 (3.6) |
| IM-2-23 | 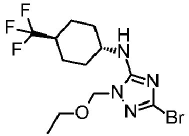 | IM-2-25: ¹H-NMR(400.2 MHz, d₆-DMSO): |
| | | δ= 6.9998 (1.8); 6.9809 (1.9); 6.7718 (0.5); 6.7583 (0.5); 5.7549 (4.6); 5.3287 (3.3); 5.2295 (11.2); 3.5277 (0.6); 3.5216 (2.2); 3.5101 (2.1); 3.5040 (7.3); 3.4925 (2.3); 3.4864 (7.4); 3.4749 (1.0); 3.4689 (2.5); 3.4541 (0.6); 3.4453 (0.6); 3.4360 (0.6); 3.4273 (0.5); 3.4175 (0.3); 3.3297 (70.8); 2.5254 (1.0); 2.5207 (1.4); 2.5120 (18.9); 2.5075 (39.3); 2.5030 (51.7); 2.4983 (36.3); 2.4937 (16.8); 2.3343 (0.4); 2.3296 (0.5); 2.3252 (0.4); 2.2969 (0.4); 2.2754 (0.5); 2.2536 (0.5); 2.2470 (0.4); 2.0744 (1.6); 2.0341 (1.7); 2.0102 (1.2); 1.9127 (1.6); 1.8976 (1.2); 1.8859 (2.3); 1.6504 (1.1); 1.6429 (1.1); 1.6308 (1.8); 1.6033 (0.3); 1.4104 (0.4); 1.4055 (0.3); 1.3780 (1.2); 1.3711 (1.0); |
| | | 1.3475 (3.5); 1.3207 (3.4); 1.2980 (0.9); 1.2919 (1.0); 1.0973 (7.6); 1.0825 (5.7); 1.0798 (16.0); 1.0648 (2.8); 1.0622 (7.4) |
| IM-2-24 | 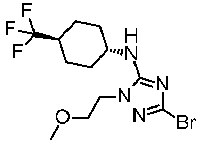 | IM-2-26: ¹H-NMR(400.2 MHz, d₆-DMSO): |
| | | δ= 6.5961 (0.8); 6.5773 (0.8); 5.7549 (1.1); 4.1212 (0.6); 4.1078 (0.4); 4.0337 (1.1); 4.0199 (2.5); 4.0062 (1.3); 3.5981 (0.4); 3.5846 (0.8); 3.5754 (1.5); 3.5684 (0.9); 3.5618 (2.8); 3.5480 (1.2); 3.3985 (0.4); 3.3898 (0.3); 3.3299 (48.4); 3.2370 (4.1); 3.2246 (16.0); 2.5249 (0.6); 2.5202 (1.0); 2.5115 (13.3); 2.5070 (27.1); 2.5024 (35.4); 2.4978 (25.0); 2.4932 (11.8); 2.0741 (0.4); 2.0337 (0.8); 2.0055 (0.6); 1.9085 (0.7); 1.8934 (0.6); 1.8797 (1.0); 1.6370 (0.4); 1.6305 (0.5); 1.6146 (0.6); 1.3733 (0.6); 1.3659 (0.5); 1.3377 (0.9); 1.3187 (0.7); 1.2994 (0.8); 1.2736 (0.4); 1.2660 (0.5) |
| IM-2-25 | 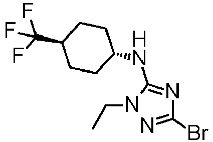 | IM-2-27: ¹H-NMR(400.2 MHz, d₆-DMSO): |
| | | δ= 8.3148 (0.4); 6.6277 (2.2); 6.6090 (2.2); 6.4155 (0.5); 6.4022 (0.5); 5.7547 (15.3); 3.9737 (0.5); 3.9559 (1.6); 3.9380 (1.6); 3.9201 (0.5); 3.8887 (1.8); 3.8708 (5.8); 3.8530 (5.9); 3.8351 (1.8); 3.7437 (0.3); 3.4167 (0.6); 3.4071 (0.7); 3.3982 (0.7); 3.3888 (0.8); 3.3794 (0.6); 3.3702 (0.4); 3.3606 (0.4); 3.3281 (53.3); 2.5255 (0.9); 2.5207 (1.4); 2.5121 (19.4); 2.5076 (40.1); 2.5030 (52.5); 2.4983 (37.0); 2.4938 (17.4); 2.3345 (0.4); 2.3297 (0.4); 2.3251 (0.4); 2.2967 (0.4); 2.2880 (0.4); 2.2747 (0.5); 2.2664 (0.6); 2.2593 (0.5); 2.2447 (0.6); 2.2371 (0.5); 2.0745 (1.4); 2.0453 (2.1); 2.0189 (1.6); 1.9117 (1.8); 1.8978 (1.3); 1.8834 (2.6); 1.6606 (0.6); 1.6521 (1.1); 1.6388 (1.4); 1.6324 (1.6); 1.6214 (0.5); 1.6075 (0.3); 1.5977 (0.4); 1.4067 (0.5); 1.4004 (0.4); 1.3741 (1.5); 1.3665 (1.3); 1.3394 (2.8); 1.3246 (1.2); 1.3078 (2.9); 1.2817 (1.1); 1.2744 (1.3); 1.2485 (0.4); 1.2411 (0.4); 1.2285 (1.8); 1.2106 (5.1); 1.2075 (7.3); 1.1897 (16.0); 1.1718 (6.7); 0.0080 (0.5); -0.0002 (17.4); -0.0086 (0.5) |

In Analogie zu den Beispielen und gemäß den oben beschriebenen Herstellverfahren lassen sich die in Tabelle 4 genannten Intermediate (IM-3) und (IM-4) erhalten:

**Tabelle 4**

| **Nr.** | **Struktur** | **NMR-Daten** |
|---|---|---|
| IM-3-01 | 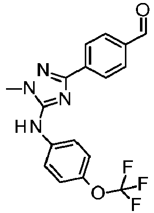 | IM-3-01: ¹H-NMR(400.2 MHz, d₆-DMSO): |
| | | δ= 10.0408 (5.4); 9.3081 (2.7); 8.1924 (3.4); 8.1719 (4.3); 8.0042 (4.4); 8.0000 (1.4); 7.9874 (1.3); 7.9832 (3.6); 7.8506 (0.4); 7.8417 (3.9); 7.8362 (1.2); 7.8245 (1.3); 7.8189 (4.4); 7.8102 (0.4); 7.3549 (2.4); 7.3339 (2.2); 3.8473 (16.0); 3.3227 (11.7); 2.6714 (0.4); 2.5249 (1.0); 2.5202 (1.6); 2.5115 (21.8); 2.5070 (44.6); 2.5025 (58.7); 2.4979 (42.1); 2.4934 (20.0); 2.3292 (0.4); 0.0080 (1.6); -0.0002 (45.9); -0.0085 (1.4) |
| IM-3-02 | 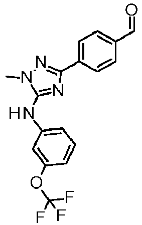 | IM-3-02: ¹H-NMR(400.2 MHz, d₆-DMSO): |
| | | δ= 10.0409 (5.3); 9.4379 (2.6); 8.1788 (3.2); 8.1583 (4.3); 8.0172 (4.4); 8.0129 (1.5); 7.9960 (3.5); 7.8805 (1.6); 7.8783 (1.6); 7.7284 (1.0); 7.7250 (0.9); 7.7235 (0.9); 7.7077 (1.2); 7.7043 (1.1); 7.4769 (1.5); 7.4564 (2.7); 7.4357 (1.4); 6.9367 (0.9); 6.9345 (0.9); 6.9313 (0.9); 6.9164 (0.8); 6.9129 (0.8); 6.9108 (0.8); 3.8568 (16.0); 3.3282 (7.8); 2.5268 (0.6); 2.5221 (0.9); 2.5134 (10.7); 2.5090 (21.8); 2.5044 (28.5); 2.4998 (20.4); 2.4953 (9.7); 0.0080 (0.4); -0.0002 (12.7); -0.0085 (0.4) |
| IM-3-03 | 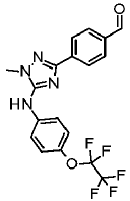 | IM-3-03: ¹H-NMR(400.2 MHz, d₆-DMSO): |
| | | δ= 10.0417 (5.7); 9.3271 (3.2); 8.1942 (3.7); 8.1736 (4.6); 8.0066 (4.7); 7.9858 (3.8); 7.8602 (0.4); 7.8516 (4.1); 7.8463 (1.4); 7.8343 (1.4); 7.8288 (4.6); 7.8201 (0.4); 7.3437 (3.3); 7.3212 (3.1); 3.8500 (16.0); 3.3344 (34.3); 2.6725 (0.3); 2.5259 (1.1); 2.5124 (19.9); 2.5081 (39.1); 2.5036 (50.9); 2.4991 (37.6); 2.4948 (18.8); 1.9898 (0.9); 1.1755 (0.5); 0.0079 (1.1); -0.0002 (28.1); -0.0084 (1.1) |
| IM-3-04 | 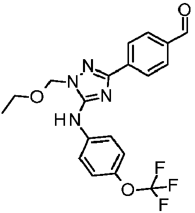 | IM-3-04: ¹H-NMR(400.2 MHz, d₆-DMSO): |
| | | δ= 10.0577 (9.9); 9.5601 (5.3); 8.2312 (6.8); 8.2106 (7.9); 8.0273 (8.7); 8.0065 (6.5); 7.9351 (2.1); 7.9267 (7.9); 7.9212 (3.0); 7.9094 (3.6); 7.9039 (8.6); 7.8952 (0.9); 7.8897 (0.7); 7.3749 (5.4); 7.3536 (4.7); 5.7574 (0.4); 5.6128 (11.6); 4.0567 (0.7); 4.0388 (2.0); 4.0210 (2.0); 4.0033 (0.6); 3.6619 (2.7); 3.6444 (7.8); 3.6268 (7.6); 3.6093 (2.3); 3.3429 (16.6); 3.3348 (95.2); 2.6779 (0.4); 2.6737 (0.5); 2.6691 (0.3); 2.5133 (45.8); 2.5090 (67.9); 2.5045 (77.1); 2.5000 (52.1); 2.4957 (23.7); 2.3360 (0.4); 2.3315 (0.5); 2.0122 (0.4); 1.9979 (1.1); 1.9901 (8.5); 1.3188 (5.1); 1.2711 (0.7); 1.2120 (0.8); 1.1938 (2.4); 1.1760 (4.8); 1.1582 (3.2); 1.1439 (8.4); 1.1342 (3.2); 1.1263 (16.0); 1.1173 (1.9); 1.1088 (7.3); 1.0706 (1.0); 0.0080 (6.7); -0.0002 (44.9); -0.0086 (1.5) |
| IM-3-05 | 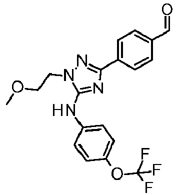 | IM-3-05: ¹H-NMR(400.2 MHz, d₆-DMSO): |
| | | δ= 10.0468 (16.0); 9.2485 (8.7); 8.3172 (0.4); 8.2047 (10.3); 8.1842 (13.1); 8.0131 (13.4); 7.9923 (10.9); 7.8713 (1.1); 7.8625 (11.7); 7.8573 (3.9); 7.8452 (3.9); 7.8398 (13.2); 7.8311 (1.3); 7.3558 (8.0); 7.3344 (7.5); 5.7572 (0.4); 4.4243 (4.6); 4.4107 (9.7); 4.3971 (5.0); 4.0559 (0.5); 4.0381 (1.6); 4.0203 (1.6); 4.0025 (0.6); 3.9346 (1.8); 3.7725 (5.5); 3.7590 (11.0); 3.7453 (5.2); 3.3308 (143.0); 3.2640 (58.2); 2.6766 (0.8); 2.6721 (1.0); 2.6676 (0.8); 2.5254 (2.8); 2.5118 (64.5); 2.5076 (131.1); 2.5031 (173.0); 2.4986 (127.6); 2.4945 (64.1); 2.3345 (0.8); 2.3299 (1.1); 2.3255 (0.8); 2.0118 (0.5); 1.9894 |
| | | (7.1); 1.3184 (1.3); 1.2695 (0.7); 1.2124 (0.7); 1.1931 (1.9); 1.1754 (3.7); 1.1576 (1.9); 1.0693 (12.4); 0.8885 (0.6); 0.8717 (0.6); 0.0079 (2.5); -0.0002 (75.4); -0.0085 (3.0); -0.1496 (0.3) |
| IM-3-06 | 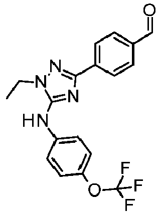 | IM-3-06: ¹H-NMR(400.2 MHz, d₆-DMSO): |
| | | δ= 10.0441 (3.5); 9.2704 (1.8); 8.2040 (2.2); 8.1834 (2.8); 8.0076 (2.9); 8.0033 (1.0); 7.9906 (1.0); 7.9865 (2.4); 7.8545 (2.6); 7.8490 (0.8); 7.8372 (0.9); 7.8317 (2.9); 7.3555 (1.6); 7.3345 (1.5); 4.2636 (0.6); 4.2457 (1.8); 4.2278 (1.8); 4.2098 (0.6); 3.9359 (2.0); 3.3354 (6.3); 2.5145 (5.7); 2.5101 (11.4); 2.5055 (14.7); 2.5009 (10.6); 2.4964 (5.1); 1.4170 (2.3); 1.3992 (5.2); 1.3812 (2.2); 1.0721 (16.0); -0.0002 (3.3) |
| IM-3-07 | 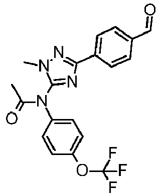 | IM-3-07: ¹H-NMR(400.2 MHz, d₆-DMSO): |
| | | δ= 10.0488 (4.3); 8.1728 (1.1); 8.1543 (1.3); 8.0170 (3.0); 7.9966 (2.4); 7.6891 (0.4); 7.6663 (0.4); 7.5115 (1.7); 7.4914 (1.4); 5.7584 (4.6); 4.0570 (0.4); 4.0391 (1.2); 4.0213 (1.2); 4.0036 (0.4); 3.9353 (0.6); 3.8744 (6.1); 3.8500 (0.3); 3.3312 (23.3); 2.5269 (0.8); 2.5136 (16.1); 2.5092 (31.8); 2.5047 (41.0); 2.5001 (29.3); 2.4956 (14.0); 2.1034 (16.0); 2.0524 (0.7); 1.9905 (5.4); 1.1940 (1.5); 1.1762 (2.9); 1.1584 (1.4); 1.0709 (3.7); 0.0080 (0.4); -0.0002 (9.2) |
| IM-3-08 | 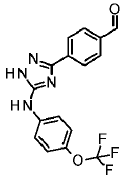 | IM-3-08: ¹H-NMR(400.2 MHz, d₆-DMSO): |
| | | δ= 10.0643 (16.0); 9.6843 (1.4); 8.3190 (0.4); 8.2067 (9.0); 8.1861 (12.0); 8.0594 (7.4); 8.0392 (5.8); 7.7130 (9.5); 7.6903 (10.7); 7.4297 (0.4); 7.2966 (6.4); 7.2752 (5.9); 3.3359 (22.6); 2.6784 (0.6); 2.6739 (0.8); 2.6693 (0.6); 2.5273 (2.6); 2.5224 (4.2); 2.5138 (45.6); 2.5094 (90.9); 2.5049 (118.2); 2.5003 (85.2); 2.4958 (41.0); 2.3362 (0.5); 2.3317 (0.7); 2.3272 (0.5); 1.2991 (0.4); 1.2590 (0.5); 1.2299 (0.3); 1.0707 (1.5); 0.0080 (2.5); -0.0002 (67.7); - 0.0085 (2.3) |
| IM-3-09 | 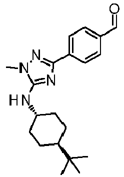 | IM-3-09: ¹H-NMR(400.2 MHz, d₆-DMSO): |
| | | δ= 10.0088 (2.6); 8.0904 (1.6); 8.0698 (2.2); 7.9439 (2.3); 7.9229 (1.8); 6.3506 (0.8); 6.3315 (0.8); 3.9305 (0.4); 3.6005 (7.1); 3.3290 (9.6); 2.5121 (4.2); 2.5078 (8.2); 2.5033 (11.0); 2.4988 (8.3); 2.4945 (4.2); 2.1124 (0.5); 2.0881 (0.6); 1.9893 (1.0); 1.8076 (0.6); 1.7771 (0.6); 1.3183 (0.3); 1.2691 (0.5); 1.2349 (0.6); 1.2029 (0.4); 1.1758 (0.6); 1.1580 (0.5); 1.1234 (0.5); 1.0973 (0.5); 1.0911 (0.5); 1.0704 (2.8); 1.0288 (0.4); 0.9997 (0.4); 0.8676 (16.0); 0.8366 (0.6); 0.0080 (0.4); -0.0002 (8.2); -0.0084 (0.4) |
| IM-3-10 | 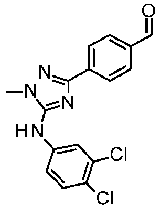 | IM-3-10: ¹H-NMR(400.2 MHz, d₆-DMSO): |
| | | δ= 10.0420 (5.6); 9.4455 (3.2); 8.1794 (3.5); 8.1588 (4.6); 8.0733 (3.2); 8.0669 (3.2); 8.0171 (4.7); 7.9962 (3.7); 7.7401 (1.5); 7.7336 (1.4); 7.7179 (1.9); 7.7114 (1.9); 7.5877 (3.6); 7.5655 (2.7); 4.0564 (0.7); 4.0386 (2.1); 4.0208 (2.2); 4.0030 (0.7); 3.8461 (16.0); 3.3294 (39.9); 2.5126 (16.8); 2.5082 (31.9); 2.5037 (41.9); 2.4991 (31.1); 2.4948 (15.3); 1.9897 (9.2); 1.1937 (2.5); 1.1760 (4.9); 1.1582 (2.4); 0.0079 (1.3); -0.0002 (26.0); -0.0085 (1.0) |
| IM-3-11 | 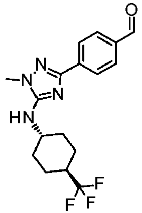 | IM-3-11: ¹H-NMR(400.2 MHz, CDCl3): |
| | | δ= 10.0308 (4.8); 8.1938 (3.3); 8.1730 (3.9); 7.9207 (4.1); 7.8999 (3.5); 7.2635 (7.8); 4.1307 (0.8); 4.1129 (0.8); 3.7816 (0.5); 3.7717 (0.6); 3.7625 (0.5); 3.7530 (0.7); 3.7433 (0.5); 3.7247 (0.4); 3.6959 (1.0); 3.6772 (0.7); 3.6466 (16.0); 2.3855 (1.1); 2.3602 (1.1); 2.3537 (1.1); 2.0731 (1.3); 2.0642 (1.1); 2.0461 (5.0); 2.0226 (0.4); 1.6101 (3.0); 1.5779 (1.1); 1.5713 (1.1); 1.5457 (1.1); 1.5383 (1.0); 1.5111 (0.3); 1.3052 (0.4); 1.2983 (0.5); 1.2774 (1.6); 1.2598 (2.0); 1.2419 (1.8); 1.2121 (0.4); 1.2038 (0.3); -0.0002 (10.8); - 0.0079 (0.5) |
| IM-3-12 | 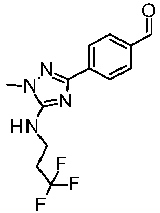 | IM-3-12: ¹H-NMR(400.2 MHz, d₆-DMSO): |
| | | δ= 10.0149 (5.2); 8.1080 (3.3); 8.0874 (4.4); 7.9577 (4.6); 7.9368 (3.5); 6.9201 (0.7); 6.9062 (1.4); 6.8922 (0.7); 3.6113 (16.0); 3.5756 (1.0); 3.5586 (2.0); 3.5436 (2.0); 3.5262 (1.0); 3.3257 (18.2); 2.6927 (0.5); 2.6818 (0.9); 2.6759 (0.6); 2.6641 (1.4); 2.6532 (1.0); 2.6467 (0.9); 2.6354 (1.4); 2.6243 (0.4); 2.6180 (0.8); 2.6068 (0.5); 2.5073 (29.6); 2.5028 (37.4); 2.4983 (26.9); 2.4941 (13.1); 1.9892 (0.4) |
| IM-3-13 | 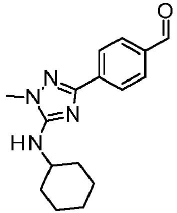 | IM-3-13: ¹H-NMR(400.2 MHz, d₆-DMSO): |
| | | δ= 10.0083 (5.2); 8.0893 (3.2); 8.0688 (4.4); 7.9451 (4.6); 7.9407 (1.5); 7.9281 (1.3); 7.9240 (3.4); 6.3400 (1.4); 6.3210 (1.4); 4.0386 (0.4); 4.0208 (0.4); 3.6071 (16.0); 3.5865 (0.4); 3.5769 (0.5); 3.5667 (0.5); 3.5591 (0.5); 3.3278 (10.4); 2.5259 (0.4); 2.5126 (8.1); 2.5082 (15.9); 2.5036 (20.5); 2.4990 (14.7); 2.4946 (7.1); 2.0152 (1.3); 1.9896 (2.6); 1.7663 (0.8); 1.7595 (0.8); 1.7354 (1.3); 1.6384 (0.5); 1.6067 (0.6); 1.3515 (0.8); 1.3188 (1.7); 1.2909 (2.2); 1.2674 (0.8); 1.2603 (0.8); 1.1936 (0.7); 1.1759 (1.2); 1.1690 (0.5); 1.1581 (0.7); 1.1391 (0.4) |
| IM-3-14 | 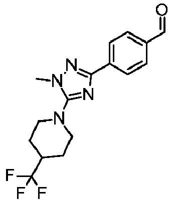 | IM-3-14: ¹H-NMR(400.2 MHz, d₆-DMSO): |
| | | δ= 10.0275 (2.9); 8.1346 (1.9); 8.1141 (2.4); 7.9805 (2.5); 7.9597 (1.9); 3.9310 (2.6); 3.7532 (8.5); 3.6032 (0.8); 3.5722 (0.8); 3.3285 (4.3); 3.0005 (0.6); 2.9740 (1.1); 2.9693 (1.1); 2.9428 (0.6); 2.5086 (7.6); 2.5042 (9.6); 2.4999 (7.0); 1.9173 (0.6); 1.8896 (0.8); 1.7050 (0.7); 1.6951 (0.7); 1.6735 (0.6); 1.6639 (0.6); 1.3188 (0.9); 1.0712 (16.0) |
| IM-3-15 | 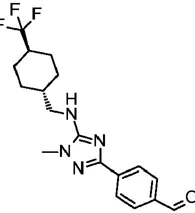 | IM-3-15: ¹H-NMR(400.2 MHz, d₆-DMSO): |
| | | δ= 10.0064 (6.3); 8.0872 (2.1); 8.0798 (3.0); 8.0665 (3.0); 8.0591 (4.1); 7.9440 (6.6); 7.9231 (4.9); 6.6620 (0.6); 6.6477 (1.3); 6.6329 (0.7); 6.6124 (0.8); 6.5979 (0.4); 3.6097 (16.0); 3.3527 (1.5); 3.3245 (78.7); 3.2048 (0.9); 3.1890 (1.4); 3.1734 (0.9); 2.6757 (0.5); 2.6712 (0.6); 2.6667 (0.5); 2.5244 (2.0); 2.5111 (40.1); 2.5067 (78.8); 2.5022 (102.2); 2.4977 (73.4); 2.4933 (35.6); 2.3335 (0.6); 2.3290 (0.8); 2.3244 (0.7); 2.2963 (0.4); 2.2895 (0.4); 2.2738 (0.4); 2.0111 (0.5); 2.0014 (0.5); 1.9928 (0.5); 1.9125 (1.3); 1.8848 (1.4); 1.6613 (1.3); 1.6326 (3.6); 1.6115 (1.4); 1.5991 (1.7); 1.5764 (1.8); 1.5597 (1.0); 1.5507 (1.3); 1.2579 (0.6); 1.2502 (0.6); 1.2255 (0.7); 1.2192 (0.6); 1.0465 (0.6); 1.0194 (0.5); 0.1458 (0.4); 0.0079 (3.4); -0.0002 (82.2); - 0.0084 (3.4); -0.1497 (0.4) |
| IM-3-16 | 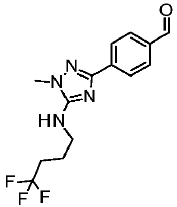 | IM-3-16: ¹H-NMR(400.2 MHz, d₆-DMSO): |
| | | δ= 10.0102 (5.4); 8.0959 (3.4); 8.0752 (4.5); 7.9512 (4.7); 7.9304 (3.6); 6.7092 (0.7); 6.6953 (1.5); 6.6815 (0.7); 3.6153 (16.0); 3.4137 (0.8); 3.3973 (2.1); 3.3821 (2.1); 3.3656 (0.9); 3.3271 (24.3); 2.5116 (14.4); 2.5075 (27.3); 2.5031 (34.6); 2.4985 (25.0); 2.4942 (12.3); 2.4060 (0.8); 2.3945 (0.6); 2.3863 (0.9); 2.3769 (1.0); 2.3658 (1.0); 2.3575 (0.9); 2.3517 (0.6); 2.3480 (0.6); 2.3368 (1.0); 2.3298 (0.5); 1.8745 (0.5); 1.8569 (1.4); 1.8369 (1.6); 1.8177 (1.3); 1.8002 (0.4); 0.0077 (1.2); -0.0002 (27.5); -0.0085 (1.2) |
| IM-3-17 | 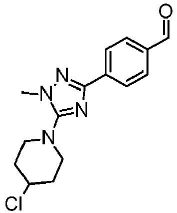 | IM-3-17: ¹H-NMR(400.2 MHz, d₆-DMSO): |
| | | δ= 10.0273 (1.9); 8.1359 (1.2); 8.1153 (1.6); 7.9795 (1.6); 7.9590 (1.3); 4.4484 (0.4); 3.9314 (2.4); 3.7502 (5.7); 3.4637 (0.3); 3.4427 (0.3); 3.4276 (0.4); 3.3309 (3.4); 3.1786 (0.4); 3.1709 (0.4); 3.1567 (0.4); 3.1479 (0.6); 3.1388 (0.3); 3.1246 (0.3); 2.5088 (5.1); 2.5045 (6.6); 2.5000 (4.7); 2.2079 (0.3); 2.1988 (0.4); 2.1918 (0.4); 2.1841 (0.4); 1.9649 (0.4); 1.9556 (0.5); 1.9439 (0.4); 1.9325 (0.4); 1.3187 (0.4); 1.0714 (16.0); -0.0002 (8.0); -0.0085 (0.3) |
| IM-3-18 | 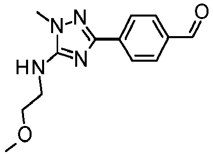 | IM-3-18: ¹H-NMR(400.2 MHz, d₆-DMSO): |
| | | δ= 10.0110 (5.3); 8.0970 (3.2); 8.0804 (1.3); 8.0764 (4.3); 7.9516 (0.9); 7.9483 (4.7); 7.9436 (1.4); 7.9314 (1.2); 7.9270 (3.5); 7.9232 (0.6); 6.7330 (0.6); 6.7191 (1.2); 6.7057 (0.6); 3.6136 (16.0); 3.5486 (0.7); 3.5444 (0.8); 3.5358 (1.0); 3.5308 (3.5); 3.5195 (2.5); 3.5180 (2.6); 3.4985 (1.2); 3.4968 (1.2); 3.4856 (2.4); 3.4719 (1.7); 3.4678 (1.0); 3.4582 (0.4); 3.4544 (0.4); 3.3297 (9.2); 3.2945 (21.1); 2.5266 (0.4); 2.5219 (0.6); 2.5132 (6.5); 2.5087 (13.1); 2.5041 (17.1); 2.4994 (12.4); 2.4949 (5.9); 0.0080 (0.8); -0.0002 (24.7); -0.0086 (0.8) |
| IM-3-19 | 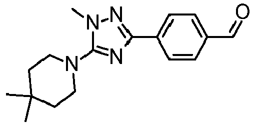 | IM-3-19: ¹H-NMR(400.2 MHz, d₆-DMSO): |
| | | δ= 10.0244 (3.1); 9.7829 (0.4); 8.1314 (1.9); 8.1108 (2.6); 7.9756 (2.7); 7.9711 (0.8); 7.9588 (0.7); 7.9545 (2.0); 7.7656 (0.4); 7.7439 (0.4); 6.9301 (0.4); 6.9087 (0.3); 3.7283 (10.1); 3.3265 (7.8); 3.1946 (1.9); 3.1806 (2.1); 3.1758 (1.5); 3.1662 (1.9); 2.5255 (0.4); 2.5207 (0.5); 2.5121 (6.9); 2.5076 (13.9); 2.5030 (18.3); 2.4984 (13.2); 2.4939 (6.3); 1.5033 (1.9); 1.4891 (2.1); 1.4748 (1.8); 1.3165 (2.1); 1.0698 (0.5); 0.9952 (16.0); 0.0080 (0.6); -0.0002 (19.1); -0.0086 (0.6) |
| IM-3-20 | 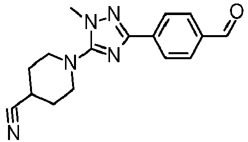 | IM-3-20: ¹H-NMR(400.2 MHz, d₆-DMSO): |
| | | δ= 10.0275 (5.1); 8.1367 (3.1); 8.1201 (1.3); 8.1161 (4.1); 7.9805 (4.4); 7.9759 (1.3); 7.9637 (1.2); 7.9593 (3.3); 5.7557 (0.8); 3.7456 (16.0); 3.3727 (0.6); 3.3638 (0.7); 3.3568 (0.7); 3.3471 (0.7); 3.3403 (0.9); 3.3265 (17.8); 3.3160 (0.9); 3.1637 (0.9); 3.1561 (1.1); 3.1488 (0.6); 3.1420 (1.2); 3.1335 (1.5); 3.1274 (1.3); 3.1167 (0.8); 3.1098 (0.9); 3.1059 (0.8); 3.1025 (0.8); 2.5258 (0.4); 2.5209 (0.6); 2.5123 (8.4); 2.5078 (17.0); 2.5033 (22.2); 2.4986 (15.9); 2.4941 (7.5); 2.0638 (0.6); 2.0555 (0.6); 2.0477 (0.6); 2.0392 (0.8); 2.0306 (0.9); 2.0228 (0.9); 2.0147 (0.8); 2.0057 (0.4); 1.9449 (0.5); 1.9362 (0.6); 1.9235 (1.0); 1.9144 (1.2); 1.9024 (0.8); 1.8909 (0.9); 1.8814 (0.6); 1.8686 (0.4); 0.0079 (0.8); -0.0002 (23.6); -0.0086 (0.7) |
| IM-3-21 | 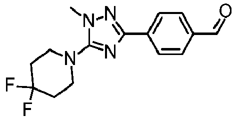 | IM-3-21: ¹H-NMR(400.2 MHz, d₆-DMSO): |
| | | δ= 10.0296 (2.4); 8.1407 (1.5); 8.1241 (0.6); 8.1201 (2.0); 7.9841 (2.1); 7.9795 (0.6); 7.9673 (0.6); 7.9629 (1.6); 3.9311 (2.6); 3.7818 (7.5); 3.3713 (1.1); 3.3575 (1.4); 3.3428 (1.2); 3.3314 (4.4); 2.5140 (1.9); 2.5095 (4.0); 2.5049 (5.3); 2.5003 (3.8); 2.4957 (1.8); 2.2087 (0.4); 2.1880 (0.6); 2.1734 (0.8); 2.1589 (0.6); 2.1380 (0.4); 1.3193 (0.5); 1.0720 (16.0); -0.0002 (2.4) |
| IM-3-22 | 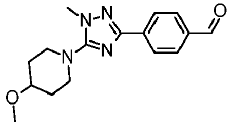 | IM-3-22: ¹H-NMR(400.2 MHz, d₆-DMSO): |
| | | δ= 10.0246 (5.0); 8.1292 (3.0); 8.1125 (1.2); 8.1086 (4.1); 7.9756 (4.3); 7.9710 (1.3); 7.9588 (1.1); 7.9545 (3.2); 5.7553 (0.4); 3.7392 (16.0); 3.5688 (0.3); 3.4279 (0.6); 3.4211 (0.9); 3.4122 (1.2); 3.4013 (1.6); 3.3903 (1.2); 3.3810 (1.2); 3.3719 (0.8); 3.3262 (27.0); 3.2873 (19.8); 3.0515 (0.8); 3.0438 (0.9); 3.0280 (0.9); 3.0201 (1.5); 3.0123 (0.8); 2.9963 (0.8); 2.9886 (0.7); 2.5254 (0.5); 2.5206 (0.7); 2.5120 (10.9); 2.5075 (22.2); 2.5030 (29.1); 2.4983 (20.7); 2.4938 (9.7); 1.9972 (0.6); 1.9894 (0.7); 1.9838 (0.6); 1.9736 (0.6); 1.9648 (0.7); 1.9587 (0.8); 1.9515 (0.7); 1.9433 (0.4); 1.6622 (0.4); 1.6530 (0.4); 1.6395 (0.7); 1.6304 (1.0); 1.6184 (0.6); 1.6079 (1.0); 1.5987 (0.6); 1.5852 (0.4); 1.5760 (0.3); 0.0079 (0.4); -0.0002 (14.0); -0.0086 (0.4) |
| IM-4-01 | 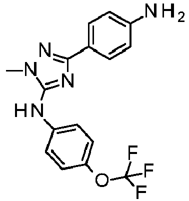 | IM-4-01: ¹H-NMR(400.2 MHz, d₆-DMSO): |
| | | δ= 9.0936 (3.0); 7.8051 (0.4); 7.7963 (4.3); 7.7908 (1.3); 7.7791 (1.3); 7.7735 (4.9); 7.7647 (0.4); 7.6517 (0.5); 7.6457 (4.0); 7.6413 (1.4); 7.6288 (1.2); 7.6242 (4.4); 7.6186 (0.6); 7.3165 (2.6); 7.2954 (2.3); 6.6043 (0.5); 6.5983 (4.2); 6.5937 (1.4); 6.5815 (1.2); 6.5768 (4.1); 6.5710 (0.5); 5.7563 (1.2); 5.3120 (3.9); 4.0561 (1.0); 4.0383 (3.1); 4.0205 (3.1); 4.0027 (1.0); 3.7397 (16.0); 3.3328 (5.5); 2.5255 (0.4); 2.5208 (0.6); 2.5121 (8.1); 2.5077 (16.7); 2.5031 (22.0); 2.4985 (15.9); 2.4940 (7.6); 1.9892 (13.9); 1.1930 (3.8); 1.1753 (7.6); 1.1575 (3.7); 0.0079 (0.9); -0.0002 (26.8); -0.0086 (0.9) |
| IM-4-02 | 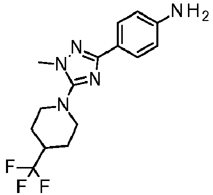 | IM-4-02: ¹H-NMR(400.2 MHz, d₆-DMSO): |
| | | δ= 7.5837 (4.1); 7.5668 (1.5); 7.5624 (4.4); 6.5682 (4.3); 6.5512 (1.4); 6.5467 (4.3); 5.2962 (4.6); 3.6357 (16.0); 3.5054 (1.3); 3.4740 (1.5); 3.3390 (18.0); 2.9375 (0.9); 2.9328 (1.1); 2.9066 (1.9); 2.9016 (1.9); 2.8754 (1.0); 2.8708 (0.9); 2.5652 (0.4); 2.5562 (0.5); 2.5443 (0.4); 2.5344 (0.5); 2.5248 (0.8); 2.5112 (6.2); 2.5068 (11.7); 2.5023 (15.1); 2.4978 (11.2); 2.4934 (5.7); 1.9891 (0.8); 1.8925 (1.1); 1.8649 (1.5); 1.7108 (0.5); 1.7007 (0.5); 1.6793 (1.2); 1.6695 (1.3); 1.6480 (1.1); 1.6382 (1.1); 1.6168 (0.4); 1.6067 (0.3); 1.1750 (0.4); 0.0078 (0.6); -0.0002 (13.2); -0.0085 (0.5) |

In Analogie zu den Beispielen und gemäß den oben beschriebenen Herstellverfahren lassen sich die in Tabelle 5 genannten Intermediate (IM-5) und (IM-6) erhalten:

**Tabelle 5**

| **Nr.** | **Struktur** | **NMR-Daten** |
|---|---|---|
| IM-5-01 | 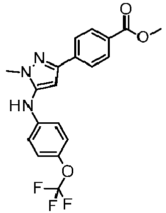 | IM-5-01: ¹H-NMR(300.1 MHz, d₆-DMSO): |
| | | δ= 8.3761 (2.9); 7.9919 (1.4); 7.9845 (0.8); 7.9626 (8.3); 7.9501 (7.9); 7.9284 (0.7); 7.9209 (1.4); 7.2423 (2.5); 7.2140 (3.1); 7.0079 (4.3); 6.9778 (3.3); 6.9660 (0.4); 6.6719 (5.0); 3.8595 (15.4); 3.7393 (16.0); 3.3304 (22.6); 2.5141 (8.5); 2.5085 (16.2); 2.5026 (21.2); 2.4967 (14.9); -0.0003 (2.9) |
| IM-5-02 | 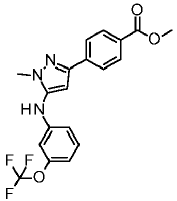 | IM-5-02: ¹H-NMR(400.2 MHz, d₆-DMSO): |
| | | δ= 8.5210 (1.2); 7.9945 (2.0); 7.9895 (1.0); 7.9729 (7.9); 7.9568 (7.4); 7.9516 (1.8); 7.9400 (1.0); 7.9350 (2.0); 7.3611 (1.2); 7.3406 (2.6); 7.3201 (1.5); 6.9384 (1.4); 6.9346 (1.2); 6.9179 (1.2); 6.9141 (1.1); 6.8226 (2.0); 6.7709 (1.2); 6.7505 (1.1); 6.7052 (5.0); 4.0570 (0.3); 4.0393 (1.0); 4.0215 (1.0); 4.0037 (0.3); 3.8628 (15.8); 3.7429 (16.0); 3.3333 (16.5); 3.3097 (2.8); 2.5134 (6.1); 2.5091 (12.6); 2.5046 (17.0); 2.5001 (12.2); 2.4957 (5.8); 1.9907 (4.3); 1.1936 (1.1); 1.1758 (2.2); 1.1580 (1.1); 0.0012 (1.6) |
| IM-6-01 | 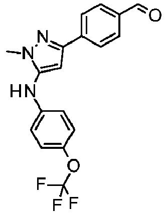 | IM-6-01: ¹H-NMR(400.2 MHz, d₆-DMSO): |
| | | δ= 10.0021 (5.8); 8.5456 (3.8); 8.0459 (3.5); 8.0254 (5.2); 7.9434 (5.4); 7.9230 (3.6); 7.3675 (1.3); 7.3471 (2.8); 7.3266 (1.6); 6.9473 (1.5); 6.9432 (1.5); 6.9269 (1.4); 6.9226 (1.4); 6.8304 (2.5); 6.7787 (1.5); 6.7625 (6.5); 3.7542 (16.0); 3.3532 (41.2); 3.3298 (4.0); 2.5051 (27.5); 1.2320 (0.6); 0.0001 (1.2) |
| IM-6-02 | 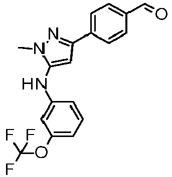 | IM-6-02: ¹H-NMR(400.2 MHz, d₆-DMSO): |
| | | δ= 10.0034 (5.7); 8.5299 (2.9); 8.0450 (3.1); 8.0243 (4.8); 7.9433 (5.1); 7.9224 (3.3); 7.8789 (0.3); 7.3668 (1.2); 7.3463 (2.6); 7.3258 (1.5); 6.9465 (1.2); 6.9424 (1.2); 6.9259 (1.1); 6.9219 (1.1); 6.8313 (1.8); 6.8296 (1.8); 6.7779 (1.1); 6.7758 (1.1); 6.7552 (5.8); 4.0575 (0.4); 4.0397 (1.1); 4.0219 (1.1); 4.0041 (0.4); 3.7555 (16.0); 3.7415 (0.4); 3.3411 (18.0); 3.3149 (0.4); 2.5146 (3.3); 2.5102 (6.8); 2.5057 (9.3); 2.5012 (6.7); 2.4968 (3.2); 1.9912 (4.7); 1.1940 (1.2); 1.1762 (2.5); 1.1584 (1.2); 0.0000 (0.4) |

### Anwendungsbeispiele

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25 ml Glasröhrchens verteilt. Bei 900 ppm Wirkstofflösung und 44,7 cm² Innenoberfläche wird bei homogener Verteilung eine Flächendosis von 5 µg/cm² erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit im Vergleich zur unbehandelten Kontrolle bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigte gute Wirkung gegen *Ctenocephalides felis,* wenn in diesem Test bei einer Aufwandmenge von 5 µg/cm² mindestens 80 % Wirkung erzielt wurde. Dabei bedeutet 100 % Wirkung, dass alle Flöhe angeschlagen oder tot waren. 0 % Wirkung bedeutet, dass keine Flöhe geschädigt wurden.

Bei diesem Test zeigten z.B. die folgenden Verbindungen der Herstellungsbeispiele eine Wirkung von 80 % bei einer Aufwandmenge von 5 µg/cm² (= 500g/ha): I-041.

### Ctenocephalides felis - Oraltest

| | |
|---|---|
| Lösungsmittel: | Dimethylsulfoxid |

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann. Der Zylinder wird auf 37 °C erwärmt während die Fliegenkammer bei Raumtemperatur gehalten wird.

Nach der gewünschten Zeit wird die Abtötung in % im Vergleich zur unbehandelten Kontrolle bestimmt. Dabei bedeutet 100 %, dass alle Flöhe abgetötet wurden; 0 % bedeutet, dass keiner der Flöhe abgetötet wurde.

Bei diesem Test zeigten z.B. die folgenden Verbindungen der Herstellungsbeispiele eine Wirkung von 100 % bei einer Aufwandmenge von 100 ppm: I-041.

Bei diesem Test zeigten z.B. die folgenden Verbindungen der Herstellungsbeispiele eine Wirkung von 90 % bei einer Aufwandmenge von 100 ppm: I-006.

### Diabrotica balteata - Sprühtest

| | |
|---|---|
| Lösungsmittel: | 78 Gewichtsteile Aceton |
| | 1,5 Gewichtsteile Dimethylformamid |
| Emulgator: | Alkylarylpol ygl ykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Vorgequollene Weizenkörner (*Triticum aestivum*) werden in einer mit Agar und etwas Wasser gefüllten Multiwell-Platte für einen Tag inkubiert (5 Saatkörner pro Kavität). Die gekeimten Weizenkörner werden mit einer Wirkstoffzubereitung der gewünschten Konzentration gespritzt. Anschließend wird jede Kavität mit 10-20 Käferlarven von *Diabrotica balteata* infiziert.

Nach 7 Tagen wird die Wirkung in % bestimmt. Dabei bedeutet 100 %, dass alle Weizenpflanzen wie in der unbehandelten, nicht infizierten Kontrolle gewachsen sind; 0 % bedeutet, dass keine Weizenpflanze gewachsen ist.

Bei diesem Test zeigten z. B. die folgenden Verbindungen der Herstellungsbeispiele Wirkung von 100 % bei einer Aufwandmenge von 100 g/ha (= 32 µg/Kavität): I-003, I-004, I-008, I-015, I-016, I-018, I-022, I-023, I-024, I-026, I-027, I-028, I-036, I-037, I-038, I-039, I-041, I-042, I-044, I-045, I-046, I-048, I-050, I-051, I-054, I-056, I-057, I-062, I-063.

Bei diesem Test zeigten z. B. die folgenden Verbindungen der Herstellungsbeispiele Wirkung von 80 % bei einer Aufwandmenge von 100 g/ha (= 32 µg/Kavität): I-009, I-017, I-029, I-031, I-043, I-047.

Bei diesem Test zeigten z. B. die folgenden Verbindungen der Herstellungsbeispiele Wirkung von 100 % bei einer Aufwandmenge von 20 g/ha (= 6,4 µg/Kavität): I-004, I-009, I-022, I-023, I-024, I-026, I-027, I-028, I-033, I-037, I-038, I-039, I-042, I-045, I-046, I-047, I-049, I-050, I-051, I-052, I-054.

Bei diesem Test zeigten z. B. die folgenden Verbindungen der Herstellungsbeispiele Wirkung von 80 % bei einer Aufwandmenge von 20 g/ha (= 6,4 µg/Kavität): I-007.

### Myzus persicae - Oraltest

| | |
|---|---|
| Lösungsmittel: | 100 Gewichtsteile Aceton |

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.

50 µl der Wirkstoffzubereitung werden in Mikrotiterplatten überführt und mit 150 µl IPL41 Insektenmedium (33 % + 15 % Zucker) auf eine Endvolumen von 200 µl aufgefüllt. Anschließend werden die Platten mit Parafilm verschlossen, durch den eine gemischte Population der Grünen Pfirsichblattlaus (*Myzus persicae*), die sich in einer zweiten Mikrotiterplatte befindet, hindurchstechen und die Lösung aufnehmen kann.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 90 % bei einer Aufwandmenge von 20 ppm: I-056, I-057, I-063.

### Nezara viridula -Sprühtest

| | |
|---|---|
| Lösungsmittel: | 78,0 Gewichtsteile Aceton |
| | 1,5 Gewichtsteile Dimethylformamid |
| Emulgator: | Alkylarylpol ygl ykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Gerstenpflanzen (*Hordeum vulgare*) werden mit einer Wirkstoffzubereitung der gewünschten Konzentration gespritzt und mit Larven der Grünen Reiswanze (*Nezara viridula*) infiziert.

Nach 4 Tagen wird die Wirkung in % bestimmt. Dabei bedeutet 100 %, dass alle Reiswanzen abgetötet wurden; 0 % bedeutet, dass keine Reiswanzen abgetötet wurden.

Bei diesem Test zeigten z. B. die folgenden Verbindungen der Herstellungsbeispiele Wirkung von 100 % bei einer Aufwandmenge von 500 g/ha: I-062, I-070.

Bei diesem Test zeigten z. B. die folgenden Verbindungen der Herstellungsbeispiele Wirkung von 90 % bei einer Aufwandmenge von 500 g/ha: I-050.

### Nilaparvata lugens -Test

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Reispflanzen (*Oryza sativa*) werden mit der Wirkstoffzubereitung der gewünschten Konzentration gespritzt und anschließend mit der Braunrückigen Reiszikade (*Nilaparvata lugens*) infiziert.

Nach 4 Tagen wird die Wirkung in % bestimmt. Dabei bedeutet 100 %, dass alle Reiszikaden abgetötet wurden; 0 % bedeutet, dass keine Reiszikaden abgetötet wurden.

Bei diesem Test zeigten z. B. die folgenden Verbindungen der Herstellungsbeispiele Wirkung von 90 % bei einer Aufwandmenge von 500 g/ha: I-070.

### Spodoptera frugiperda - Sprühtest

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigten z. B. die folgenden Verbindungen der Herstellungsbeispiele Wirkung von 100 % bei einer Aufwandmenge von 100 g/ha: I-002, I-003, I-004, I-007, I-008, I-009, I-010, I-012, I-013, I-014, I-015, I-016, I-017, I-018, I-019, I-020, I-021, I-022, I-023, I-024, I-025, I-026, I-027, I-028, I-029, I-030, I-032, I-033, I-035, I-036, I-037, I-038, I-039, I-040, I-041, I-042, I-043, I-044, I-045, I-046, I-047, I-049, I-050, I-051, I-052, I-053, I-054, I-057, I-058, I-059, I-060, I-061, I-062, I-063, I-070, I-071, I-072, I-073, I-074, I-075, I-076, I-077, I-078, I-079, I-84, I-85, I-86, I-87, I-88, I-89, I-90, I-91.

Bei diesem Test zeigten z. B. die folgenden Verbindungen der Herstellungsbeispiele Wirkung von 83 % bei einer Aufwandmenge von 100 g/ha: I-011.

## Patentansprüche

1. Verbindung der Formel (I) 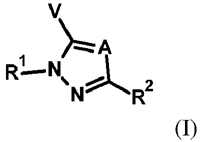 worin
A für N oder CR^{A} steht; dabei steht
R^{A} für H, Halogen, Cyano, Nitro, SF₅, NR^{d}R^{e}, OR^{a}, S(O)ₙR^{a} oder SO₂NR^{b}R^{c}; oder für C₁-C₆-Alkyl, C₂-C₆-Alkenyl, C₂-C₆-Alkinyl oder C₃-C₇-Cycloalkyl, die alle jeweils unsubstituiert sein können, oder einfach bis 13fach mit Halogen und/oder gegebenenfalls mit 1 bis 3 R^{f} substituiert sein können;
V für -NR^{V1}R^{V12}, -N(R^{V1})C(Q¹)R^{b}, -N(R^{b})C(Q¹)R^{V3}, -N(R^{V1})C(Q¹)NR^{b}R^{c}, -N(R^{V1})C(O)OR^{a}, -N(R^{V1})C(O)C(O)R^{b}, -N(R^{V1})SO₂R^{a}, -N(R^{b})SO₂R^{V3} oder -N(R^{b})C(O)N(R^{b})R^{V1} steht; dabei steht
R^{V1} für C₁-C₆₋Alkyl, das jeweils unsubstituiert sein kann, oder gegebenenfalls einfach bis 13fach mit Halogen und/oder mit 1 bis 2 R^{h} substituiert sein kann; oder für C₂-C₆-Alkenyl, C₂-C₆-Alkinyl, C₃-C₇-Cycloalkyl oder C₅-C₇-Cycloalkenyl, die alle jeweils unsubstituiert sein können, oder einfach bis 13fach mit Halogen und/oder gegebenenfalls mit 1 bis 2 Rⁱ substituiert sein können; oder für Phenyl oder für einen 4- bis 7-gliedrigen gesättigen, teilweise gesättigten oder aromatischen Heterocyclus mit 1 bis 3 Heteroatomen, jeweils unsubstituiert oder einfach bis 5fach substituiert mit Halogen und/oder gegebenenfalls mit 1 bis 2 Rⁱ;
R^{V2} für H; oder für C₁-C₆-Alkyl, C₂-C₆-Alkenyl, C₂-C₆-Alkinyl, C₁-C₆-Alkoxy oder C₃-C₇-Cycloalkyl, alle jeweils unsubstituiert oder einfach bis 13fach substituiert mit Halogen und/oder gegebenenfalls mit 1 bis 3 R^{g};
oder
R^{V1} und R^{V2} zusammen mit dem Stickstoffatom, an das sie gebunden sind, für einen gesättigten, teilweise gesättigten oder aromatischen Heterocyclus mit 3 bis 7 Ringatomen steht, der gegebenenfalls durch weitere Heteroatome aus der Reihe N, O, S(O)ₙ und/oder durch ein oder zwei C=O-Gruppen unterbrochen sein kann und der jeweils unsubstituiert ist oder einfach bis 7fach mit Halogen und/oder gegebenenfalls mit 1 bis 2 Rⁱ substituiert sein kann;
R^{V3} für C₁-C₆-Alkyl, C₂-C₆-Alkenyl, C₂-C₆-Alkinyl oder C₃-C₇-Cycloalkyl, die alle jeweils unsubstituiert sein können, oder einfach bis 13fach mit Halogen und/oder gegebenenfalls mit 1 bis 2 Rⁱ substituiert sein können; oder für einen 4- bis 7-gliedrigen gesättigen oder teilweise gesättigten Heterocyclus mit 1 bis 3 Heteroatomen, jeweils unsubstituiert oder einfach bis 5fach substituiert mit Halogen und/oder gegebenenfalls mit 1 bis 2 Rⁱ;
R¹ für H steht; oder für C₁-C₆-Alkyl, C₂-C₆-Alkenyl, C₂-C₆-Alkinyl, C₃-C₇-Cycloalkyl, C₁-C₆-Alkylcarbonyl, C₃-C₇-Cycloalkylcarbonyl, C₁-C₆-Alkoxycarbonyl, C₃-C₇-Cycloalkoxyylcarbonyl, C₁-C₆-Alkylsulfonyl oder C₃-C₇-Cycloalkylsulfonyl steht, die alle jeweils unsubstituiert sein können, oder einfach bis 13fach mit Halogen und/oder gegebenenfalls mit 1 bis 2 Rⁱ substituiert sein können;
R² für die Substruktur der allgemeinen Formel -X-Y-Z steht; dabei steht
X für Phenyl oder einen 5- oder 6-gliedrigen heteroaromatischen Ring, alle jeweils unsubstituiert oder substituiert mit 1 bis 3 R^{X}; dabei steht jedes
R^{X} unabhängig voneinander für Halogen, Cyano, Nitro, C(Q¹)R^{a}, C(O)OR^{a}, C(Q¹)NR^{b}R^{c}, NR^{d}R^{e}, OR^{a}, S(O)ₙR^{a} oder SO₂NR^{b}R^{c}; oder für C₁-C₆-Alkyl, C₂-C₆-Alkenyl, C₂-C₆-Alkinyl oder C₃-C₇-Cycloalkyl, alle jeweils unsubstituiert oder einfach bis 13fach substituiert mit Halogen und/oder gegebenenfalls mit 1 bis 3 R^{f};
Y für -CR^{Y1}=N-, wobei N an Z gebunden ist, oder für -NR^{Y2}-C(=Q^{Y})-, wobei C an Z gebunden ist; dabei steht jedes
R^{Y1} und R^{Y2} für H; oder für C₁-C₆-Alkyl, C₂-C₆-Alkenyl, C₂-C₆-Alkinyl oder C₃-C₇-Cycloalkyl, alle jeweils unsubstituiert oder substituiert mit 1 bis 7 R^{Y11}; dabei steht jedes
R^{Y11} unabhängig voneinander für Halogen, Cyano, C₁-C₄-Alkyl, C₃-C₆-Cycloalkyl, C₁-C₄-Haloalkyl, C₁-C₄-Alkoxy, oder C₁-C₄-Haloalkoxy;
Q^{Y} für O oder S;
Z für die Fragmente der allgemeinen Formel (A1), (A2) oder (A3); 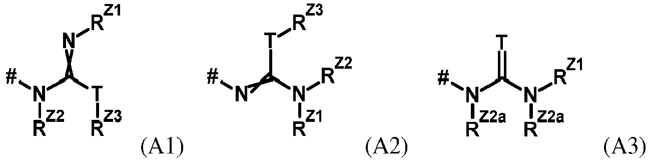 dabei ist # der Anknüpfungspunkt zu Y und dabei steht jedes
T für O oder S;
R^{Z1} unabhängig voneinander für einen 5- bis 10-gliedrigen aromatischen oder heteroaromatischen Ring oder ein bicyclisches Ringsystem, jeweils unsubstituiert oder substituiert mit 1 bis 4 R^{Z11}; dabei steht jedes
R^{Z11} unabhängig voneinander für Halogen, Cyano, Nitro, SF₅, C(Q¹)R^{a}, C(O)OR^{a}, C(Q¹)NR^{b}R^{c}, NR^{d}R^{e}, OR^{a}, S(O)ₙR^{a} oder SO₂NR^{b}R^{c}; oder für C₁-C₆-Alkyl, C₂-C₆-Alkenyl, C₂-C₆-Alkinyl oder C₃-C₇-Cycloalkyl, alle jeweils unsubstituiert oder substituiert mit 1 bis 5 R^{Z1a}; oder für Phenyl oder einen 5- oder 6-gliedrigen heteroaromatischen Ring, alle jeweils unsubstituiert oder substituiert mit 1 bis 3 R^{Z1a}; oder zwei benachbarte R^{Z11} bilden zusammen eine unsubstituierte oder mit 1 bis 6 R^{Z1a} substituierte lineare C₃-C₅-Alkylen-Gruppe, wobei unabhängig voneinander 1 CH₂-Einheit durch Carbonyl und 1 bis 2 CH₂-Einheiten durch O, S, NH oder N(CH₃) ersetzt sein können; dabei steht jedes
R^{Z1a} für Halogen, Cyano, C₁-C₄-Alkyl, C₁-C₄-Alkoxy, C₁-C₄-Thioalkyl, C₁-C₄-Haloalkyl oder C₁-C₄-Haloalkoxy;
R^{Z2}, R^{Z2a} und R^{Z3} unabhängig voneinander für H; oder für C(O)R^{a}, C(O)OR^{a}, C(O)NR^{b}R^{c}, S(O)ₙR^{a}; oder für C₁-C₆-Alkyl, C₂-C₆-Alkenyl, C₂-C₆-Alkinyl oder C₃-C₇-Cycloalkyl, alle jeweils unsubstituiert oder substituiert mit 1 bis 5 R^{Z21}; oder für Phenyl, Benzyl oder einen 5- oder 6-gliedrigen heteroaromatischen Ring, alle jeweils unsubstituiert oder substituiert mit 1 bis 4 R^{Z21}; dabei steht jedes
R^{Z21} unabhängig voneinander für Halogen, Cyano, Nitro, SF₅, C(Q¹)R^{a}, C(O)OR^{a}, C(Q¹)NR^{b}R^{c}, NR^{d}R^{e}, OR^{a}, S(O)ₙR^{a} oder SO₂NR^{b}R^{c}; oder für C₁-C₆-Alkyl, C₂-C₆-Alkenyl, C₂-C₆-Alkinyl oder C₃-C₇-Cycloalkyl; oder
R^{Z2} und R^{Z3} bilden zusammen mit der T-C-N-Einheit einen 5- bis 7-gliedrigen Ring; dabei bestehen die R^{Z2}-R^{Z3}-Ringglieder aus Kohlenstoff-Atomen und gegebenenfalls 1 Sauerstoff- oder Schwefel- oder Stickstoff-Atom; dabei ist das Heteroatom nicht direkt an T gebunden; dabei können bis zu 2 Kohlenstoff-Atom-Ringglieder unabhängig voneinander aus C(=O) und C(=S) bestehen und das Schwefel-Atom-Ringglied aus S, S(O) oder S(O)₂ bestehen; dabei ist diese R^{Z2}-R^{Z3}-Einheit unsubstituiert oder substituiert mit 1 bis 4 R^{Z22}; dabei steht jedes
R^{Z22} unabhängig voneinander für Halogen, Hydroxy, Cyano, C₁-C₆-Alkyl, C₃-C₆-Cycloalkyl, C₁-C₆-Haloalkyl, C₁-C₄-Alkenyl, C₁-C₆-Alkoxy, oder C₁-C₆-Haloalkoxy; oder für spirocyclisch gebundenes Heterocyclyl mit 2 bis 7 Ringatomen, wobei 1 bis 2 Sauerstoffatome als Heteroatome enthalten sind;
oder
R^{Z2a} und ein zweites R^{Z2a} bilden zusammen mit der N-C-N-Einheit einen 5- bis 7-gliedrigen Ring; dabei bestehen die R^{Z2a}-R^{Z2a}-Ringglieder aus Kohlenstoff-Atomen und bis zu 2 Heteroatomen, die unabhängig voneinander aus 1 Sauerstoff-Atom, 1 Schwefel-Atom und bis zu 2 Stickstoff-Atomen ausgewählt werden können; dabei können bis zu 2 Kohlenstoff-Atom-Ringglieder unabhängig voneinander aus C(=O) und C(=S) bestehen und das Schwefel-Atom-Ringglied aus S, S(O) oder S(O)₂ bestehen; dabei ist diese R^{Z2}-R^{Z3}-Einheit unsubstituiert oder substituiert mit 1 bis 5 R^{Z22};
dabei steht jedes
R^{a} unabhängig voneinander für C₁-C₆-Alkyl, C₃-C₆-Cycloalkyl, C₂-C₆-Alkenyl, C₂-C₆-Alkinyl, die alle jeweils unsubstituiert sein können, oder einfach bis 13fach mit Halogen und/oder gegebenenfalls mit 1 bis 3 R^{f} substituiert sein können; oder für Phenyl, unsubstituiert oder substituiert mit 1 bis 5 R^{g};
R^{b} und R^{c} unabhängig voneinander für H; oder für C₁-C₆-Alkyl, C₃-C₆-Cycloalkyl, C₂-C₆-Alkenyl, C₂-C₆-Alkinyl, die alle jeweils unsubstituiert sein können, oder einfach bis 13fach mit Halogen und/oder gegebenenfalls mit 1 bis 3 R^{f} substituiert sein können; oder R^{b} und R^{c} bilden zusammen einen 3- bis 7-gliedrigen Ring;
R^{d} und R^{e} unabhängig voneinander für H, C(Q¹)R^{a}, C(O)OR^{a}; oder für C₁-C₆-Alkyl, C₃-C₆-Cycloalkyl, C₂-C₆-Alkenyl, C₂-C₆-Alkinyl, die alle jeweils unsubstituiert sein können, oder einfach bis 13fach mit Halogen und/oder gegebenenfalls mit 1 bis 3 R^{f} substituiert sein können; oder für Phenyl, unsubstituiert oder substituiert mit 1 bis 5 R^{g}; oder R^{d} und R^{e} bilden zusammen einen 3- bis 7-gliedrigen Ring;
R^{f} unabhängig voneinander für Halogen, Cyano, Nitro, C₃-C₆-Cycloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl, C₁-C₄-Alkoxycarbonyl; oder für Phenyl oder einen 5- oder 6-gliedrigen heteroaromatischen Ring, alle jeweils unsubstituiert oder substituiert mit 1 bis 7 R^{g};
R^{g} unabhängig voneinander für Halogen, Cyano, Nitro, C₁-C₄-Alkyl, C₃-C₆-Cycloalkyl, C₁-C₄-Haloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl oder C₁-C₄-Alkoxycarbonyl;
R^{h} unabhängig voneinander für Halogen, Cyano, Nitro, Hydroxy, OSO₂R^{a}, SO₂NR^{b}R^{c}, N(R^{d})(R^{e}), C(Q¹)NR^{b}R^{c}, N(R^{b})C(Q¹)R^{a}, C(O)OR^{b} OC(O)R^{b} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl, spirocyclisch gebundenes C₂-C₆-Cycloalkyl, spirocyclisch gebundenes Heterocyclyl mit 3 bis 7 Ringatomen, C₃-C₆-Cycloalkylthio, C₃-C₆-Cycloalkoxy, C₃-C₆-Cycloalkylsulfinyl oder C₃-C₆-Cycloalkylsulfonyl, die alle jeweils unsubstituiert sein können oder einfach bis 13fach mit Halogen und/oder gegebenenfalls mit 1 bis 2 R^{g} substituiert sein können; oder für einen 4- bis 7-gliedrigen gesättigen oder teilweise gesättigten Heterocyclus mit 1 bis 4 Heteroatomen, die alle jeweils unsubstituiert sein können oder einfach bis 5fach mit Halogen und/oder gegebenenfalls mit 1 bis 2 R^{g} substituiert sein können;
Rⁱ unabhängig voneinander für Halogen, Cyano, Nitro, Hydroxy, OSO₂R^{a}, SO₂NR^{b}R^{c}, N(R^{d})(R^{e}), C(Q¹)NR^{b}R^{c}, N(R^{b})C(Q¹)R^{a}, C(O)R^{a}, C(=N-OR^{b})R^{a}, C(O)OR^{b}, OC(O)R^{a} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl, spirocyclisch gebundenes C₂-C₆-Cycloalkyl, spirocyclisch gebundenes Heterocyclyl mit 3 bis 7 Ringatomen, C₃-C₆-Cycloalkylthio, C₃-C₆-Cycloalkoxy, C₃-C₆-Cycloalkylsulfinyl oder C₃-C₆-Cycloalkylsulfonyl, die alle jeweils unsubstituiert sein können oder einfach bis 13fach mit Halogen und/oder gegebenenfalls mit 1 bis 2 R^{g} substituiert sein können; oder für Phenyl oder einen 4- bis 7-gliedrigen gesättigen, teilweise gesättigten oder aromatischen Heterocyclus mit 1 bis 4 Heteroatomen, die alle jeweils unsubstituiert sein können oder einfach bis 5fach mit Halogen und/oder gegebenenfalls mit 1 bis 2 R^{g} substituiert sein können;
Q¹ unabhängig voneinander für O, S, NOR^{a} oder NCN;
Q² unabhängig voneinander für O oder NOR^{a};
n unabhängig voneinander für 0, 1 oder 2.

2. Verbindungen nach Anspruch 1, worin
A für N oder CR^{A} steht; dabei steht
R^{A} für H, Halogen, Cyano oder SF₅; oder für C₁-C₄-Alkyl, C₂-C₄-Alkenyl, C₂-C₆-Alkinyl oder C₃-C₆-Cycloalkyl, die alle jeweils unsubstituiert sein können, oder einfach bis siebenfach mit Halogen und/oder gegebenenfalls mit einem R^{f} substituiert sein können;
V für -NR^{V1}R¹², -N(R^{V1})C(Q¹)R^{b}, -N(R^{b})C(Q¹)R^{V3}, -N(R^{V1})C(Q¹)NR^{b}R^{c}, -N(R^{V1})C(O)OR^{a}, -N(R^{V1})C(O)C(O)R^{b} oder -N(R^{V1})SO₂R^{a} steht; dabei steht
R^{V1} für C₁-C₆-Alkyl, das jeweils unsubstituiert sein kann, oder einfach bis 5fach mit Halogen und/oder gegebenenfalls mit 1 bis 2 R^{h} substituiert sein kann; oder für C₂-C₆-Alkenyl, C₂-C₆-Alkinyl oder C₃-C₇-Cycloalkyl, die alle jeweils unsubstituiert sein können, oder einfach bis fünffach mit Halogen und/oder gegebenenfalls mit 1 bis 2 Rⁱ substituiert sein können; oder für Phenyl oder für einen Heterocylus aus der Reihe Furanyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidyl, Pyridazinyl, Pyrazinyl, 1,2,3-Triazinyl, 1,2,4-Triazinyl, 1,3,5-Triazinyl, Oxetanyl, Oxolanyl, Oxanyl, Dioxanyl, Thiethanyl, Thiethanyl-1-oxide, Thiethanyl-1,1-oxide, Thiolanyl, Thiolanyl-1-oxide, Thiolanyl-1,1-oxide, Thianyl, Thianyl-1-oxide, Thianyl-1,1-oxide, Pyrrolidinyl, Piperidinyl oder Dihydroisoxazolyl, die alle jeweils unsubstituiert sein können oder gegebenenfalls einfach bis 5fach mit Halogen und/oder mit 1 bis 2 Rⁱ substituiert sein können;
R^{V2} für H; oder für C₁-C₄-Alkyl, C₂-C₄-Alkenyl, C₂-C₄-Alkinyl, C₁-C₄-Alkoxy oder C₃-C₆-Cycloalkyl, alle jeweils alle jeweils unsubstituiert oder einfach bis 5fach substituiert mit Halogen und/oder gegebenenfalls mit 1 bis 3 R^{g};
oder
R^{V1} und R^{V2} zusammen mit dem Stickstoffatom, an das sie gebunden sind, für einen Heterocyclus aus der Reihe U-01 bis U-06 steht 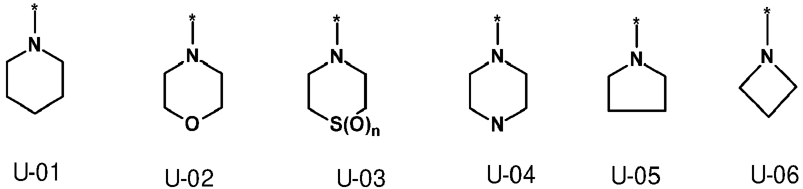 wobei alle U-01 bis U-06 jeweils unsubstituiert sein können oder gegebenenfalls einfach bis 5fach mit Halogen substituiert sein können und/oder gegebenenfalls mit 1 bis 2 Rⁱ
R^{V3} für C₁-C₆-Alkyl, C₂-C₆-Alkenyl, C₂-C₆-Alkinyl oder C₃-C₆-Cycloalkyl, die alle jeweils unsubstituiert sein können, oder einfach bis fünffach mit Halogen und/oder gegebenenfalls mit 1 bis 2 Rⁱ substituiert sein können; oder für einen Heterocylus aus der Reihe Oxetanyl, Oxolanyl, Oxanyl, Dioxanyl, Thiethanyl, Thiolanyl, Thianyl, Pyrrolidin-3-yl, Piperidin-4-yl oder Dihydroisoxazolyl jeweils unsubstituiert oder substituiert mit 1 bis 2 Rⁱ
R¹ für H steht; oder für C₁-C₄-Alkyl, C₂-C₄-Alkenyl, C₂-C₆-Alkinyl oder C₃-C₆-Cycloalkyl steht, die alle jeweils unsubstituiert sein können, oder einfach bis fünffach mit Halogen und/oder gegebenenfalls mit 1 bis 2 Rⁱ substituiert sein können;
R² für die Substruktur der allgemeinen Formel -X-Y-Z steht; dabei steht
X für Phenyl, Pyridyl, Pyrimidyl, Pyridazinyl oder Thienyl, alle jeweils unsubstituiert oder substituiert mit 1 bis 3 R^{X}; dabei steht
R^{X} unabhängig voneinander für Halogen, Cyano, Nitro, C(Q¹)R^{a}, C(O)OR^{a}; oder für C₁-C₄-Alkyl, C₂-C₄-Alkenyl, C₁-C₄-Alkoxy, C₂-C₆-Alkinyl oder C₃-C₆-Cycloalkyl, alle jeweils unsubstituiert oder substituiert mit 1 bis 3 R^{f};
Y für -CR^{Y1}=N-, wobei N an Z gebunden ist, oder für -NR^{Y2}-C(=Q^{Y})-, wobei C an Z gebunden ist; dabei steht jedes
R^{Y1} und R^{Y2} für H; oder für C₁-C₄-Alkyl, C₂-C₆-Alkenyl, C₂-C₄-Alkinyl oder C₃-C₇-Cycloalkyl, alle jeweils unsubstituiert oder substituiert mit 1 bis 3 R^{Y11}; dabei steht jedes
R^{Y11} unabhängig voneinander für Halogen, Cyano, C₁-C₄-Alkyl, C₃-C₆-Cycloalkyl, C₁-C₄-Haloalkyl, C₁-C₄-Alkoxy, oder C₁-C₄-Haloalkoxy;
Q^{Y} für O oder S;
Z für die Fragmente der allgemeinen Formel (A1), (A2) oder (A3); 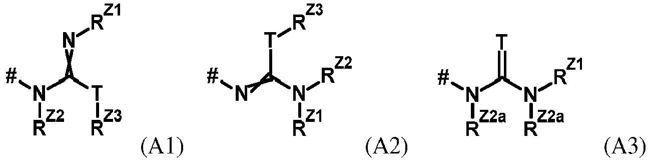 dabei ist # der Anknüpfungspunkt zu Y und dabei steht jedes
T für O oder S;
R^{Z1} für Phenyl, unsubstituiert oder substituiert mit 1 bis 4 R^{Z11}; dabei steht jedes
R^{Z11} unabhängig voneinander für Halogen, Cyano, Nitro, SF₅, C(Q¹)R^{a}, C(O)OR^{a}, C(Q¹)NR^{b}R^{c}, NR^{d}R^{e}, OR^{a}, S(O)ₙR^{a} oder SO₂NR^{b}R^{c}; oder für C₁-C₆-Alkyl, C₂-C₆-Alkenyl, C₂-C₆-Alkinyl oder C₃-C₇-Cycloalkyl, alle jeweils unsubstituiert oder substituiert mit 1 bis 5 R^{Z1a}; dabei steht jedes
R^{Z1a} für Halogen, Cyano, C₁-C₃-Alkyl, C₁-C₃-Alkoxy, C₁-C₃-Thioalkyl, C₁-C₃-Haloalkyl oder C₁-C₃- Haloalkoxy;
R^{Z2}, R^{Z2a} und R^{Z3} unabhängig voneinander für H; oder für C(O)R^{a}, C(O)OR^{a}, C(O)NR^{b}R^{c}, S(O)ₙR^{a}; oder für C₁-C₆-Alkyl, C₂-C₆-Alkenyl, C₂-C₆-Alkinyl oder C₃-C₆-Cycloalkyl, alle jeweils unsubstituiert oder substituiert mit 1 bis 4 R^{Z21}; oder für Phenyl oder Benzyl alle jeweils unsubstituiert oder substituiert mit 1 bis 3 R^{Z21}; dabei steht jedes
R^{Z21} unabhängig voneinander für Halogen, Cyano, Nitro, SF₅, C(Q¹)R^{a}, C(O)OR^{a}, C(Q¹)NR^{b}R^{c}, NR^{d}R^{e}, OR^{a}, S(O)ₙR^{a} oder SO₂NR^{b}R^{c}; oder für C₁-C₄-Alkyl, C₁-C₄-Haloalkyl, C₁-C₄-Alkenyl, C₂-C₄-Alkinyl oder C₃-C₆-Cycloalkyl; oder
R^{Z2} und R^{Z3} bilden zusammen mit der T-C-N-Einheit einen 5- bis 7-gliedrigen Ring; dabei bestehen die R^{Z2}-R^{Z3}-Ringglieder aus Kohlenstoff-Atomen; dabei können bis zu 2 Kohlenstoff-Atom-Ringglieder unabhängig voneinander aus C(=O) und C(=S) bestehen; dabei ist diese R^{Z2}-R^{Z3}-Einheit unsubstituiert oder substituiert mit 1 bis 4 R^{Z22} , dabei steht jedes
R^{Z22} unabhängig voneinander für Halogen, Hydroxy, Cyano, C₁-C₆-Alkyl, C₃-C₆-Cycloalkyl, C₁-C₆-Haloalkyl, C₁-C₄-Alkenyl, C₁-C₆-Alkoxy, oder C₁-C₆-Haloalkoxy;
oder
R^{Z2a} und ein zweites R^{Z2a} bilden zusammen mit der N-C-N-Einheit einen 5- bis 7-gliedrigen Ring; dabei bestehen die R^{Z2a}-R^{Z2a}-Ringglieder aus Kohlenstoff-Atomen; dabei können bis zu 2 Kohlenstoff-Atom-Ringglieder unabhängig voneinander aus C(=O) und C(=S) bestehen; dabei ist diese R^{Z2}-R^{Z3}-Einheit unsubstituiert oder substituiert mit 1 bis 4 R^{Z22};
dabei steht jedes
R^{a} unabhängig voneinander für C₁-C₆-Alkyl, C₃-C₆-Cycloalkyl, C₂-C₆-Alkenyl, C₂-C₆-Alkinyl, die alle jeweils unsubstituiert sein können, oder einfach bis 5fach mit Halogen und/oder gegebenenfalls mit einem R^{f}; oder für Phenyl, unsubstituiert oder substituiert mit 1 bis 3 R^{g};
R^{b} und R^{c} unabhängig voneinander für H; oder für C₁-C₄-Alkyl, C₃-C₆-Cycloalkyl, C₂-C₄-Alkenyl, C₂-C₄-Alkinyl, die alle jeweils unsubstituiert sein können, oder einfach bis 5fach mit Halogen und/oder gegebenenfalls mit 1 bis 2 R^{f} substituiert sein können; oder R^{b} und R^{c} bilden zusammen einen 3- bis 6-gliedrigen Ring;
R^{d} und R^{e} unabhängig voneinander für H, C(Q¹)R^{a}, C(O)OR^{a}; oder für C₁-C₄-Alkyl, C₃-C₆-Cycloalkyl, C₂-C₄-Alkenyl, C₂-C₄-Alkinyl, die alle jeweils unsubstituiert sein können, oder einfach bis 5fach mit Halogen und/oder gegebenenfalls mit 1 bis 2 R^{f} substituiert sein können; oder für Phenyl, unsubstituiert oder substituiert mit 1 bis 3 R^{g}; oder R^{d} und R^{e} bilden zusammen einen 3- bis 6-gliedrigen Ring;
R^{f} unabhängig voneinander für Halogen, Cyano, Nitro, C₃-C₆-Cycloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl, C₁-C₄-Alkoxycarbonyl; oder für Phenyl, oder einen 5- oder 6-gliedrigen heteroaromatischen Ring, alle jeweils unsubstituiert oder substituiert mit 1 bis 3 R^{g};
R^{g} unabhängig voneinander für Halogen, Cyano, Nitro, C₁-C₄-Alkyl, C₃-C₆-Cycloalkyl, C₁-C₄-Haloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl oder C₁-C₄-Alkoxycarbonyl;
R^{h} unabhängig voneinander für Halogen, Cyano, Nitro, Hydroxy, C(Q¹)NR^{b}R^{c}, N(R^{b})C(Q¹)R^{a}, C(O)R^{a}, OC(O)R^{b} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl, spirocyclisch gebundenes C₃-C₆-Cycloalkyl, C₃-C₆-Cycloalkoxy, C₃-C₆-Cycloalkylthio, C₃-C₆-Cycloalkylsulfinyl oder C₃-C₆-Cycloalkylsulfonyl, die alle jeweils unsubstituiert sein können, oder einfach bis siebenfach mit Halogen und/oder gegebenenfalls mit 1 bis 2 R^{g} substituiert sein können; oder für einen Heterocyclus aus der Reihe Oxetanyl, Oxolanyl, Dioxolanyl, Oxanyl, Dioxanyl, Thiethanyl, Thiolanyl, Thianyl oder Dihydroisoxazolyl, die alle jeweils unsubstituiert sein können oder einfach bis 5fach mit Halogen und/oder gegebenenfalls mit 1 R^{g} substituiert sein können;
Rⁱ unabhängig voneinander für Halogen, Cyano, Nitro, Hydroxy, C(Q¹)NR^{b}R^{c}, N(R^{b})C(Q¹)R^{a}, C(O)R^{a}, C(=N-OR^{b})R^{a}, C(O)OR^{b}, OC(O)R^{a} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl, spirocyclisch gebundenes C₃-C₆-Cycloalkyl, C₃-C₆-Cycloalkoxy, C₃-C₆-Cycloalkylthio, C₃-C₆-Cycloalkylsulfinyl oder C₃-C₆-Cycloalkylsulfonyl, die alle jeweils unsubstituiert sein können, oder einfach bis siebenfach mit Halogen und/oder gegebenenfalls mit 1 bis 2 R^{g} substituiert sein können; oder für Phenyl oder für einen Heterocyclus aus der Reihe Furanyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidyl, Pyridazinyl, Pyrazinyl, 1,2,3-Triazinyl, 1,2,4-Triazinyl, 1,3,5-Triazinyl, Oxetanyl, Oxolanyl, Dioxolanyl, Oxanyl, Dioxanyl, Thiethanyl, Thiolanyl, Thianyl, oder Dihydroisoxazolyl, alle jeweils unsubstituiert oder substituiert mit 1 bis 3 R^{g};
Q¹ unabhängig voneinander für O, S, NOR^{a} oder NCN;
Q² unabhängig voneinander für O oder NOR^{a};
n unabhängig voneinander für 0, 1 oder 2.

3. Verbindungen nach einem der Ansprüche 1 bis 2, worin
A für N oder CR^{A} steht; dabei steht
R^{A} für H, Halogen oder C₁-C₄-Alkyl;
V für -NR^{V1}R^{V2}, -N(R^{V1})C(Q¹)R^{b}, -N(R^{b})C(Q¹)R^{V3} oder -N(R^{V1})C(O)OR^{a} steht; dabei steht
R^{V1} für C₁-C₆-Alkyl, das jeweils unsubstituiert sein kann, oder einfach bis 5fach mit Halogen und/oder gegebenenfalls mit 1 bis 2 R^{h} substituiert sein kann; oder für C₂-C₆-Alkenyl, C₂-C₆-Alkinyl oder C₃-C₆-Cycloalkyl, die alle jeweils unsubstituiert sein können, oder einfach bis 5fach mit Halogen und/oder gegebenenfalls mit 1 bis 2 Rⁱ substituiert sein können; oder für Phenyl oder für einen Heterocylus aus der Reihe Furanyl, Thienyl, Pyrrolyl, Pyrazolyl, Imidazolyl, 1,2,3-Triazolyl, 1,2,4-Triazolyl, Oxazolyl, Isoxazolyl, Thiazolyl, Pyridyl, Pyrimidyl, Oxetanyl, Oxolanyl, Oxan-4-yl, Thiethanyl, Thiethanyl-1-oxide, Thiethanyl-1,1-oxide, Thianyl, Thianyl-1-oxide, Thianyl-1,1-oxide, oder Piperidin-4-yl, die alle jeweils unsubstituiert sein können oder die gegebenenfalls einfach bis 5fach mit Halogen und/oder mit 1 bis 2 Rⁱ substituiert sein können.
R^{V2} für H; oder für C₁-C₄-Alkyl, C₂-C₄-Alkenyl, C₂-C₄-Alkinyl oder C₃-C₆-Cycloalkyl, alle jeweils unsubstituiert oder substituiert mit 1 bis 3 R^{g};
oder
R^{V1} und R^{V2} zusammen mit dem Stickstoffatom, an das sie gebunden sind, für einen Heterocyclus aus der Reihe U-01 bis U-06 steht 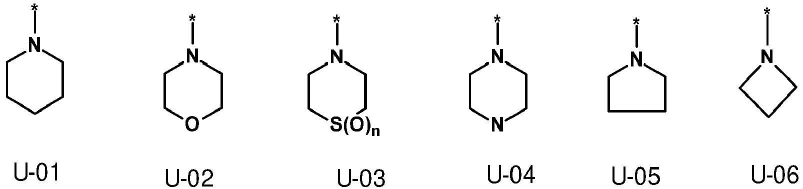
wobei alle U-01 bis U-06 jeweils unsubstituiert sein können oder gegebenenfalls einfach bis 5fach mit Halogen substituiert sein können und/oder gegebenenfalls mit 1 bis 2 Rⁱ
R^{V3} für C₁-C₄-Alkyl, C₂-C₄-Alkenyl oder C₃-C₆-Cycloalkyl, die alle jeweils unsubstituiert sein können, oder einfach bis fünffach mit Halogen und/oder gegebenenfalls mit 1 bis 2 Rⁱ substituiert sein können; oder für einen Heterocylus aus der Reihe Oxanyl, Dioxanyl, Thianyl oder Piperidin-4-yl jeweils unsubstituiert oder substituiert mit 1 bis 2 Rⁱ
R¹ für H steht; oder für C₁-C₄-Alkyl oder C₂-C₄-Alkenyl steht, die alle jeweils unsubstituiert sein können, oder einfach bis fünffach mit Halogen und/oder gegebenenfalls mit 1 bis 2 Rⁱ substituiert sein können;
R² für die Substruktur der allgemeinen Formel -X-Y-Z steht; dabei steht
X für Phenyl oder Pyridyl, alle jeweils unsubstituiert oder substituiert mit 1 bis 3 R^{X}; dabei steht
R^{X} unabhängig voneinander für Halogen, Cyano, oder für C₁-C₄-Alkyl, jeweils unsubstituiert oder substituiert mit 1 bis 2 R^{f};
Y für -CR^{Y1}=N-, wobei N an Z gebunden ist, oder für -NR^{Y2}-C(=Q^{Y})-, wobei C an Z gebunden ist; dabei steht jedes
R^{Y1} und R^{Y2} für H; oder für C₁-C₂-Alkyl jeweils unsubstituiert oder substituiert mit 1 bis 3 R^{Y11}; dabei steht jedes
R^{Y11} unabhängig voneinander für Halogen, Cyano, C₁-C₂-Alkyl oder C₁-C₂-Alkoxy;
Q^{Y} für O oder S;
Z für die Fragmente der allgemeinen Formel (A1), (A2) oder (A3); 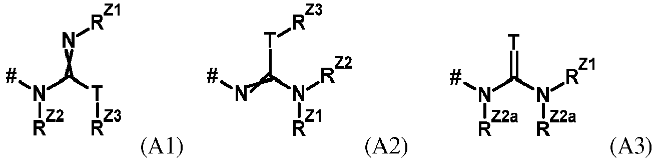 dabei ist # der Anknüpfungspunkt zu Y und dabei steht jedes
T für O oder S;
R^{Z1} für Phenyl, substituiert mit 1 bis 3 R^{Z11}, steht; dabei steht jedes
R^{Z11} unabhängig voneinander für Halogen, Cyano, OR^{a}, SR^{a}, oder für C₁-C₄-Alkyl, C₃-C₆-Cycloalkyl, alle jeweils unsubstituiert oder substituiert mit 1 bis 3 R^{Z1a}; dabei steht jedes
R^{Z1a} für Halogen, C₁-C₃-Alkoxy oder C₁-C₃-Thioalkyl;
R^{Z2}, R^{Z2a} und R^{Z3} unabhängig voneinander für H; oder für C₁-C₄-Alkyl, jeweils unsubstituiert oder substituiert mit 1 bis 3 R^{Z21}; dabei steht jedes
R^{Z21} unabhängig voneinander für Halogen oder C₁-C₃-Alkoxy;
oder
R^{Z2} und R^{Z3} bilden zusammen mit der T-C-N-Einheit einen 5- bis 6-gliedrigen Ring; dabei bestehen die R^{Z2}-R^{Z3}-Ringglieder aus Kohlenstoff-Atomen; dabei kann 1 Kohlenstoff-Atom-Ringglied aus C(=O) bestehen; dabei ist diese R^{Z2}-R^{Z3}-Einheit unsubstituiert oder substituiert mit 1 bis 3 R^{Z22}; dabei steht jedes
R^{Z22} unabhängig voneinander für Halogen, C₁-C₃-Alkyl, C₁-C₃-Alkenyl, oder C₁-C₃-Alkoxy;
dabei steht jedes
R^{a} unabhängig voneinander für C₁-C₄-Alkyl, C₃-C₆-Cycloalkyl, C₂-C₄-Alkenyl, C₂-C₄-Alkinyl, die alle jeweils unsubstituiert sein können,oder einfach bis 5fach mit Halogen und/oder gegebenenfalls mit einem R^{f} substituiert sein können;
R^{b} unabhängig voneinander für H; oder für C₁-C₄-Alkyl, C₃-C₆-Cycloalkyl, C₂-C₄-Alkenyl, C₂-C₄-Alkinyl, die alle jeweils unsubstituiert sein können, oder einfach bis 5fach mit Halogen und/oder gegebenenfalls mit 1 bis 3 R^{f} substituiert sein können;
R^{f} unabhängig voneinander für Halogen, Cyano, Nitro, C₃-C₆-Cycloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl, C₁-C₄-Alkoxycarbonyl; oder für Phenyl oder einen 5- oder 6-gliedrigen heteroaromatischen Ring, alle jeweils unsubstituiert oder substituiert mit 1 bis 3 R^{g};
R^{g} unabhängig voneinander für Halogen, Cyano, Nitro, C₁-C₄-Alkyl, C₃-C₆-Cycloalkyl, C₁-C₄-Haloalkyl, C₁-C₄-Alkoxy, C₁-C₄-Haloalkoxy, C₁-C₄-Alkylthio, C₁-C₄-Haloalkylthio, C₁-C₄-Alkylsulfinyl, C₁-C₄-Haloalkylsulfinyl, C₁-C₄-Alkylsulfonyl, C₁-C₄-Haloalkylsulfonyl, C₁-C₄-Alkylcarbonyl, C₁-C₄-Alkoxycarbonyl;
R^{h} unabhängig voneinander für Halogen, Cyano, Nitro, Hydroxy, C(Q¹)NR^{b}R^{c}, N(R^{b})C(Q¹)R^{a}, C(O)OR^{b} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl oder C₃-C₆-Cycloalkoxy, die alle jeweils unsubstituiert sein können, oder einfach bis fünfach mit Halogen und/oder gegebenenfalls mit 1 R^{g} substituiert sein können;
Rⁱ unabhängig voneinander für Halogen, Cyano, Nitro, Hydroxy, C(Q¹)NR^{b}R^{c}, N(R^{b})C(Q¹)R^{a}, C(O)OR^{b} oder =Q²; oder für C₁-C₄-Alkyl, C₁-C₄-Alkoxy, C₁-C₄-Alkylthio, C₁-C₄-Alkylsulfinyl, C₁-C₄-Alkylsulfonyl, C₃-C₆-Cycloalkyl oder C₃-C₆-Cycloalkoxy, die alle jeweils unsubstituiert sein können, oder einfach bis fünfach mit Halogen und/oder gegebenenfalls mit 1 R^{g} substituiert sein können; oder für Phenyl oder für einen Heterocyclus aus der Reihe, Thienyl, Pyrazolyl, Pyridyl, Pyrimidyl, Oxetanyl, Oxolanyl, Dioxolanyl, Oxanyl oder Dioxanyl, alle jeweils unsubstituiert oder substituiert mit 1 bis 3 R^{g};
Q¹ für O;
Q² unabhängig voneinander für O oder NOR^{a};
n für 0,1,2.

4. Verbindungen nach einem deer Ansprüche 1 bis 3, worin
A für N oder CR^{A} steht; dabei steht
R^{A} für H;
V für -NR^{V1}R^{V2}, -N(R^{V1})C(O)R^{b} oder -N(R^{b})C(O)R^{V3} steht; dabei steht
R^{V1} für C₁-C₄-Alkyl, das jeweils unsubstituiert sein kann, oder gegebenenfalls einfach bis 5-fach mit Halogen und mit 1 R^{h} substituiert sein kann; oder für C₂-C₄-Alkenyl, C₂-C₆-Alkinyl oder C₃-C₆-Cycloalkyl, die alle jeweils unsubstituiert sein können, oder einfach bis fünffach mit Halogen und/oder gegebenenfalls mit 1 Rⁱ substituiert sein können; oder für Phenyl oder für einen Heterocylus aus der Reihe, Pyridyl, Pyrimidyl, Oxan-4-yl, Thian-4-yl, Thian-1,1-oxid-4-yl oder Piperidin-4-yl die alle jeweils unsubstituiert sein können oder die gegebenenfalls einfach bis 5fach mit Halogen und/oder mit 1 Rⁱ substituiert sein können;
R^{V2} für H; oder für C₁-C₄-Alkyl, unsubstituiert oder substituiert mit 1 bis 3 R^{g};
oder
R^{V1} und R^{V2} zusammen mit dem Stickstoffatom, an das sie gebunden sind, für einen Heterocyclus aus der Reihe U-01, U-05 oder U-06 steht; 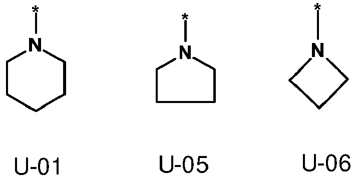 wobei U-01, U-05 und U-06 jeweils unsubstituiert sein können oder gegebenenfalls einfach bis 5fach mit Halogen substituiert sein können und/oder gegebenenfalls mit 1 bis 2 Rⁱ
R^{V3} für C₁-C₄-Alkyl oder C₃-C₆-Cycloalkyl, die alle jeweils unsubstituiert sein können, oder einfach bis fünffach mit Halogen und/oder gegebenenfalls mit 1 bis 2 Rⁱ substituiert sein können;
R¹ für H steht; oder für C₁-C₄-Alkyl steht, das jeweils unsubstituiert sein kann, oder einfach bis dreifach mit Halogen und/oder gegebenenfalls mit 1 bis 2 Rⁱ substituiert sein kann;
R² für die Substruktur der allgemeinen Formel -X-Y-Z steht; dabei steht
X für Phenyl, unsubstituiert oder substituiert mit 1 bis 3 R^{x}; dabei steht
R^{x} unabhängig voneinander für Halogen, Cyano, Methyl
Y für -CR^{Y1}=N-, wobei N an Z gebunden ist, oder für -NR^{Y2}-C(=Q^{Y})-, wobei C an Z gebunden ist; dabei steht jedes
R^{Y1} und R^{Y2} für H;
Q^{Y} für O;
Z für die Fragmente der allgemeinen Formel (A1), (A2), (A3); 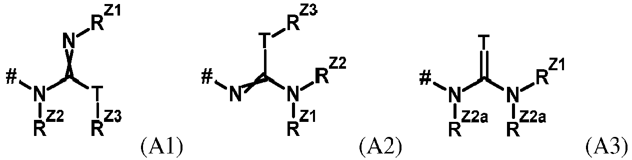 dabei ist # der Anknüpfungspunkt zu Y und dabei steht jedes
T für S;
R^{Z1} für Phenyl, substituiert mit 1 bis 3 R^{Z11}, steht; dabei befindet sich 1 R^{Z11} in 2-Position und dabei steht jedes
R^{Z11} unabhängig voneinander für F, CI, Br, CH₃, CH₂CH₃, CH₂CH₂CH₃, CH(CH₃)₂, Cyclopropyl, OCH₃, OCH₂CH₃, OCH₂CH₂CH₃, OCH(CH₃)₂, CH₂OCH₃ oder CH(CH₃)OCH₃;
R^{Z2}, R^{Z2a} und R^{Z3} unabhängig voneinander für H; oder
R^{Z2} und R^{Z3} bilden zusammen mit der T-C-N-Einheit einen 5- bis 6-gliedrigen Ring; dabei bestehen die R^{Z2}-R^{Z3}-Ringglieder aus Kohlenstoff-Atomen; dabei kann 1 Kohlenstoff-Atom-Ringglied aus C(=O) bestehen; dabei ist diese R^{Z2}-R^{Z3}-Einheit unsubstituiert oder substituiert mit 1 bis 2 R^{Z22}; dabei steht jedes
R^{Z22} unabhängig voneinander für Methyl;
dabei steht jedes
R^{b} unabhängig voneinander für H; oder für C₁-C₄-Alkyl, jeweils unsubstituiert oder substituiert mit 1 bis 3 R^{f};
R^{g} unabhängig voneinander für Halogen, Cyano, C₁-C₄-Alkyl, C₃-C₆-Cycloalkyl, C₁-C₄-Haloalkyl, C₁-C₄-Alkoxy oder C₁-C₄-Haloalkoxy;
R^{h} unabhängig voneinander für Halogen, Cyano, Hydroxy; oder für C₁-C₄-Alkyl, C₁-C₄-Alkoxy oder C₃-C₆-Cycloalkyl, die alle unsubstituiert sein können, oder einfach bis fünfach mit Halogen und/oder gegebenenfalls mit 1 R^{g} substituiert sein können;
Rⁱ unabhängig voneinander für Halogen, Cyano, Hydroxy; oder für C₁-C₄-Alkyl, C₁-C₄-Alkoxy oder C₃-C₆-Cycloalkyl, die alle unsubstituiert sein können, oder einfach bis fünfach mit Halogen und/oder gegebenenfalls mit 1 R^{g} substituiert sein können; oder für Phenyl, Thienyl oder Pyridyl, die alle jeweils unsubstituiert sein können oder einfach bis fünfach mit Halogen und/oder gegebenenfalls mit 1 R^{g} substituiert sein können.

5. Verbindungen nach einem der Ansprüche 1 bis 4, worin
A für N oder CR^{A} steht; dabei steht
R^{A} für H;
V für -NR^{V1}R^{V2}, -N(R^{V1})C(=O)R^{b} oder -N(R^{b})C(=O)R^{V3} steht; dabei steht
R^{V1} für C₁-C₄-Alkyl, das jeweils unsubstituiert ist oder das gegebenenfalls einfach bis 5-fach mit F oder Cl, und/oder oder gegebenenfalls einfach mit R^{h} substituiert sein kann; oder für C₃-C₆-Cycloalkyl, das jeweils unsubstituiert sein kann, oder einfach bis dreifach mit F oder Cl, und/oder oder gegebenenfalls einfach mit Rⁱ substituiert sein kann; oder für Phenyl das gegebenenfalls einfach bis dreifach Halogen und/oder mit 1 R^{g}, substituiert sein kann;
R^{V2} für H, Methyl oder Ethyl;
R^{V1} und R^{V2} zusammen mit dem Stickstoffatom, an das sie gebunden sind, für eine Heterocyclus aus der Reihe U-01, U-05 oder U-06 stehen; 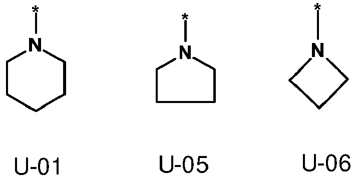 wobei U-01, U-05 und U-06 jeweils unsubstituiert sein können oder gegebenenfalls einfach bis 3fach mit Fluor oder Chlor oder einfach mit CN, C₁-C₄-Alkyl, C₁-C₄-Alkoxy, CF₃ oder C₂F₅ substituiert sein können;
R^{V3} für C₁-C₄-Alkyl oder C₃-C₆-Cycloalkyl, die alle jeweils unsubstituiert sein können, oder einfach bis dreifach mit F oder Cl, oder gegebenenfalls einfach bis zweifach mit R^{h} substituiert sein können;
Rⁱ für H steht; oder für C₁-C₄-Alkyl steht, das jeweils unsubstituiert sein kann, oder einfach bis 3fach mit Fluor oder einfach mit CN oder C₁-C₄-Alkoxy, substituiert sein kann;
R² für die Substruktur der allgemeinen Formel -X-Y-Z steht; dabei steht
X für Phenyl, unsubstituiert oder substituiert mit 1 bis 2 R^{X}; dabei steht
R^{X} unabhängig voneinander für Fluor, Chlor, Cyano oder Methyl;
Y für -CR^{Y1}=N-, wobei N an Z gebunden ist, oder für -NR^{Y2}-C(=Q^{Y})-, wobei C an Z gebunden ist; dabei steht jedes
R^{Y1} und R^{Y2} für H;
Q^{Y} für O;
Z für die Fragmente der allgemeinen Formel (A2) oder (A3); 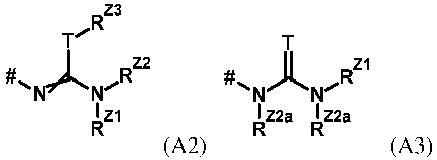 dabei ist # der Anknüpfungspunkt zu Y und dabei steht jedes
T für S;
R^{Z1} für Phenyl, substituiert mit 1 bis 3 R^{Z11}, steht; dabei befindet sich 1 R^{Z11} in 2-Position und dabei steht jedes
R^{Z11} unabhängig voneinander für F, CI, Br, CH₃, CH₂CH₃, CH₂CH₂CH₃, CH(CH₃)₂, Cyclopropyl, OCH₃, OCH₂CH₃, OCH₂CH₂CH₃, OCH(CH₃)₂ oder CH₂OCH₃;
R^{Z2}, R^{Z2a} und R^{Z3} für H; oder
R^{Z2} und R^{Z3} bilden zusammen -C(O)CH₂-; dabei steht jedes
R^{b} und R^{c} unabhängig voneinander für H; oder für Methyl, Ethyl, n-Propyl, iso-Propyl oder Cyclopropyl;
R^{g} unabhängig voneinander für Fluor, Chlor, Cyano, Methyl, Ethyl, n-Propyl, i-Propyl, Methoxy, Ethoxy, CF₃, C₂F₅, OCF₃ oder OC₂F₅;
R^{h} unabhängig voneinander für Fluor, Chlor, Cyano, Methyl, Ethyl, n-Propyl, i-Propyl, Methoxy, Ethoxy, CF₃, C₂F₅, OCF₃ oder OC₂F₅; oder für Cyclohexyl, das unsubstituiert sein kann oder das gegebenenfalls einfach bis 5-fach mit F und/oder gegebenenfalls einfach mit CF₃ substituiert sein kann;
Rⁱ unabhängig voneinander für Fluor, Chlor, Cyano, Methyl, Ethyl, n-Propyl, i-Propyl, t-Butyl, -CF3, C₂F₅, Methoxy, Ethoxy oder OCF₃.

6. Verbindungen nach einem der Ansprüche 1 bis 5, worin
A für N oder CH steht;
V für -NR^{V1}R^{V2}, -N(R^{V1})C(=O)CH₃ oder -N(H)C(=O)R^{V3} steht; dabei steht
R^{V1} für 4,4,4-Trifluorbutyl, 3,3,3-Trifluorpropyl, Methoxyethyl, Cyclohexyl, 4-(Trifluormethyl)cyclohexyl, 4-(Trifluormethyl)cyclohexylmethyl, 4-(tert.Butyl)cyclohexyl, 4,4-Difluorcyclohexyl, oder für 4-(Trifluormethoxy)phenyl, 4-(Pentafluorethoxy)phenyl, 3-(Trifluormethoxy)phenyl, 3,4-Dichlorphenyl;
R^{V2} für H;
oder
R^{V1} und R^{V2} zusammen mit dem Stickstoffatom, an das sie gebunden sind, für 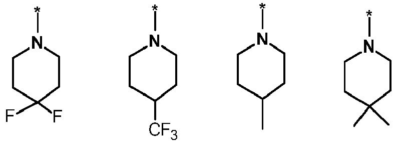 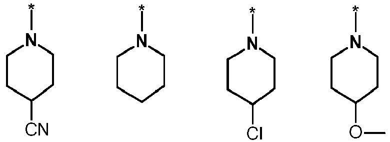
stehen;
R^{V3} für 4-(Trifluormethyl)cyclohexyl steht;
R¹ für H, Methyl, Ethyl, Ethoxymethyl oder Methoxyethyl steht.
R² für die Substruktur der allgemeinen Formel -X-Y-Z steht; dabei steht
X für Phenyl
Y für -CH=N-, wobei N an Z gebunden ist, oder für -NH-C(=O)-, wobei C an Z gebunden ist; dabei steht jedes
Z für die Fragmente der allgemeinen Formel (A2-1) oder (A3-1); 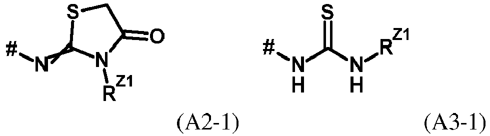 dabei ist # der Anknüpfungspunkt zu Y und dabei steht jedes
R^{Z1} für Phenyl, substituiert mit 1 bis 2 R^{Z11}, steht; dabei befindet sich 1 R^{Z11} in 2-Position und dabei steht jedes
R^{Z11} unabhängig voneinander für Methyl, Methoxy oder iso-Propyl.

7. Formulierung, insbesondere agrochemische Formulierung, umfassend mindestens eine Verbindung der Formel (I) gemäß einem der Ansprüche 1 bis 6.

8. Formulierung nach Anspruch 7 ferner umfassend mindestens ein Streckmittel und/oder mindestens eine oberflächenaktive Substanz

9. Formulierung nach Anspruch 7 oder 8, **dadurch gekennzeichnet, dass** die Verbindung der Formel (I) in Mischung mit mindestens einem weiteren Wirkstoff vorliegt.

10. Verfahren zur Bekämpfung von Schädlingen, insbesondere tierischen Schädlingen, **dadurch gekennzeichnet, dass** man eine Verbindung der Formel (I) gemäß einem der Ansprüche 1 bis 6 oder eine Formulierung gemäß einem der Ansprüche 6 bis 8 auf die Schädlinge und/oder ihren Lebensraum einwirken lässt.

11. Verfahren nach Anspruch 10, **dadurch gekennzeichnet, dass** der Schädling ein tierischer Schädling ist und ein Insekt oder ein Spinnentier umfasst, oder dass der Schädling ein Insekt oder ein Spinnentier ist.

12. Verwendung einer Verbindung der Formel (I) gemäß einem der Ansprüche 1 bis 6 oder einer Formulierung gemäß einem der Ansprüche 7 bis 9 zur Bekämpfung von tierischen Schädlingen.

13. Verwendung nach Anspruch 12, **dadurch gekennzeichnet, dass** der tierische Schädling ein Insekt oder ein Spinnentier umfasst, oder dass der tierische Schädling ein Insekt oder ein Spinnentier ist.

14. Verwendung nach Anspruch 12 oder 13 im Pflanzenschutz.

15. Verfahren zum Schutz eines Saatgutes oder einer keimenden Pflanze vor Schädlingen, insbesondere tierischen Schädlingen, umfassend einen Verfahrensschritt, in welchem das Saatgut in Kontakt mit einer Verbindung der Formel (I) gemäß einem der Ansprüche 1 bis 6 oder mit einer Formulierung gemäß einem der Ansprüche 7 bis 9 gebracht wird.

16. Saatgut, erhalten durch ein Verfahren gemäß Anspruch 15.
